(19) Europäisches Patentamt
European Patent Office
Office européen des brevets

(11) **EP 1 767 645 B1**

(12) **EUROPEAN PATENT SPECIFICATION**

(45) Date of publication and mention
of the grant of the patent:
**30.12.2009 Bulletin 2009/53**

(51) Int Cl.:
***C12P 19/18*** *(2006.01)* ***C12P 21/00*** *(2006.01)*

(21) Application number: **05020823.0**

(22) Date of filing: **23.09.2005**

(54) **Transglycosylation method and method for producing glycoprotein**

Transglykosylierungsverfahren und die Herstellung eines Glykoproteins

Procédé de transglycosylation et la production de une glycoprotéine

(84) Designated Contracting States:
**AT BE BG CH CY CZ DE DK EE ES FI FR GB GR HU IE IS IT LI LT LU LV MC NL PL PT RO SE SI SK TR**

(43) Date of publication of application:
**28.03.2007 Bulletin 2007/13**

(73) Proprietor: **Ajinomoto Co., Inc.**
**Chuo-ku,**
**Tokyo (JP)**

(72) Inventors:
• **Yamaguchi, Masanori**
  **A201 Guran Kuryu Takajyou**
  **Hirosaki, Aomori 036-8271 (JP)**
• **Ozawa, Hayato**
  **203 Guddhi Ichijyouji**
  **Sakyo-ku, Kyoto 606-8186 (JP)**
• **Fujita, Kiyotaka**
  **402 Erudoberu Sakuragi**
  **Kagoshima 890-0055 (JP)**
• **Katayama, Takane**
  **Kanazawa, Ishikawa 921-8171 (JP)**
• **Yamamoto, Kenji**
  **Ootu, Shiga 520-0248 (JP)**

• **Suzuki, Shunichi**
  **c/o Ajinomoto Co., Inc.**
  **A.S. Lab.**
  **Kanagawa 210-8681 (JP)**

(74) Representative: **Strehl Schübel-Hopf & Partner**
**Maximilianstrasse 54**
**80538 München (DE)**

(56) References cited:
• **KATAYAMA ET AL: "Novel bifidobacterial glycosidases acting on sugar chains of mucin glycoproteins" JOURNAL OF BIOSCIENCE AND BIOENGINEERING, vol. 99, May 2005 (2005-05), pages 457-465, XP004997036**
• **FUJITA ET AL: "Identification and molecular cloning of a novel glycoside hydrolase family of core 1 type O-Glycan-specific enda-alpha-N-acetylgalactosaminidase from Bifidobacterium longum" THE JOURNAL OF BIOLOGICAL CHEMISTRY, vol. 280, 1 September 2005 (2005-09-01), pages 37415-37422, XP002383896**
• **XU ET AL: "Site-specific incorporation of the mucin-type N-acetylgalactosamine-alpha-O-threonine into protein in Escherichia coli" JOURNAL OF THE AMERICAN CHEMICAL SOCIETY, vol. 48, November 2004 (2004-11), pages 15654-15655, XP002515396**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

**Description**

BACKGROUND OF THE INVENTION

1) Field of the Invention

The present invention relates to a transglycosylation method, a method for producing glycoprotein and applications thereof.

2) Description of the Related Art

Endo-$\alpha$-$N$-acetylgalactosaminidase (hereinafter, "Endo-$\alpha$" or "Endo-$\alpha$-GalNAc-ase") is an enzyme which recognizes a core 1 structure of an $O$-linked sugar chain in glycoprotein, that is, a structure having a galactosyl$\beta$1→3$N$-acetylgalactosamine (hereinafter, "Gal$\beta$1-3GalNAc") disaccharide bound at the $\alpha$-position to serine or threonine, and which releases the disaccharide unit by hydrolysis. The $O$-linked sugar chain is also called a mucin-type sugar chain and is contained in a large amount in mucin with which the surfaces of animal digestive tracts and organs are covered.

It has been reported that Endo-$\alpha$ producing bacteria are found in a culture of, for example, *Diplococcus pneumoniae* and bacteria belonging to the genus *Clostridium* (for example, Bhavanandan, V. P. et al., Biochem. Biophys. Res. Commun., 70, 738-745 (1976)). It has also been reported that in some Endo-$\alpha$ studied up to now, there is a transglycosylation activity of transferring and adding a disaccharide to an entering suitable acceptor in place of a water molecule upon hydrolysis (For example, Bardales, R. M. and Bhavanandan, V.P. , J. Biol. Chem. , 264, 19893-19897 (1989)). Among microorganisms known as the Endo-$\alpha$-producing bacteria, there are microorganisms whose total genome sequence has been clarified.

The presence of the Endo-$\alpha$-producing microorganism is known, but there is no report on the cloning of a gene for an enzyme having the Endo-$\alpha$ activity. That is, the amino acid sequence of the Endo-$\alpha$ is not revealed, and which gene is a gene encoding the Endo-$\alpha$ has not been specified.

J. of Bioscience and Bioengineering, May 2005, pages 457 to 465, and J. of Biological Chemistry, 1 September 2005 (online), pages 37415 to 37422 disclose glycosidases from Bifidobacterium.

SUMMARY OF THE INVENTION

Sugar chain are very important in the living body or the like, and in study on sugar chains, production of desired sugar chains, and production of glycoprotein, enzymes capable of transferring and adding various kinds of sugar chains have been desired. Under such circumstances, the object of the present invention is to provide a method for transferring a sugar chain, i.e. transglycosilation, utilizing a novel enzyme capable of transferring and adding a sugar chain.

The Endo-$\alpha$ is generally a hydrolase, and the present inventors have found an enzyme having extremely high transglycosylation activity from the Endo-$\alpha$ enzymes. Further, they have succeeded in specifying its gene to complete the present invention. The present invention also provides a method for producing a glycoprotein, to be described below. Thus, the invention refers to the following embodiments, which correspond to claims 1-10.

1. A method for transglycosylation, comprising a reaction of transferring a galactosyl$\beta$1→3$N$-acetylgalactosaminyl group from a sugar donor containing a sugar chain having a galactosylp1$\beta$1→3$N$-acetylgalactosaminyl group binding in the configuration of an $\alpha$ anomer to an amino acid or a polypeptide as a sugar chain acceptor, in the presence of a protein having endo-$\alpha$-$N$-acetylgalactosaminidase activity and transglycosylation activity, wherein the protein is selected from the group of (A) and (B):

(A) a protein having an amino acid sequence described in SEQ ID NO:2, and
(B) a protein having an endo-$\alpha$-$N$-acetylgalactosaminidase activity and a transglycosylation activity, and having the amino acid sequence that includes one to fifty amino acids mutation in the amino acid sequence described in SEQ ID NO: 2, the mutation being selected from the group consisting of substitution, deletion, insertion, addition, and inversion.

2. The method for transglycosylation according to item 1, wherein the protein is derived from a microorganism belonging to the genus *Bifidobacterium.*

3. The method for transglycosylation according to item 1, comprising:

cultivating a cell transformed with a polynucleotide encoding the protein in a suitable medium for the expression of the protein,
forming the protein in the medium and/or the cell,
and mixing the sugar donor and the sugar chain receptor with the medium and/or the cell.

4. A method of modifying a food comprising the method of item 1, wherein the food before modification contains an amino acid or a polypeptide as a sugar chain acceptor.

5. A method of modifying a drug comprising the method of item 1, wherein the drug before modification contains an amino acid or a polypeptide as a sugar chain acceptor.

6. A method for producing a glycoprotein comprising the method of item 1.

7. The method according to item 6, wherein the protein is derived from a microorganism belonging to the genus *Bifidobacterium.*

8. The method according to item 6, wherein said step of transferring comprises:

cultivating a cell transformed with a polynucleotide encoding the protein in a suitable medium for the expression of the protein,
forming the protein in the medium and/or the cell,
and mixing the sugar donor and the sugar chain receptor with the medium and/or the cell.

9. The method according to item 8, wherein said cell is derived from a microorganism belonging to the genus Escherichia.

10. The method according to item 3, wherein said cell is derived from a microorganism belonging to the genus Escherichia.

According to the present invention, there are provided a new transglycosylation method and a protein catalyzing a transglycosylation reaction. The present invention may be utilized for industry related to the sugar chain engineering such as production for glycoprotein.

## BRIEF DESCRIPTION OF THE DRAWINGS

Fig. 1 is a schematic diagram of the transglycosylation reaction.
Fig. 2 shows the results of measurement of hydrolysis activity.
Fig. 3 shows the results of measurement of transglycosylation activity.
Fig. 4 shows the results of measurement of transglycosylation activity into 1-alkanol.
Fig. 5 shows the results of transglycosylation activities on peptide-T, PAMP-12 and bradykinin measured by HPLC.
Fig. 6 shows a result of a transglycosylation activity on peptide-T, PAMP-12 and bradykinin measured by MALDI-TOF-MS.

MALDI-TOF-MS.

## DETAILED DESCRIPTIONS

Hereinafter, the present invention is described in more detail with reference to exemplary embodiments. For carrying out biochemical or genetic engineering techniques in the present invention, reference is made to various experimental manuals such as Molecular Cloning: A LABORATORY MANUAL, 3rd edition, Cold Spring Harbor Laboratory Press, Cold Spring Harbor, New York (2001) ; "Shin Idenshi Kogaku Handbook" (New Genetic Engineering Handbook), by Masami MURAMATSU, et al, Yodosha, Experimental Medicine, extra issue, 3rd edition, 1999; "Tanpakushitsu Jikken No Susumekata (How to Advance Protein Experiment), by Masato OKADA and Kaori MIYAZAKI, Yodosha, 1st edition, 1998; and "Tanpakushitsu Jikken Note (Protein Experimental Note) by Masato OKADA and Kaori MIYAZAKI, Yodosha, 2nd edition, 1999.
In the transglycosylation method of the present invention, a protein having endo-$\alpha$-N-acetylgalactosaminidase activity and transglycosylation activity is used as a catalyst to transfer a sugar chain from a sugar chain donor to a sugar chain acceptor. The sugar chain is transferred from the sugar chain donor and fed to a transfer reaction system, and the sugar chain acceptor accepts the sugar chain supplied by the sugar chain donor. The sugar chain donor contains a galactosyl $\beta1\rightarrow3N$-acetylgalactosaminyl $\alpha$-group. Galactosyl $\beta1\rightarrow3N$-acetylgalactosamine is a disaccharide having galactose and $N$-acetylgalactosamine bound via $\beta$ $(1\rightarrow3)$. In this specification, galactosyl $\beta1\rightarrow3N$-acetylgalactosamine is also referred to hereinafter as Gal$\beta$1-3GalNAc, and the galactosyl $\beta1\rightarrow3N$-acetylgalactosaminyl $\alpha$-group is also referred to hereinafter as Gal$\beta$1-3GalNAc$\alpha$-group.

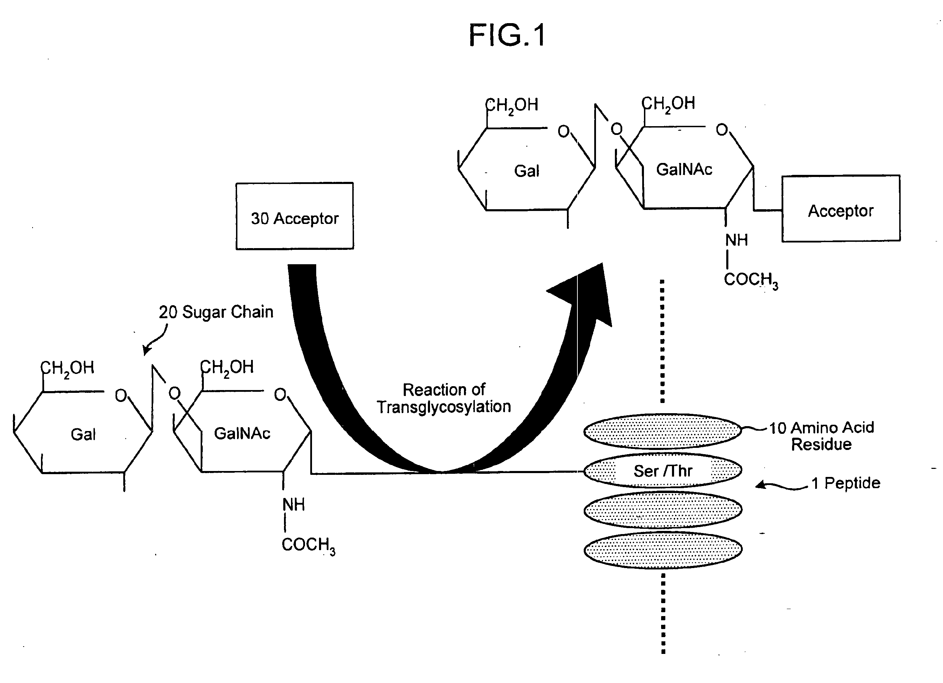

The protein used in the method for transglycosylation of the present invention has at least two activities, that is, endo-$\alpha$-$N$-acetylgalactosaminidase activity and transglycosylation activity. Endo-$\alpha$-$N$-acetylgalactosaminidase is an enzyme which recognizes a core 1 structure of an $O$-linked sugar chain in glycoprotein, that is, a structure having a Gal$\beta$1-3GalNAc disaccharide bound in the configuration of an $\alpha$ anomer to serine or threonine, and which has at least an activity of catalyzing a reaction of releasing the disaccharide unit by hydrolysis. The $O$-linked sugar chain is also called a mucin-type sugar chain and is contained in a large amount in mucin with which the surfaces of animal digestive tracts and organs are covered. The protein used in the present invention also has transglycosylation activity. As shonw in Fig. 1, the protein catalyzing the transglycosylation reaction in the transglycosylation method of the present invention can recognize at least a structure wherein a sugar chain moiety 20 of Gal$\beta$1-3GalNAc group in a glycoprotein (peptide 1) is bound in the configuration of an $\alpha$ anomer to serine or threonine, and which has an activity of transferring the Gal$\beta$1-3GalNAc group to an acceptor 30. The transglycosylation activity of the protein used in the present invention is high. The protein exhibiting a transglycosylation degree (%) of at least 2%, preferably 5% or more, more preferably 10% or more, still more preferably 15% or more, as determined by a method shown later in the Examples, is used.

The terms "in the presence of the protein" means that the reaction system is placed under conditions where the transglycosylation reaction can be carried out by the catalytic action of this protein. The protein may be supplied for example by adding a microorganism and/or the enzyme to the reaction system. That is, the microorganism and/or the enzyme may be allowed to be present in the reaction system in any form, provided that the microorganism and/or the enzyme effects the reaction of transferring galactosyl $\beta1\rightarrow3N$-acetylgalactosaminyl group from a galactosyl $\beta1\rightarrow3N$-acetylgalactosaminyl $\alpha$-group-containing sugar chain donor to a sugar chain acceptor. Either of the microorganism or enzyme may be used, or both of them may be present.

The "microorganism and/or the enzyme" may be in the following form. The specific formmay include a culture of the microorganisms, microbial cells separated from the culture, and a treated microbial cell product. The culture of the microorganisms is a material obtained by cultivating the microorganisms, and refers more specifically to a mixture of the microbial cells, a culture used in cultivating the microorganisms, and substances formed by the microorganism. The microbial cells may be used as a washed microorganisms after washing the cells. The treated microbial cell product may include materials obtained by disrupting, lyzing or freeze-drying the bacterium, as well as a crudely purified enzyme recovered by treating the microorganism etc. or a purified enzyme obtained by further purification. As the enzyme subjected to purification treatment, it is possible to use a enzyme such as partially purified enzyme obtained by various purification methods or an immobilized enzyme having such enzyme immobilized by a covalent bonding method, an adsorption method, an inclusion method or the like. Some of the used microorganisms are lyzed during cultivation, and in this case, a supernatant of the culture may also be utilized as a material containing the protein having transglycosylation activity.

The conditions in the reaction system using the microorganism and/or the enzyme may be suitably regulated depending on the condition such as specific type of microorganism, enzyme and starting material used. The amount of the microorganism and/or enzyme used may be an amount (effective amount) to exhibit the desired effect. This effective amount can be easily determined in a preliminary experiment which can be carried out easily by those skilled in the art; for example, when the enzyme is used, about 0.01 to 100 units (U) is preferable, and when the washed microorganisms are used, about 0.1 to 500 g/L is preferable. The reaction is carried at a temperature in the range where the enzyme used is active; that is, the reaction temperature is preferably in the range of 10 to 70°C, more preferably 20 to 65°C, still more preferably 25 to 60°C. The pH value of the enzyme reaction solution is regulated usually in the range of 2 to 12, preferably 4 to 10, more preferably 5 to 6.

The protein catalyzing the reaction described above may be obtained from a microorganism belonging to the genus *Bifidobacterium*. More specific example may include preferably *Bifidobacterium longum*, more preferably *Bifidobacterium longum* JCM1217. The stains given JCM numbers have been deposited with Japan Collection of Microorganisms, Riken Bioresource Center, (Hirosawa 2-1, Wako City, Saitama Prefecture, 351-0198, Japan), and may be obtained according to a predetermined procedure.

The protein catalyzing the transglycosylation reaction is selected from the group of protein (A) and (B) :

(A) a protein having an amino acid sequence of SEQ ID NO:2, or
(B) a protein having an endo-$\alpha$-$N$-acetylgalactosaminidase activity and a transglycosylation activity, and having the amino acid sequence that includes one to fifty amino acids mutation in the amino acid sequence of SEQ ID NO: 2, the mutation being selected from the group consisting of substitution, deletion, insertion, addition, and inversion.

The present inventors newly isolated a protein having the amino acid sequence of SEQ ID NO:2 from *Bifidobacterium longum* JCM1217, and specified the amino acid sequence of the protein catalyzing the above reaction.

In the production method of the present invention, a protein which is substantially the same as the protein shown in (A) above may also be used. Specifically, the protein shown in (B) is provided. Herein, the term "several" refers to 2 to 50. Note that it is desirable that among those of protein groups (B), the protein having the amino acid sequence containing mutation of one to fifty amino acid residues selected from the group of consisting of substitution, deletion, insertion, addition, and inversion, retain approximately 50% or more, more preferably 80% or more, and further preferably 90% or more of enzyme activity of the protein containing no mutation under the conditions, 50°C and pH8.

The mutation in amino acids as shown in the aforementioned (B) may be induced by modifying the nucleotide sequence so that the amino acids at the corresponding sites of the enzyme gene may be substituted, deleted, inserted, added, and/or inverted using, for example, the site-specific mutation method. Alternatively, the polynucleotide having modified nucleotide sequence may be obtained by the conventionally known mutation process. The mutation process may include a technique involving a step for in vitro inducing the mutation in the DNA encoding the amino acids of the (A) under the treatment of hydroxylamine and a technique involving a step for introducing the mutation in a bacterium belonging to the genus *Escherichia* containing the DNA encoding the amino acids of the (A) by UV irradiation, or under the treatment with *N*-methyl-*N'*-nitro-*N*-nitrosoguanidine (NTG) or any of mutating agents generally used for mutation engineering, such as nitrous acid.

Mutations such as substitution, deletion, insertion, addition and inversion in amino acids, accompanying the modification to the nucleotide sequence, may include naturally occurring mutations such as differences due to microbial species or microbial strain. By expressing such mutated DNA in a suitable cell and examining the activity of the present enzyme in the expression product, a DNA encoding substantially the same protein as the protein of SEQ ID NO:2 is obtained.

The polynucleotide encoding the protein used in the transglycosylation method of the present invention includes a polynucleotide encoding the amino acid sequence shown in SEQ ID NO:2. By codon degeneracy, there can be a plurality of nucleotide sequences encoding one amino acid sequence. That is, the polynucleotide used in the present invention includes polynucleotides having nucleotide sequences encoding the following proteins:

(A) a protein having an amino acid sequence of SEQ ID NO:2, and
(B) a protein having an endo-$\alpha$-*N*-acetylgalactosaminidase activity and a transglycosylation activity, and having the amino acid sequence that includes one to fifty amino acids mutation in the amino acid sequence of SEQ ID NO: 2 the mutation being selected from the group consisting of substitution, deletion, insertion, addition, and inversion.

The nucleotide sequence encoding the amino acid sequence of SEQ ID NO:2 is exemplified by the nucleotide sequence of SEQ ID NO:1. Further, as the polynucleotide which is substantially the same as the DNA having the nucleotide sequence of SEQ ID NO: 1, there may be mentioned a polynucleotide substantially the same as the polynucleotide having the nucleotide sequence shown in SEQ ID NO:1 and obtained by isolating a polynucleotide which hybridizes under stringent conditions with a polynucleotide consisting of, or with a probe prepared from, a nucleotide sequence complementary to the nucleotide sequence shown in SEQ ID NO: 1 and which encodes a protein having an endo-$\alpha$-*N*-acetylgalactosaminidase activity and a transglycosylation activity, from a polynucleotide encoding the protein having the amino acid sequence shown in SEQ ID NO:2 or from a cell retaining the polynucleotide.

Specific examples of the polynucleotide used in the present invention preferably include the following polynucleotides (a) and (b):

(a) a polynucleotide having a nucleotide sequence shown in SEQ ID NO:1 in the Sequence Listing, and
(b) a polynucleotide which hybridizes under stringent conditions with a polynucleotide consisting of a nucleotide sequence complementary to the nucleotide sequence of SEQ ID NO: 1, and which encodes a protein having an endo-$\alpha$-*N*-acetylgalactosaminidase activity and a transglycosylation activity.

The probe may be prepared in an ordinary method for example on the basis of the nucleotide sequence shown in SEQ ID NO:1. The method of probing, which the desired polynucleotide is isolated by screening a polynucleotide hybridizing with the probe, may also be carried out in an ordinary method. For example, the DNA probe may be prepared by amplifying the nucleotide sequence cloned in a plasmid or a phage vector, cleaving with a restriction enzyme a nucleotide sequence intended to be used as the probe, and extracting it. The site to be cleaved off may be regulated depending on the desired DNA.

As used herein, the "stringent conditions" refers to those conditions under which a specific hybrid is formed whereas an unspecific hybrid is not formed. These conditions are hardly expressed explicitly numerically, but by way of example, there may be mentioned those conditions under which DNA molecules having higher homology e.g. preferably 50% or more, more preferably 80 % or more, still more preferably 90 % or more, further more preferably 95% or more homology, hybridize with each other while DNA molecules having lower homology do not hybridize with each other, or those conditions under which hybridization occurs under usual washing conditions in Southern hybridization, that is, at a salt concentration corresponding to 1 × SSC and 0.1 % SDS at 60°C, preferably 0.1 × SSC and 0.1 % SDS at 60°C. Among the genes hybridizing under such conditions, there are genes having a stop codon in their sequence or encoding enzymes whose activity is lost due to a mutation in active center, and such genes can be easily removed by ligating each of them into a commercial expression vector, expressing it in a suitable host, and measuring the enzyme activity of each expression product by a method described later in the Examples.

As described above, a polynucleotide having the nucleotide sequence encoding the protein (B), and the polynucleotide (b), desirably keep about at least half of the enzyme activity of a protein having the amino acid sequence of SEQ ID NO:1, more preferably 80% or more, still more preferably 90% or more of the transglycosylation activity.

The DNA having the nucleotide sequence in SEQ ID NO:1 may be obtained from chromosomal DNA of *Bifidobacterium longum* JCM1217 or from a DNA library by PCR (polymerase chain reaction; see White, T. J. et al. in Trends Genet. 5, 185 (1989)) or by hybridization. The primers used in PCR may be designed based on an internal amino acid sequence determined on the basis of, for example, the purified protein having a peptide synthesizing activity. As PCR primers, those having sequences corresponding to the 5'-non-translating region and 3'-non-translating region respectively may be used to amplify a full-length coding region of the present protein.

The primers may be synthesized in a usual manner according to, for example, the phosphoamidite method (see Tetrahedron Letters (1981), 22, 1859) in a DNA synthesizer model 380B manufactured by Applied Biosystems. The PCR reaction may be carried out using, for example, Gene Amp PCR system 9600 (supplied from PERKIN ELMER) and TaKaRa La PCR in vitro Cloning Kit (supplied from TAKARA BIO INC.) by a method prescribed by a supplier such as each manufacturer.

Furthermore, for homologues of the protein having transglycosylation activity derived from *Bifidobacterium longum* JCM1217, nucleotide sequences for the protein in Example (8-4) below were specified by the present inventors.

The sugar chain donor used in the transglycosylation method of the present invention may be a compound having a Galβ1-3GalNAc group as a sugar chain, and examples may include mucin, a protein having a Galβ1-3GalNAc group as' a sugar chain, more specifically a protein having a structure where a disaccharide Galβ1-3GalNAc is bound at the α-position to serine or threonine, as well as Galβ1-3GalNAc group-containing p-nitrophenol (referred to hereinafter as Galβ1-3GalNAc α1-pNP; p-Nitrophenol is referred to as pNP).

The sugar chain acceptor used in the transglycosylation method of the present invention includes amino acids and polypeptides.

In one preferable embodiment of the transglycosylation method of the present invention, a transformant expressing the protein is prepared and used in the transglycosylation method. That is, transformed cells into which a polynucleotide encoding the protein (A) or (B) described above, or the polynucleotide (a) or (b) described above, was introduced are cultivated in a medium to accumulate a protein having an endo-α-*N*-acetylgalactosaminidase activity and a transglycosylation activity in the medium and/or the transformed cells thereby supplying the protein to the reaction system for transglycosylation.

As the host for expressing the protein specified by the polynucleotide having the nucleotide of SEQ ID NO: 1, it is possible to use various procaryotic cells including bacteria of the genus *Escherichia*, such as *Escherichia coli*, those of the genus *Empedobacter*, those of the genus *Sphingobacterium*, those of the genus *Flavobacterium*, and *Bacillus subtilis*, as well as various eucaryotic cells including *Saccharomyces cerevisiae, Pichia stipitis*, and *Aspergillus oryzae.*

A recombinant DNA used in introducing the DNA having the nucleotide sequence in SEQ ID NO:1 into a host can be prepared by inserting the DNA into a vector compatible with the host in such a form that the protein encoded by the DNA can be expressed. When a promoter inherent in the gene encoding the above protein derived from *Bifidobacterium longum* acts in the intended host cell, the promoter may be used for expressing the protein. If necessary, another promoter working in the host cell may be ligated to the DNA of SEQ ID NO:1 and expressed under its control.

The transformation method of introducing a recombinant such as an expression vector into a host cell may include the D. M. Morrison method (Methods in Enzymology 68, 326 (1979)), a method that involves treating a recipient microbial cell with calcium chloride to increase the permeability of DNA (Mandel, M. and Higa, A., J. Mol. Biol., 53, 159 (1970)).

The transglycosylation method of the present invention is used preferably in modifying foods, pharmaceutical preparations and various physiologically active substances. In a preferable embodiment of the method of modifying a food or pharmaceutical preparation, a sugar chain is transferred and added to a part or the whole of a component incorporated into the food or pharmaceutical preparation, to produce a modified food or pharmaceutical preparation containing the component having the sugar chain added thereto. The component to which the sugar chain is to be added is a sugar chain acceptor. Preferable examples of the sugar chain acceptor and donor include those described above.

In accordance with the method for transglycosylation of the present invention, a desirable glycoprotein (a sugar-amino acid compound and a sugar-polypeptide compound) may be produced.

Examples

Hereinafter, the present invention is described in more detail with reference to the following Examples, but the present invention is not limited thereto.

(1) Analysis of sugar chain by TLC (thin layer chromatography)

TLC was performed by using Silica gel 60 (MERCK) as the plate with a mixture of developing solvents of chloroform/methanol/water (3/3/1) and a coloration reagent with the composition shown in Table 1.

Table 1

| | |
|---|---|
| Acetone | 10 ml |
| Aniline | 100 $\mu$l |
| Diphenylamine | 0.1 g |
| Phosphoric acid | 1 ml |

(2) Analysis of a reaction product by HPLC (high performance liquid chromatography)

A reaction mixture was subjected to HPLC (supplied from Hitachi D-7420 UV-VIS Detector, D-7100 Pump, D-7500 Integrator) to analyze the reaction product. Analysis was performed under the following conditions according to a manual attached to the apparatus. A normal-phase column was used with Solvent A/Solvent B (25/75) as the solvent.

Column; TSK-gel amide-80
Solvent A; 100% $H_2O$
Solvent B; 100% $CH_3CN$
Column temp; 40°C
Flow rate; 1 mL/min
Wavelength; 214 nm

(3) Determination of enzyme activity

(3-1) Determination of hydrolysis activity

The enzymatic reaction was performed using Gal$\beta$1-3GalNAc$\alpha$1-pNP was used as the substrate, and liberated pNP (p-nitrophenol) was quantified colorimetrically under alkali conditions. Specifically, a sample was reacted at 37°C for a predetermined time, and $Na_2CO_3$ was added to terminate the reaction, followed by measurement of the absorbance at 400 nm. The composition of the reaction solution is shown below (Table 2). When another substrate was used to examine substrate specificity, it was used at the same concentration as that of Gal$\beta$1-3GalNAc$\alpha$1-pNP below.

Table 2

| | |
|---|---|
| 10 mM Gal$\beta$1-3GalNAc$\alpha$1-pNP | 0.5 $\mu$l |
| 1 M acetate buffer (pH 5.0) | 1.0 $\mu$l |
| Water | 16 $\mu$l |
| Enzyme | 2.5 $\mu$l |
| + | |
| $Na_2CO_3$ | 30 $\mu$l |

One unit was defined as the amount of the enzyme necessary to release 1 $\mu$mol pNP at 37°C per minute, and the activity was acquired according to the following equation (I):

$$\text{Units/ml} = A/(18.3 \ TV) \qquad \cdots \ (I),$$

wherein

A = absorbance (nm)

T = reaction time (min)

V = amount of the enzyme in the total volume including the termination solution

18.3 = millimolar absorptivity coefficient of pNP at pH 10 or more

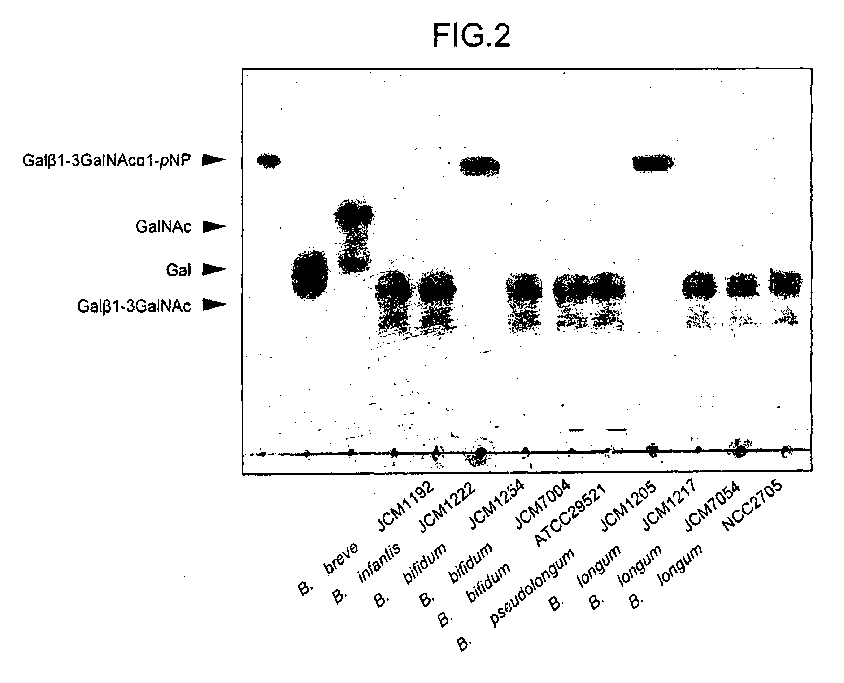

Various bifidobacteria were examined by using galactosyl β1→3*N*-acetylgalactosaminyl α1-*p*-nitrophenol (Galβ1-3GalNAc α1-pNP) as the substrate, to determine whether they had the activity of the present enzyme or not. The bifidobacteria were cultivated by stationary culture in GAM bouillon (supplied from Nissui Pharmaceutical) at 37°C for 24 hours under anaerobic conditions. When the reaction solution was subjected to TLC, the activity of the present enzyme in decomposing the substrate not into monosaccharide units but into disaccharide units was recognized in many strains, as shown in Fig. 2. In Fig. 2, the 4th lane (Galβ1-3GalNAc) from the left is a control of disaccharide obtained by cutting the same substrate with an enzyme derived from a bacterium of the genus *Bacillus* known as an endo-α-*N*-acetylgalactosaminidase-producing bacterium.

With respect to this reaction product and GalNAc, it appears that there are two separated spots, but this is estimated due to a difference in anomer. The present inventors separately confirmed that one spot appeared when a developing solvent and coloration reagent different from those described above were used.

(3-2) Determination of transglycosylation activity

The present enzyme derived from *Bifidobacterium longum* JCM1217 was examined for its transglycosylation activity in the following manner. In the reaction system for determining the transglycosylation activity, the pNP substrate was used as the sugar chain donor in every case. The reaction was performed using the sugar chain donor (final concentration, 2 mM), the acceptor (final concentration, 1 M) and the enzyme (recombinant endo-α-GalNAc-ase, final concentration, 61 units/L) in a 10 mM acetate buffer, pH 5.0. After the reaction was conducted at 37°C for 20 minutes, the reaction mixture was boiled for 3 minutes to inactivate the enzyme, then passed through a filter at 4°C and subjected to HPLC. The degree of transglycosylation was acquired by the following equation (II) :

```
Degree of transglycosylation (%) =

(Area of transglycosylation product peak/(Area of hydrolysis

product peak + Area of transglycosylation product peak)) x 100

                                                        ··· (II)
```

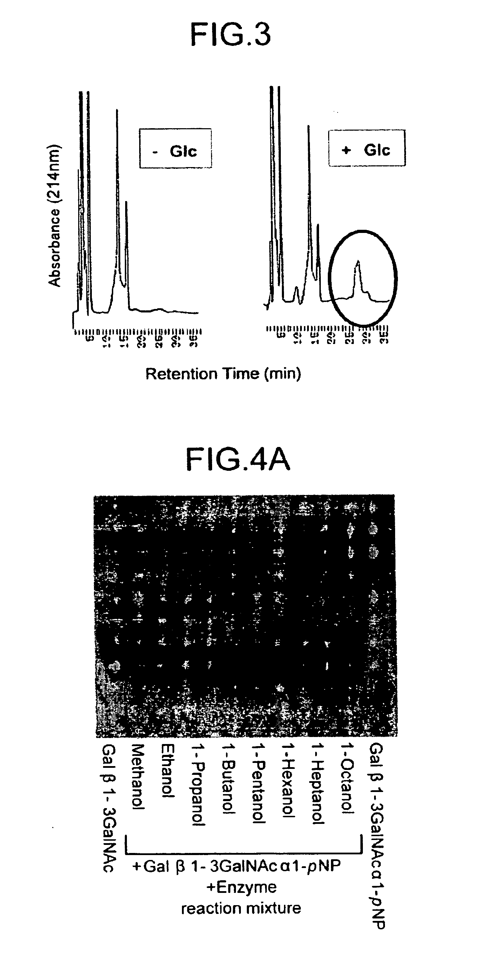

The reaction was carried out by using glucose as the acceptor and Galβ1-3GalNAcα1-pNP as the sugar chain donor, and the reaction solution was subjected to HPLC. The results are shown in Fig. 3. The left chromatograph (-Glc) is the glucose-free control, and the right chromatograph (+Glc) is the reaction solution. In the right chromatograph (+Glc), a new peak shown as the enclosed region was detected, and thus this region was isolated and analyzed by MALDI-TOF-MS (matrix assisted laser desorption ionization (MALDI); time of flight (TOF); mass spectrometry (MS)).

As a result, a peak indicating a trisaccharide having Glc added to Gal-GalNAc released from the sugar chain donor was obtained, thus revealing that the isolated enzyme had transglycosylation activity. The same HPLC analysis revealed that the sugar chain was also transferred to galactose, mannose and arabinose. The degree of transglycosylation to each acceptor under the predetermined reaction conditions is also shown (Table 3). The degree of transglycosylation to galactose, particularly to mannose, was lower than to glucose. It is estimated that a hydroxyl group at the 2-or 4-position is involved in the transglycosylation reaction.

Table 3

| Sugar chain acceptor | Degree of transglycosylation (%) |
| --- | --- |
| Glucose | 19.7 |
| Galactose | 8.3 |
| Mannose | 2.5 |
| Arabinose | 18.0 |
| Maltose | 14.4 |

(4) Method of cultivating *E. coli*

As the medium used, a liquid medium LB broth (supplied from DIFCO) was used, and when an agar medium was used, the medium was prepared by adding agar at a final concentration of 2% to the above liquid medium. When ampicilin was used, it was added to a final concentration of 150 $\mu$m/ml.

(5) Construction of expression plasmid

On the basis of the nucleotide sequence of ORF encoding protein BL0464 of unknown functions, which was estimated as Endo-$\alpha$-GalNAc-ase in *Bifidobacterium longum* NCC2705, primers capable of amplifying it by PCR were designed (Table 4). A restriction enzyme site was integrated in the primer to facilitate cloning. Using *Bifidobacterium longum* JCM1217 genome as a template, PCR was conducted using TaKaRa Ex Taq™ DNA Pblymerase (supplied from TAKARA BIO INC.) in a reaction solution having the following composition and under the following conditions.

Composition of the reaction solution: template DNA, 0.01 to 0.1 $\mu$g; 2.5 units TaKaRa Ex Taq™ DNA Polymerase; 10 $\mu$L 10X Ex Taq™ buffer (supplied from TAKARA BIO INC.); 0.2 mM dNTP mixture (supplied from TAKARA BIO INC.); and 1 $\mu$M primers.

Reaction conditions: 94°C, 30 seconds → (94°C, 30 seconds → 60°C, 30 seconds → 72°C, 6 minutes) × 30 cycles → 72°C, 6 minutes.

Table 4

| | |
|---|---|
| BL0464 forward primer ( NcoI-endo-$\alpha$-GalNAc-ase) | 5'-TTAACCTCCATGGGCAGCGGGGGAGG-3' :SEQ ID NO; 13 |
| BL0464 reverse primer (Endo-$\alpha$-GalNAc-ase-His$_6$-tag-NotI) | 5'-AACCTGCGGCCGCCAGTTGCTCGCGATTGC-3' SEQ ID NO; 14 |

The resulting PCR amplification product (about 6 kbp) was subjected to TA cloning, and then cleaved with restriction enzymes *Nco* I (supplied from Toyobo) and *Not* I (supplied from Toyobo) simultaneously, and the resulting fragment (about 6 kb) was inserted between *Nco* I/*Not* I sites in *E. coli* expression vector pET-23d(+) (supplied from Novagen). Ligation was carried out using Ligation High (supplied from TAKARA BIO INC.).

(6) Transformation of *E. coli*

Transformation of *E. coli* was conducted by adding the ligation reaction solution (about 20 $\mu$L) to competent cells (100 to 200 $\mu$L) then giving heat shock to the cells at 42°C for 45 seconds, adding 800 to 900 $\mu$L SOC medium to the cells and cultivating the cells at 37°C for 1 hour. The resultant culture was suitably diluted and applied onto an antibiotic-containing LB agar plate, and whether transformation occurred or not was confirmed by subjecting the growing *E. coli* to colony PCR, or by plasmid extraction and subsequent treatment with restriction enzymes. In extraction of the plasmid, Wizard plus SV Minipreps (supplied from Promega) was used. The *E. coli* competent cells were prepared by the Inoue method and stored at -80°C.

(7) Expression and purification of recombinant Endo-$\alpha$-GalNAc-ase

*E. coli* BL21 (DE3)/pET-23d-Endo-$\alpha$-GalNAc-ase was subjected to shake culture in LB-ampicilin medium at 37°C until the OD600 of the culture reached 0.4, and then IPTG was added at a final concentration of 0.4 mM, and shake culture was continued for 3 hours, whereby the desired Endo-$\alpha$-GalNAc-ase was expressed in a large amount. The bacterial cells were collected and used to prepare a cell-free extract fraction with Bug Buster reagent (supplied from Novagen), and its activity was confirmed.

For purification, the cell-free extract fraction was added to a nickel column HiTrap™ Chelating HP (supplied from Amersham) previously equilibrated with a buffer (2 mM Tris-HCl buffer (pH 8.0), 0.5 M NaCl) containing 5 mM imidazole, and an excess of protein in the column was washed away with the same buffer containing 5 mM imidazole, and subsequently the column was connected to FPLC. In FPLC, a buffer containing 20 mM imidazole, in an amount of 10cv (column volume), was passed through the column and then the desired Endo-$\alpha$-GalNAc-ase was eluted with a buffer containing 5cv 1 M imidazole. The eluted fractions were dialyzed against a 10 mM KPB buffer, pH 7.0, and the desired Endo-$\alpha$-GalNAc-ase was identified by activity measurement and SDS-PAGE.

(8) Analysis of DNA sequence

Gene manipulation was carried out mainly in accordance with the method of Sambrook et al. The analysis of DNA sequence was carried out according to the method of Sanger et al. by using BigDye terminator v3.0 cycle sequencing ready reaction kit and ABI prism™ 310 NT genetic analyzer (Applied Biosystems).
The full-length nucleotide sequence of ORF encoding the Endo-α-GalNAc-ase in the genome of *Bifidobacterium longum* JCM1217 was determined.

(8-1) Sequence of the gene fragment integrated in the vector

For sequencing of the whole region of the desired gene fragment integrated in the vector, a primer binding region in the vector was utilized, and using T7 promoter primer and M13 reverse primer, sequences at both ends of the gene fragment were decoded. On the basis of the revealed sequences, primers directing towards the inside of the gene fragment were designed to advance decoding. By repeating this procedure, the nucleotide sequence of the whole region of the gene fragment integrated in the vector was determined. Given the gene fragment integrated in the vector, PCR amplification was initiated from the primer sequence, and thus the N-terminal region and C-terminal region of full-length ORF encoding Endo-α-GalNAc-ase derived from *Bifidobacterium longum* JCM1217 could not be completely determined.

(8-2) Sequence of C-terminal region

To utilize homology among *Bifidobacterium longum* species, a primer (BL0464-C152 reverse primer: 5'-TGCGATTCATCGCCTAGCAG-3' (SEQ ID NO:15)) corresponding to the slightly outside of the C-terminus to the inside of ORF was designed on the basis of information on a nucleotide sequence of *Bifidobacterium longum* NCC2705. This primer, and the primer in Endo-α-GalNAc-ase gene fragment integrated in the vector, were used for PCR with *Bifidobacterium longum* JCM1217 genome as the template thereby to amplify the gene fragment containing the C-terminal region, which was then subj ected to TA cloning using T easy vector. By sequencing, the nucleotide sequence of the complete C-terminal region in ORF encoding Endo-α-GalNAc-ase derived from *Bifidobacterium longum* JCM1217 was determined.

(8-3) Sequence of N-terminal region

Because the outside of the N-terminus was composed of a repeat region, a suitable primer could not be designed, so that by using Southern blotting, a group of N-terminal region-containing gene fragments was obtained by restriction enzyme treatment from the genome of *Bifidobacterium longum* JCM1217. A vector having such fragment ligated therein was transformed into a competent cell. From the growing *E. coli*, positive clones were selected by colony PCR. By sequencing, the nucleotide sequence of the complete N-terminal region in ORF encoding Endo-α-GalNAc-ase derived from *Bifidobacterium longum* JCM1217 was determined.

(8-4)

On the basis of the information on the nucleotide sequence or amino acid sequence of the protein specified in *Bifidobacterium longum* JCM1217, homologue sequences were specified in the following microbial strains. Each microbial strain and the sequence number of its nucleotide sequence are shown. Each sequence can be searched from a database such as DDBJ (DNA Data Bank of Japan) by using each accession number etc.
*Streptococcus pneumoniae* R6 (Accession No. AE008413) ; SEQ ID NO:3 *Bifidobacterium longum* NCC2705 (Accession No. AE014666); SEQ ID NO:5
*Clostridium perfringens* str.13 (Accession No. AP003187); SEQ ID NO:7
*Streptomyces coelicolor* A3 (2) (Accession No. AL939127) ; SEQ ID NO:9 *Enterococcus faecalis* V583 (Accession No. AE016952) ; SEQ ID NO:11

(9) Transglycosylation into 1-alkanol

The transglycosylation experiment described in Example (3-2) was performed with various kinds of 1-alkanol as the acceptor. The reaction system for measurement of transglycosylation activity was carried out by using the sugar chain donor Galβ1-3GalNAcα1-pNP at a final concentration of 2 mM, the acceptor 1-alkanol at a final concentration of 15% (v/v) and the purified enzyme (recombinant endo-α-GalNAc-ase) at a final concentration of 6.8 units/L in a 50 mM potassium phosphate buffer, pH 7.0. After the reaction at 37°C for 60 minutes, 3 μl of the reaction solution was spotted on a TLC plate, developed with a mixture of solvent of chloroform/methanol/water (7/6.2/2), and colored with naphthoresorcinol in sulfuric acid (0.2% (w/v) naphthoresorcinol in sulfuric acid : ethanol (5:95 (v/v)). The results are shown in Fig. 4A. As shown in the drawing, when 1-alkanol was added as the acceptor, new spots were detected in addition to Galβ1-3GalNAc as a hydrolysis product of the sugar chain donor, and these spots were detected at different mobility depending on the type of 1-alkanol. Therefore it was considered that the spots was due to the substance of Galβ1-3GalNAc transferred to each alkanol.

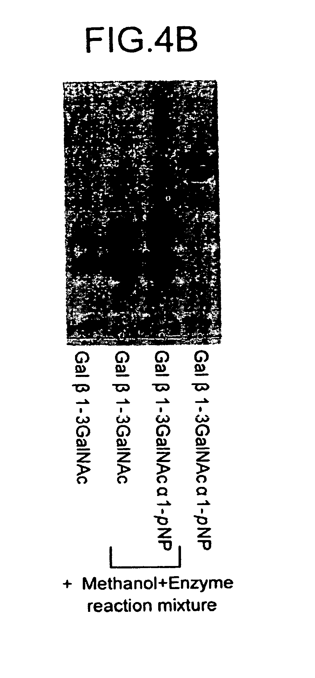

Using methanol as the sugar chain acceptor, the reaction with Galβ1-3GalNAc and the reaction with Galβ1-3GalNAcα1-pNP were then carried out respectively in order to confirm whether the formation of these substances by the enzyme reaction was due to the transglycosylation reaction or due to the reverse reaction of hydrolysis reaction. The reaction conditions were the same as described above except that Galβ1-3GalNAc and Galβ1-3GalNAcα1-pNP were used at a final concentration of 1.2 mM respectively. The results are shown in Fig. 4B. As shown in the drawing, the spot formed in Fig. 4A was not formed in the reaction of Galβ1-3GalNAc with methanol, but formed in the reaction of Galβ1-3GalNAcα1-pNP with methanol, thus revealing that the products were formed not by the reverse reaction of hydrolysis reaction but by the transglycosylation reaction.

(10) Transglycosylation into polypeptide

The transglycosylation activity by endo-α-N-acetylgalactosaminidase (endo-α-GalNAc-ase) when using various polypeptides as the sugar chain acceptor was examined. (10-1) Enzymatic reaction

First, an enzymatic reaction of recombinant endo-α-GalNAc-ase purified in (7) was performed with Galβ1-3GalNAcα1-pNP as the sugar chain donor and peptide-T (see SEQ ID: 16), PAMP-12 (an amino acid sequence shown in SEQ ID: 17 with an amino group attached at C-end of the sequence) and bradykinin (see SEQ ID: 18) as the sugar chain acceptor.

That is, 10 μl of Galβ1-3GalNAcα1-pNP, 6 μl solution of each polypeptide as the acceptor and 1.5 μl of NaP (sodium phosphate) buffer (500 mM, pH 6) were mixed. The sugar chain donor, Galβ1-3GalNAcα1-pNP was used at a concentration of 10 mM, and for the peptide solution, each of 50 mM peptide-T, 50 mM bradykinin, and 10 mM PAMP-12 was used. A solution obtained by mixing was warmed up to 37°C, subsequently 2. 5 μl of endo-α-GalNAc-ase (1.55 unit/ml) was added thereto, which was then incubated in a water bath at 37°C for one hour. Subsequently, the enzyme was inactivated by boiling for 3 to 5 minutes.

(10-2) Analysis and fractionation by HPLC

Each reaction solution obtained in the aforementioned enzymatic reaction was applied to an HPLC analysis, and reaction products were analyzed and fractionated. For both the analysis and fractionation, Pump L-7100, UV-VIS Detector L-7240 and Integrator D-7500 suppled from Hitachi were used an HPLC apparatus system, and COSMOSIL 5C$_{18}$-AR-II column (nakalai tesque): 10 x 250 mm was used as a column. An elution was performed under a condition of a temperature at 40°C using 0 to 25% acetonitrile (0.1% trifluoroacetic acid) as a solvent. UV (wavelength 214 nm) spectra were detected.

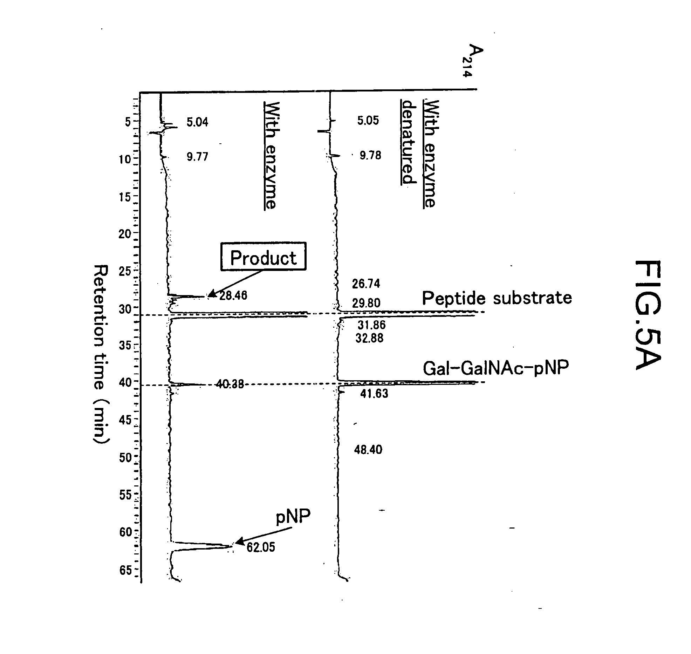

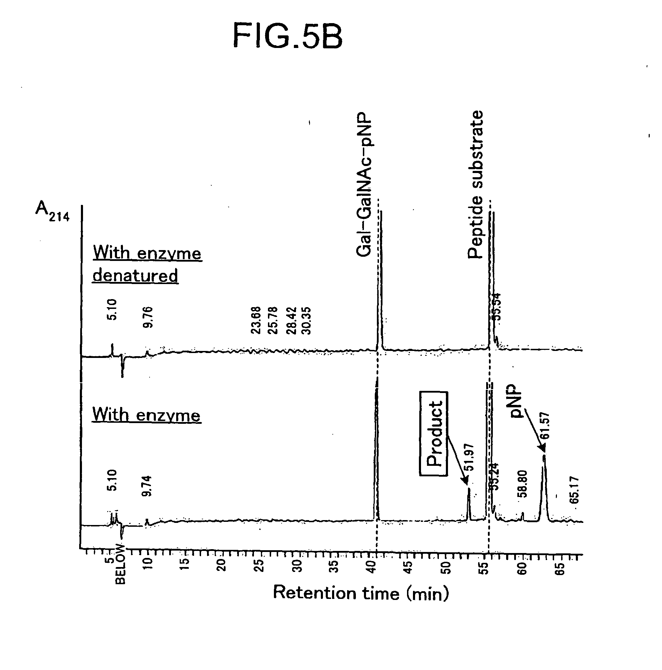

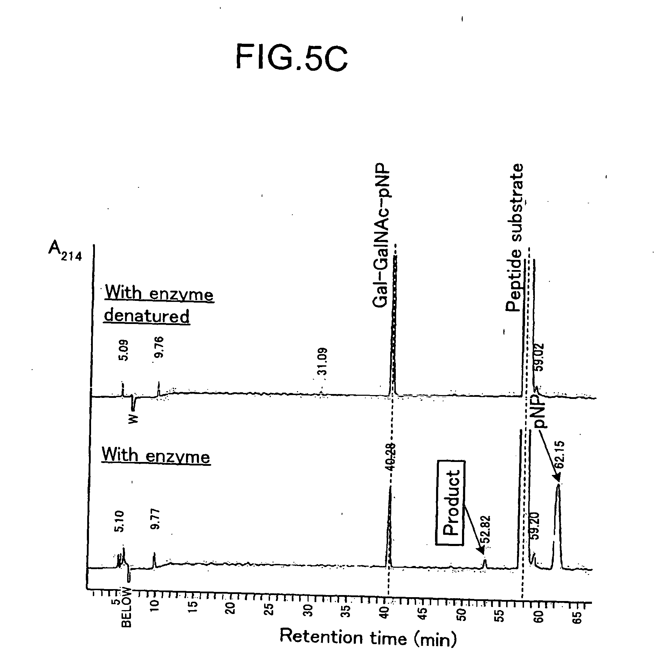

Detected results of each reaction solution for peptide-T, PAMP-12 and bradykinin are shown in Figs. 5A, 5B and 5C, respectively. Lower chromatogram in each of Figs. 5A, 5B and 5C shows the result of the experiment with the enzyme for each reaction solution obtained in the enzymatic reaction as described above. Upper chromatogram shows the result of the control with the denatured enzyme for each reaction solution obtained from the enzymatic reaction performed by adding the inactivated enzyme obtained by boiling the enzyme 5 minutes before the reaction, in place of the active enzyme.

As is evident from Figs. 5A, 5B and 5C, it was demonstrated that the sugar chain was transferred to each peptide because peaks of transglycosylation products ("product" portion in each Figure) were observed. Thus, these peak portions (fractions) were fractionated and applied to the following analysis.

(10-3) Analysis by mass spectrometry (MS)

The fractions fractionated by the aforementioned HPLC were analyzed by MALDI-TOF-MS. BRUKER Daltons Autofex-G MALDI-TOF mass spectrometer was used as an MALDI-TOF-MS apparatus.

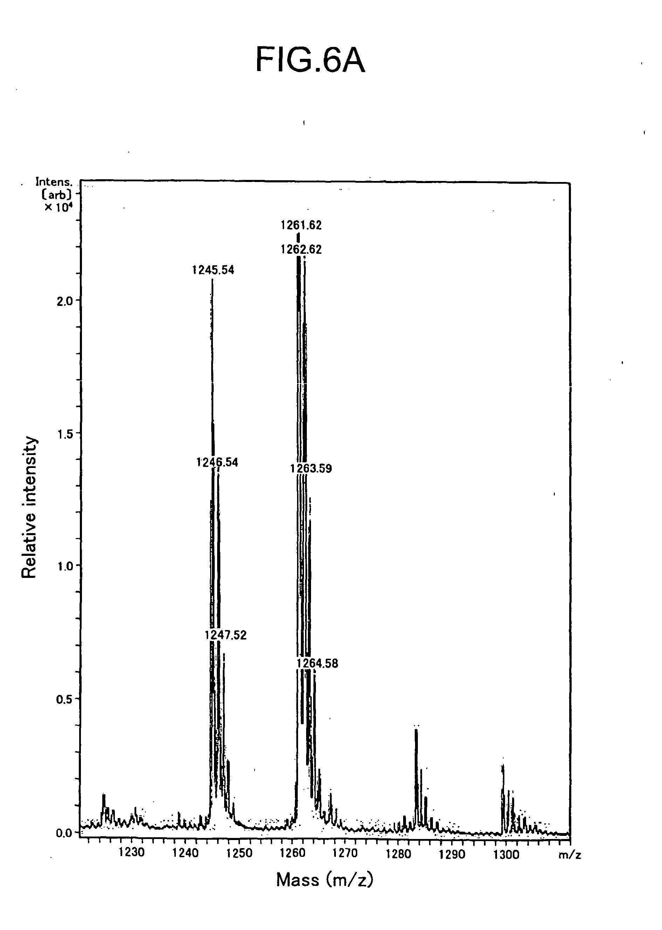

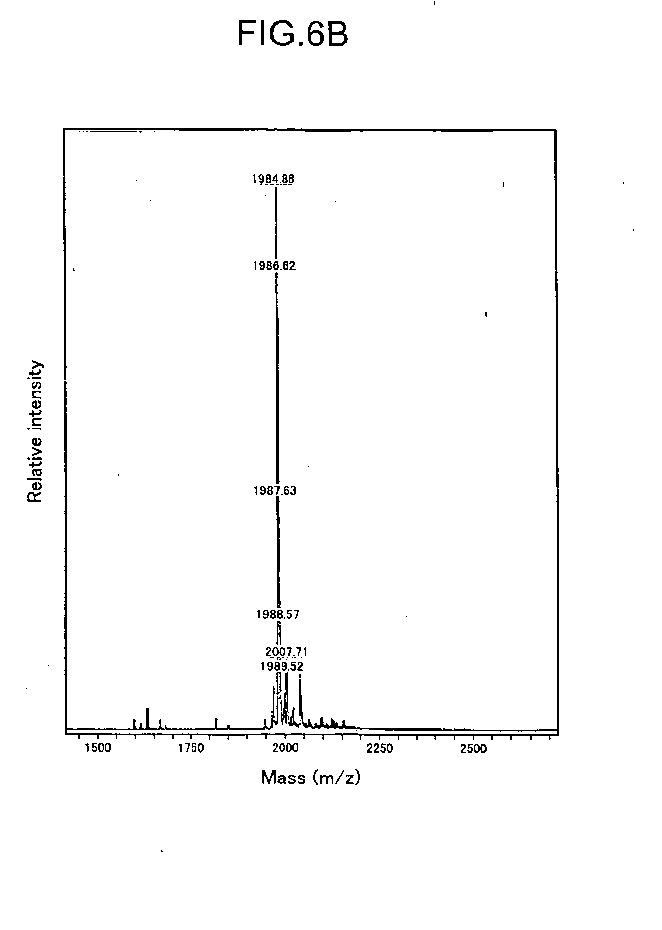

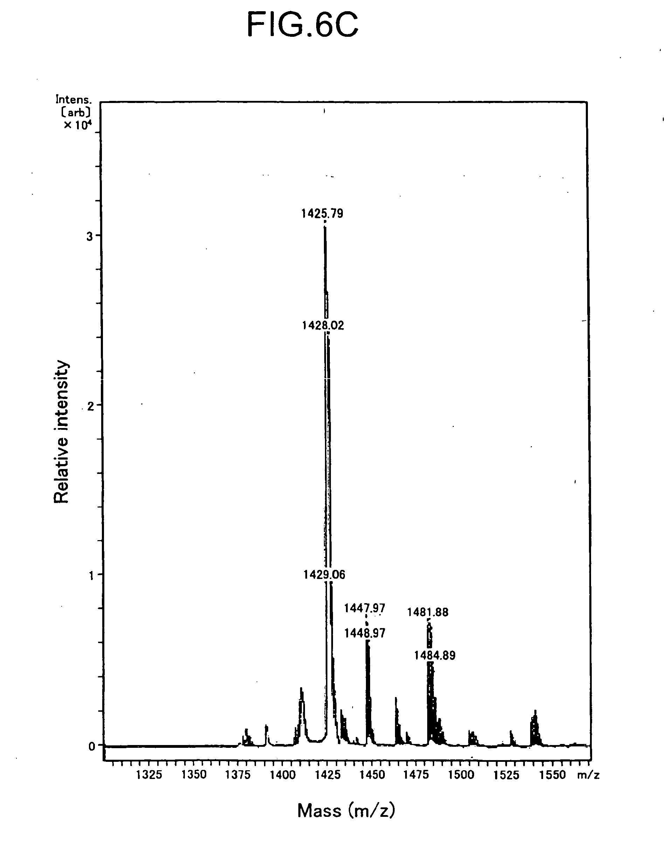

Detection results for MS spectra of the fractions fractionated from the reaction solutions of peptide-T, PAMP-12 and bradykinin are shown in Figs. 6A, 6B and 6C, respectively. As is evident from Figs 6A, 6B and 6C, the peaks corresponding to those in which disaccharide (Gal-GalNAc) had been transferred were obtained for all of the peptides. Each peak was as follows: Peptide-T: 1245.54 (M+Na)[+], 1261.62 (M+K)[+]; PAMP-12: 1984.68 (M+H)[+] ;Bradykinin: 1425.79 (M+H)[+]

## Claims

1. A method for transglycosylation, comprising a reaction of transferring a galactosylβ1→3N-acetylgalactosaminyl group from a sugar donor containing a sugar chain having a galactosylβ1→3N-acetylgalactosaminyl group binding in the configuration of an α anomer to an amino acid or a polypeptide as a sugar chain acceptor, in the presence of a protein having endo-α-N-acetylgalactosaminidase activity and transglycosylation activity, wherein the protein is selected from the group of (A) and (B):

   (A) a protein having an amino acid sequence described in SEQ ID NO:2, and
   (B) a protein having an endo-α-N-acetylgalactosaminidase activity and a transglycosylation activity, and having the amino acid sequence that includes one to fifty amino acids mutation in the amino acid sequence described in SEQ ID NO: 2, the mutation being selected from the group consisting of substitution, deletion, insertion, addition, and inversion.

2. The method for transglycosylation according to claim 1, wherein the protein is derived from a microorganism belonging to the genus *Bifidobacterium.*

3. The method for transglycosylation according to claim 1, comprising:

   cultivating a cell transformed with a polynucleotide encoding the protein in a suitable medium for the expression of the protein,
   forming the protein in the medium and/or the cell,
   and mixing the sugar donor and the sugar chain receptor with the medium and/or the cell.

4. A method of modifying a food comprising the method of claim 1, wherein the food before modification contains an amino acid or a polypeptide as a sugar chain acceptor.

5. A method of modifying a drug comprising the method of claim 1, wherein the drug before modification contains an amino acid or a polypeptide as a sugar chain acceptor.

6. A method for producing a glycoprotein comprising the method of claim 1.

7. The method according to claim 6, wherein the protein is derived from a microorganism belonging to the genus *Bifidobacterium.*

8. The method according to claim 6, wherein said step of transferring comprises:

   cultivating a cell transformed with a polynucleotide encoding the protein in a suitable medium for the expression of the protein,
   forming the protein in the medium and/or the cell,
   and mixing the sugar donor and the sugar chain receptor with the medium and/or the cell.

9. The method according to claim 8, wherein said cell is derived from a microorganism belonging to the genus Escherichia.

10. The method according to claim 3, wherein said cell is derived from a microorganism belonging to the genus Escherichia.

**Patentansprüche**

1. Verfahren zur Transglycosylierung, welches eine Reaktion zur Übertragung einer Galactosylβ1→3N-acetylgalactosaminylgruppe von einem Zuckerdonor, der eine Zuckerkette mit einer in der Konfiguration eines α-Anomers bindenden Galactosylβ1→3N-acetylgalactosaminylgruppe enthält, zu einer Aminosäure oder einem Polypeptid als Zuckerkettenakzeptor in Anwesenheit eines Proteins mit Endo-α-N-acetylgalactosaminidase-Aktivität und Transglycosylierungsaktivität umfasst,
wobei das Protein aus der aus (A) und (B) bestehenden Gruppe ausgewählt ist:

   (A) ein Protein mit einer in Sequenz ID NO:2 beschriebenen Aminosäuresequenz, und
   (B) ein Protein mit einer Endo-α-N-acetylgalactosaminidase-Aktivität und einer Transglycosylierungsaktivität und der Aminosäuresequenz, die in der Aminosäuresequenz der Sequenz ID NO: 2 eine Mutation von 1 bis 50 Aminosäuren umfasst, wobei die Mutation aus der Gruppe ausgewählt ist, die aus Substitution, Deletion, Insertion, Addition oder Inversion besteht.

2. Verfahren zur Transglycosylierung nach Anspruch 1,
wobei das Protein von einem Mikroorganismus der Gattung Bifidobacterium abgeleitet ist.

3. Verfahren zur Transglycosylierung nach Anspruch 1, welches umfasst:

   Kultivieren einer mit einem für das Protein kodierenden Polynucleotid transformierten Zelle in einem geeigneten Medium für die Expression des Proteins,
   Bilden des Proteins in dem Medium und/oder der Zelle und
   Mischen des Zuckerdonors und des Zuckerkettenakzeptors mit dem Medium und/oder der Zelle.

4. Verfahren zum Modifizieren eines Nahrungsmittels, welches das Verfahren nach Anspruch 1 umfasst, wobei das Nahrungsmittel vor der Modifikation eine Aminosäure oder ein Polypeptid als Zuckerkettenakzeptor enthält.

5. Verfahren zum Modifizieren eines Arzneimittels, welches das Verfahren nach Anspruch 1 umfasst, wobei das Arzneimittel vor der Modifikation eine Aminosäure oder ein Polypeptid als Zuckerkettenakzeptor enthält.

6. Verfahren zum Herstellen eines Glycoproteins, welches das Verfahren nach Anspruch 1 umfasst.

7. Verfahren nach Anspruch 6, wobei das Protein von einem Mikroorganismus der Gattung Bifidobacterium abgeleitet ist.

8. Verfahren nach Anspruch 6, wobei die Stufe der Übertragung folgendes umfasst:

   Kultivieren einer mit einem für das Protein kodierenden Polynucleotid transformierten Zelle in einem geeigneten Medium für die Expression des Proteins,
   Bilden des Proteins in dem Medium und/oder der Zelle und
   Mischen des Zuckerdonors und des Zuckerkettenakzeptors mit dem Medium und/oder der Zelle.

9. Verfahren nach Anspruch 8, wobei die Zelle von einem Mikroorganismus der Gattung Escherichia abgeleitet ist.

10. Verfahren nach Anspruch 3, wobei die Zelle von einem Mikroorganismus der Gattung Escherichia abgeleitet ist.


**Revendications**

1. Procédé de transglycosylation comprenant une réaction de transfert d'un groupe galactosylβ1→3*N*-acétylgalactosaminyle d'un donneur de sucre contenant une chaîne de sucre ayant un groupe galactosylβ1→3*N*-acétylgalactosaminyle lié dans la configuration d'un anomère α à un aminoacide ou un polypeptide à titre d'accepteur de chaîne de sucre, en présence d'une protéine ayant une activité d'endo-α-*N*-acétylgalactosaminidase et une activité de transglycosylation,
où la protéine est choisie dans le groupe de (A) et (B) :

   (A) une protéine ayant une séquence d'aminoacides décrite dans SEQ ID NO:2, et
   (B) une protéine ayant une activité d'endo-α-*N*-acétylgalactosa-minidase et une activité de transglycosylation, et ayant la séquence d'aminoacides qui inclut une mutation d'un à cinquante aminoacides dans la séquence d'aminoacides décrite dans SEQ ID NO:2, la mutation étant choisie dans le groupe consistant en une substitution, une délétion, une insertion, une addition et une inversion.

2. Procédé de transglycosylation selon la revendication 1 où la protéine est dérivée d'un micro-organisme appartenant au genre *Bifidobacterium.*

3. Procédé de transglycosylation selon la revendication 1 comprenant :

la culture d'une cellule transformée avec un polynucléotide codant la protéine dans un milieu approprié pour l'expression de la protéine,
la formation de la protéine dans le milieu et/ou la cellule,
et le mélange du donneur de sucre et du récepteur de chaîne de sucre avec le milieu et/ou la cellule.

4. Procédé de modification d'un aliment comprenant le procédé selon la revendication 1 où l'aliment avant la modification contient un aminoacide ou un polypeptide en tant qu'accepteur de chaîne de sucre.

5. Procédé de modification d'un médicament comprenant le procédé selon la revendication 1 où le médicament avant la modification contient un aminoacide ou un polypeptide en tant qu'accepteur de chaîne de sucre.

6. Procédé de production d'une glycoprotéine comprenant le procédé selon la revendication 1.

7. Procédé selon la revendication 6 où la protéine est dérivée d'un micro-organisme appartenant au genre *Bifidobacterium.*

8. Procédé selon la revendication 6 où ladite étape de transfert comprend :

la culture d'une cellule transformée avec un polynucléotide codant la protéine dans un milieu approprié pour l'expression de la protéine,
la formation de la protéine dans le milieu et/ou la cellule,
et le mélange du donneur de sucre et du récepteur de chaîne de sucre avec le milieu et/ou la cellule.

9. Procédé selon la revendication 8 où ladite cellule est dérivée d'un micro-organisme appartenant au genre Escherichia.

10. Procédé selon la revendication 3 où ladite cellule est dérivée d'un micro-organisme appartenant au genre Escherichia.

# FIG.1

30 Acceptor

20 Sugar Chain

Gal

CH₂OH

GalNAc

CH₂OH

NH
COCH₃

Reaction of
Transglycosylation

Gal

CH₂OH

GalNAc

CH₂OH

NH
COCH₃

Acceptor

10 Amino Acid
Residue

Ser /Thr

1 Peptide

EP 1 767 645 B1

FIG.2

# FIG.3

Retention Time (min)

# FIG.4A

# FIG.4B

Gal β 1-3GalNAc

Gal β 1-3GalNAc

Gal β 1-3GalNAc α 1-*p*NP

Gal β 1-3GalNAc α 1-*p*NP

+ Methanol+Enzyme
reaction mixture

EP 1 767 645 B1

# FIG.5A

# FIG.5B

# FIG.5C

# FIG.6A

# FIG.6B

# FIG.6C

**EP 1 767 645 B1**

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Non-patent literature cited in the description**

- **Bhavanandan, V. P. et al.** *Biochem. Biophys. Res. Commum.,* 1976, vol. 70, 738-745
- **Bardales, R. M. ; Bhavanandan, V.P.** *J. Biol. Chem.,* 1989, vol. 264, 19893-19897
- *J. of Bioscience and Bioengineering,* May 2005, 457-465
- *J. of Biological Chemistry,* 01 September 2005, 37415-37422
- Molecular Cloning: A LABORATORY MANUAL. Cold Spring Harbor Laboratory Press, 2001
- Experimental Medicine. **Masami MURAMATSU et al.** Shin Idenshi Kogaku Handbook. Yodosha, 1999
- **Masato OKADA ; Kaori MIYAZAKI.** Tanpakushitsu Jikken No Susumekata. Yodosha, 1998
- **Masato OKADA ; Kaori MIYAZAKI.** Tanpakushitsu Jikken Note. Yodosha, 1999
- **White, T. J. et al.** *Trends Genet.,* 1989, vol. 5, 185
- *Tetrahedron Letters,* 1981, vol. 22, 1859
- *Methods in Enzymology,* 1979, vol. 68, 326
- **Mandel, M. ; Higa, A.** *J. Mol. Biol.,* 1970, vol. 53, 159

SEQUENCE LISTING

<110> Ajinomoto Co., Inc.

<120> Transglycosylation method and method for producing glycoprotein

<130> PAMA-05004-EP

<160> 18

<170> PatentIn version 3.1

<210> 1
<211> 5901
<212> DNA
<213> Bifidobacterium lomgum JCM1217

<220>
<221> CDS
<222> (1).. (5901)

<400> 1

```
atg aaa aag aag aag act ata tcg gct gcg ctg gca aca gcg tta gcc       48
Met Lys Lys Lys Lys Thr Ile Ser Ala Ala Leu Ala Thr Ala Leu Ala
1               5                   10                  15

tta acc tgc atg ggc agc ggg gga ggt act gcg ttc gca gtg ccc ctg       96
Leu Thr Cys Met Gly Ser Gly Gly Gly Thr Ala Phe Ala Val Pro Leu
                20                  25                  30

tct gat gct gac ttg cag act ttg gca agt cag att cag caa atc aac      144
Ser Asp Ala Asp Leu Gln Thr Leu Ala Ser Gln Ile Gln Gln Ile Asn
            35                  40                  45

gat act tct gat tct gca act gct tcc gag act cct tcc gca caa gcc      192
Asp Thr Ser Asp Ser Ala Thr Ala Ser Glu Thr Pro Ser Ala Gln Ala
        50                  55                  60

gat gcg gtt gaa ggc tgg act att gat tcc aac atc gct cag ggc ggc      240
Asp Ala Val Glu Gly Trp Thr Ile Asp Ser Asn Ile Ala Gln Gly Gly
65                  70                  75                  80

gaa atc ctg gag atg gca aac ggt tgg ctg cac ctc aag tcc act gcc      288
Glu Ile Leu Glu Met Ala Asn Gly Trp Leu His Leu Lys Ser Thr Ala
                85                  90                  95

tct aac ggt aat gcg gca gcg aac ccc agc tcc agc aac aac tgg ccg      336
Ser Asn Gly Asn Ala Ala Ala Asn Pro Ser Ser Ser Asn Asn Trp Pro
                100                 105                 110

gca gta gcc gta tgg ggc aca gat tac gac ttc tcc aag gcc ggc tcc      384
Ala Val Ala Val Trp Gly Thr Asp Tyr Asp Phe Ser Lys Ala Gly Ser
            115                 120                 125

ttc cac gcc acc atc aaa tcc ccg cag gaa ggc tcc gcc aac cgc ttc      432
Phe His Ala Thr Ile Lys Ser Pro Gln Glu Gly Ser Ala Asn Arg Phe
        130                 135                 140

ggc ttc tac ctg ggc tac aac gac ccg ggc agc ggc ctg ttc atc ggc      480
Gly Phe Tyr Leu Gly Tyr Asn Asp Pro Gly Ser Gly Leu Phe Ile Gly
145                 150                 155                 160

tac gat tcg ggc ggc tgg ttc tgg cag acc tac acc ggt ggc ggt agc      528
Tyr Asp Ser Gly Gly Trp Phe Trp Gln Thr Tyr Thr Gly Gly Gly Ser
                165                 170                 175

ggc agc tgg tac agc ggt gct cgt atc gct gct ccg agc gcc aac gaa      576
Gly Ser Trp Tyr Ser Gly Ala Arg Ile Ala Ala Pro Ser Ala Asn Glu
                180                 185                 190

gag cac gac att cgg gtc tcc tgg acc gac gcc aag gtc gcc aca ctg      624
Glu His Asp Ile Arg Val Ser Trp Thr Asp Ala Lys Val Ala Thr Leu
            195                 200                 205
```

```
acc gtg gat ggc cag aag gca ttc gat gtc gat tac tcc gca atg acg      672
Thr Val Asp Gly Gln Lys Ala Phe Asp Val Asp Tyr Ser Ala Met Thr
    210             215             220

aac ctc tcc aac aag ctt gcc atc aag gcc ggc tcc tgg aag gag ctg      720
Asn Leu Ser Asn Lys Leu Ala Ile Lys Ala Gly Ser Trp Lys Glu Leu
225             230             235             240

aac gag gtc acc gac gtg tac atc aag gac ttc ccg gag gtt gtc gaa      768
Asn Glu Val Thr Asp Val Tyr Ile Lys Asp Phe Pro Glu Val Val Glu
            245             250             255

gcc gcc aag cac gcg gtt tcc ggc aag gtt gtg gac gct gga ggc gct      816
Ala Ala Lys His Ala Val Ser Gly Lys Val Val Asp Ala Gly Gly Ala
        260             265             270

gcc att gaa ggc gca aca gtg cgt ttg gat aag acc aag gtg aag acc      864
Ala Ile Glu Gly Ala Thr Val Arg Leu Asp Lys Thr Lys Val Lys Thr
        275             280             285

ggt gca gat ggc acc ttc tcc ttc gcc gac att gaa gaa ggc gaa cac      912
Gly Ala Asp Gly Thr Phe Ser Phe Ala Asp Ile Glu Glu Gly Glu His
    290             295             300

acg ctt tcg att gcc aag gaa ggc tat gag gac gtc tcc cag cag gtg      960
Thr Leu Ser Ile Ala Lys Glu Gly Tyr Glu Asp Val Ser Gln Gln Val
305             310             315             320

acg gtc tcc ggc gcg gat ctt gcc atc gat ccc atc acc ctg aac aaa     1008
Thr Val Ser Gly Ala Asp Leu Ala Ile Asp Pro Ile Thr Leu Asn Lys
            325             330             335

acc gtt cag gtc gcc tcc gaa acg ctg aag acc aag aag atg gaa gtt     1056
Thr Val Gln Val Ala Ser Glu Thr Leu Lys Thr Lys Lys Met Glu Val
            340             345             350

cag att aag aag aac ttc ccc tct gtg ctg cag tac acg atg acc gac     1104
Gln Ile Lys Lys Asn Phe Pro Ser Val Leu Gln Tyr Thr Met Thr Asp
        355             360             365

ggc aag gtg atg tac ggc cag tcc aag gat gtg cgc acc gtc gaa atc     1152
Gly Lys Val Met Tyr Gly Gln Ser Lys Asp Val Arg Thr Val Glu Ile
    370             375             380

aac ggc acc aac atc gaa ctg ggc gat gac gac gtg acc ttc aag aag     1200
Asn Gly Thr Asn Ile Glu Leu Gly Asp Asp Asp Val Thr Phe Lys Lys
385             390             395             400

gtt tcc gac acc gag gcc acc tac acg ctg aag gtc aag gat gag gcc     1248
Val Ser Asp Thr Glu Ala Thr Tyr Thr Leu Lys Val Lys Asp Glu Ala
            405             410             415

aag aag att gac gcg gtg atc acc gtt cag atc acg gtc aag gcc aac     1296
Lys Lys Ile Asp Ala Val Ile Thr Val Gln Ile Thr Val Lys Ala Asn
            420             425             430

cag ctg cac ctc aac gtc acc aag atc aag aac aac ctg tcc gaa ggc     1344
Gln Leu His Leu Asn Val Thr Lys Ile Lys Asn Asn Leu Ser Glu Gly
        435             440             445

att cct gag ggc aac ggt gtg gag gag aac gcc atc cag acg ctg tcc     1392
Ile Pro Glu Gly Asn Gly Val Glu Glu Asn Ala Ile Gln Thr Leu Ser
    450             455             460

ttc ccg aac cag agt ctc gtt tcc gtg cgt tcc agc cag gaa aat gcc     1440
Phe Pro Asn Gln Ser Leu Val Ser Val Arg Ser Ser Gln Glu Asn Ala
465             470             475             480

caa ttc act ggt gct cgt atg tct tcc aac acg cag aag cct ggc gat     1488
Gln Phe Thr Gly Ala Arg Met Ser Ser Asn Thr Gln Lys Pro Gly Asp
            485             490             495

acc aac ttc gca gtg acc gaa gat act aac gtc acc gat agc gac tac     1536
Thr Asn Phe Ala Val Thr Glu Asp Thr Asn Val Thr Asp Ser Asp Tyr
        500             505             510
```

14

EP 1 767 645 B1

```
acc tac ggc ttc atc tcc ggt gct ggc ctg agt gcc ggc ctg tgg agc    1584
Thr Tyr Gly Phe Ile Ser Gly Ala Gly Leu Ser Ala Gly Leu Trp Ser
        515               520               525

aac tcc gag cac gat ggc acc tat gtg gcg gct cct gtg cgc ggc ggc    1632
Asn Ser Glu His Asp Gly Thr Tyr Val Ala Ala Pro Val Arg Gly Gly
    530               535               540

agc cag aac acg cgt gtc tac gcc acc acc cag cag act ggt gac gcc    1680
Ser Gln Asn Thr Arg Val Tyr Ala Thr Thr Gln Gln Thr Gly Asp Ala
545               550               555               560

acc tcc ctg ggc ctg gcc agc gct ccg tgg tac tac cac cgc acg gtc    1728
Thr Ser Leu Gly Leu Ala Ser Ala Pro Trp Tyr Tyr His Arg Thr Val
                565               570               575

acc gat tcc aag ggc aag aag tac acc gtg gcc gaa acc gct ctg ccg    1776
Thr Asp Ser Lys Gly Lys Lys Tyr Thr Val Ala Glu Thr Ala Leu Pro
            580               585               590

cag atg gcc gtg gcc atc gcc ggc gac gag aac gaa gac ggt gcc gtc    1824
Gln Met Ala Val Ala Ile Ala Gly Asp Glu Asn Glu Asp Gly Ala Val
        595               600               605

aac tgg cag gat ggc gca atc gcc tac cgc gac atc atg aac aac ccg    1872
Asn Trp Gln Asp Gly Ala Ile Ala Tyr Arg Asp Ile Met Asn Asn Pro
    610               615               620

tac aag tcc gag gaa gtt ccc gaa ctg gtg gca tgg cgt atc gcc atg    1920
Tyr Lys Ser Glu Glu Val Pro Glu Leu Val Ala Trp Arg Ile Ala Met
625               630               635               640

aac ttc ggc tcc cag gcg cag aac ccg ttc ctc acc acg ctt gac aac    1968
Asn Phe Gly Ser Gln Ala Gln Asn Pro Phe Leu Thr Thr Leu Asp Asn
                645               650               655

gtc aag aag gtg gcc ttg aac acc gac ggc ctc ggc cag tcc gtg ctg    2016
Val Lys Lys Val Ala Leu Asn Thr Asp Gly Leu Gly Gln Ser Val Leu
                660               665               670

ctc aag ggc tac ggc aat gaa ggc cac gac tcc ggc cac ccg gac tac    2064
Leu Lys Gly Tyr Gly Asn Glu Gly His Asp Ser Gly His Pro Asp Tyr
                675               680               685

ggc gat atc ggc cag cgt ctc ggc ggc gcc gac gac atg aac acc atg    2112
Gly Asp Ile Gly Gln Arg Leu Gly Gly Ala Asp Asp Met Asn Thr Met
    690               695               700

atg gaa gag ggc tcc aag tat ggc gct cgc ttc ggt gtg cac gtc aac    2160
Met Glu Glu Gly Ser Lys Tyr Gly Ala Arg Phe Gly Val His Val Asn
705               710               715               720

gcc tcc gaa atg tat ccg gaa gcc aag gcc ttc agc gag gac atg gtg    2208
Ala Ser Glu Met Tyr Pro Glu Ala Lys Ala Phe Ser Glu Asp Met Val
                725               730               735

cgc cgc aac tct gca ggc ggc ctg agc tac ggc tgg aac tgg ctt gat    2256
Arg Arg Asn Ser Ala Gly Gly Leu Ser Tyr Gly Trp Asn Trp Leu Asp
                740               745               750

cag ggt gtc ggc atc gac ggc atc tac gat ctg gca tcc ggt tct cgt    2304
Gln Gly Val Gly Ile Asp Gly Ile Tyr Asp Leu Ala Ser Gly Ser Arg
        755               760               765

gta agc cgt ttc gct gac ctc agc aag gaa gtc ggc gac aac atg gac    2352
Val Ser Arg Phe Ala Asp Leu Ser Lys Glu Val Gly Asp Asn Met Asp
    770               775               780

ttc atc tac ctc gat gtg tgg ggc aac ctg act tct tcc ggt tcg gaa    2400
Phe Ile Tyr Leu Asp Val Trp Gly Asn Leu Thr Ser Ser Gly Ser Glu
785               790               795               800

gat tct tgg gaa acc cgc aag atg agc aag atg atc aac gac aac ggc    2448
Asp Ser Trp Glu Thr Arg Lys Met Ser Lys Met Ile Asn Asp Asn Gly
                805               810               815
```

15

```
tgg cgt atg acc acc gaa tgg ggt tcc ggc aac gag tac gac tcc acc    2496
Trp Arg Met Thr Thr Glu Trp Gly Ser Gly Asn Glu Tyr Asp Ser Thr
            820             825                 830

ttc cag cac tgg gca gct gat ctg acc tac ggc ggc tac acc tcc aag    2544
Phe Gln His Trp Ala Ala Asp Leu Thr Tyr Gly Gly Tyr Thr Ser Lys
        835                 840                 845

ggc gag aac tcc gaa gtg atg cgc ttc ctg cgc aac cac cag aag gac    2592
Gly Glu Asn Ser Glu Val Met Arg Phe Leu Arg Asn His Gln Lys Asp
        850                 855                 860

agc tgg gtt ggc gac tac ccg caa tac ggc ggc gct gcc aac gcc ccg    2640
Ser Trp Val Gly Asp Tyr Pro Gln Tyr Gly Gly Ala Ala Asn Ala Pro
865                 870                 875                 880

ctg ctc ggc ggc tac aac atg aag gac ttc gaa ggc tgg cag ggc cgc    2688
Leu Leu Gly Gly Tyr Asn Met Lys Asp Phe Glu Gly Trp Gln Gly Arg
                885                 890                 895

aac gac tat gcc gcc tac atc aag aac ctg tac acc cat gat gtg tcc    2736
Asn Asp Tyr Ala Ala Tyr Ile Lys Asn Leu Tyr Thr His Asp Val Ser
                900                 905                 910

act aag ttc atc cag cac ttc aag gtg acc cgc tgg gtc aac aac ccg    2784
Thr Lys Phe Ile Gln His Phe Lys Val Thr Arg Trp Val Asn Asn Pro
            915                 920                 925

ctg ctg acc gcc gac aat ggc aat gcc gct gcc gtg tcc gac ccg aac    2832
Leu Leu Thr Ala Asp Asn Gly Asn Ala Ala Ala Val Ser Asp Pro Asn
        930                 935                 940

acg aac aac ggc aac gag cag att acc ctg aag gat tcc aac ggc aac    2880
Thr Asn Asn Gly Asn Glu Gln Ile Thr Leu Lys Asp Ser Asn Gly Asn
945                 950                 955                 960

gtt gta gta gtc tcc cgt ggt tcc aac gac acc tct agc gca gcc tac    2928
Val Val Val Val Ser Arg Gly Ser Asn Asp Thr Ser Ser Ala Ala Tyr
                965                 970                 975

cgc cag cgc acc atc acc ttc aac ggc gtg aag gtc gca tcc ggt gtg    2976
Arg Gln Arg Thr Ile Thr Phe Asn Gly Val Lys Val Ala Ser Gly Val
            980                 985                 990

gtc tcc gca ggc gat ggc agc gcc   act ggc gac gag tcc  tac ctg ctg    3024
Val Ser Ala Gly Asp Gly Ser Ala   Thr Gly Asp Glu Ser  Tyr Leu Leu
            995             1000                1005

ccg tgg  atg tgg gat tcc ttc  acc ggc aag ctg gtc  aag gat tcc    3069
Pro Trp  Met Trp Asp Ser Phe  Thr Gly Lys Leu Val  Lys Asp Ser
    1010                1015                1020

gag cag  aag ctc tac cac tgg  aac acc aag ggc ggc  acc acc acc    3114
Glu Gln  Lys Leu Tyr His Trp  Asn Thr Lys Gly Gly  Thr Thr Thr
    1025                1030                1035

tgg acg  ctg ccg gac agc tgg  aag aac ctc tcc agc  gta aag gtg    3159
Trp Thr  Leu Pro Asp Ser Trp  Lys Asn Leu Ser Ser  Val Lys Val
    1040                1045                1050

tac cag  ctc acc gat cag ggc  aag acc aac gag cag  acc gtt gcc    3204
Tyr Gln  Leu Thr Asp Gln Gly  Lys Thr Asn Glu Gln  Thr Val Ala
    1055                1060                1065

gtc tcc  ggc ggc aag gtg acg  ctt acc gct gat gcc  gaa acc ccg    3249
Val Ser  Gly Gly Lys Val Thr  Leu Thr Ala Asp Ala  Glu Thr Pro
    1070                1075                1080

tac gtg  gtg tac aag ggc gaa  gcc aag cag atc cag  gtc aac tgg    3294
Tyr Val  Val Tyr Lys Gly Glu  Ala Lys Gln Ile Gln  Val Asn Trp
    1085                1090                1095

agc gaa  ggc atg cat gtg gta  gac gcc ggc ttc aac  ggc ggc tcc    3339
Ser Glu  Gly Met His Val Val  Asp Ala Gly Phe Asn  Gly Gly Ser
    1100                1105                1110
```

```
aac acc ctc acc gac aac tgg  acc gtc agc ggc tcc  ggc aag gcc      3384
Asn Thr Leu Thr Asp Asn Trp  Thr Val Ser Gly Ser  Gly Lys Ala
    1115                1120                1125

gaa gtt gaa ggc gac aac aac  gcc atg ctg cgc ctg  acc ggc aag      3429
Glu Val Glu Gly Asp Asn Asn  Ala Met Leu Arg Leu  Thr Gly Lys
    1130                1135                1140

gtc gat gtc tcc cag cgt ctg  acc gat ctc aag gct  ggc cag aag      3474
Val Asp Val Ser Gln Arg Leu  Thr Asp Leu Lys Ala  Gly Gln Lys
    1145                1150                1155

tac gcg ctg tat gtt ggc gtc  gac aac cgc tcc acc  ggc gat gca      3519
Tyr Ala Leu Tyr Val Gly Val  Asp Asn Arg Ser Thr  Gly Asp Ala
    1160                1165                1170

tcc gtc acc gta acc agc ggc  ggc aag gtg ctg gcc  acc aac tcc      3564
Ser Val Thr Val Thr Ser Gly  Gly Lys Val Leu Ala  Thr Asn Ser
    1175                1180                1185

acc ggc aag tcc atc gcc aag  aac tac atc aag gca  tac ggc cac      3609
Thr Gly Lys Ser Ile Ala Lys  Asn Tyr Ile Lys Ala  Tyr Gly His
    1190                1195                1200

aac acg aac agc aat acg gaa  aat ggc tcc agc tac  ttc cag aac      3654
Asn Thr Asn Ser Asn Thr Glu  Asn Gly Ser Ser Tyr  Phe Gln Asn
    1205                1210                1215

atg tac gtg ttc ttc acc gcg  cct gag aac ggc gat  gcc acg gta      3699
Met Tyr Val Phe Phe Thr Ala  Pro Glu Asn Gly Asp  Ala Thr Val
    1220                1225                1230

acc ctg tct cac aag agc acc  gac gga gca cac acc  tac ttc gac      3744
Thr Leu Ser His Lys Ser Thr  Asp Gly Ala His Thr  Tyr Phe Asp
    1235                1240                1245

gat gtg cgc atc gtg gag aac  cag tac tcc ggc atc  acc tat gag      3789
Asp Val Arg Ile Val Glu Asn  Gln Tyr Ser Gly Ile  Thr Tyr Glu
    1250                1255                1260

aag gac ggc acg ctg aag tcc  ctc acc aac gga ttc  gaa aac aac      3834
Lys Asp Gly Thr Leu Lys Ser  Leu Thr Asn Gly Phe  Glu Asn Asn
    1265                1270                1275

gcc cag ggc atc tgg ccg ttc  gtg gtc tcc ggt tcc  gaa ggc gtt      3879
Ala Gln Gly Ile Trp Pro Phe  Val Val Ser Gly Ser  Glu Gly Val
    1280                1285                1290

gag gac aac cgc atc cac ctc  tcc gag ctg cat gct  ccg ttc acg      3924
Glu Asp Asn Arg Ile His Leu  Ser Glu Leu His Ala  Pro Phe Thr
    1295                1300                1305

cgg gcc ggt tgg gat gtc aag  aag atg gac gat gtg  ctc gat ggc      3969
Arg Ala Gly Trp Asp Val Lys  Lys Met Asp Asp Val  Leu Asp Gly
    1310                1315                1320

act tgg tct gtg aag gtt aac  ggc ctg acc cag aag  ggc acg ctg      4014
Thr Trp Ser Val Lys Val Asn  Gly Leu Thr Gln Lys  Gly Thr Leu
    1325                1330                1335

gtc tac cag acg atc ccg cag  aac gtg aag ttc gag  gcg ggt gcc      4059
Val Tyr Gln Thr Ile Pro Gln  Asn Val Lys Phe Glu  Ala Gly Ala
    1340                1345                1350

aag tac aag gtg agc ttc gac  tac cag tcc ggt tcc  gat gac atc      4104
Lys Tyr Lys Val Ser Phe Asp  Tyr Gln Ser Gly Ser  Asp Asp Ile
    1355                1360                1365

tac gcc atc gct gtg ggc cag  ggt gaa tac tct gcc  ggc agc gtg      4149
Tyr Ala Ile Ala Val Gly Gln  Gly Glu Tyr Ser Ala  Gly Ser Val
    1370                1375                1380

aag ctg acc aac ctg aag aag  gct ctg ggt gag acc  ggc aag gcc      4194
Lys Leu Thr Asn Leu Lys Lys  Ala Leu Gly Glu Thr  Gly Lys Ala
    1385                1390                1395
```

17

```
gag ttc  gag ctg acc ggt ggc  gtc aac ggc gat tcc  tgg ttc ggt    4239
Glu Phe  Glu Leu Thr Gly Gly  Val Asn Gly Asp Ser  Trp Phe Gly
    1400             1405              1410

att tac  tcg acc gca acc gca  cct gat ctg cag ggt  tcc acc ggc    4284
Ile Tyr  Ser Thr Ala Thr Ala  Pro Asp Leu Gln Gly  Ser Thr Gly
    1415             1420              1425

aat gca  cag gac ttc ggc gga  tac aag gac ttc gtg  ctc gac aac    4329
Asn Ala  Gln Asp Phe Gly Gly  Tyr Lys Asp Phe Val  Leu Asp Asn
    1430             1435              1440

ctg aag  atc gag cgc atc gag  tcc cag acc cgc acc  aag gcc gaa    4374
Leu Lys  Ile Glu Arg Ile Glu  Ser Gln Thr Arg Thr  Lys Ala Glu
    1445             1450              1455

gcg cag  gac aag gtc aag gaa  atc cgc ggc aag tac  gat tcc aag    4419
Ala Gln  Asp Lys Val Lys Glu  Ile Arg Gly Lys Tyr  Asp Ser Lys
    1460             1465              1470

cgt gct  gag ctc tcc gat gcc  gca tgg cag cag tat  cag gac acc    4464
Arg Ala  Glu Leu Ser Asp Ala  Ala Trp Gln Gln Tyr  Gln Asp Thr
    1475             1480              1485

ttg gtc  aag gct cgc gtg ctc  atc aac aag aat ggc  gca acc gct    4509
Leu Val  Lys Ala Arg Val Leu  Ile Asn Lys Asn Gly  Ala Thr Ala
    1490             1495              1500

gag gac  ttc acc aag gca tac  gac att ctc gtg gcc  ctc gac gag    4554
Glu Asp  Phe Thr Lys Ala Tyr  Asp Ile Leu Val Ala  Leu Asp Glu
    1505             1510              1515

tac atg  aag acc gct ccc ggc  aac gag agc agc gac  aag tac gac    4599
Tyr Met  Lys Thr Ala Pro Gly  Asn Glu Ser Ser Asp  Lys Tyr Asp
    1520             1525              1530

gtg gca  gct gac ggc tcc gat  gag ctg ggt ggc tac  acc gtg gcc    4644
Val Ala  Ala Asp Gly Ser Asp  Glu Leu Gly Gly Tyr  Thr Val Ala
    1535             1540              1545

acg ggc  agc gaa gag cct acc  gca ggc ctg ccg agc  gaa ggc ccg    4689
Thr Gly  Ser Glu Glu Pro Thr  Ala Gly Leu Pro Ser  Glu Gly Pro
    1550             1555              1560

gcc gat  ctg gca cag gat ggc  aac gac agc acc cac  tgg cac acc    4734
Ala Asp  Leu Ala Gln Asp Gly  Asn Asp Ser Thr His  Trp His Thr
    1565             1570              1575

agc tgg  agc gag aac gca gtc  ggc aac ggc acc gca  tgg tat cag    4779
Ser Trp  Ser Glu Asn Ala Val  Gly Asn Gly Thr Ala  Trp Tyr Gln
    1580             1585              1590

ttc aac  ctc aac gaa ccg acc  acc atc aac ggc ctg  cgc tac ctg    4824
Phe Asn  Leu Asn Glu Pro Thr  Thr Ile Asn Gly Leu  Arg Tyr Leu
    1595             1600              1605

ccg cgc  tcc gga ggt atg aac  gcc aac ggc aag atc  aag ggc tac    4869
Pro Arg  Ser Gly Gly Met Asn  Ala Asn Gly Lys Ile  Lys Gly Tyr
    1610             1615              1620

aag atc  acg ctc act ctg gcg  gat ggc acc acc aag  gat gtc gtc    4914
Lys Ile  Thr Leu Thr Leu Ala  Asp Gly Thr Thr Lys  Asp Val Val
    1625             1630              1635

acc gat  gct gag ttc tcc acc  acc acc atg tgg cag  aag gcc agc    4959
Thr Asp  Ala Glu Phe Ser Thr  Thr Thr Met Trp Gln  Lys Ala Ser
    1640             1645              1650

ttc gac  gcc gtc gag aat gtg  acc gcc gta cgc tcg  acc gtc ctg    5004
Phe Asp  Ala Val Glu Asn Val  Thr Ala Val Arg Ser  Thr Val Leu
    1655             1660              1665

tct tcc  gca ggc cag agc gac  tcc cag gcc aac aag  ttc gca tcc    5049
Ser Ser  Ala Gly Gln Ser Asp  Ser Gln Ala Asn Lys  Phe Ala Ser
    1670             1675              1680
```

```
gct gcc gaa ctg cgt ttg acc acg gac cgc gag gtt gag gaa gag    5094
Ala Ala Glu Leu Arg Leu Thr Thr Asp Arg Glu Val Glu Glu Glu
    1685              1690              1695

act gtc gct ccg gac aag acc gac ctc aac gac acc atc gcc aag    5139
Thr Val Ala Pro Asp Lys Thr Asp Leu Asn Asp Thr Ile Ala Lys
    1700              1705              1710

gct aac ggt ctt aag gaa tcc gac tac acg gct gaa agc tgg act    5184
Ala Asn Gly Leu Lys Glu Ser Asp Tyr Thr Ala Glu Ser Trp Thr
    1715              1720              1725

gct ctg gtc aag gcc cgc gaa gct gca cag gcc gtg gcg gat aac    5229
Ala Leu Val Lys Ala Arg Glu Ala Ala Gln Ala Val Ala Asp Asn
    1730              1735              1740

gat aag gcc acc gct tac gat gtg gct ctg gcg ctg acg aac ctc    5274
Asp Lys Ala Thr Ala Tyr Asp Val Ala Leu Ala Leu Thr Asn Leu
    1745              1750              1755

gaa tcc gct atc gct ggt ctc gag aag acc ggt gag gag cct ggc    5319
Glu Ser Ala Ile Ala Gly Leu Glu Lys Thr Gly Glu Glu Pro Gly
    1760              1765              1770

cca ggc ccg gtt gag gtg aac aag acc gac ctg cag act gca gtg    5364
Pro Gly Pro Val Glu Val Asn Lys Thr Asp Leu Gln Thr Ala Val
    1775              1780              1785

aac aag gca agc aag ctc gag aag gcc gat tac acg acc aac tcg    5409
Asn Lys Ala Ser Lys Leu Glu Lys Ala Asp Tyr Thr Thr Asn Ser
    1790              1795              1800

tgg gaa gct ttc gcc gag gca ctg aag gct gca cag cag gtg ctc    5454
Trp Glu Ala Phe Ala Glu Ala Leu Lys Ala Ala Gln Gln Val Leu
    1805              1810              1815

gac aac aag aac gcc acc cag cag gat gtg gat acc gca ctg agc    5499
Asp Asn Lys Asn Ala Thr Gln Gln Asp Val Asp Thr Ala Leu Ser
    1820              1825              1830

gct ctt cag gac gcc atc tcc aag ctg gaa gct gcc acc gag ccg    5544
Ala Leu Gln Asp Ala Ile Ser Lys Leu Glu Ala Ala Thr Glu Pro
    1835              1840              1845

aag ccg aat ccg gaa ccg ggc gtg gtg gac aag gct gct ctg aac    5589
Lys Pro Asn Pro Glu Pro Gly Val Val Asp Lys Ala Ala Leu Asn
    1850              1855              1860

gcg acc atc aac aag gcc gcc gcc atc aac ctg ggt ctc tac acc    5634
Ala Thr Ile Asn Lys Ala Ala Ala Ile Asn Leu Gly Leu Tyr Thr
    1865              1870              1875

gac gac tcc gcc aac gct ctg cgc gcc gcg ctg aag aag gcc cgt    5679
Asp Asp Ser Ala Asn Ala Leu Arg Ala Ala Leu Lys Lys Ala Arg
    1880              1885              1890

gag gtc tcc gac aac agc aac gcc acg cag aag cag gtc gac gca    5724
Glu Val Ser Asp Asn Ser Asn Ala Thr Gln Lys Gln Val Asp Ala
    1895              1900              1905

gct cgt gaa gcc ctg gag aag gca att gcc gct ctg gtg aag cgt    5769
Ala Arg Glu Ala Leu Glu Lys Ala Ile Ala Ala Leu Val Lys Arg
    1910              1915              1920

ccc gct gcc aag ggt gat ggc aac gtt gtt tcc aac acg ggc tcc    5814
Pro Ala Ala Lys Gly Asp Gly Asn Val Val Ser Asn Thr Gly Ser
    1925              1930              1935

gat gtc gcc acg atc gct ctg gct gga ctg ctg ctg gca ggt gct    5859
Asp Val Ala Thr Ile Ala Leu Ala Gly Leu Leu Leu Ala Gly Ala
    1940              1945              1950

ggc gcc gcc atc gcc tac cgt cgc aat cgc gag caa ctg tga        5901
Gly Ala Ala Ile Ala Tyr Arg Arg Asn Arg Glu Gln Leu
    1955              1960              1965
```

19

<210> 2
<211> 1966
<212> PRT
<213> Bifidobacterium lomgum JCM1217

<400> 2

Met Lys Lys Lys Lys Thr Ile Ser Ala Ala Leu Ala Thr Ala Leu Ala
1                 5                 10                15

Leu Thr Cys Met Gly Ser Gly Gly Thr Ala Phe Ala Val Pro Leu
            20              25              30

Ser Asp Ala Asp Leu Gln Thr Leu Ala Ser Gln Ile Gln Gln Ile Asn
        35              40              45

Asp Thr Ser Asp Ser Ala Thr Ala Ser Glu Thr Pro Ser Ala Gln Ala
    50              55              60

Asp Ala Val Glu Gly Trp Thr Ile Asp Ser Asn Ile Ala Gln Gly Gly
65              70              75              80

Glu Ile Leu Glu Met Ala Asn Gly Trp Leu His Leu Lys Ser Thr Ala
            85              90              95

Ser Asn Gly Asn Ala Ala Ala Asn Pro Ser Ser Ser Asn Asn Trp Pro
        100             105             110

Ala Val Ala Val Trp Gly Thr Asp Tyr Asp Phe Ser Lys Ala Gly Ser
    115             120             125

Phe His Ala Thr Ile Lys Ser Pro Gln Glu Gly Ser Ala Asn Arg Phe
    130             135             140

Gly Phe Tyr Leu Gly Tyr Asn Asp Pro Gly Ser Gly Leu Phe Ile Gly
145             150             155             160

Tyr Asp Ser Gly Gly Trp Phe Trp Gln Thr Tyr Thr Gly Gly Gly Ser
            165             170             175

Gly Ser Trp Tyr Ser Gly Ala Arg Ile Ala Ala Pro Ser Ala Asn Glu
        180             185             190

Glu His Asp Ile Arg Val Ser Trp Thr Asp Ala Lys Val Ala Thr Leu
    195             200             205

Thr Val Asp Gly Gln Lys Ala Phe Asp Val Asp Tyr Ser Ala Met Thr
    210             215             220

Asn Leu Ser Asn Lys Leu Ala Ile Lys Ala Gly Ser Trp Lys Glu Leu
225             230             235             240

Asn Glu Val Thr Asp Val Tyr Ile Lys Asp Phe Pro Glu Val Val Glu
        245             250             255

Ala Ala Lys His Ala Val Ser Gly Lys Val Val Asp Ala Gly Gly Ala
        260             265             270

21

```
Ala Ile Glu Gly Ala Thr Val Arg Leu Asp Lys Thr Lys Val Lys Thr
        275                 280             285

Gly Ala Asp Gly Thr Phe Ser Phe Ala Asp Ile Glu Glu Gly Glu His
        290                 295             300

Thr Leu Ser Ile Ala Lys Glu Gly Tyr Glu Asp Val Ser Gln Gln Val
305                 310             315                 320

Thr Val Ser Gly Ala Asp Leu Ala Ile Asp Pro Ile Thr Leu Asn Lys
                325                 330             335

Thr Val Gln Val Ala Ser Glu Thr Leu Lys Thr Lys Lys Met Glu Val
                340                 345             350

Gln Ile Lys Lys Asn Phe Pro Ser Val Leu Gln Tyr Thr Met Thr Asp
        355                 360             365

Gly Lys Val Met Tyr Gly Gln Ser Lys Asp Val Arg Thr Val Glu Ile
        370                 375             380

Asn Gly Thr Asn Ile Glu Leu Gly Asp Asp Asp Val Thr Phe Lys Lys
385                 390             395                 400

Val Ser Asp Thr Glu Ala Thr Tyr Thr Leu Lys Val Lys Asp Glu Ala
                405                 410             415

Lys Lys Ile Asp Ala Val Ile Thr Val Gln Ile Thr Val Lys Ala Asn
        420                 425             430

Gln Leu His Leu Asn Val Thr Lys Ile Lys Asn Asn Leu Ser Glu Gly
        435                 440             445

Ile Pro Glu Gly Asn Gly Val Glu Glu Asn Ala Ile Gln Thr Leu Ser
        450                 455             460

Phe Pro Asn Gln Ser Leu Val Ser Val Arg Ser Ser Gln Glu Asn Ala
465                 470             475                 480

Gln Phe Thr Gly Ala Arg Met Ser Ser Asn Thr Gln Lys Pro Gly Asp
                485                 490             495

Thr Asn Phe Ala Val Thr Glu Asp Thr Asn Val Thr Asp Ser Asp Tyr
                500                 505             510

Thr Tyr Gly Phe Ile Ser Gly Ala Gly Leu Ser Ala Gly Leu Trp Ser
        515                 520             525

Asn Ser Glu His Asp Gly Thr Tyr Val Ala Ala Pro Val Arg Gly Gly
        530                 535             540

Ser Gln Asn Thr Arg Val Tyr Ala Thr Thr Gln Gln Thr Gly Asp Ala
545                 550             555                 560

Thr Ser Leu Gly Leu Ala Ser Ala Pro Trp Tyr Tyr His Arg Thr Val
                565                 570             575
```

22

```
Thr Asp Ser Lys Gly Lys Lys Tyr Thr Val Ala Glu Thr Ala Leu Pro
        580             585             590

Gln Met Ala Val Ala Ile Ala Gly Asp Glu Asn Glu Asp Gly Ala Val
        595         600             605

Asn Trp Gln Asp Gly Ala Ile Ala Tyr Arg Asp Ile Met Asn Asn Pro
    610             615             620

Tyr Lys Ser Glu Glu Val Pro Glu Leu Val Ala Trp Arg Ile Ala Met
625             630             635             640

Asn Phe Gly Ser Gln Ala Gln Asn Pro Phe Leu Thr Thr Leu Asp Asn
            645             650             655

Val Lys Lys Val Ala Leu Asn Thr Asp Gly Leu Gly Gln Ser Val Leu
        660             665             670

Leu Lys Gly Tyr Gly Asn Glu Gly His Asp Ser Gly His Pro Asp Tyr
        675             680             685

Gly Asp Ile Gly Gln Arg Leu Gly Gly Ala Asp Asp Met Asn Thr Met
    690             695             700

Met Glu Glu Gly Ser Lys Tyr Gly Ala Arg Phe Gly Val His Val Asn
705             710             715             720

Ala Ser Glu Met Tyr Pro Glu Ala Lys Ala Phe Ser Glu Asp Met Val
            725             730             735

Arg Arg Asn Ser Ala Gly Gly Leu Ser Tyr Gly Trp Asn Trp Leu Asp
        740             745             750

Gln Gly Val Gly Ile Asp Gly Ile Tyr Asp Leu Ala Ser Gly Ser Arg
        755             760             765

Val Ser Arg Phe Ala Asp Leu Ser Lys Glu Val Gly Asp Asn Met Asp
    770             775             780

Phe Ile Tyr Leu Asp Val Trp Gly Asn Leu Thr Ser Ser Gly Ser Glu
785             790             795             800

Asp Ser Trp Glu Thr Arg Lys Met Ser Lys Met Ile Asn Asp Asn Gly
            805             810             815

Trp Arg Met Thr Thr Glu Trp Gly Ser Gly Asn Glu Tyr Asp Ser Thr
        820             825             830

Phe Gln His Trp Ala Ala Asp Leu Thr Tyr Gly Gly Tyr Thr Ser Lys
        835             840             845

Gly Glu Asn Ser Glu Val Met Arg Phe Leu Arg Asn His Gln Lys Asp
    850             855             860

Ser Trp Val Gly Asp Tyr Pro Gln Tyr Gly Gly Ala Ala Asn Ala Pro
865             870             875             880
```

23

Leu Leu Gly Gly Tyr Asn Met Lys Asp Phe Glu Gly Trp Gln Gly Arg
            885              890               895

Asn Asp Tyr Ala Ala Tyr Ile Lys Asn Leu Tyr Thr His Asp Val Ser
        900              905              910

Thr Lys Phe Ile Gln His Phe Lys Val Thr Arg Trp Val Asn Asn Pro
        915              920              925

Leu Leu Thr Ala Asp Asn Gly Asn Ala Ala Ala Val Ser Asp Pro Asn
    930              935              940

Thr Asn Asn Gly Asn Glu Gln Ile Thr Leu Lys Asp Ser Asn Gly Asn
945              950              955              960

Val Val Val Val Ser Arg Gly Ser Asn Asp Thr Ser Ser Ala Ala Tyr
            965              970              975

Arg Gln Arg Thr Ile Thr Phe Asn Gly Val Lys Val Ala Ser Gly Val
            980              985              990

Val Ser Ala Gly Asp Gly Ser Ala  Thr Gly Asp Glu Ser  Tyr Leu Leu
        995              1000              1005

Pro Trp  Met Trp Asp Ser Phe  Thr Gly Lys Leu Val  Lys Asp Ser
    1010              1015              1020

Glu Gln  Lys Leu Tyr His Trp  Asn Thr Lys Gly Gly  Thr Thr Thr
    1025              1030              1035

Trp Thr  Leu Pro Asp Ser Trp  Lys Asn Leu Ser Ser  Val Lys Val
    1040              1045              1050

Tyr Gln  Leu Thr Asp Gln Gly  Lys Thr Asn Glu Gln  Thr Val Ala
    1055              1060              1065

Val Ser  Gly Gly Lys Val Thr  Leu Thr Ala Asp Ala  Glu Thr Pro
    1070              1075              1080

Tyr Val  Val Tyr Lys Gly Glu  Ala Lys Gln Ile Gln  Val Asn Trp
    1085              1090              1095

Ser Glu  Gly Met His Val Val  Asp Ala Gly Phe Asn  Gly Gly Ser
    1100              1105              1110

Asn Thr  Leu Thr Asp Asn Trp  Thr Val Ser Gly Ser  Gly Lys Ala
    1115              1120              1125

Glu Val  Glu Gly Asp Asn Asn  Ala Met Leu Arg Leu  Thr Gly Lys
    1130              1135              1140

Val Asp  Val Ser Gln Arg Leu  Thr Asp Leu Lys Ala  Gly Gln Lys
    1145              1150              1155

Tyr Ala  Leu Tyr Val Gly Val  Asp Asn Arg Ser Thr  Gly Asp Ala
    1160              1165              1170

24

```
Ser Val  Thr Val Thr Ser Gly  Gly Lys Val Leu Ala  Thr Asn Ser
    1175                1180                1185

Thr Gly  Lys Ser Ile Ala Lys  Asn Tyr Ile Lys Ala  Tyr Gly His
    1190                1195                1200

Asn Thr  Asn Ser Asn Thr Glu  Asn Gly Ser Ser Tyr  Phe Gln Asn
    1205                1210                1215

Met Tyr  Val Phe Phe Thr Ala  Pro Glu Asn Gly Asp  Ala Thr Val
    1220                1225                1230

Thr Leu  Ser His Lys Ser Thr  Asp Gly Ala His Thr  Tyr Phe Asp
    1235                1240                1245

Asp Val  Arg Ile Val Glu Asn  Gln Tyr Ser Gly Ile  Thr Tyr Glu
    1250                1255                1260

Lys Asp  Gly Thr Leu Lys Ser  Leu Thr Asn Gly Phe  Glu Asn Asn
    1265                1270                1275

Ala Gln  Gly Ile Trp Pro Phe  Val Val Ser Gly Ser  Glu Gly Val
    1280                1285                1290

Glu Asp  Asn Arg Ile His Leu  Ser Glu Leu His Ala  Pro Phe Thr
    1295                1300                1305

Arg Ala  Gly Trp Asp Val Lys  Lys Met Asp Asp Val  Leu Asp Gly
    1310                1315                1320

Thr Trp  Ser Val Lys Val Asn  Gly Leu Thr Gln Lys  Gly Thr Leu
    1325                1330                1335

Val Tyr  Gln Thr Ile Pro Gln  Asn Val Lys Phe Glu  Ala Gly Ala
    1340                1345                1350

Lys Tyr  Lys Val Ser Phe Asp  Tyr Gln Ser Gly Ser  Asp Asp Ile
    1355                1360                1365

Tyr Ala  Ile Ala Val Gly Gln  Gly Glu Tyr Ser Ala  Gly Ser Val
    1370                1375                1380

Lys Leu  Thr Asn Leu Lys Lys  Ala Leu Gly Glu Thr  Gly Lys Ala
    1385                1390                1395

Glu Phe  Glu Leu Thr Gly Gly  Val Asn Gly Asp Ser  Trp Phe Gly
    1400                1405                1410

Ile Tyr  Ser Thr Ala Thr Ala  Pro Asp Leu Gln Gly  Ser Thr Gly
    1415                1420                1425

Asn Ala  Gln Asp Phe Gly Gly  Tyr Lys Asp Phe Val  Leu Asp Asn
    1430                1435                1440

Leu Lys  Ile Glu Arg Ile Glu  Ser Gln Thr Arg Thr  Lys Ala Glu
    1445                1450                1455
```

```
Ala Gln  Asp Lys Val Lys Glu  Ile Arg Gly Lys Tyr  Asp Ser Lys
    1460                1465                1470


Arg Ala  Glu Leu Ser Asp Ala  Ala Trp Gln Gln Tyr  Gln Asp Thr
    1475                1480                1485


Leu Val  Lys Ala Arg Val Leu  Ile Asn Lys Asn Gly  Ala Thr Ala
    1490                1495                1500


Glu Asp  Phe Thr Lys Ala Tyr  Asp Ile Leu Val Ala  Leu Asp Glu
    1505                1510                1515


Tyr Met  Lys Thr Ala Pro Gly  Asn Glu Ser Ser Asp  Lys Tyr Asp
    1520                1525                1530


Val Ala  Ala Asp Gly Ser Asp  Glu Leu Gly Gly Tyr  Thr Val Ala
    1535                1540                1545


Thr Gly  Ser Glu Glu Pro Thr  Ala Gly Leu Pro Ser  Glu Gly Pro
    1550                1555                1560


Ala Asp  Leu Ala Gln Asp Gly  Asn Asp Ser Thr His  Trp His Thr
    1565                1570                1575


Ser Trp  Ser Glu Asn Ala Val  Gly Asn Gly Thr Ala  Trp Tyr Gln
    1580                1585                1590


Phe Asn  Leu Asn Glu Pro Thr  Thr Ile Asn Gly Leu  Arg Tyr Leu
    1595                1600                1605


Pro Arg  Ser Gly Gly Met Asn  Ala Asn Gly Lys Ile  Lys Gly Tyr
    1610                1615                1620


Lys Ile  Thr Leu Thr Leu Ala  Asp Gly Thr Thr Lys  Asp Val Val
    1625                1630                1635


Thr Asp  Ala Glu Phe Ser Thr  Thr Thr Met Trp Gln  Lys Ala Ser
    1640                1645                1650


Phe Asp  Ala Val Glu Asn Val  Thr Ala Val Arg Ser  Thr Val Leu
    1655                1660                1665


Ser Ser  Ala Gly Gln Ser Asp  Ser Gln Ala Asn Lys  Phe Ala Ser
    1670                1675                1680


Ala Ala  Glu Leu Arg Leu Thr  Thr Asp Arg Glu Val  Glu Glu Glu
    1685                1690                1695


Thr Val  Ala Pro Asp Lys Thr  Asp Leu Asn Asp Thr  Ile Ala Lys
    1700                1705                1710


Ala Asn  Gly Leu Lys Glu Ser  Asp Tyr Thr Ala Glu  Ser Trp Thr
    1715                1720                1725


Ala Leu  Val Lys Ala Arg Glu  Ala Ala Gln Ala Val  Ala Asp Asn
    1730                1735                1740
```

26

EP 1 767 645 B1

```
Asp Lys  Ala Thr Ala Tyr Asp  Val Ala Leu Ala Leu  Thr Asn Leu
    1745                 1750                 1755

Glu Ser  Ala Ile Ala Gly Leu  Glu Lys Thr Gly Glu  Glu Pro Gly
    1760                 1765                 1770

Pro Gly  Pro Val Glu Val Asn  Lys Thr Asp Leu Gln  Thr Ala Val
    1775                 1780                 1785

Asn Lys  Ala Ser Lys Leu Glu  Lys Ala Asp Tyr Thr  Thr Asn Ser
    1790                 1795                 1800

Trp Glu  Ala Phe Ala Glu Ala  Leu Lys Ala Ala Gln  Gln Val Leu
    1805                 1810                 1815

Asp Asn  Lys Asn Ala Thr Gln  Gln Asp Val Asp Thr  Ala Leu Ser
    1820                 1825                 1830

Ala Leu  Gln Asp Ala Ile Ser  Lys Leu Glu Ala Ala  Thr Glu Pro
    1835                 1840                 1845

Lys Pro  Asn Pro Glu Pro Gly  Val Val Asp Lys Ala  Ala Leu Asn
    1850                 1855                 1860

Ala Thr  Ile Asn Lys Ala Ala  Ala Ile Asn Leu Gly  Leu Tyr Thr
    1865                 1870                 1875

Asp Asp  Ser Ala Asn Ala Leu  Arg Ala Ala Leu Lys  Lys Ala Arg
    1880                 1885                 1890

Glu Val  Ser Asp Asn Ser Asn  Ala Thr Gln Lys Gln  Val Asp Ala
    1895                 1900                 1905

Ala Arg  Glu Ala Leu Glu Lys  Ala Ile Ala Ala Leu  Val Lys Arg
    1910                 1915                 1920

Pro Ala  Ala Lys Gly Asp Gly  Asn Val Val Ser Asn  Thr Gly Ser
    1925                 1930                 1935

Asp Val  Ala Thr Ile Ala Leu  Ala Gly Leu Leu Leu  Ala Gly Ala
    1940                 1945                 1950

Gly Ala  Ala Ile Ala Tyr Arg  Arg Asn Arg Glu Gln  Leu
    1955                 1960                 1965
```

<210> 3

<211> 5304

<212> DNA

<213> Streptococcus pneumoniae R6

<220>

<221> CDS

<222> (1).. (5304)

<400> 3

27

```
atg aat aaa gga tta ttt gaa aaa cgt tgt aaa tat agt att cgg aaa      48
Met Asn Lys Gly Leu Phe Glu Lys Arg Cys Lys Tyr Ser Ile Arg Lys
1               5                   10                  15

ttt tca tta ggt gtt gct tct gtt atg att gga gct aca ttc ttt ggg      96
Phe Ser Leu Gly Val Ala Ser Val Met Ile Gly Ala Thr Phe Phe Gly
```

```
                    20                   25                   30
    aca agt ccg gtt ctt gca gat agc gtg cag tct ggt tcc acg gcg aac    144
    Thr Ser Pro Val Leu Ala Asp Ser Val Gln Ser Gly Ser Thr Ala Asn
            35                   40                   45

    tta cca gct gat tta gct act gct ctt gca aca gca aaa gag aat gat    192
    Leu Pro Ala Asp Leu Ala Thr Ala Leu Ala Thr Ala Lys Glu Asn Asp
        50                   55                   60

    ggg cat gat ttt gaa gcg cct aag gtg gga gaa gac caa ggt tct cca    240
    Gly His Asp Phe Glu Ala Pro Lys Val Gly Glu Asp Gln Gly Ser Pro
    65                   70                   75                   80

    gaa gtt aca gat gga cct aag aca gaa gaa gaa cta tta gca ctt gaa    288
    Glu Val Thr Asp Gly Pro Lys Thr Glu Glu Glu Leu Leu Ala Leu Glu
                    85                   90                   95

    aaa gaa aaa ccg gct gaa gaa aaa cca aaa gag gat aaa cct gca gct    336
    Lys Glu Lys Pro Ala Glu Glu Lys Pro Lys Glu Asp Lys Pro Ala Ala
                100                  105                  110

    gct aaa cct gaa aca cct aag acg gta acc cct gaa tgg caa acg gta    384
    Ala Lys Pro Glu Thr Pro Lys Thr Val Thr Pro Glu Trp Gln Thr Val
                115                  120                  125

    gag aaa aaa gaa caa cag gga aca gtc act atc cga gaa gaa aaa ggt    432
    Glu Lys Lys Glu Gln Gln Gly Thr Val Thr Ile Arg Glu Glu Lys Gly
            130                  135                  140

    gtc cgc tac aac caa tta tcc tca act gct caa aat gat aac gca ggt    480
    Val Arg Tyr Asn Gln Leu Ser Ser Thr Ala Gln Asn Asp Asn Ala Gly
    145                  150                  155                  160

    aaa cca gcc ctg ttt gaa aag aag ggc ttg acc gtt gat gcc aat gga    528
    Lys Pro Ala Leu Phe Glu Lys Lys Gly Leu Thr Val Asp Ala Asn Gly
                    165                  170                  175

    aat gca act gtt gat tta acc ttc aaa gat gat tct gaa aag ggc aaa    576
    Asn Ala Thr Val Asp Leu Thr Phe Lys Asp Asp Ser Glu Lys Gly Lys
                180                  185                  190

    tca cgc ttt ggt gtc ttc ttg aaa ttt aaa gat acc aag aat aat gtt    624
    Ser Arg Phe Gly Val Phe Leu Lys Phe Lys Asp Thr Lys Asn Asn Val
            195                  200                  205

    ttt gtc ggt tac gac aag gat ggc tgg ttc tgg gag tat aaa tct cca    672
    Phe Val Gly Tyr Asp Lys Asp Gly Trp Phe Trp Glu Tyr Lys Ser Pro
        210                  215                  220

    aca act agc act tgg tat aga ggt agt cgt gtt gct gct cct gaa aca    720
    Thr Thr Ser Thr Trp Tyr Arg Gly Ser Arg Val Ala Ala Pro Glu Thr
    225                  230                  235                  240

    gga tca aca aac cgt ctc tct atc act ctc aag tca gac ggt cag cta    768
    Gly Ser Thr Asn Arg Leu Ser Ile Thr Leu Lys Ser Asp Gly Gln Leu
                    245                  250                  255

    aat gcc agc aat aac gat gtc aat ctc ttt gac aca gtg act cta cca    816
    Asn Ala Ser Asn Asn Asp Val Asn Leu Phe Asp Thr Val Thr Leu Pro
                260                  265                  270

    gct gcg gtc aat gac cat ctt aaa aat gag aag aag att ctt ctc aag    864
    Ala Ala Val Asn Asp His Leu Lys Asn Glu Lys Lys Ile Leu Leu Lys
                275                  280                  285

    gcg ggc tct tat gac gat gag cga aca gtt gtt agc gtt aaa acg gat    912
    Ala Gly Ser Tyr Asp Asp Glu Arg Thr Val Val Ser Val Lys Thr Asp
            290                  295                  300

    aac caa gag ggg gta aaa aca gag gat acc cct gct gaa aaa gaa aca    960
    Asn Gln Glu Gly Val Lys Thr Glu Asp Thr Pro Ala Glu Lys Glu Thr
    305                  310                  315                  320

    ggt cct gaa gtt gat gat agc aag gtg act tat gac acg att cag tct    1008
    Gly Pro Glu Val Asp Asp Ser Lys Val Thr Tyr Asp Thr Ile Gln Ser
```

29

```
                    325                 330                 335
aag gtt ctc aaa gca gtg att gac caa gcc ttc cct cgt gtc aag gaa    1056
Lys Val Leu Lys Ala Val Ile Asp Gln Ala Phe Pro Arg Val Lys Glu
            340                 345                 350

tac agc ttg aat gga cat act ttg cca gga cag gtt caa cag ttc aac    1104
Tyr Ser Leu Asn Gly His Thr Leu Pro Gly Gln Val Gln Gln Phe Asn
            355                 360                 365

caa gtc ttt atc aat aac cac cga atc acc cct gaa gtc act tat aag    1152
Gln Val Phe Ile Asn Asn His Arg Ile Thr Pro Glu Val Thr Tyr Lys
        370                 375                 380

aaa atc aat gag aca aca gca gag tac ttg atg aag ctt cgc gat gat    1200
Lys Ile Asn Glu Thr Thr Ala Glu Tyr Leu Met Lys Leu Arg Asp Asp
385                 390                 395                 400

gct cac tta atc aat gcg gaa atg aca gta cgc ttg caa gtt gtg gac    1248
Ala His Leu Ile Asn Ala Glu Met Thr Val Arg Leu Gln Val Val Asp
                405                 410                 415

aat caa ttg cac ttt gat gtg acc aag att gtc aac cac aat caa gtc    1296
Asn Gln Leu His Phe Asp Val Thr Lys Ile Val Asn His Asn Gln Val
            420                 425                 430

act cca ggt caa aag att gat gac gaa aga aaa cta ctt tct tct att    1344
Thr Pro Gly Gln Lys Ile Asp Asp Glu Arg Lys Leu Leu Ser Ser Ile
            435                 440                 445

agt ttc ctc ggc aat gct tta gtc tct gtt tct agt gat caa act ggt    1392
Ser Phe Leu Gly Asn Ala Leu Val Ser Val Ser Ser Asp Gln Thr Gly
        450                 455                 460

gct aag ttt gat ggg gca acc atg tca aac aat acg cat gtc agc gga    1440
Ala Lys Phe Asp Gly Ala Thr Met Ser Asn Asn Thr His Val Ser Gly
465                 470                 475                 480

gat gat cat atc gat gta acc aat cca atg aaa gat cta gcc aag ggt    1488
Asp Asp His Ile Asp Val Thr Asn Pro Met Lys Asp Leu Ala Lys Gly
                485                 490                 495

tac atg tat gga ttt gtt tct aca gat aag ctt gct gct ggt gtt tgg    1536
Tyr Met Tyr Gly Phe Val Ser Thr Asp Lys Leu Ala Ala Gly Val Trp
            500                 505                 510

agt aac tct caa aac agc tat ggt ggt ggt tcg aat gac tgg act cgt    1584
Ser Asn Ser Gln Asn Ser Tyr Gly Gly Gly Ser Asn Asp Trp Thr Arg
            515                 520                 525

ttg aca gcc tat aaa gaa aca gtc gga aat gcc aac tat gta gga atc    1632
Leu Thr Ala Tyr Lys Glu Thr Val Gly Asn Ala Asn Tyr Val Gly Ile
        530                 535                 540

cac agc tct gaa tgg caa tgg gaa aaa gct tat aag ggc att gtt ttc    1680
His Ser Ser Glu Trp Gln Trp Glu Lys Ala Tyr Lys Gly Ile Val Phe
545                 550                 555                 560

cca gaa tac acg aag gaa ctt cca agt gct aag gtt gtt atc act gaa    1728
Pro Glu Tyr Thr Lys Glu Leu Pro Ser Ala Lys Val Val Ile Thr Glu
                565                 570                 575

gat gcc aat gca gac aag aaa gtc gat tgg cag gat ggt gcc att gct    1776
Asp Ala Asn Ala Asp Lys Lys Val Asp Trp Gln Asp Gly Ala Ile Ala
                580                 585                 590

tat cgt agc att atg aac aat cct caa ggt tgg aaa aaa gtt aag gat    1824
Tyr Arg Ser Ile Met Asn Asn Pro Gln Gly Trp Lys Lys Val Lys Asp
            595                 600                 605

atc aca gct tac cgt atc gcg atg aac ttt ggt tct caa gca caa aac    1872
Ile Thr Ala Tyr Arg Ile Ala Met Asn Phe Gly Ser Gln Ala Gln Asn
        610                 615                 620

cca ttc ctt atg acc ttg gat ggt atc aag aaa atc aat ctc cac aca    1920
Pro Phe Leu Met Thr Leu Asp Gly Ile Lys Lys Ile Asn Leu His Thr
```

|  | 625 |  |  | 630 |  |  | 635 |  |  | 640 |  |  |
|---|---|---|---|---|---|---|---|---|---|---|---|---|

gat ggt ctt ggg caa ggt gtt ctc ctt aaa gga tat ggt agc gaa ggc    1968
Asp Gly Leu Gly Gln Gly Val Leu Leu Lys Gly Tyr Gly Ser Glu Gly
                    645              650              655

cat gac tct ggt cac ttg aac tat gct gat att ggt aag cgt atc ggt    2016
His Asp Ser Gly His Leu Asn Tyr Ala Asp Ile Gly Lys Arg Ile Gly
                660              665              670

ggt gtc gaa gac ttc aag acc cta att gag aag gct aag aaa tat gga    2064
Gly Val Glu Asp Phe Lys Thr Leu Ile Glu Lys Ala Lys Lys Tyr Gly
            675              680              685

gct cat cta ggt atc cac gtt aac gct tca gaa act tat cct gag tct    2112
Ala His Leu Gly Ile His Val Asn Ala Ser Glu Thr Tyr Pro Glu Ser
        690              695              700

aaa tac ttc aat gaa aaa att ctc cgt aag aat cca gat gga agc tat    2160
Lys Tyr Phe Asn Glu Lys Ile Leu Arg Lys Asn Pro Asp Gly Ser Tyr
705              710              715              720

agc tat ggt tgg aac tgg cta gat caa ggt atc aac att gat gct gcc    2208
Ser Tyr Gly Trp Asn Trp Leu Asp Gln Gly Ile Asn Ile Asp Ala Ala
                725              730              735

tat gac cta gct cat ggt cgt ttg gca cgt tgg gaa gat ttg aag aaa    2256
Tyr Asp Leu Ala His Gly Arg Leu Ala Arg Trp Glu Asp Leu Lys Lys
                740              745              750

aaa ctt ggt gac ggt ctc gac ttt atc tat gtg gac gtt tgg ggt aat    2304
Lys Leu Gly Asp Gly Leu Asp Phe Ile Tyr Val Asp Val Trp Gly Asn
            755              760              765

ggt caa tca ggt gat aac ggt gcc tgg gct acc cac gtt ctt gct aaa    2352
Gly Gln Ser Gly Asp Asn Gly Ala Trp Ala Thr His Val Leu Ala Lys
        770              775              780

gaa att aac aaa caa ggc tgg cgc ttt gcg atc gag tgg ggc cat ggt    2400
Glu Ile Asn Lys Gln Gly Trp Arg Phe Ala Ile Glu Trp Gly His Gly
785              790              795              800

ggt gag tac gac tct acc ttc cat cac tgg gca gct gac ttg acc tac    2448
Gly Glu Tyr Asp Ser Thr Phe His His Trp Ala Ala Asp Leu Thr Tyr
                805              810              815

ggt ggc tac acc aat aaa ggt atc aac agt gcc atc acc cgc ttt ata    2496
Gly Gly Tyr Thr Asn Lys Gly Ile Asn Ser Ala Ile Thr Arg Phe Ile
                820              825              830

cgt aac cac caa aaa gat gct tgg gta ggg gac tac aga agt tat ggt    2544
Arg Asn His Gln Lys Asp Ala Trp Val Gly Asp Tyr Arg Ser Tyr Gly
            835              840              845

ggt gca gcc aac tat cca ctg cta ggt ggc tac agc atg aaa gac ttt    2592
Gly Ala Ala Asn Tyr Pro Leu Leu Gly Gly Tyr Ser Met Lys Asp Phe
        850              855              860

gaa ggc tgg caa gga aga agt gac tac aat ggc tat gta act aac tta    2640
Glu Gly Trp Gln Gly Arg Ser Asp Tyr Asn Gly Tyr Val Thr Asn Leu
865              870              875              880

ttt gcc cat gac gtc atg acc aag tac ttc caa cac ttc act gta agt    2688
Phe Ala His Asp Val Met Thr Lys Tyr Phe Gln His Phe Thr Val Ser
                885              890              895

aaa tgg gaa aat ggt aca ccg gtg act atg acc gat aac ggt agc acc    2736
Lys Trp Glu Asn Gly Thr Pro Val Thr Met Thr Asp Asn Gly Ser Thr
                900              905              910

tat aaa tgg act cca gaa atg cga gtg gaa ttg gta gat gct gac aat    2784
Tyr Lys Trp Thr Pro Glu Met Arg Val Glu Leu Val Asp Ala Asp Asn
            915              920              925

aat aaa gta gtt gta act cgt aag tca aat gat gtc aat agt cca caa    2832
Asn Lys Val Val Val Thr Arg Lys Ser Asn Asp Val Asn Ser Pro Gln

```
                930                   935                   940

        tat cgc gaa cgt aca gta act ctc aac gga cgt gtc atc caa gat ggt      2880
        Tyr Arg Glu Arg Thr Val Thr Leu Asn Gly Arg Val Ile Gln Asp Gly
        945                   950                   955                   960

        tca gct tac ttg act cct tgg aac tgg gat gca aat ggt aag aaa ctt      2928
        Ser Ala Tyr Leu Thr Pro Trp Asn Trp Asp Ala Asn Gly Lys Lys Leu
                        965                   970                   975

        tct act gat aag gaa aag atg tac tac ttc aat acg cag gcc ggt gca      2976
        Ser Thr Asp Lys Glu Lys Met Tyr Tyr Phe Asn Thr Gln Ala Gly Ala
                        980                   985                   990

        aca act tgg acc ctt cca agc gat  tgg gca aag agc aag  gtt tac ctt    3024
        Thr Thr Trp Thr Leu Pro Ser Asp  Trp Ala Lys Ser Lys  Val Tyr Leu
                        995                   1000                  1005

        tac aag cta act gac caa ggt  aag aca gaa gag caa  gaa cta act         3069
        Tyr Lys Leu Thr Asp Gln Gly  Lys Thr Glu Glu Gln  Glu Leu Thr
                1010                  1015                  1020

        gta aaa gat ggt aaa att acc  cta gat ctt cta gca  aat caa cca        3114
        Val Lys Asp Gly Lys Ile Thr  Leu Asp Leu Leu Ala  Asn Gln Pro
                1025                  1030                  1035

        tac gtt ctc tat cgt tcg aaa  caa acc aat cct gaa  atg tca tgg        3159
        Tyr Val Leu Tyr Arg Ser Lys  Gln Thr Asn Pro Glu  Met Ser Trp
                1040                  1045                  1050

        agt gaa ggc atg cac atc tat  gac caa gga ttt aac  agt ggt acc        3204
        Ser Glu Gly Met His Ile Tyr  Asp Gln Gly Phe Asn  Ser Gly Thr
                1055                  1060                  1065

        ttg aaa cat tgg acc att tca  ggc gat gct tct aag  gca gaa att        3249
        Leu Lys His Trp Thr Ile Ser  Gly Asp Ala Ser Lys  Ala Glu Ile
                1070                  1075                  1080

        gtc aag tct caa ggg gca aac  gat atg ctt cgt att  caa gga aac        3294
        Val Lys Ser Gln Gly Ala Asn  Asp Met Leu Arg Ile  Gln Gly Asn
                1085                  1090                  1095

        aaa gaa aaa gtt agt ctc act  cag aaa tta act ggc  ttg aaa cca        3339
        Lys Glu Lys Val Ser Leu Thr  Gln Lys Leu Thr Gly  Leu Lys Pro
                1100                  1105                  1110

        aat acc aag tat gcc gtt tat  gtc ggt gtc gat aac  cgt agt aat        3384
        Asn Thr Lys Tyr Ala Val Tyr  Val Gly Val Asp Asn  Arg Ser Asn
                1115                  1120                  1125

        gcc aag gcg agc atc act gta  aat act ggt gaa aaa  gaa gtg act        3429
        Ala Lys Ala Ser Ile Thr Val  Asn Thr Gly Glu Lys  Glu Val Thr
                1130                  1135                  1140

        act tat acc aat aag tct ctc  gcc ctc aac tat gta  aaa gcc tat        3474
        Thr Tyr Thr Asn Lys Ser Leu  Ala Leu Asn Tyr Val  Lys Ala Tyr
                1145                  1150                  1155

        gcc cac aat aca cgt cgt aac  aat gct aca gtt gac  gat aca agt        3519
        Ala His Asn Thr Arg Arg Asn  Asn Ala Thr Val Asp  Asp Thr Ser
                1160                  1165                  1170

        tac ttc caa aac atg tac gcc  ttc ttt aca act gga  tcg gac gta        3564
        Tyr Phe Gln Asn Met Tyr Ala  Phe Phe Thr Thr Gly  Ser Asp Val
                1175                  1180                  1185

        tca aat gtt act ctg aca ttg  agt cgt gaa gct ggt  gat gaa gca        3609
        Ser Asn Val Thr Leu Thr Leu  Ser Arg Glu Ala Gly  Asp Glu Ala
                1190                  1195                  1200

        act tac ttt gat gaa att cgt  acc ttt gaa aac aat  tca agc atg        3654
        Thr Tyr Phe Asp Glu Ile Arg  Thr Phe Glu Asn Asn  Ser Ser Met
                1205                  1210                  1215

        tac gga gac aag cat gat aca  ggt aaa ggc acc ttc  aag caa gac        3699
        Tyr Gly Asp Lys His Asp Thr  Gly Lys Gly Thr Phe  Lys Gln Asp
```

32

```
                1220                    1225                    1230
        ttt gaa  aat gtt gct cag ggt  atc ttc cca ttt gta  gtg ggt ggt    3744
        Phe Glu  Asn Val Ala Gln Gly  Ile Phe Pro Phe Val  Val Gly Gly
             1235                    1240                   1245

        gtc gaa  ggt gtc gaa gac aac  cgc act cac ttg tct  gaa aaa cac    3789
        Val Glu  Gly Val Glu Asp Asn  Arg Thr His Leu Ser  Glu Lys His
             1250                    1255                   1260

        gat cca  tat aca caa cgt ggt  tgg aat ggt aag aaa  gtc gat gat    3834
        Asp Pro  Tyr Thr Gln Arg Gly  Trp Asn Gly Lys Lys  Val Asp Asp
             1265                    1270                   1275

        gtt atc  gaa gga aat tgg tca  ctc aag aca aat gga  cta gtg agc    3879
        Val Ile  Glu Gly Asn Trp Ser  Leu Lys Thr Asn Gly  Leu Val Ser
             1280                    1285                   1290

        cgt cgt  aac ttg gtt tac caa  act att ccg caa aac  ttc cgt ttt    3924
        Arg Arg  Asn Leu Val Tyr Gln  Thr Ile Pro Gln Asn  Phe Arg Phe
             1295                    1300                   1305

        gaa gca  ggt aag acc tac cgt  gta acc ttt gaa tac  gaa gca ggt    3969
        Glu Ala  Gly Lys Thr Tyr Arg  Val Thr Phe Glu Tyr  Glu Ala Gly
             1310                    1315                   1320

        tca gac  aat acc tat gct ttt  gta gtc ggt aag gga  gaa ttc cag    4014
        Ser Asp  Asn Thr Tyr Ala Phe  Val Val Gly Lys Gly  Glu Phe Gln
             1325                    1330                   1335

        tca ggt  cgt cgt ggt act caa  gca agc aac ttg gaa  atg cat gaa    4059
        Ser Gly  Arg Arg Gly Thr Gln  Ala Ser Asn Leu Glu  Met His Glu
             1340                    1345                   1350

        ttg cca  aat act tgg aca gat  tct aag aaa gcc aag  aag gca acc    4104
        Leu Pro  Asn Thr Trp Thr Asp  Ser Lys Lys Ala Lys  Lys Ala Thr
             1355                    1360                   1365

        ttc ctc  gtg aca ggt gca gaa  aca ggg gat act tgg  gta ggt atc    4149
        Phe Leu  Val Thr Gly Ala Glu  Thr Gly Asp Thr Trp  Val Gly Ile
             1370                    1375                   1380

        tac tca  act gga aat gca agt  aat act cgt ggt gat  tct ggt gga    4194
        Tyr Ser  Thr Gly Asn Ala Ser  Asn Thr Arg Gly Asp  Ser Gly Gly
             1385                    1390                   1395

        aat gcc  aac ttc cgt ggt tat  aac gac ttc atg atg  gat aat ctt    4239
        Asn Ala  Asn Phe Arg Gly Tyr  Asn Asp Phe Met Met  Asp Asn Leu
             1400                    1405                   1410

        caa atc  gaa gaa att acc cta  aca ggt aag atg ttg  aca gaa aat    4284
        Gln Ile  Glu Glu Ile Thr Leu  Thr Gly Lys Met Leu  Thr Glu Asn
             1415                    1420                   1425

        gct ctg  aag aac tac ttg cca  acg gtt gcc atg act  aac tac acc    4329
        Ala Leu  Lys Asn Tyr Leu Pro  Thr Val Ala Met Thr  Asn Tyr Thr
             1430                    1435                   1440

        aaa gag  tct atg gat gct ttg  aaa gag gcg gtc ttt  aac ctc agt    4374
        Lys Glu  Ser Met Asp Ala Leu  Lys Glu Ala Val Phe  Asn Leu Ser
             1445                    1450                   1455

        cag gcc  gat gat gat atc agt  gtg gaa gaa gcg cgt  gca gag att    4419
        Gln Ala  Asp Asp Asp Ile Ser  Val Glu Glu Ala Arg  Ala Glu Ile
             1460                    1465                   1470

        gcc aag  att gaa gcc ttg aag  aat gct ttg gtt cag  aag aaa acg    4464
        Ala Lys  Ile Glu Ala Leu Lys  Asn Ala Leu Val Gln  Lys Lys Thr
             1475                    1480                   1485

        gct ttg  gta gca gat gac ttt  gca agt ctt aca gct  cct gct cag    4509
        Ala Leu  Val Ala Asp Asp Phe  Ala Ser Leu Thr Ala  Pro Ala Gln
             1490                    1495                   1500

        gct caa  gaa ggt ctt gca aat  gcc ttt gat gga aac  tta tct agt    4554
        Ala Gln  Glu Gly Leu Ala Asn  Ala Phe Asp Gly Asn  Leu Ser Ser
```

```
                  1505                    1510                      1515

        tta  tgg  cat  aca  tca  tgg  ggc  gga  gga  gat  gta  ggc  aag  cct  gca      4599
        Leu  Trp  His  Thr  Ser  Trp  Gly  Gly  Gly  Asp  Val  Gly  Lys  Pro  Ala
             1520                    1525                      1530

        acc  atg  gtc  ttg  aaa  gaa  gca  act  gaa  atc  act  gga  ctt  cgt  tat      4644
        Thr  Met  Val  Leu  Lys  Glu  Ala  Thr  Glu  Ile  Thr  Gly  Leu  Arg  Tyr
             1535                    1540                      1545

        gtt  cca  cgt  gga  tca  ggt  tca  aat  ggt  aac  ttg  cgt  gat  gtg  aaa      4689
        Val  Pro  Arg  Gly  Ser  Gly  Ser  Asn  Gly  Asn  Leu  Arg  Asp  Val  Lys
             1550                    1555                      1560

        ctt  gtt  gtg  aca  gat  gag  tct  ggc  aag  gag  cat  acc  ttt  act  gca      4734
        Leu  Val  Val  Thr  Asp  Glu  Ser  Gly  Lys  Glu  His  Thr  Phe  Thr  Ala
             1565                    1570                      1575

        act  gat  tgg  cca  gat  aac  aat  aag  cca  aaa  gac  att  gat  ttt  ggt      4779
        Thr  Asp  Trp  Pro  Asp  Asn  Asn  Lys  Pro  Lys  Asp  Ile  Asp  Phe  Gly
             1580                    1585                      1590

        aag  aca  att  aag  gct  aag  aaa  att  gtc  ctt  aca  ggt  act  aag  act      4824
        Lys  Thr  Ile  Lys  Ala  Lys  Lys  Ile  Val  Leu  Thr  Gly  Thr  Lys  Thr
             1595                    1600                      1605

        tac  gga  gat  ggt  ggc  gat  aaa  tac  caa  tct  gca  gcg  gaa  ctc  atc      4869
        Tyr  Gly  Asp  Gly  Gly  Asp  Lys  Tyr  Gln  Ser  Ala  Ala  Glu  Leu  Ile
             1610                    1615                      1620

        ttt  act  cgt  cca  cag  gta  gca  gaa  aca  cct  ctt  gac  ttg  tca  ggc      4914
        Phe  Thr  Arg  Pro  Gln  Val  Ala  Glu  Thr  Pro  Leu  Asp  Leu  Ser  Gly
             1625                    1630                      1635

        tat  gaa  gca  gct  ttg  gct  aag  gct  cag  aaa  tta  aca  gac  aaa  gac      4959
        Tyr  Glu  Ala  Ala  Leu  Ala  Lys  Ala  Gln  Lys  Leu  Thr  Asp  Lys  Asp
             1640                    1645                      1650

        aat  caa  gag  gaa  gta  gct  agc  gtt  cag  gca  agc  atg  aaa  tat  gcg      5004
        Asn  Gln  Glu  Glu  Val  Ala  Ser  Val  Gln  Ala  Ser  Met  Lys  Tyr  Ala
             1655                    1660                      1665

        acg  gat  aac  cat  ctc  ttg  acg  gaa  aga  atg  gtg  gaa  tac  ttt  gca      5049
        Thr  Asp  Asn  His  Leu  Leu  Thr  Glu  Arg  Met  Val  Glu  Tyr  Phe  Ala
             1670                    1675                      1680

        gat  tat  ctc  aac  caa  tta  aaa  gat  tct  gct  acg  aaa  cca  gat  gct      5094
        Asp  Tyr  Leu  Asn  Gln  Leu  Lys  Asp  Ser  Ala  Thr  Lys  Pro  Asp  Ala
             1685                    1690                      1695

        cca  act  gta  gag  aaa  cct  gag  ttt  aaa  ctt  agc  tct  gta  gct  tcc      5139
        Pro  Thr  Val  Glu  Lys  Pro  Glu  Phe  Lys  Leu  Ser  Ser  Val  Ala  Ser
             1700                    1705                      1710

        gat  caa  ggt  aag  acg  cca  gat  tat  aag  caa  gaa  ata  gct  aga  cca      5184
        Asp  Gln  Gly  Lys  Thr  Pro  Asp  Tyr  Lys  Gln  Glu  Ile  Ala  Arg  Pro
             1715                    1720                      1725

        gaa  aca  cct  gaa  caa  atc  ttg  cca  gca  aca  ggt  gag  agt  caa  ttt      5229
        Glu  Thr  Pro  Glu  Gln  Ile  Leu  Pro  Ala  Thr  Gly  Glu  Ser  Gln  Phe
             1730                    1735                      1740

        gac  aca  gcc  ctc  ttc  cta  gca  agt  gtt  agc  cta  gcc  cta  tct  gct      5274
        Asp  Thr  Ala  Leu  Phe  Leu  Ala  Ser  Val  Ser  Leu  Ala  Leu  Ser  Ala
             1745                    1750                      1755

        ctc  ttt  gta  gta  aaa  acg  aag  aaa  gac  tag                              5304
        Leu  Phe  Val  Val  Lys  Thr  Lys  Lys  Asp
             1760                    1765
```

<210> 4  
<211> 1767

34

<212> PRT
<213> Streptococcus pneumoniae R6

<400> 4

Met Asn Lys Gly Leu Phe Glu Lys Arg Cys Lys Tyr Ser Ile Arg Lys
1                 5                   10                  15

Phe Ser Leu Gly Val Ala Ser Val Met Ile Gly Ala Thr Phe Phe Gly
            20              25              30

Thr Ser Pro Val Leu Ala Asp Ser Val Gln Ser Gly Ser Thr Ala Asn
        35              40              45

Leu Pro Ala Asp Leu Ala Thr Ala Leu Ala Thr Ala Lys Glu Asn Asp
    50              55              60

Gly His Asp Phe Glu Ala Pro Lys Val Gly Glu Asp Gln Gly Ser Pro
65              70              75                  80

Glu Val Thr Asp Gly Pro Lys Thr Glu Glu Glu Leu Leu Ala Leu Glu
            85              90                  95

Lys Glu Lys Pro Ala Glu Glu Lys Pro Lys Glu Asp Lys Pro Ala Ala
            100             105             110

Ala Lys Pro Glu Thr Pro Lys Thr Val Thr Pro Glu Trp Gln Thr Val
    115             120             125

Glu Lys Lys Glu Gln Gln Gly Thr Val Thr Ile Arg Glu Glu Lys Gly
    130             135             140

Val Arg Tyr Asn Gln Leu Ser Ser Thr Ala Gln Asn Asp Asn Ala Gly
145             150             155             160

Lys Pro Ala Leu Phe Glu Lys Lys Gly Leu Thr Val Asp Ala Asn Gly
            165             170             175

Asn Ala Thr Val Asp Leu Thr Phe Lys Asp Asp Ser Glu Lys Gly Lys
        180             185             190

Ser Arg Phe Gly Val Phe Leu Lys Phe Lys Asp Thr Lys Asn Asn Val
    195             200             205

Phe Val Gly Tyr Asp Lys Asp Gly Trp Phe Trp Glu Tyr Lys Ser Pro
    210             215             220

Thr Thr Ser Thr Trp Tyr Arg Gly Ser Arg Val Ala Ala Pro Glu Thr
225             230             235             240

Gly Ser Thr Asn Arg Leu Ser Ile Thr Leu Lys Ser Asp Gly Gln Leu
            245             250             255

Asn Ala Ser Asn Asn Asp Val Asn Leu Phe Asp Thr Val Thr Leu Pro
            260             265             270

Ala Ala Val Asn Asp His Leu Lys Asn Glu Lys Lys Ile Leu Leu Lys
    275             280             285

Ala Gly Ser Tyr Asp Asp Glu Arg Thr Val Val Ser Val Lys Thr Asp
    290             295             300

```
Asn Gln Glu Gly Val Lys Thr Glu Asp Thr Pro Ala Glu Lys Glu Thr
305             310         315             320

Gly Pro Glu Val Asp Asp Ser Lys Val Thr Tyr Asp Thr Ile Gln Ser
            325             330             335

Lys Val Leu Lys Ala Val Ile Asp Gln Ala Phe Pro Arg Val Lys Glu
            340             345             350

Tyr Ser Leu Asn Gly His Thr Leu Pro Gly Gln Val Gln Gln Phe Asn
            355             360             365

Gln Val Phe Ile Asn Asn His Arg Ile Thr Pro Glu Val Thr Tyr Lys
        370             375             380

Lys Ile Asn Glu Thr Thr Ala Glu Tyr Leu Met Lys Leu Arg Asp Asp
385             390             395             400

Ala His Leu Ile Asn Ala Glu Met Thr Val Arg Leu Gln Val Val Asp
            405             410             415

Asn Gln Leu His Phe Asp Val Thr Lys Ile Val Asn His Asn Gln Val
            420             425             430

Thr Pro Gly Gln Lys Ile Asp Asp Glu Arg Lys Leu Leu Ser Ser Ile
            435             440             445

Ser Phe Leu Gly Asn Ala Leu Val Ser Val Ser Ser Asp Gln Thr Gly
        450             455             460

Ala Lys Phe Asp Gly Ala Thr Met Ser Asn Asn Thr His Val Ser Gly
465             470             475             480

Asp Asp His Ile Asp Val Thr Asn Pro Met Lys Asp Leu Ala Lys Gly
            485             490             495

Tyr Met Tyr Gly Phe Val Ser Thr Asp Lys Leu Ala Ala Gly Val Trp
            500             505             510

Ser Asn Ser Gln Asn Ser Tyr Gly Gly Gly Ser Asn Asp Trp Thr Arg
            515             520             525

Leu Thr Ala Tyr Lys Glu Thr Val Gly Asn Ala Asn Tyr Val Gly Ile
            530             535             540

His Ser Ser Glu Trp Gln Trp Glu Lys Ala Tyr Lys Gly Ile Val Phe
545             550             555             560

Pro Glu Tyr Thr Lys Glu Leu Pro Ser Ala Lys Val Val Ile Thr Glu
            565             570             575

Asp Ala Asn Ala Asp Lys Lys Val Asp Trp Gln Asp Gly Ala Ile Ala
            580             585             590

Tyr Arg Ser Ile Met Asn Asn Pro Gln Gly Trp Lys Lys Val Lys Asp
            595             600             605
```

37

```
Ile Thr Ala Tyr Arg Ile Ala Met Asn Phe Gly Ser Gln Ala Gln Asn
    610               615               620

Pro Phe Leu Met Thr Leu Asp Gly Ile Lys Lys Ile Asn Leu His Thr
625               630               635               640

Asp Gly Leu Gly Gln Gly Val Leu Leu Lys Gly Tyr Gly Ser Glu Gly
                645               650               655

His Asp Ser Gly His Leu Asn Tyr Ala Asp Ile Gly Lys Arg Ile Gly
            660               665               670

Gly Val Glu Asp Phe Lys Thr Leu Ile Glu Lys Ala Lys Lys Tyr Gly
        675               680               685

Ala His Leu Gly Ile His Val Asn Ala Ser Glu Thr Tyr Pro Glu Ser
    690               695               700

Lys Tyr Phe Asn Glu Lys Ile Leu Arg Lys Asn Pro Asp Gly Ser Tyr
705               710               715               720

Ser Tyr Gly Trp Asn Trp Leu Asp Gln Gly Ile Asn Ile Asp Ala Ala
            725               730               735

Tyr Asp Leu Ala His Gly Arg Leu Ala Arg Trp Glu Asp Leu Lys Lys
        740               745               750

Lys Leu Gly Asp Gly Leu Asp Phe Ile Tyr Val Asp Val Trp Gly Asn
        755               760               765

Gly Gln Ser Gly Asp Asn Gly Ala Trp Ala Thr His Val Leu Ala Lys
        770               775               780

Glu Ile Asn Lys Gln Gly Trp Arg Phe Ala Ile Glu Trp Gly His Gly
785               790               795               800

Gly Glu Tyr Asp Ser Thr Phe His His Trp Ala Ala Asp Leu Thr Tyr
            805               810               815

Gly Gly Tyr Thr Asn Lys Gly Ile Asn Ser Ala Ile Thr Arg Phe Ile
        820               825               830

Arg Asn His Gln Lys Asp Ala Trp Val Gly Asp Tyr Arg Ser Tyr Gly
        835               840               845

Gly Ala Ala Asn Tyr Pro Leu Leu Gly Gly Tyr Ser Met Lys Asp Phe
        850               855               860

Glu Gly Trp Gln Gly Arg Ser Asp Tyr Asn Gly Tyr Val Thr Asn Leu
865               870               875               880

Phe Ala His Asp Val Met Thr Lys Tyr Phe Gln His Phe Thr Val Ser
            885               890               895

Lys Trp Glu Asn Gly Thr Pro Val Thr Met Thr Asp Asn Gly Ser Thr
            900               905               910
```

```
Tyr Lys Trp Thr Pro Glu Met Arg Val Glu Leu Val Asp Ala Asp Asn
        915             920             925

Asn Lys Val Val Val Thr Arg Lys Ser Asn Asp Val Asn Ser Pro Gln
        930             935             940

Tyr Arg Glu Arg Thr Val Thr Leu Asn Gly Arg Val Ile Gln Asp Gly
945             950             955             960

Ser Ala Tyr Leu Thr Pro Trp Asn Trp Asp Ala Asn Gly Lys Lys Leu
        965             970             975

Ser Thr Asp Lys Glu Lys Met Tyr Tyr Phe Asn Thr Gln Ala Gly Ala
        980             985             990

Thr Thr Trp Thr Leu Pro Ser Asp Trp Ala Lys Ser Lys Val Tyr Leu
        995             1000            1005

Tyr Lys Leu Thr Asp Gln Gly Lys Thr Glu Glu Gln Glu Leu Thr
    1010            1015            1020

Val Lys Asp Gly Lys Ile Thr Leu Asp Leu Leu Ala Asn Gln Pro
    1025            1030            1035

Tyr Val Leu Tyr Arg Ser Lys Gln Thr Asn Pro Glu Met Ser Trp
    1040            1045            1050

Ser Glu Gly Met His Ile Tyr Asp Gln Gly Phe Asn Ser Gly Thr
    1055            1060            1065

Leu Lys His Trp Thr Ile Ser Gly Asp Ala Ser Lys Ala Glu Ile
    1070            1075            1080

Val Lys Ser Gln Gly Ala Asn Asp Met Leu Arg Ile Gln Gly Asn
    1085            1090            1095

Lys Glu Lys Val Ser Leu Thr Gln Lys Leu Thr Gly Leu Lys Pro
    1100            1105            1110

Asn Thr Lys Tyr Ala Val Tyr Val Gly Val Asp Asn Arg Ser Asn
    1115            1120            1125

Ala Lys Ala Ser Ile Thr Val Asn Thr Gly Glu Lys Glu Val Thr
    1130            1135            1140

Thr Tyr Thr Asn Lys Ser Leu Ala Leu Asn Tyr Val Lys Ala Tyr
    1145            1150            1155

Ala His Asn Thr Arg Arg Asn Asn Ala Thr Val Asp Asp Thr Ser
    1160            1165            1170

Tyr Phe Gln Asn Met Tyr Ala Phe Phe Thr Thr Gly Ser Asp Val
    1175            1180            1185

Ser Asn Val Thr Leu Thr Leu Ser Arg Glu Ala Gly Asp Glu Ala
    1190            1195            1200
```

```
Thr Tyr  Phe Asp Glu Ile Arg  Thr Phe Glu Asn Asn  Ser Ser Met
    1205             1210             1215

Tyr Gly  Asp Lys His Asp Thr  Gly Lys Gly Thr Phe  Lys Gln Asp
    1220             1225             1230

Phe Glu  Asn Val Ala Gln Gly  Ile Phe Pro Phe Val  Val Gly Gly
    1235             1240             1245

Val Glu  Gly Val Glu Asp Asn  Arg Thr His Leu Ser  Glu Lys His
    1250             1255             1260

Asp Pro  Tyr Thr Gln Arg Gly  Trp Asn Gly Lys Lys  Val Asp Asp
    1265             1270             1275

Val Ile  Glu Gly Asn Trp Ser  Leu Lys Thr Asn Gly  Leu Val Ser
    1280             1285             1290

Arg Arg  Asn Leu Val Tyr Gln  Thr Ile Pro Gln Asn  Phe Arg Phe
    1295             1300             1305

Glu Ala  Gly Lys Thr Tyr Arg  Val Thr Phe Glu Tyr  Glu Ala Gly
    1310             1315             1320

Ser Asp  Asn Thr Tyr Ala Phe  Val Val Gly Lys Gly  Glu Phe Gln
    1325             1330             1335

Ser Gly  Arg Arg Gly Thr Gln  Ala Ser Asn Leu Glu  Met His Glu
    1340             1345             1350

Leu Pro  Asn Thr Trp Thr Asp  Ser Lys Lys Ala Lys  Lys Ala Thr
    1355             1360             1365

Phe Leu  Val Thr Gly Ala Glu  Thr Gly Asp Thr Trp  Val Gly Ile
    1370             1375             1380

Tyr Ser  Thr Gly Asn Ala Ser  Asn Thr Arg Gly Asp  Ser Gly Gly
    1385             1390             1395

Asn Ala  Asn Phe Arg Gly Tyr  Asn Asp Phe Met Met  Asp Asn Leu
    1400             1405             1410

Gln Ile  Glu Glu Ile Thr Leu  Thr Gly Lys Met Leu  Thr Glu Asn
    1415             1420             1425

Ala Leu  Lys Asn Tyr Leu Pro  Thr Val Ala Met Thr  Asn Tyr Thr
    1430             1435             1440

Lys Glu  Ser Met Asp Ala Leu  Lys Glu Ala Val Phe  Asn Leu Ser
    1445             1450             1455

Gln Ala  Asp Asp Asp Ile Ser  Val Glu Glu Ala Arg  Ala Glu Ile
    1460             1465             1470

Ala Lys  Ile Glu Ala Leu Lys  Asn Ala Leu Val Gln  Lys Lys Thr
    1475             1480             1485
```

Ala Leu Val Ala Asp Asp Phe Ala Ser Leu Thr Ala Pro Ala Gln
     1490                1495                1500

Ala Gln Glu Gly Leu Ala Asn Ala Phe Asp Gly Asn Leu Ser Ser
     1505                1510                1515

Leu Trp His Thr Ser Trp Gly Gly Gly Asp Val Gly Lys Pro Ala
     1520                1525                1530

Thr Met Val Leu Lys Glu Ala Thr Glu Ile Thr Gly Leu Arg Tyr
     1535                1540                1545

Val Pro Arg Gly Ser Gly Ser Asn Gly Asn Leu Arg Asp Val Lys
     1550                1555                1560

Leu Val Val Thr Asp Glu Ser Gly Lys Glu His Thr Phe Thr Ala
     1565                1570                1575

Thr Asp Trp Pro Asp Asn Asn Lys Pro Lys Asp Ile Asp Phe Gly
     1580                1585                1590

Lys Thr Ile Lys Ala Lys Lys Ile Val Leu Thr Gly Thr Lys Thr
     1595                1600                1605

Tyr Gly Asp Gly Gly Asp Lys Tyr Gln Ser Ala Ala Glu Leu Ile
     1610                1615                1620

Phe Thr Arg Pro Gln Val Ala Glu Thr Pro Leu Asp Leu Ser Gly
     1625                1630                1635

Tyr Glu Ala Ala Leu Ala Lys Ala Gln Lys Leu Thr Asp Lys Asp
     1640                1645                1650

Asn Gln Glu Glu Val Ala Ser Val Gln Ala Ser Met Lys Tyr Ala
     1655                1660                1665

Thr Asp Asn His Leu Leu Thr Glu Arg Met Val Glu Tyr Phe Ala
     1670                1675                1680

Asp Tyr Leu Asn Gln Leu Lys Asp Ser Ala Thr Lys Pro Asp Ala
     1685                1690                1695

Pro Thr Val Glu Lys Pro Glu Phe Lys Leu Ser Ser Val Ala Ser
     1700                1705                1710

Asp Gln Gly Lys Thr Pro Asp Tyr Lys Gln Glu Ile Ala Arg Pro
     1715                1720                1725

Glu Thr Pro Glu Gln Ile Leu Pro Ala Thr Gly Glu Ser Gln Phe
     1730                1735                1740

Asp Thr Ala Leu Phe Leu Ala Ser Val Ser Leu Ala Leu Ser Ala
     1745                1750                1755

Leu Phe Val Val Lys Thr Lys Lys Asp
     1760                1765

<210> 5
<211> 5901
<212> DNA
<213> Bifidobacterium longum NCC2705

<220>
<221> CDS
<222> (1).. (5901)

<400> 5

```
atg aaa aag aag aag act ata tcg gct gcg ctg gca aca gcg tta gcc      48
Met Lys Lys Lys Lys Thr Ile Ser Ala Ala Leu Ala Thr Ala Leu Ala
1               5               10              15

tta acc tgc atg ggc agc ggg gga ggt act gcg ttc gca gtg ccc ctg      96
Leu Thr Cys Met Gly Ser Gly Gly Gly Thr Ala Phe Ala Val Pro Leu
                20              25              30

tct gat gct gac ttg cag act ttg gca agt cag att cag caa atc aac     144
Ser Asp Ala Asp Leu Gln Thr Leu Ala Ser Gln Ile Gln Gln Ile Asn
                35              40              45

gat act tct gat tct gca act gct tcc gag act cct tcc gca caa gcc     192
Asp Thr Ser Asp Ser Ala Thr Ala Ser Glu Thr Pro Ser Ala Gln Ala
        50              55              60

gat gcg gtt gaa ggc tgg act att gat tcc aac atc gct cag ggc gac     240
Asp Ala Val Glu Gly Trp Thr Ile Asp Ser Asn Ile Ala Gln Gly Asp
65              70              75              80

gaa atc ctg gag atg gca aac ggt tgg ctg cac ctc aag tcc act gcc     288
Glu Ile Leu Glu Met Ala Asn Gly Trp Leu His Leu Lys Ser Thr Ala
                85              90              95

tct aac ggt aat gcg gca gcg aac ccc agc tcc agc aac aac tgg ccg     336
Ser Asn Gly Asn Ala Ala Ala Asn Pro Ser Ser Ser Asn Asn Trp Pro
                100             105             110

gca gta gcc gta tgg ggc aca gat tac gac ttc tcc aag gcc ggc tcc     384
Ala Val Ala Val Trp Gly Thr Asp Tyr Asp Phe Ser Lys Ala Gly Ser
                115             120             125

ttc cac gcc acc atc aaa tcc ccg cag gaa ggc tcc gcc aac cgc ttc     432
Phe His Ala Thr Ile Lys Ser Pro Gln Glu Gly Ser Ala Asn Arg Phe
        130             135             140

ggc ttc tac ctg ggc tac aac gac ccg ggc agc ggc ctg ttc atc ggc     480
Gly Phe Tyr Leu Gly Tyr Asn Asp Pro Gly Ser Gly Leu Phe Ile Gly
145             150             155             160

tac gat tcg gac ggc tgg ttc tgg cag acc tac acc ggt ggc ggt agc     528
Tyr Asp Ser Asp Gly Trp Phe Trp Gln Thr Tyr Thr Gly Gly Gly Ser
                165             170             175

ggc agc tgg tac agc ggt gct cgt atc gct gct ccg agc gcc aac gaa     576
Gly Ser Trp Tyr Ser Gly Ala Arg Ile Ala Ala Pro Ser Ala Asn Glu
                180             185             190

gag cac gac att cag gtc tcc tgg acc gac gcc aag gtc gcc aca ctg     624
Glu His Asp Ile Gln Val Ser Trp Thr Asp Ala Lys Val Ala Thr Leu
                195             200             205

acc gtg gat ggc cag aag gca ttc gat gtc gat tac tcc gca atg acg     672
Thr Val Asp Gly Gln Lys Ala Phe Asp Val Asp Tyr Ser Ala Met Thr
        210             215             220

aac ctc tcc aac aag ctt gcc atc aag gcc ggc tcc tgg aag ggg ctg     720
Asn Leu Ser Asn Lys Leu Ala Ile Lys Ala Gly Ser Trp Lys Gly Leu
225             230             235             240

aac cag gtc acc gac gtg tac atc aag gac ttc ccg gag gtt gtc gaa     768
Asn Gln Val Thr Asp Val Tyr Ile Lys Asp Phe Pro Glu Val Val Glu
        245             250             255
```

```
gcc gcc aag cac gcg gtt tcc ggc aag gtt gtg gac gct gga ggc gct     816
Ala Ala Lys His Ala Val Ser Gly Lys Val Val Asp Ala Gly Gly Ala
        260             265             270

gcc att gaa ggc gca aca gtg cgt ttg gat aag acc aag gtg aag acc     864
Ala Ile Glu Gly Ala Thr Val Arg Leu Asp Lys Thr Lys Val Lys Thr
        275             280             285

ggt gca gat ggc acc ttc tcc ttc gcc gac att gaa gaa ggc gaa cac     912
Gly Ala Asp Gly Thr Phe Ser Phe Ala Asp Ile Glu Glu Gly Glu His
        290             295             300

acg ctt tcg att gcc aag gaa ggc tat gag gac gtc tcc cag cag gtg     960
Thr Leu Ser Ile Ala Lys Glu Gly Tyr Glu Asp Val Ser Gln Gln Val
305             310             315             320

gcg gtc tcc ggc gcg gac ctt gcc atc gat ccc atc acc ctg aac aaa     1008
Ala Val Ser Gly Ala Asp Leu Ala Ile Asp Pro Ile Thr Leu Asn Lys
                325             330             335

acc gtt cag gtc gcc tcc gaa acg ctg aag acc aag aag atg gaa gtt     1056
Thr Val Gln Val Ala Ser Glu Thr Leu Lys Thr Lys Lys Met Glu Val
            340             345             350

cag att aag aag aac ttc ccc tct gtg ctg cag tac acg atg acc gac     1104
Gln Ile Lys Lys Asn Phe Pro Ser Val Leu Gln Tyr Thr Met Thr Asp
        355             360             365

ggc aag gtg atg tac ggc caa acc aag gat gtg cgc acc gtt gag atc     1152
Gly Lys Val Met Tyr Gly Gln Thr Lys Asp Val Arg Thr Val Glu Ile
        370             375             380

aac ggc acc aac atc gaa ctg acc gac gat gac gtg acc ttc aag aag     1200
Asn Gly Thr Asn Ile Glu Leu Thr Asp Asp Asp Val Thr Phe Lys Lys
385             390             395             400

gtt tcc gat acc gaa gcc acc tac acg ctg aag gtc aag gat gag gcc     1248
Val Ser Asp Thr Glu Ala Thr Tyr Thr Leu Lys Val Lys Asp Glu Ala
                405             410             415

aag aag att gac gcg gtg atc acc gtt cag atc acg gtc aag gcc aac     1296
Lys Lys Ile Asp Ala Val Ile Thr Val Gln Ile Thr Val Lys Ala Asn
            420             425             430

cag ctg cac ctc aac gtc acc aag atc aag aac aac ctg tcc gaa ggc     1344
Gln Leu His Leu Asn Val Thr Lys Ile Lys Asn Asn Leu Ser Glu Gly
        435             440             445

att cct gag ggc aac ggt gtg gag gag aac gcc atc cag acg ctg tcc     1392
Ile Pro Glu Gly Asn Gly Val Glu Glu Asn Ala Ile Gln Thr Leu Ser
        450             455             460

ttc ccg aac cag agt ctc gtt tcc gtg cgt tcc agc cag gaa aat gcc     1440
Phe Pro Asn Gln Ser Leu Val Ser Val Arg Ser Ser Gln Glu Asn Ala
465             470             475             480

caa ttc act ggt gct cgt atg tct tcc aac acg cag aag cct ggc gat     1488
Gln Phe Thr Gly Ala Arg Met Ser Ser Asn Thr Gln Lys Pro Gly Asp
                485             490             495

acc aac ttc gca gtg acc gaa gat act aac gtc acc gat agc gac tac     1536
Thr Asn Phe Ala Val Thr Glu Asp Thr Asn Val Thr Asp Ser Asp Tyr
                500             505             510

acc tac ggc ttc atc tcc ggt gct ggc ctg agt gcc ggc ctg tgg agc     1584
Thr Tyr Gly Phe Ile Ser Gly Ala Gly Leu Ser Ala Gly Leu Trp Ser
            515             520             525

aac tcc gag cac gat ggc acc tat gtg gcg gct cct gtg cgc ggc ggc     1632
Asn Ser Glu His Asp Gly Thr Tyr Val Ala Ala Pro Val Arg Gly Gly
530             535             540

agc cag aac acg cgt gtc tac gcc acc acc cag cag act ggt gac gcc     1680
Ser Gln Asn Thr Arg Val Tyr Ala Thr Thr Gln Gln Thr Gly Asp Ala
545             550             555             560
```

44

```
acc tcc ctg ggc ctg gcc agc gct ccg tgg tac tac cac cgc acg gtc    1728
Thr Ser Leu Gly Leu Ala Ser Ala Pro Trp Tyr Tyr His Arg Thr Val
            565                 570                 575

acc gat tcc aag ggc aag aag tac acc gtg gcc gaa acc gct ctg ccg    1776
Thr Asp Ser Lys Gly Lys Lys Tyr Thr Val Ala Glu Thr Ala Leu Pro
            580                 585                 590

cag atg gcc gtg gcc atc gcc ggc gac gag aac ggt gac ggt gcc gtc    1824
Gln Met Ala Val Ala Ile Ala Gly Asp Glu Asn Gly Asp Gly Ala Val
        595                 600                 605

aac tgg cag gat ggc gca atc gcc tac cgc gac atc atg aac aac ccg    1872
Asn Trp Gln Asp Gly Ala Ile Ala Tyr Arg Asp Ile Met Asn Asn Pro
    610                 615                 620

tac aag tcc gag gaa gtt ccc gaa ctg gtg gca tgg cgt atc gcc atg    1920
Tyr Lys Ser Glu Glu Val Pro Glu Leu Val Ala Trp Arg Ile Ala Met
625                 630                 635                 640

aac ttc ggc tcc cag gcg cag aac ccg ttc ctc acc acg ctt gac aac    1968
Asn Phe Gly Ser Gln Ala Gln Asn Pro Phe Leu Thr Thr Leu Asp Asn
                645                 650                 655

gtc aag aag gtg gcc ttg aac acc gac ggc ctc ggc cag tcc gtg ctg    2016
Val Lys Lys Val Ala Leu Asn Thr Asp Gly Leu Gly Gln Ser Val Leu
            660                 665                 670

ctc aag ggc tac ggc aat gaa ggc cac gac tcc ggc cac ccg gac tac    2064
Leu Lys Gly Tyr Gly Asn Glu Gly His Asp Ser Gly His Pro Asp Tyr
            675                 680                 685

ggc gat atc ggc cag cgt ctc ggc ggc gcc gac gac atg aac acc atg    2112
Gly Asp Ile Gly Gln Arg Leu Gly Gly Ala Asp Asp Met Asn Thr Met
    690                 695                 700

atg gaa gag ggc tcc aag tat ggc gct cgc ttc ggt gtg cac gtc aac    2160
Met Glu Glu Gly Ser Lys Tyr Gly Ala Arg Phe Gly Val His Val Asn
705                 710                 715                 720

gcc tcc gaa atg tat ccg gaa gcc aag gcc ttc agc gag gac atg gtg    2208
Ala Ser Glu Met Tyr Pro Glu Ala Lys Ala Phe Ser Glu Asp Met Val
                725                 730                 735

cgc cgc aac tct gca ggc ggc ctg agc tac ggc tgg aac tgg ctt gat    2256
Arg Arg Asn Ser Ala Gly Gly Leu Ser Tyr Gly Trp Asn Trp Leu Asp
            740                 745                 750

cag ggt gtc ggc atc gac ggc atc tac gat ctg gca tcc ggt tct cgt    2304
Gln Gly Val Gly Ile Asp Gly Ile Tyr Asp Leu Ala Ser Gly Ser Arg
            755                 760                 765

gta agc cgt ttc gct gac ctc agc aag gaa gtc ggc gac aac atg gac    2352
Val Ser Arg Phe Ala Asp Leu Ser Lys Glu Val Gly Asp Asn Met Asp
        770                 775                 780

ttc atc tac ctc gat gtg tgg ggc aac ctg act tct tcc ggt tcg gaa    2400
Phe Ile Tyr Leu Asp Val Trp Gly Asn Leu Thr Ser Ser Gly Ser Glu
785                 790                 795                 800

gat tct tgg gaa acc cgc aag atg agc aag atg atc aac gac aac ggc    2448
Asp Ser Trp Glu Thr Arg Lys Met Ser Lys Met Ile Asn Asp Asn Gly
                805                 810                 815

tgg cgt atg acc acc gaa tgg ggt tcc ggc aac gag tac gac tcc acc    2496
Trp Arg Met Thr Thr Glu Trp Gly Ser Gly Asn Glu Tyr Asp Ser Thr
            820                 825                 830

ttc cag cac tgg gca gct gat ctg acc tac ggc ggc tac acc tcc aag    2544
Phe Gln His Trp Ala Ala Asp Leu Thr Tyr Gly Gly Tyr Thr Ser Lys
            835                 840                 845

ggc gag aac tcc gaa gtg atg cgc ttc ctg cgc aac cac cag aag gac    2592
Gly Glu Asn Ser Glu Val Met Arg Phe Leu Arg Asn His Gln Lys Asp
850                 855                 860
```

45

```
agc tgg gtt ggc gac tac ccg caa tac ggc ggc gct gcc aac gcc ccg      2640
Ser Trp Val Gly Asp Tyr Pro Gln Tyr Gly Gly Ala Ala Asn Ala Pro
865             870             875                 880

ctg ctc ggc ggc tac aac atg aag gac ttc gaa ggc tgg cag ggc cgc      2688
Leu Leu Gly Gly Tyr Asn Met Lys Asp Phe Glu Gly Trp Gln Gly Arg
                885             890             895

aac gac tat gcc gcc tac atc aag aac ctg tac acc cat gat gtg tcc      2736
Asn Asp Tyr Ala Ala Tyr Ile Lys Asn Leu Tyr Thr His Asp Val Ser
            900             905             910

act aag ttc atc cag cac ttc aag gtg acc cgc tgg gtc aac aac ccg      2784
Thr Lys Phe Ile Gln His Phe Lys Val Thr Arg Trp Val Asn Asn Pro
            915             920             925

ctg ctg acc gcc gac aat ggc aat gcc gct gcc gtg tcc gac ccg aac      2832
Leu Leu Thr Ala Asp Asn Gly Asn Ala Ala Ala Val Ser Asp Pro Asn
    930             935             940

acg aac aac ggc aac gag cag att acc ctg aag gat tcc aac ggc aac      2880
Thr Asn Asn Gly Asn Glu Gln Ile Thr Leu Lys Asp Ser Asn Gly Asn
945             950             955                 960

gtt gta gta gtc tcc cgt ggt tcc aac gac acc tct agc gca gcc tac      2928
Val Val Val Val Ser Arg Gly Ser Asn Asp Thr Ser Ser Ala Ala Tyr
                965             970             975

cgc cag cgc acc atc acc ttc aac ggc gtg aag gtc gca tcc ggt gtg      2976
Arg Gln Arg Thr Ile Thr Phe Asn Gly Val Lys Val Ala Ser Gly Val
            980             985             990

gtc tcc gca ggc gat ggc agc gcc  act ggc gac gag tcc  tac ctg ctg    3024
Val Ser Ala Gly Asp Gly Ser Ala  Thr Gly Asp Glu Ser  Tyr Leu Leu
            995             1000             1005

ccg tgg atg tgg gat tcc ttc  acc ggc aag ctg gtc  aag gat tcc        3069
Pro Trp Met Trp Asp Ser Phe  Thr Gly Lys Leu Val  Lys Asp Ser
    1010             1015             1020

gag cag aag ctc tac cac tgg aac acc aag ggc ggc  acc acc acc         3114
Glu Gln Lys Leu Tyr His Trp Asn Thr Lys Gly Gly  Thr Thr Thr
    1025             1030             1035

tgg acg ctg ccg gac agc tgg  aag aac ctc tcc agc  gta aag gtg        3159
Trp Thr Leu Pro Asp Ser Trp  Lys Asn Leu Ser Ser  Val Lys Val
    1040             1045             1050

tac cag ctc acc gat cag ggc  aag acc aac gag cag  acc gtt gcc        3204
Tyr Gln Leu Thr Asp Gln Gly  Lys Thr Asn Glu Gln  Thr Val Ala
    1055             1060             1065

gtc tcc ggc ggc aag gtg acg  ctt acc gct gat gcc  gaa acc ccg        3249
Val Ser Gly Gly Lys Val Thr  Leu Thr Ala Asp Ala  Glu Thr Pro
    1070             1075             1080

tac gtg gtg tac aag ggc gaa  gcc aag cag atc cag  gtc aac tgg        3294
Tyr Val Val Tyr Lys Gly Glu  Ala Lys Gln Ile Gln  Val Asn Trp
    1085             1090             1095

agc gaa ggc atg cat gtg gta  gac gcc ggc ttc aac  ggc ggc tcc        3339
Ser Glu Gly Met His Val Val  Asp Ala Gly Phe Asn  Gly Gly Ser
    1100             1105             1110

aac acc ctc acc gac aac tgg  acc gtc gcc gga acc  ggc aag gcc        3384
Asn Thr Leu Thr Asp Asn Trp  Thr Val Ala Gly Thr  Gly Lys Ala
    1115             1120             1125

gaa gtt gaa ggc gac aac aac  gcc atg ctg cgc ctg  acc ggc aag        3429
Glu Val Glu Gly Asp Asn Asn  Ala Met Leu Arg Leu  Thr Gly Lys
    1130             1135             1140

gtc gat gtc tcc cag cgt ctg  acc gat ctc aag gct  ggc cag aag        3474
Val Asp Val Ser Gln Arg Leu  Thr Asp Leu Lys Ala  Gly Gln Lys
    1145             1150             1155
```

46

```
tac gcg ctg tat gtt ggc gtc  gac aac cgc tcc acc  ggc gat gca    3519
Tyr Ala Leu Tyr Val Gly Val  Asp Asn Arg Ser Thr  Gly Asp Ala
    1160            1165                1170

tcc gtc acc gta acc agc ggc  ggc aag gtg ctg gcc  gcc aac tcc    3564
Ser Val Thr Val Thr Ser Gly  Gly Lys Val Leu Ala  Ala Asn Ser
    1175            1180                1185

acc ggc aag tcc atc gcc aag  aac tac atc aag gca  tac ggc cac    3609
Thr Gly Lys Ser Ile Ala Lys  Asn Tyr Ile Lys Ala  Tyr Gly His
    1190            1195                1200

aac acg aac agc aat acg gaa  aat ggc tcc agc tac  ttc cag aac    3654
Asn Thr Asn Ser Asn Thr Glu  Asn Gly Ser Ser Tyr  Phe Gln Asn
    1205            1210                1215

atg tac gtg ttc ttc acc gcg  cct gag aac ggc gat  gcc acg gta    3699
Met Tyr Val Phe Phe Thr Ala  Pro Glu Asn Gly Asp  Ala Thr Val
    1220            1225                1230

acc ctg tct cac aag agc acc  gac gga gca cac acc  tac ttc gac    3744
Thr Leu Ser His Lys Ser Thr  Asp Gly Ala His Thr  Tyr Phe Asp
    1235            1240                1245

gat gtg cgc atc gtg gag aac  cag tac tcc ggc atc  acc tat gag    3789
Asp Val Arg Ile Val Glu Asn  Gln Tyr Ser Gly Ile  Thr Tyr Glu
    1250            1255                1260

aag gac ggc acg ctg aag tcc  ctc acc aac gga ttc  gaa aac aac    3834
Lys Asp Gly Thr Leu Lys Ser  Leu Thr Asn Gly Phe  Glu Asn Asn
    1265            1270                1275

gcc cag ggc atc tgg ccg ttc  gtg gtc tcc ggt tcc  gaa ggc gtt    3879
Ala Gln Gly Ile Trp Pro Phe  Val Val Ser Gly Ser  Glu Gly Val
    1280            1285                1290

gag gac aac cgc atc cac ctc  tcc gag ctg cat gct  ccg ttc acg    3924
Glu Asp Asn Arg Ile His Leu  Ser Glu Leu His Ala  Pro Phe Thr
    1295            1300                1305

cag gcc ggt tgg gat gtc aag  aag atg gac gat gtg  ctc gat ggc    3969
Gln Ala Gly Trp Asp Val Lys  Lys Met Asp Asp Val  Leu Asp Gly
    1310            1315                1320

act tgg tct gtg aag gtt aac  ggc ctg acc cag aag  ggc acg ctg    4014
Thr Trp Ser Val Lys Val Asn  Gly Leu Thr Gln Lys  Gly Thr Leu
    1325            1330                1335

gtc tac cag acg atc ccg cag  aac gtg aag ttc gag  gcg ggt gcc    4059
Val Tyr Gln Thr Ile Pro Gln  Asn Val Lys Phe Glu  Ala Gly Ala
    1340            1345                1350

aag tac aag gtg agc ttc gac  tac cag tcc ggt tcc  gat gac atc    4104
Lys Tyr Lys Val Ser Phe Asp  Tyr Gln Ser Gly Ser  Asp Asp Ile
    1355            1360                1365

tac gcc atc gct gtg ggc cag  ggt gaa tac tct gcc  ggc agc gtg    4149
Tyr Ala Ile Ala Val Gly Gln  Gly Glu Tyr Ser Ala  Gly Ser Val
    1370            1375                1380

aag ctg acc aac ctg aag aag  gct ctg ggt gag acc  ggc aag gcc    4194
Lys Leu Thr Asn Leu Lys Lys  Ala Leu Gly Glu Thr  Gly Lys Ala
    1385            1390                1395

gag ttc gag ctg acc ggt ggc  gtc aac ggc gat tcc  tgg ttc ggt    4239
Glu Phe Glu Leu Thr Gly Gly  Val Asn Gly Asp Ser  Trp Phe Gly
    1400            1405                1410

att tac tcg acc gca acc gca  cct gat ctg cag ggt  tcc acc ggc    4284
Ile Tyr Ser Thr Ala Thr Ala  Pro Asp Leu Gln Gly  Ser Thr Gly
    1415            1420                1425

aat gca cag gac ttc ggc gga  tac aag gac ttc gtg  ctc gac aac    4329
Asn Ala Gln Asp Phe Gly Gly  Tyr Lys Asp Phe Val  Leu Asp Asn
    1430            1435                1440
```

47

```
ctg aag atc gag cgc atc gag tcc cag acc cgc acc aag gcc gaa    4374
Leu Lys Ile Glu Arg Ile Glu Ser Gln Thr Arg Thr Lys Ala Glu
    1445             1450             1455

gcg cag gac aag gtc aag gaa atc cgc ggc aag tac gat tcc aag    4419
Ala Gln Asp Lys Val Lys Glu Ile Arg Gly Lys Tyr Asp Ser Lys
    1460             1465             1470

cgt gct gag ctc tcc gat gcc gca tgg cag cag tat cag gac acc    4464
Arg Ala Glu Leu Ser Asp Ala Ala Trp Gln Gln Tyr Gln Asp Thr
    1475             1480             1485

ttg gtc aag gct cgc gtg ctc atc aac aag aat ggc gca acc gct    4509
Leu Val Lys Ala Arg Val Leu Ile Asn Lys Asn Gly Ala Thr Ala
    1490             1495             1500

gag gac ttc acc aag gca tac gac att ctc gtg gcc ctc gac gag    4554
Glu Asp Phe Thr Lys Ala Tyr Asp Ile Leu Val Ala Leu Asp Glu
    1505             1510             1515

tac atg aag acc gct ccc ggc aac gag agc agc gac aag tac gac    4599
Tyr Met Lys Thr Ala Pro Gly Asn Glu Ser Ser Asp Lys Tyr Asp
    1520             1525             1530

gtg gca gct gac ggc tcc gat gag ctg ggt ggc tac acc gtg gcc    4644
Val Ala Ala Asp Gly Ser Asp Glu Leu Gly Gly Tyr Thr Val Ala
    1535             1540             1545

acg ggc agc gaa gag cct acc gca ggc ctg ccg agc gaa ggc ccg    4689
Thr Gly Ser Glu Glu Pro Thr Ala Gly Leu Pro Ser Glu Gly Pro
    1550             1555             1560

gcc gat ctg gca cag gat ggc aac gac agc acc cac tgg cac acc    4734
Ala Asp Leu Ala Gln Asp Gly Asn Asp Ser Thr His Trp His Thr
    1565             1570             1575

agc tgg agc gag aac gca gtc ggc aac ggc acc gca tgg tat cag    4779
Ser Trp Ser Glu Asn Ala Val Gly Asn Gly Thr Ala Trp Tyr Gln
    1580             1585             1590

ttc aac ctc aac gaa ccg acc acc atc aac ggc ctg cgc tac ctg    4824
Phe Asn Leu Asn Glu Pro Thr Thr Ile Asn Gly Leu Arg Tyr Leu
    1595             1600             1605

ccg cgc tcc gga ggt atg aac gcc aac ggc aag atc aag ggc tac    4869
Pro Arg Ser Gly Gly Met Asn Ala Asn Gly Lys Ile Lys Gly Tyr
    1610             1615             1620

aag atc acg ctc act ctg gcg gat ggc acc acc aag gat gtc gtc    4914
Lys Ile Thr Leu Thr Leu Ala Asp Gly Thr Thr Lys Asp Val Val
    1625             1630             1635

acc gat gct gag ttc tcc acc acc acc atg tgg cag aag gcc agc    4959
Thr Asp Ala Glu Phe Ser Thr Thr Thr Met Trp Gln Lys Ala Ser
    1640             1645             1650

ttc gac gcc gtc gag aat gtg acc gcc gta cgc ttg acc gtc ctg    5004
Phe Asp Ala Val Glu Asn Val Thr Ala Val Arg Leu Thr Val Leu
    1655             1660             1665

tct tcc gca ggc cag agc gac tcc cag gcc aac aag ttc gca tcc    5049
Ser Ser Ala Gly Gln Ser Asp Ser Gln Ala Asn Lys Phe Ala Ser
    1670             1675             1680

gct gcc gaa ctg cgt ttg acc acg gac cgc gag gtt gag gaa gag    5094
Ala Ala Glu Leu Arg Leu Thr Thr Asp Arg Glu Val Glu Glu Glu
    1685             1690             1695

act gtc gct ccg gac aag acc gac ctc aac gac acc atc gcc aag    5139
Thr Val Ala Pro Asp Lys Thr Asp Leu Asn Asp Thr Ile Ala Lys
    1700             1705             1710

gct aac ggt ctt aag gaa tcc gac tac acg gct gaa agc tgg act    5184
Ala Asn Gly Leu Lys Glu Ser Asp Tyr Thr Ala Glu Ser Trp Thr
    1715             1720             1725
```

```
gct ctg gtc aag gcc cgc gaa gct gca cag gcc gtg gcg gat aac        5229
Ala Leu Val Lys Ala Arg Glu Ala Ala Gln Ala Val Ala Asp Asn
    1730            1735            1740

gat aag gcc acc gct tac gat gtg gct ctg gcg ctg acg aac ctc        5274
Asp Lys Ala Thr Ala Tyr Asp Val Ala Leu Ala Leu Thr Asn Leu
    1745            1750            1755

gaa tcc gct atc gct ggt ctc gag aag acc ggt gag gag cct ggc        5319
Glu Ser Ala Ile Ala Gly Leu Glu Lys Thr Gly Glu Glu Pro Gly
    1760            1765            1770

cca ggc ccg gtt gag gtg aac aag acc gac ctg cag act gca gtg        5364
Pro Gly Pro Val Glu Val Asn Lys Thr Asp Leu Gln Thr Ala Val
    1775            1780            1785

aac aag gca agc aag ctc gag aag gcc gat tac acg acc aac tcg        5409
Asn Lys Ala Ser Lys Leu Glu Lys Ala Asp Tyr Thr Thr Asn Ser
    1790            1795            1800

tgg gaa gct ttc gcc gag gca ctg aag gct gca cag cag gtg ctc        5454
Trp Glu Ala Phe Ala Glu Ala Leu Lys Ala Ala Gln Gln Val Leu
    1805            1810            1815

gac aac aag aac gcc acc cag cag gat gtg gat acc gca ctg agc        5499
Asp Asn Lys Asn Ala Thr Gln Gln Asp Val Asp Thr Ala Leu Ser
    1820            1825            1830

gct ctt cag gac gcc atc tcc aag ctg gaa gct acc gcc gag ccg        5544
Ala Leu Gln Asp Ala Ile Ser Lys Leu Glu Ala Thr Ala Glu Pro
    1835            1840            1845

aag ccg aat ccg gaa ccg ggc gtg gtg gac aag gct gct ctg aac        5589
Lys Pro Asn Pro Glu Pro Gly Val Val Asp Lys Ala Ala Leu Asn
    1850            1855            1860

gcg acc atc aac aag gcc gcc gcc atc aac ctg ggt ctc tac acc        5634
Ala Thr Ile Asn Lys Ala Ala Ala Ile Asn Leu Gly Leu Tyr Thr
    1865            1870            1875

gac gac tcc gcc aac gct ctg cgc gcc gcg ctg aag aag gcc cgt        5679
Asp Asp Ser Ala Asn Ala Leu Arg Ala Ala Leu Lys Lys Ala Arg
    1880            1885            1890

gag gtc tcc gac aac agc aac gcc acg cag aag cag gtc gac gca        5724
Glu Val Ser Asp Asn Ser Asn Ala Thr Gln Lys Gln Val Asp Ala
    1895            1900            1905

gct cgc gag gct ctc gag aag gca att gcc ggc ttg gtg aag cgt        5769
Ala Arg Glu Ala Leu Glu Lys Ala Ile Ala Gly Leu Val Lys Arg
    1910            1915            1920

acc gct gcc aag ggt gat ggc aac gtt gtt tcc aac acg ggc tcc        5814
Thr Ala Ala Lys Gly Asp Gly Asn Val Val Ser Asn Thr Gly Ser
    1925            1930            1935

gat gtc gcc acg atc gct ctg gct gga ctg ctg ctg gca ggt gct        5859
Asp Val Ala Thr Ile Ala Leu Ala Gly Leu Leu Leu Ala Gly Ala
    1940            1945            1950

ggc gcc gcc atc gcc tac cgt cgc aat cgc gag caa ctg tga            5901
Gly Ala Ala Ile Ala Tyr Arg Arg Asn Arg Glu Gln Leu
    1955            1960            1965
```

<210> 6
<211> 1966
<212> PRT
<213> Bifidobacterium longum NCC2705

<400> 6

49

Met Lys Lys Lys Lys Thr Ile Ser Ala Ala Leu Ala Thr Ala Leu Ala
1               5                   10                  15

EP 1 767 645 B1

```
Leu Thr Cys Met Gly Ser Gly Gly Gly Thr Ala Phe Ala Val Pro Leu
            20              25              30

Ser Asp Ala Asp Leu Gln Thr Leu Ala Ser Gln Ile Gln Gln Ile Asn
        35              40              45

Asp Thr Ser Asp Ser Ala Thr Ala Ser Glu Thr Pro Ser Ala Gln Ala
        50              55              60

Asp Ala Val Glu Gly Trp Thr Ile Asp Ser Asn Ile Ala Gln Gly Asp
65              70              75              80

Glu Ile Leu Glu Met Ala Asn Gly Trp Leu His Leu Lys Ser Thr Ala
            85              90              95

Ser Asn Gly Asn Ala Ala Ala Asn Pro Ser Ser Ser Asn Asn Trp Pro
        100             105             110

Ala Val Ala Val Trp Gly Thr Asp Tyr Asp Phe Ser Lys Ala Gly Ser
        115             120             125

Phe His Ala Thr Ile Lys Ser Pro Gln Glu Gly Ser Ala Asn Arg Phe
        130             135             140

Gly Phe Tyr Leu Gly Tyr Asn Asp Pro Gly Ser Gly Leu Phe Ile Gly
145             150             155             160

Tyr Asp Ser Asp Gly Trp Phe Trp Gln Thr Tyr Thr Gly Gly Gly Ser
            165             170             175

Gly Ser Trp Tyr Ser Gly Ala Arg Ile Ala Ala Pro Ser Ala Asn Glu
        180             185             190

Glu His Asp Ile Gln Val Ser Trp Thr Asp Ala Lys Val Ala Thr Leu
        195             200             205

Thr Val Asp Gly Gln Lys Ala Phe Asp Val Asp Tyr Ser Ala Met Thr
        210             215             220

Asn Leu Ser Asn Lys Leu Ala Ile Lys Ala Gly Ser Trp Lys Gly Leu
225             230             235             240

Asn Gln Val Thr Asp Val Tyr Ile Lys Asp Phe Pro Glu Val Val Glu
            245             250             255

Ala Ala Lys His Ala Val Ser Gly Lys Val Val Asp Ala Gly Gly Ala
            260             265             270

Ala Ile Glu Gly Ala Thr Val Arg Leu Asp Lys Thr Lys Val Lys Thr
            275             280             285

Gly Ala Asp Gly Thr Phe Ser Phe Ala Asp Ile Glu Glu Gly Glu His
            290             295             300

Thr Leu Ser Ile Ala Lys Glu Gly Tyr Glu Asp Val Ser Gln Gln Val
305             310             315             320
```

```
Ala Val Ser Gly Ala Asp Leu Ala Ile Asp Pro Ile Thr Leu Asn Lys
            325             330             335

Thr Val Gln Val Ala Ser Glu Thr Leu Lys Thr Lys Lys Met Glu Val
            340             345             350

Gln Ile Lys Lys Asn Phe Pro Ser Val Leu Gln Tyr Thr Met Thr Asp
            355             360             365

Gly Lys Val Met Tyr Gly Gln Thr Lys Asp Val Arg Thr Val Glu Ile
            370             375             380

Asn Gly Thr Asn Ile Glu Leu Thr Asp Asp Asp Val Thr Phe Lys Lys
385                 390             395             400

Val Ser Asp Thr Glu Ala Thr Tyr Thr Leu Lys Val Lys Asp Glu Ala
            405             410             415

Lys Lys Ile Asp Ala Val Ile Thr Val Gln Ile Thr Val Lys Ala Asn
            420             425             430

Gln Leu His Leu Asn Val Thr Lys Ile Lys Asn Asn Leu Ser Glu Gly
            435             440             445

Ile Pro Glu Gly Asn Gly Val Glu Glu Asn Ala Ile Gln Thr Leu Ser
450                 455             460

Phe Pro Asn Gln Ser Leu Val Ser Val Arg Ser Ser Gln Glu Asn Ala
465             470             475             480

Gln Phe Thr Gly Ala Arg Met Ser Ser Asn Thr Gln Lys Pro Gly Asp
            485             490             495

Thr Asn Phe Ala Val Thr Glu Asp Thr Asn Val Thr Asp Ser Asp Tyr
            500             505             510

Thr Tyr Gly Phe Ile Ser Gly Ala Gly Leu Ser Ala Gly Leu Trp Ser
            515             520             525

Asn Ser Glu His Asp Gly Thr Tyr Val Ala Ala Pro Val Arg Gly Gly
            530             535             540

Ser Gln Asn Thr Arg Val Tyr Ala Thr Thr Gln Gln Thr Gly Asp Ala
545             550             555             560

Thr Ser Leu Gly Leu Ala Ser Ala Pro Trp Tyr Tyr His Arg Thr Val
            565             570             575

Thr Asp Ser Lys Gly Lys Lys Tyr Thr Val Ala Glu Thr Ala Leu Pro
            580             585             590

Gln Met Ala Val Ala Ile Ala Gly Asp Glu Asn Gly Asp Gly Ala Val
            595             600             605

Asn Trp Gln Asp Gly Ala Ile Ala Tyr Arg Asp Ile Met Asn Asn Pro
            610             615             620
```

Tyr Lys Ser Glu Glu Val Pro Glu Leu Val Ala Trp Arg Ile Ala Met
625                630                635                640

Asn Phe Gly Ser Gln Ala Gln Asn Pro Phe Leu Thr Thr Leu Asp Asn
               645                650                655

Val Lys Lys Val Ala Leu Asn Thr Asp Gly Leu Gly Gln Ser Val Leu
          660                665                670

Leu Lys Gly Tyr Gly Asn Glu Gly His Asp Ser Gly His Pro Asp Tyr
          675                680                685

Gly Asp Ile Gly Gln Arg Leu Gly Gly Ala Asp Asp Met Asn Thr Met
          690                695                700

Met Glu Glu Gly Ser Lys Tyr Gly Ala Arg Phe Gly Val His Val Asn
705                710                715                720

Ala Ser Glu Met Tyr Pro Glu Ala Lys Ala Phe Ser Glu Asp Met Val
               725                730                735

Arg Arg Asn Ser Ala Gly Gly Leu Ser Tyr Gly Trp Asn Trp Leu Asp
          740                745                750

Gln Gly Val Gly Ile Asp Gly Ile Tyr Asp Leu Ala Ser Gly Ser Arg
          755                760                765

Val Ser Arg Phe Ala Asp Leu Ser Lys Glu Val Gly Asp Asn Met Asp
          770                775                780

Phe Ile Tyr Leu Asp Val Trp Gly Asn Leu Thr Ser Ser Gly Ser Glu
785                790                795                800

Asp Ser Trp Glu Thr Arg Lys Met Ser Lys Met Ile Asn Asp Asn Gly
               805                810                815

Trp Arg Met Thr Thr Glu Trp Gly Ser Gly Asn Glu Tyr Asp Ser Thr
               820                825                830

Phe Gln His Trp Ala Ala Asp Leu Thr Tyr Gly Gly Tyr Thr Ser Lys
          835                840                845

Gly Glu Asn Ser Glu Val Met Arg Phe Leu Arg Asn His Gln Lys Asp
          850                855                860

Ser Trp Val Gly Asp Tyr Pro Gln Tyr Gly Gly Ala Ala Asn Ala Pro
865                870                875                880

Leu Leu Gly Gly Tyr Asn Met Lys Asp Phe Glu Gly Trp Gln Gly Arg
               885                890                895

Asn Asp Tyr Ala Ala Tyr Ile Lys Asn Leu Tyr Thr His Asp Val Ser
          900                905                910

Thr Lys Phe Ile Gln His Phe Lys Val Thr Arg Trp Val Asn Asn Pro
          915                920                925

53

Leu Leu Thr Ala Asp Asn Gly Asn Ala Ala Ala Val Ser Asp Pro Asn
930                935                940

Thr Asn Asn Gly Asn Glu Gln Ile Thr Leu Lys Asp Ser Asn Gly Asn
945                950                955                960

Val Val Val Val Ser Arg Gly Ser Asn Asp Thr Ser Ser Ala Ala Tyr
965                970                975

Arg Gln Arg Thr Ile Thr Phe Asn Gly Val Lys Val Ala Ser Gly Val
980                985                990

Val Ser Ala Gly Asp Gly Ser Ala Thr Gly Asp Glu Ser Tyr Leu Leu
995                1000                1005

Pro Trp Met Trp Asp Ser Phe Thr Gly Lys Leu Val Lys Asp Ser
1010                1015                1020

Glu Gln Lys Leu Tyr His Trp Asn Thr Lys Gly Gly Thr Thr Thr
1025                1030                1035

Trp Thr Leu Pro Asp Ser Trp Lys Asn Leu Ser Ser Val Lys Val
1040                1045                1050

Tyr Gln Leu Thr Asp Gln Gly Lys Thr Asn Glu Gln Thr Val Ala
1055                1060                1065

Val Ser Gly Gly Lys Val Thr Leu Thr Ala Asp Ala Glu Thr Pro
1070                1075                1080

Tyr Val Val Tyr Lys Gly Glu Ala Lys Gln Ile Gln Val Asn Trp
1085                1090                1095

Ser Glu Gly Met His Val Val Asp Ala Gly Phe Asn Gly Gly Ser
1100                1105                1110

Asn Thr Leu Thr Asp Asn Trp Thr Val Ala Gly Thr Gly Lys Ala
1115                1120                1125

Glu Val Glu Gly Asp Asn Asn Ala Met Leu Arg Leu Thr Gly Lys
1130                1135                1140

Val Asp Val Ser Gln Arg Leu Thr Asp Leu Lys Ala Gly Gln Lys
1145                1150                1155

Tyr Ala Leu Tyr Val Gly Val Asp Asn Arg Ser Thr Gly Asp Ala
1160                1165                1170

Ser Val Thr Val Thr Ser Gly Gly Lys Val Leu Ala Ala Asn Ser
1175                1180                1185

Thr Gly Lys Ser Ile Ala Lys Asn Tyr Ile Lys Ala Tyr Gly His
1190                1195                1200

Asn Thr Asn Ser Asn Thr Glu Asn Gly Ser Ser Tyr Phe Gln Asn
1205                1210                1215

```
Met Tyr  Val Phe Phe Thr Ala  Pro Glu Asn Gly Asp  Ala Thr Val
    1220              1225              1230

Thr Leu  Ser His Lys Ser Thr  Asp Gly Ala His Thr  Tyr Phe Asp
    1235              1240              1245

Asp Val  Arg Ile Val Glu Asn  Gln Tyr Ser Gly Ile  Thr Tyr Glu
    1250              1255              1260

Lys Asp  Gly Thr Leu Lys Ser  Leu Thr Asn Gly Phe  Glu Asn Asn
    1265              1270              1275

Ala Gln  Gly Ile Trp Pro Phe  Val Val Ser Gly Ser  Glu Gly Val
    1280              1285              1290

Glu Asp  Asn Arg Ile His Leu  Ser Glu Leu His Ala  Pro Phe Thr
    1295              1300              1305

Gln Ala  Gly Trp Asp Val Lys  Lys Met Asp Asp Val  Leu Asp Gly
    1310              1315              1320

Thr Trp  Ser Val Lys Val Asn  Gly Leu Thr Gln Lys  Gly Thr Leu
    1325              1330              1335

Val Tyr  Gln Thr Ile Pro Gln  Asn Val Lys Phe Glu  Ala Gly Ala
    1340              1345              1350

Lys Tyr  Lys Val Ser Phe Asp  Tyr Gln Ser Gly Ser  Asp Asp Ile
    1355              1360              1365

Tyr Ala  Ile Ala Val Gly Gln  Gly Glu Tyr Ser Ala  Gly Ser Val
    1370              1375              1380

Lys Leu  Thr Asn Leu Lys Lys  Ala Leu Gly Glu Thr  Gly Lys Ala
    1385              1390              1395

Glu Phe  Glu Leu Thr Gly Gly  Val Asn Gly Asp Ser  Trp Phe Gly
    1400              1405              1410

Ile Tyr  Ser Thr Ala Thr Ala  Pro Asp Leu Gln Gly  Ser Thr Gly
    1415              1420              1425

Asn Ala  Gln Asp Phe Gly Gly  Tyr Lys Asp Phe Val  Leu Asp Asn
    1430              1435              1440

Leu Lys  Ile Glu Arg Ile Glu  Ser Gln Thr Arg Thr  Lys Ala Glu
    1445              1450              1455

Ala Gln  Asp Lys Val Lys Glu  Ile Arg Gly Lys Tyr  Asp Ser Lys
    1460              1465              1470

Arg Ala  Glu Leu Ser Asp Ala  Ala Trp Gln Gln Tyr  Gln Asp Thr
    1475              1480              1485

Leu Val  Lys Ala Arg Val Leu  Ile Asn Lys Asn Gly  Ala Thr Ala
    1490              1495              1500
```

```
Glu Asp  Phe Thr Lys Ala Tyr  Asp Ile Leu Val Ala  Leu Asp Glu
    1505                1510                1515

Tyr Met  Lys Thr Ala Pro Gly  Asn Glu Ser Ser Asp  Lys Tyr Asp
    1520                1525                1530

Val Ala  Ala Asp Gly Ser Asp  Glu Leu Gly Gly Tyr  Thr Val Ala
    1535                1540                1545

Thr Gly  Ser Glu Glu Pro Thr  Ala Gly Leu Pro Ser  Glu Gly Pro
    1550                1555                1560

Ala Asp  Leu Ala Gln Asp Gly  Asn Asp Ser Thr His  Trp His Thr
    1565                1570                1575

Ser Trp  Ser Glu Asn Ala Val  Gly Asn Gly Thr Ala  Trp Tyr Gln
    1580                1585                1590

Phe Asn  Leu Asn Glu Pro Thr  Thr Ile Asn Gly Leu  Arg Tyr Leu
    1595                1600                1605

Pro Arg  Ser Gly Gly Met Asn  Ala Asn Gly Lys Ile  Lys Gly Tyr
    1610                1615                1620

Lys Ile  Thr Leu Thr Leu Ala  Asp Gly Thr Thr Lys  Asp Val Val
    1625                1630                1635

Thr Asp  Ala Glu Phe Ser Thr  Thr Thr Met Trp Gln  Lys Ala Ser
    1640                1645                1650

Phe Asp  Ala Val Glu Asn Val  Thr Ala Val Arg Leu  Thr Val Leu
    1655                1660                1665

Ser Ser  Ala Gly Gln Ser Asp  Ser Gln Ala Asn Lys  Phe Ala Ser
    1670                1675                1680

Ala Ala  Glu Leu Arg Leu Thr  Thr Asp Arg Glu Val  Glu Glu Glu
    1685                1690                1695

Thr Val  Ala Pro Asp Lys Thr  Asp Leu Asn Asp Thr  Ile Ala Lys
    1700                1705                1710

Ala Asn  Gly Leu Lys Glu Ser  Asp Tyr Thr Ala Glu  Ser Trp Thr
    1715                1720                1725

Ala Leu  Val Lys Ala Arg Glu  Ala Ala Gln Ala Val  Ala Asp Asn
    1730                1735                1740

Asp Lys  Ala Thr Ala Tyr Asp  Val Ala Leu Ala Leu  Thr Asn Leu
    1745                1750                1755

Glu Ser  Ala Ile Ala Gly Leu  Glu Lys Thr Gly Glu  Glu Pro Gly
    1760                1765                1770

Pro Gly  Pro Val Glu Val Asn  Lys Thr Asp Leu Gln  Thr Ala Val
    1775                1780                1785
```

```
        Asn Lys  Ala Ser Lys Leu Glu  Lys Ala Asp Tyr Thr  Thr Asn Ser
            1790              1795              1800

        Trp Glu  Ala Phe Ala Glu Ala  Leu Lys Ala Ala Gln  Gln Val Leu
            1805              1810              1815

        Asp Asn  Lys Asn Ala Thr Gln  Gln Asp Val Asp Thr  Ala Leu Ser
            1820              1825              1830

        Ala Leu  Gln Asp Ala Ile Ser  Lys Leu Glu Ala Thr  Ala Glu Pro
            1835              1840              1845

        Lys Pro  Asn Pro Glu Pro Gly  Val Val Asp Lys Ala  Ala Leu Asn
            1850              1855              1860

        Ala Thr  Ile Asn Lys Ala Ala  Ala Ile Asn Leu Gly  Leu Tyr Thr
            1865              1870              1875

        Asp Asp  Ser Ala Asn Ala Leu  Arg Ala Ala Leu Lys  Lys Ala Arg
            1880              1885              1890

        Glu Val  Ser Asp Asn Ser Asn  Ala Thr Gln Lys Gln  Val Asp Ala
            1895              1900              1905

        Ala Arg  Glu Ala Leu Glu Lys  Ala Ile Ala Gly Leu  Val Lys Arg
            1910              1915              1920

        Thr Ala  Ala Lys Gly Asp Gly  Asn Val Val Ser Asn  Thr Gly Ser
            1925              1930              1935

        Asp Val  Ala Thr Ile Ala Leu  Ala Gly Leu Leu Leu  Ala Gly Ala
            1940              1945              1950

        Gly Ala  Ala Ile Ala Tyr Arg  Arg Asn Arg Glu Gln  Leu
            1955              1960              1965
```

<210> 7
<211> 5061
<212> DNA
<213> Clostridium perfringens str. 13

<220>
<221> CDS
<222> (1).. (5061)

<400> 7

```
atg ggt aga aaa tgc atg aat aag aag att gcc gca ata ata gca gct      48
Met Gly Arg Lys Cys Met Asn Lys Lys Ile Ala Ala Ile Ile Ala Ala
1               5                   10                  15

gca gtt att gta gga caa tta cca att tca gta ctt gct aca cct gtt      96
Ala Val Ile Val Gly Gln Leu Pro Ile Ser Val Leu Ala Thr Pro Val
            20                  25                  30

aat gaa gct gga gat gag att aat agt gaa tca gct gaa att tta act     144
Asn Glu Ala Gly Asp Glu Ile Asn Ser Glu Ser Ala Glu Ile Leu Thr
        35                  40                  45

aat agt gat gaa gaa gct gag gct tat att caa aac tat gat aga cca     192
Asn Ser Asp Glu Glu Ala Glu Ala Tyr Ile Gln Asn Tyr Asp Arg Pro
    50                  55                  60

gag ggg att act tgg aca aaa tta gca ggc tca gga agt gtt gag gta     240
Glu Gly Ile Thr Trp Thr Lys Leu Ala Gly Ser Gly Ser Val Glu Val
```

```
        65                    70                    75                    80
act gat gga ttt tta tca gtt act aat aat gga gat tat aga att atg       288
Thr Asp Gly Phe Leu Ser Val Thr Asn Asn Gly Asp Tyr Arg Ile Met
                85                    90                    95

gaa gat caa tcg cct aat ata aaa aat ggt gag tta gaa agt aaa ttt       336
Glu Asp Gln Ser Pro Asn Ile Lys Asn Gly Glu Leu Glu Ser Lys Phe
                100                   105                   110

aca gtt gga ggt tct caa act gga ata ata ttt aga gca act gag tca       384
Thr Val Gly Gly Ser Gln Thr Gly Ile Ile Phe Arg Ala Thr Glu Ser
                115                   120                   125

aac tat gga atg atc aac tat aac tca ggt act ggc tgg gtt ata gaa       432
Asn Tyr Gly Met Ile Asn Tyr Asn Ser Gly Thr Gly Trp Val Ile Glu
                130                   135                   140

aat aaa aat agt tgg gag gat att aca gga cca aaa cta aat aat gga       480
Asn Lys Asn Ser Trp Glu Asp Ile Thr Gly Pro Lys Leu Asn Asn Gly
145                   150                   155                   160

gat gtt gta aca gtt aag gct act ttt gtt gaa aag cat tta act gtt       528
Asp Val Val Thr Val Lys Ala Thr Phe Val Glu Lys His Leu Thr Val
                165                   170                   175

aat gtt tct gta aat gat gga gaa ttt gaa act ata tat gat aaa gaa       576
Asn Val Ser Val Asn Asp Gly Glu Phe Glu Thr Ile Tyr Asp Lys Glu
                180                   185                   190

tca gat tta att cca tta caa gct ggt aaa gtt gga tat aga gga tgg       624
Ser Asp Leu Ile Pro Leu Gln Ala Gly Lys Val Gly Tyr Arg Gly Trp
                195                   200                   205

ggt aac gct aaa act acc aaa ttt gat tat att aag tat gct cct atg       672
Gly Asn Ala Lys Thr Thr Lys Phe Asp Tyr Ile Lys Tyr Ala Pro Met
                210                   215                   220

act ata gat aag gga cct ata gta tca ata aat gaa gta aat gta gaa       720
Thr Ile Asp Lys Gly Pro Ile Val Ser Ile Asn Glu Val Asn Val Glu
225                   230                   235                   240

act tat cca aga gtt aag cct att tta cca tca agt gtt aca gta aat       768
Thr Tyr Pro Arg Val Lys Pro Ile Leu Pro Ser Ser Val Thr Val Asn
                245                   250                   255

cat gaa aat ggt atg tct agt att aag gat gtt tct tgg aat tat ata       816
His Glu Asn Gly Met Ser Ser Ile Lys Asp Val Ser Trp Asn Tyr Ile
                260                   265                   270

cct aag gaa agt tat tca aag cca ggt aca ttc aaa gtt gaa ggt aca       864
Pro Lys Glu Ser Tyr Ser Lys Pro Gly Thr Phe Lys Val Glu Gly Thr
                275                   280                   285

gtt gaa ggt aca gat gta aaa gca ata gct aat gta act gta agt tca       912
Val Glu Gly Thr Asp Val Lys Ala Ile Ala Asn Val Thr Val Ser Ser
                290                   295                   300

gat tta gca tat tat gaa act aat ttt gaa aca gaa gaa aca aga gga       960
Asp Leu Ala Tyr Tyr Glu Thr Asn Phe Glu Thr Glu Glu Thr Arg Gly
305                   310                   315                   320

gat tgg caa gtt gta caa gga gga gga tct cca agt tat gaa gag ggt      1008
Asp Trp Gln Val Val Gln Gly Gly Gly Ser Pro Ser Tyr Glu Glu Gly
                325                   330                   335

aaa gta aaa ata cct atg aat gga gta tca atc gct gtt gat atg aat      1056
Lys Val Lys Ile Pro Met Asn Gly Val Ser Ile Ala Val Asp Met Asn
                340                   345                   350

tct cca gag gtt aag aac ttt act tat gaa act gat ttt tct gtt gat      1104
Ser Pro Glu Val Lys Asn Phe Thr Tyr Glu Thr Asp Phe Ser Val Asp
                355                   360                   365

aat aat gga gga aga ata ggt cta tta ttt aga tat gta tca gaa act      1152
Asn Asn Gly Gly Arg Ile Gly Leu Leu Phe Arg Tyr Val Ser Glu Thr
```

|  | 370 |  | 375 |  | 380 |  |
|---|---|---|---|---|---|---|

gag tgg gga gct gtt tgc tat gat aat ggt tct tgg gta tgg aaa act   1200
Glu Trp Gly Ala Val Cys Tyr Asp Asn Gly Ser Trp Val Trp Lys Thr
385             390                 395            400

gga gat ggc aaa tat ggt aat ttc cca gga aca ttt aca cca gag cca   1248
Gly Asp Gly Lys Tyr Gly Asn Phe Pro Gly Thr Phe Thr Pro Glu Pro
            405              410             415

gga aaa act tac aga ata aag ctt aaa gta gaa gat aca aat att act   1296
Gly Lys Thr Tyr Arg Ile Lys Leu Lys Val Glu Asp Thr Asn Ile Thr
           420            425           430

atg tgg gtt gat gga gag aaa ata ggg caa gtt gca gta tct aat tta   1344
Met Trp Val Asp Gly Glu Lys Ile Gly Gln Val Ala Val Ser Asn Leu
           435          440           445

cca gat gta aga gga aaa gtt ggc tta act gga tgg ttt gga aat aaa   1392
Pro Asp Val Arg Gly Lys Val Gly Leu Thr Gly Trp Phe Gly Asn Lys
    450           455          460

aat gtt act tta gat aat ctt gtt gtt gag gaa tta ggt gga ata atg   1440
Asn Val Thr Leu Asp Asn Leu Val Val Glu Glu Leu Gly Gly Ile Met
465           470          475          480

gca cca gaa gta ggt cca tta gaa gaa caa agt ata gaa tca gat tct   1488
Ala Pro Glu Val Gly Pro Leu Glu Glu Gln Ser Ile Glu Ser Asp Ser
           485          490          495

atg aaa gtt gtt tta gat aat aga ttc cca act gtt att aga tat gag   1536
Met Lys Val Val Leu Asp Asn Arg Phe Pro Thr Val Ile Arg Tyr Glu
           500         505          510

tgg aaa gga act gaa gat gtt tta tca gga gca tct gta gat gat tta   1584
Trp Lys Gly Thr Glu Asp Val Leu Ser Gly Ala Ser Val Asp Asp Leu
           515         520          525

gaa gct caa tat atg gtt gaa att aat ggt gaa aag aga att cca aaa   1632
Glu Ala Gln Tyr Met Val Glu Ile Asn Gly Glu Lys Arg Ile Pro Lys
    530           535          540

gta act agt gaa ttt gca aat aat gaa ggt ata tat aca tta aac ttt   1680
Val Thr Ser Glu Phe Ala Asn Asn Glu Gly Ile Tyr Thr Leu Asn Phe
545           550          555          560

gaa gat ata gga atg act att act tta aag atg act gtt aat gaa aat   1728
Glu Asp Ile Gly Met Thr Ile Thr Leu Lys Met Thr Val Asn Glu Asn
           565         570          575

aaa tta aga atg gaa gtt act gat att caa gaa ggg gat gtt aaa ctt   1776
Lys Leu Arg Met Glu Val Thr Asp Ile Gln Glu Gly Asp Val Lys Leu
           580         585          590

caa aca tta aat ttc cca aat cat agt tta gct tca gta agc agt tta   1824
Gln Thr Leu Asn Phe Pro Asn His Ser Leu Ala Ser Val Ser Ser Leu
           595         600          605

aat aat ggt aaa aca gcc tct gtt cta aca act ggt gac tgg aat aac   1872
Asn Asn Gly Lys Thr Ala Ser Val Leu Thr Thr Gly Asp Trp Asn Asn
          610          615          620

ata aat gaa gag ttt aca gat gtt gct aag gca aaa cca ggg gtt aag   1920
Ile Asn Glu Glu Phe Thr Asp Val Ala Lys Ala Lys Pro Gly Val Lys
625           630         635          640

ggt aaa act tat gca ttt ata aat gat gat aag ttt gct gtt act ata   1968
Gly Lys Thr Tyr Ala Phe Ile Asn Asp Asp Lys Phe Ala Val Thr Ile
           645         650          655

aat aat aat act att gaa ggt gga aat agg gtt gta tta aca aca gaa   2016
Asn Asn Asn Thr Ile Glu Gly Gly Asn Arg Val Val Leu Thr Thr Glu
          660          665          670

aat gat act ctt cct gat aat aca aac tat aag aaa gtt gga ata tca   2064
Asn Asp Thr Leu Pro Asp Asn Thr Asn Tyr Lys Lys Val Gly Ile Ser

```
                675                 680                 685
aac ggt act tgg act tat aaa gaa ata ctt caa gat aca aca gat caa        2112
Asn Gly Thr Trp Thr Tyr Lys Glu Ile Leu Gln Asp Thr Thr Asp Gln
    690                 695                 700

gga agt aag cta tat caa ggt gaa aag cca tgg tca gaa gta att ata        2160
Gly Ser Lys Leu Tyr Gln Gly Glu Lys Pro Trp Ser Glu Val Ile Ile
705                 710                 715                 720

gca aga gat gag aat gaa gat ggt caa gta gat tgg caa gat ggt gct        2208
Ala Arg Asp Glu Asn Glu Asp Gly Gln Val Asp Trp Gln Asp Gly Ala
                725                 730                 735

att caa tat aga aaa aat atg aaa att cca gta ggt gga gaa gaa ata        2256
Ile Gln Tyr Arg Lys Asn Met Lys Ile Pro Val Gly Gly Glu Glu Ile
                740                 745                 750

aaa aat caa atg tca tat att gat ttt aat att gga tac act caa aat        2304
Lys Asn Gln Met Ser Tyr Ile Asp Phe Asn Ile Gly Tyr Thr Gln Asn
                755                 760                 765

cca ttc tta aga tca tta gat aca att aaa aag ctt tca aat tat aca        2352
Pro Phe Leu Arg Ser Leu Asp Thr Ile Lys Lys Leu Ser Asn Tyr Thr
                770                 775                 780

gat gga ttt gga caa tta gtt ctt cat aag gga tat caa gga gaa gga        2400
Asp Gly Phe Gly Gln Leu Val Leu His Lys Gly Tyr Gln Gly Glu Gly
785                 790                 795                 800

cat gac gac tct cat cca gac tat ggc gga cat att ggt atg aga caa        2448
His Asp Asp Ser His Pro Asp Tyr Gly Gly His Ile Gly Met Arg Gln
                805                 810                 815

ggt ggt aag gaa gac ttc aat acc tta ata gaa caa ggt aag gaa tat        2496
Gly Gly Lys Glu Asp Phe Asn Thr Leu Ile Glu Gln Gly Lys Glu Tyr
                820                 825                 830

aat gct aaa ata ggt gtt cat ata aat gca acc gaa tat act atg gat        2544
Asn Ala Lys Ile Gly Val His Ile Asn Ala Thr Glu Tyr Thr Met Asp
                835                 840                 845

gcg ttt gaa tat cca act gaa tta gtt aac gaa aat gct cca gga tgg        2592
Ala Phe Glu Tyr Pro Thr Glu Leu Val Asn Glu Asn Ala Pro Gly Trp
    850                 855                 860

gga tgg tta gac caa gct tat tat gta aat caa aga gga gat ata act        2640
Gly Trp Leu Asp Gln Ala Tyr Tyr Val Asn Gln Arg Gly Asp Ile Thr
865                 870                 875                 880

agt ggt gaa tta ttc aga aga ctt gat atg tta atg gaa gat gca cca        2688
Ser Gly Glu Leu Phe Arg Arg Leu Asp Met Leu Met Glu Asp Ala Pro
                885                 890                 895

gaa cta gga tgg att tac gtt gac gtt tat aca ggt aat gga tgg aat        2736
Glu Leu Gly Trp Ile Tyr Val Asp Val Tyr Thr Gly Asn Gly Trp Asn
                900                 905                 910

gct cat caa tta gga gaa aag ata aat gat tat gga att atg att gca        2784
Ala His Gln Leu Gly Glu Lys Ile Asn Asp Tyr Gly Ile Met Ile Ala
    915                 920                 925

aca gaa atg aat gga cca tta gag caa cat gtt cca tgg act cac tgg        2832
Thr Glu Met Asn Gly Pro Leu Glu Gln His Val Pro Trp Thr His Trp
    930                 935                 940

ggt gga gat cct gca tat cca aac aag gga aat gca agt aaa ata atg        2880
Gly Gly Asp Pro Ala Tyr Pro Asn Lys Gly Asn Ala Ser Lys Ile Met
945                 950                 955                 960

aga ttt atg aaa aat gat act caa gat tca ttc tta gca gat cca tta        2928
Arg Phe Met Lys Asn Asp Thr Gln Asp Ser Phe Leu Ala Asp Pro Leu
                965                 970                 975

gta aaa ggt aat aag cat tta tta tca ggt gga tgg gga act aga cat        2976
Val Lys Gly Asn Lys His Leu Leu Ser Gly Gly Trp Gly Thr Arg His
```

```
                      980                    985                   990
    gat ata gaa ggt gct tat ggt aca  gaa gta ttc tat aat  caa gta tta   3024
    Asp Ile Glu Gly Ala Tyr Gly Thr  Glu Val Phe Tyr Asn  Gln Val Leu
                995                   1000                 1005

    cct act aag tat tta caa cac  ttc caa ata act aag  atg agc gaa       3069
    Pro Thr Lys Tyr Leu Gln His  Phe Gln Ile Thr Lys  Met Ser Glu
        1010                1015                1020

    aat gaa gta tta ttt gaa aat  gga gtt aag gct gtt  aga gaa aac       3114
    Asn Glu Val Leu Phe Glu Asn  Gly Val Lys Ala Val  Arg Glu Asn
        1025                1030                1035

    tct aat ata aat tac tat aga  aat gat aga tta gtt  gct act act       3159
    Ser Asn Ile Asn Tyr Tyr Arg  Asn Asp Arg Leu Val  Ala Thr Thr
        1040                1045                1050

    cca gaa aat tca att gga aat  act ggt ata ggg gat  act caa tta       3204
    Pro Glu Asn Ser Ile Gly Asn  Thr Gly Ile Gly Asp  Thr Gln Leu
        1055                1060                1065

    ttc tta cca tgg aat cct gtt  gat gag gca aat agt  gaa aag ata       3249
    Phe Leu Pro Trp Asn Pro Val  Asp Glu Ala Asn Ser  Glu Lys Ile
        1070                1075                1080

    tat cac tgg aat cca tta gga  act act tca gaa tgg  act tta cca       3294
    Tyr His Trp Asn Pro Leu Gly  Thr Thr Ser Glu Trp  Thr Leu Pro
        1085                1090                1095

    gaa gga tgg act tct aat gac  aaa gtt tat tta tat  gaa tta tca       3339
    Glu Gly Trp Thr Ser Asn Asp  Lys Val Tyr Leu Tyr  Glu Leu Ser
        1100                1105                1110

    gat tta gga aga act tta gtt  aag gaa gta cca gtt  gta gat gga       3384
    Asp Leu Gly Arg Thr Leu Val  Lys Glu Val Pro Val  Val Asp Gly
        1115                1120                1125

    aaa gtt aat tta gaa gta aaa  caa gat act cct tat  ata gtt act       3429
    Lys Val Asn Leu Glu Val Lys  Gln Asp Thr Pro Tyr  Ile Val Thr
         1130               1135                1140

    aag gaa aaa gtt gaa gag aag  aga ata gag gat tgg  gga tat ggc       3474
    Lys Glu Lys Val Glu Glu Lys  Arg Ile Glu Asp Trp  Gly Tyr Gly
        1145                1150                1155

    tca gaa ata gct gat cca gga  ttt gat tct caa act  ttt gat aag       3519
    Ser Glu Ile Ala Asp Pro Gly  Phe Asp Ser Gln Thr  Phe Asp Lys
        1160                1165                1170

    tgg aat aaa gaa tct act gct  gaa aat aca gat cat  ata act att       3564
    Trp Asn Lys Glu Ser Thr Ala  Glu Asn Thr Asp His  Ile Thr Ile
        1175                1180                1185

    gaa aat gaa agt gtt caa aag  aga tta ggt aat gat  gtt ctt aaa       3609
    Glu Asn Glu Ser Val Gln Lys  Arg Leu Gly Asn Asp  Val Leu Lys
        1190                1195                1200

    ata agt gga aat gaa ggc gct  gat gct aaa att tct  caa agc ata       3654
    Ile Ser Gly Asn Glu Gly Ala  Asp Ala Lys Ile Ser  Gln Ser Ile
        1205                1210                1215

    agt gga tta gaa gaa gga gta  act tat tca gta tct  gca tgg gtt       3699
    Ser Gly Leu Glu Glu Gly Val  Thr Tyr Ser Val Ser  Ala Trp Val
        1220                1225                1230

    aaa aat gat aat aat aga gaa  gtt aca cta gga gtt  aat gtt ggt       3744
    Lys Asn Asp Asn Asn Arg Glu  Val Thr Leu Gly Val  Asn Val Gly
        1235                1240                1245

    gga aaa gat ttc act aat gta  ata aca agt ggt ggt  aaa gta aga       3789
    Gly Lys Asp Phe Thr Asn Val  Ile Thr Ser Gly Gly  Lys Val Arg
        1250                1255                1260

    caa gga gaa ggt gtt aag tat  att gac gat act ttc  gtg aga atg       3834
    Gln Gly Glu Gly Val Lys Tyr  Ile Asp Asp Thr Phe  Val Arg Met
```

```
        1265                    1270                    1275
gaa gta  gaa ttt act gta cct  aaa gga gta aat tca  gct gat gtt      3879
Glu Val  Glu Phe Thr Val Pro  Lys Gly Val Asn Ser  Ala Asp Val
    1280                1285                1290

tac tta  aag gct tca gaa gga  gat gct gac tca gtt  gta cta gtt      3924
Tyr Leu  Lys Ala Ser Glu Gly  Asp Ala Asp Ser Val  Val Leu Val
    1295                1300                1305

gat gac  ttt aga ata tgg gat  cat cca gga cac act  aat aga gat      3969
Asp Asp  Phe Arg Ile Trp Asp  His Pro Gly His Thr  Asn Arg Asp
    1310                1315                1320

gga tat  gta ttc tac gaa gat  ttt gaa aat gta gat  gaa ggt ata      4014
Gly Tyr  Val Phe Tyr Glu Asp  Phe Glu Asn Val Asp  Glu Gly Ile
    1325                1330                1335

tca cca  ttt tat tta tct cca  gga aga gga cat tca  aat aga tct      4059
Ser Pro  Phe Tyr Leu Ser Pro  Gly Arg Gly His Ser  Asn Arg Ser
    1340                1345                1350

cac tta  gct gag aag gat ata  tct ata gat gct aat  caa aga atg      4104
His Leu  Ala Glu Lys Asp Ile  Ser Ile Asp Ala Asn  Gln Arg Met
    1355                1360                1365

aac tgg  gta ctt gat gga aga  ttc tca tta aaa tct  aac caa caa      4149
Asn Trp  Val Leu Asp Gly Arg  Phe Ser Leu Lys Ser  Asn Gln Gln
    1370                1375                1380

cca aag  gaa ata ggt gaa atg  ttg act acg gat gtt  tca tca ttc      4194
Pro Lys  Glu Ile Gly Glu Met  Leu Thr Thr Asp Val  Ser Ser Phe
    1385                1390                1395

aaa tta  gaa cca aat aaa act  tat gaa ttt gga ttc  tta tat tca      4239
Lys Leu  Glu Pro Asn Lys Thr  Tyr Glu Phe Gly Phe  Leu Tyr Ser
    1400                1405                1410

tta gaa  aat gct gct cca gga  tat tct gta aat att  aag aat aga      4284
Leu Glu  Asn Ala Ala Pro Gly  Tyr Ser Val Asn Ile  Lys Asn Arg
    1415                1420                1425

gat ggt  gaa aag att gta agt  att cct tta gag gct  act ggt tca      4329
Asp Gly  Glu Lys Ile Val Ser  Ile Pro Leu Glu Ala  Thr Gly Ser
    1430                1435                1440

aat tat  gca caa gat att ttc  act aaa act aaa tct  gta act cac      4374
Asn Tyr  Ala Gln Asp Ile Phe  Thr Lys Thr Lys Ser  Val Thr His
    1445                1450                1455

gag ttt  aca act gga gat ttt  gct gga gat tac tat  att act tta      4419
Glu Phe  Thr Thr Gly Asp Phe  Ala Gly Asp Tyr Tyr  Ile Thr Leu
    1460                1465                1470

gaa aaa  ggt gat gga ttt aaa  gaa gta atc tta gat  aat att tat      4464
Glu Lys  Gly Asp Gly Phe Lys  Glu Val Ile Leu Asp  Asn Ile Tyr
    1475                1480                1485

gtt aag  gaa ata gat aag tca  att gaa tca cct gaa  tta gct cat      4509
Val Lys  Glu Ile Asp Lys Ser  Ile Glu Ser Pro Glu  Leu Ala His
    1490                1495                1500

gta aac  tta aat aca gta gaa  cat gac tta gaa gtt  gga caa agc      4554
Val Asn  Leu Asn Thr Val Glu  His Asp Leu Glu Val  Gly Gln Ser
    1505                1510                1515

gtt cca  ttt gct ata aat gca  tta atg aat aat ggt  gca aat gtt      4599
Val Pro  Phe Ala Ile Asn Ala  Leu Met Asn Asn Gly  Ala Asn Val
    1520                1525                1530

aac tta  gaa gaa gct gaa gtt  gaa tat aaa gtt tca  aaa cca gaa      4644
Asn Leu  Glu Glu Ala Glu Val  Glu Tyr Lys Val Ser  Lys Pro Glu
    1535                1540                1545

gtt tta  act att gaa aat gga  atg atg act gga gct  tct gaa ggc      4689
Val Leu  Thr Ile Glu Asn Gly  Met Met Thr Gly Ala  Ser Glu Gly
```

63

```
                1550                  1555                  1560
ttt act gat gtc caa gta aat  att act gtt aat gga  aat aaa gtt        4734
Phe Thr Asp Val Gln Val Asn  Ile Thr Val Asn Gly  Asn Lys Val
1565                  1570                  1575

tct tca aat aca gta aga gtt  aag gtt gga aat cca  gaa gtt gag        4779
Ser Ser Asn Thr Val Arg Val  Lys Val Gly Asn Pro  Glu Val Glu
1580                  1585                  1590

gaa gaa gaa gtt ata gta aac  cca gta aga aac ttt  aag gtt act        4824
Glu Glu Glu Val Ile Val Asn  Pro Val Arg Asn Phe  Lys Val Thr
1595                  1600                  1605

gat aag act aag aag aat gta  act gta agc tgg gaa  gag cca gaa        4869
Asp Lys Thr Lys Lys Asn Val  Thr Val Ser Trp Glu  Glu Pro Glu
1610                  1615                  1620

aaa act tat gga tta gaa ggc  tat gtt ctt tat aaa  gat ggt aag        4914
Lys Thr Tyr Gly Leu Glu Gly  Tyr Val Leu Tyr Lys  Asp Gly Lys
1625                  1630                  1635

aaa gtt aaa gaa ata ggt gct  gat aaa act gaa ttt  aca ttt aag        4959
Lys Val Lys Glu Ile Gly Ala  Asp Lys Thr Glu Phe  Thr Phe Lys
1640                  1645                  1650

gga tta aac aga cac act att  tat aac ttt aag att  gct gct aaa        5004
Gly Leu Asn Arg His Thr Ile  Tyr Asn Phe Lys Ile  Ala Ala Lys
1655                  1660                  1665

tat tct aat ggt gaa ctt tca  act aag gaa tca ata  act gtt aga        5049
Tyr Ser Asn Gly Glu Leu Ser  Thr Lys Glu Ser Ile  Thr Val Arg
1670                  1675                  1680

act gca aga taa                                                      5061
Thr Ala Arg
1685
```

<210> 8
<211> 1686
<212> PRT
<213> Clostridium perfringens str. 13

<400> 8

```
Met Gly Arg Lys Cys Met Asn Lys Lys Ile Ala Ala Ile Ile Ala Ala
1               5                   10                  15

Ala Val Ile Val Gly Gln Leu Pro Ile Ser Val Leu Ala Thr Pro Val
            20              25                  30

Asn Glu Ala Gly Asp Glu Ile Asn Ser Glu Ser Ala Glu Ile Leu Thr
        35              40                  45

Asn Ser Asp Glu Glu Ala Glu Ala Tyr Ile Gln Asn Tyr Asp Arg Pro
    50              55                  60

Glu Gly Ile Thr Trp Thr Lys Leu Ala Gly Ser Gly Ser Val Glu Val
65              70              75                  80

Thr Asp Gly Phe Leu Ser Val Thr Asn Asn Gly Asp Tyr Arg Ile Met
            85              90                  95

Glu Asp Gln Ser Pro Asn Ile Lys Asn Gly Glu Leu Glu Ser Lys Phe
            100             105                 110

Thr Val Gly Gly Ser Gln Thr Gly Ile Ile Phe Arg Ala Thr Glu Ser
        115             120                 125
```

Asn Tyr Gly Met Ile Asn Tyr Asn Ser Gly Thr Gly Trp Val Ile Glu
    130              135              140

Asn Lys Asn Ser Trp Glu Asp Ile Thr Gly Pro Lys Leu Asn Asn Gly
145              150              155              160

Asp Val Val Thr Val Lys Ala Thr Phe Val Glu Lys His Leu Thr Val
              165              170              175

Asn Val Ser Val Asn Asp Gly Glu Phe Glu Thr Ile Tyr Asp Lys Glu
          180              185              190

Ser Asp Leu Ile Pro Leu Gln Ala Gly Lys Val Gly Tyr Arg Gly Trp
        195              200              205

Gly Asn Ala Lys Thr Thr Lys Phe Asp Tyr Ile Lys Tyr Ala Pro Met
    210              215              220

Thr Ile Asp Lys Gly Pro Ile Val Ser Ile Asn Glu Val Asn Val Glu
225              230              235              240

Thr Tyr Pro Arg Val Lys Pro Ile Leu Pro Ser Ser Val Thr Val Asn
              245              250              255

His Glu Asn Gly Met Ser Ser Ile Lys Asp Val Ser Trp Asn Tyr Ile
              260              265              270

Pro Lys Glu Ser Tyr Ser Lys Pro Gly Thr Phe Lys Val Glu Gly Thr
        275              280              285

Val Glu Gly Thr Asp Val Lys Ala Ile Ala Asn Val Thr Val Ser Ser
    290              295              300

Asp Leu Ala Tyr Tyr Glu Thr Asn Phe Glu Thr Glu Glu Thr Arg Gly
305              310              315              320

Asp Trp Gln Val Val Gln Gly Gly Gly Ser Pro Ser Tyr Glu Glu Gly
              325              330              335

Lys Val Lys Ile Pro Met Asn Gly Val Ser Ile Ala Val Asp Met Asn
              340              345              350

Ser Pro Glu Val Lys Asn Phe Thr Tyr Glu Thr Asp Phe Ser Val Asp
        355              360              365

Asn Asn Gly Gly Arg Ile Gly Leu Leu Phe Arg Tyr Val Ser Glu Thr
    370              375              380

Glu Trp Gly Ala Val Cys Tyr Asp Asn Gly Ser Trp Val Trp Lys Thr
385              390              395              400

Gly Asp Gly Lys Tyr Gly Asn Phe Pro Gly Thr Phe Thr Pro Glu Pro
              405              410              415

Gly Lys Thr Tyr Arg Ile Lys Leu Lys Val Glu Asp Thr Asn Ile Thr
              420              425              430

66

Met Trp Val Asp Gly Glu Lys Ile Gly Gln Val Ala Val Ser Asn Leu
435                440              445

Pro Asp Val Arg Gly Lys Val Gly Leu Thr Gly Trp Phe Gly Asn Lys
450              455              460

Asn Val Thr Leu Asp Asn Leu Val Val Glu Glu Leu Gly Gly Ile Met
465              470              475              480

Ala Pro Glu Val Gly Pro Leu Glu Glu Gln Ser Ile Glu Ser Asp Ser
485              490              495

Met Lys Val Val Leu Asp Asn Arg Phe Pro Thr Val Ile Arg Tyr Glu
500              505              510

Trp Lys Gly Thr Glu Asp Val Leu Ser Gly Ala Ser Val Asp Asp Leu
515              520              525

Glu Ala Gln Tyr Met Val Glu Ile Asn Gly Glu Lys Arg Ile Pro Lys
530              535              540

Val Thr Ser Glu Phe Ala Asn Asn Glu Gly Ile Tyr Thr Leu Asn Phe
545              550              555              560

Glu Asp Ile Gly Met Thr Ile Thr Leu Lys Met Thr Val Asn Glu Asn
565              570              575

Lys Leu Arg Met Glu Val Thr Asp Ile Gln Glu Gly Asp Val Lys Leu
580              585              590

Gln Thr Leu Asn Phe Pro Asn His Ser Leu Ala Ser Val Ser Ser Leu
595              600              605

Asn Asn Gly Lys Thr Ala Ser Val Leu Thr Thr Gly Asp Trp Asn Asn
610              615              620

Ile Asn Glu Glu Phe Thr Asp Val Ala Lys Ala Lys Pro Gly Val Lys
625              630              635              640

Gly Lys Thr Tyr Ala Phe Ile Asn Asp Asp Lys Phe Ala Val Thr Ile
645              650              655

Asn Asn Asn Thr Ile Glu Gly Gly Asn Arg Val Val Leu Thr Thr Glu
660              665              670

Asn Asp Thr Leu Pro Asp Asn Thr Asn Tyr Lys Lys Val Gly Ile Ser
675              680              685

Asn Gly Thr Trp Thr Tyr Lys Glu Ile Leu Gln Asp Thr Thr Asp Gln
690              695              700

Gly Ser Lys Leu Tyr Gln Gly Glu Lys Pro Trp Ser Glu Val Ile Ile
705              710              715              720

Ala Arg Asp Glu Asn Glu Asp Gly Gln Val Asp Trp Gln Asp Gly Ala
725              730              735

67

```
Ile Gln Tyr Arg Lys Asn Met Lys Ile Pro Val Gly Gly Glu Glu Ile
         740               745               750

Lys Asn Gln Met Ser Tyr Ile Asp Phe Asn Ile Gly Tyr Thr Gln Asn
         755               760               765

Pro Phe Leu Arg Ser Leu Asp Thr Ile Lys Lys Leu Ser Asn Tyr Thr
770               775               780

Asp Gly Phe Gly Gln Leu Val Leu His Lys Gly Tyr Gln Gly Glu Gly
785               790               795               800

His Asp Asp Ser His Pro Asp Tyr Gly Gly His Ile Gly Met Arg Gln
             805               810               815

Gly Gly Lys Glu Asp Phe Asn Thr Leu Ile Glu Gln Gly Lys Glu Tyr
         820               825               830

Asn Ala Lys Ile Gly Val His Ile Asn Ala Thr Glu Tyr Thr Met Asp
         835               840               845

Ala Phe Glu Tyr Pro Thr Glu Leu Val Asn Glu Asn Ala Pro Gly Trp
    850               855               860

Gly Trp Leu Asp Gln Ala Tyr Tyr Val Asn Gln Arg Gly Asp Ile Thr
865               870               875               880

Ser Gly Glu Leu Phe Arg Arg Leu Asp Met Leu Met Glu Asp Ala Pro
             885               890               895

Glu Leu Gly Trp Ile Tyr Val Asp Val Tyr Thr Gly Asn Gly Trp Asn
         900               905               910

Ala His Gln Leu Gly Glu Lys Ile Asn Asp Tyr Gly Ile Met Ile Ala
    915               920               925

Thr Glu Met Asn Gly Pro Leu Glu Gln His Val Pro Trp Thr His Trp
    930               935               940

Gly Gly Asp Pro Ala Tyr Pro Asn Lys Gly Asn Ala Ser Lys Ile Met
945               950               955               960

Arg Phe Met Lys Asn Asp Thr Gln Asp Ser Phe Leu Ala Asp Pro Leu
             965               970               975

Val Lys Gly Asn Lys His Leu Leu Ser Gly Gly Trp Gly Thr Arg His
             980               985               990

Asp Ile Glu Gly Ala Tyr Gly Thr Glu Val Phe Tyr Asn Gln Val Leu
    995              1000              1005

Pro Thr Lys Tyr Leu Gln His Phe Gln Ile Thr Lys Met Ser Glu
    1010              1015              1020

Asn Glu Val Leu Phe Glu Asn Gly Val Lys Ala Val Arg Glu Asn
    1025              1030              1035
```

68

EP 1 767 645 B1

```
Ser Asn  Ile Asn Tyr Tyr Arg  Asn Asp Arg Leu Val  Ala Thr Thr
    1040             1045             1050

Pro Glu  Asn Ser Ile Gly Asn  Thr Gly Ile Gly Asp  Thr Gln Leu
    1055             1060             1065

Phe Leu  Pro Trp Asn Pro Val  Asp Glu Ala Asn Ser  Glu Lys Ile
    1070             1075             1080

Tyr His  Trp Asn Pro Leu Gly  Thr Thr Ser Glu Trp  Thr Leu Pro
    1085             1090             1095

Glu Gly  Trp Thr Ser Asn Asp  Lys Val Tyr Leu Tyr  Glu Leu Ser
    1100             1105             1110

Asp Leu  Gly Arg Thr Leu Val  Lys Glu Val Pro Val  Val Asp Gly
    1115             1120             1125

Lys Val  Asn Leu Glu Val Lys  Gln Asp Thr Pro Tyr  Ile Val Thr
    1130             1135             1140

Lys Glu  Lys Val Glu Glu Lys  Arg Ile Glu Asp Trp  Gly Tyr Gly
    1145             1150             1155

Ser Glu  Ile Ala Asp Pro Gly  Phe Asp Ser Gln Thr  Phe Asp Lys
    1160             1165             1170

Trp Asn  Lys Glu Ser Thr Ala  Glu Asn Thr Asp His  Ile Thr Ile
    1175             1180             1185

Glu Asn  Glu Ser Val Gln Lys  Arg Leu Gly Asn Asp  Val Leu Lys
    1190             1195             1200

Ile Ser  Gly Asn Glu Gly Ala  Asp Ala Lys Ile Ser  Gln Ser Ile
    1205             1210             1215

Ser Gly  Leu Glu Glu Gly Val  Thr Tyr Ser Val Ser  Ala Trp Val
    1220             1225             1230

Lys Asn  Asp Asn Asn Arg Glu  Val Thr Leu Gly Val  Asn Val Gly
    1235             1240             1245

Gly Lys  Asp Phe Thr Asn Val  Ile Thr Ser Gly Gly  Lys Val Arg
    1250             1255             1260

Gln Gly  Glu Gly Val Lys Tyr  Ile Asp Asp Thr Phe  Val Arg Met
    1265             1270             1275

Glu Val  Glu Phe Thr Val Pro  Lys Gly Val Asn Ser  Ala Asp Val
    1280             1285             1290

Tyr Leu  Lys Ala Ser Glu Gly  Asp Ala Asp Ser Val  Val Leu Val
    1295             1300             1305

Asp Asp  Phe Arg Ile Trp Asp  His Pro Gly His Thr  Asn Arg Asp
    1310             1315             1320
```

Gly Tyr Val Phe Tyr Glu Asp Phe Glu Asn Val Asp Glu Gly Ile
1325 1330 1335

Ser Pro Phe Tyr Leu Ser Pro Gly Arg Gly His Ser Asn Arg Ser
1340 1345 1350

His Leu Ala Glu Lys Asp Ile Ser Ile Asp Ala Asn Gln Arg Met
1355 1360 1365

Asn Trp Val Leu Asp Gly Arg Phe Ser Leu Lys Ser Asn Gln Gln
1370 1375 1380

Pro Lys Glu Ile Gly Glu Met Leu Thr Thr Asp Val Ser Ser Phe
1385 1390 1395

Lys Leu Glu Pro Asn Lys Thr Tyr Glu Phe Gly Phe Leu Tyr Ser
1400 1405 1410

Leu Glu Asn Ala Ala Pro Gly Tyr Ser Val Asn Ile Lys Asn Arg
1415 1420 1425

Asp Gly Glu Lys Ile Val Ser Ile Pro Leu Glu Ala Thr Gly Ser
1430 1435 1440

Asn Tyr Ala Gln Asp Ile Phe Thr Lys Thr Lys Ser Val Thr His
1445 1450 1455

Glu Phe Thr Thr Gly Asp Phe Ala Gly Asp Tyr Tyr Ile Thr Leu
1460 1465 1470

Glu Lys Gly Asp Gly Phe Lys Glu Val Ile Leu Asp Asn Ile Tyr
1475 1480 1485

Val Lys Glu Ile Asp Lys Ser Ile Glu Ser Pro Glu Leu Ala His
1490 1495 1500

Val Asn Leu Asn Thr Val Glu His Asp Leu Glu Val Gly Gln Ser
1505 1510 1515

Val Pro Phe Ala Ile Asn Ala Leu Met Asn Asn Gly Ala Asn Val
1520 1525 1530

Asn Leu Glu Glu Ala Glu Val Glu Tyr Lys Val Ser Lys Pro Glu
1535 1540 1545

Val Leu Thr Ile Glu Asn Gly Met Met Thr Gly Ala Ser Glu Gly
1550 1555 1560

Phe Thr Asp Val Gln Val Asn Ile Thr Val Asn Gly Asn Lys Val
1565 1570 1575

Ser Ser Asn Thr Val Arg Val Lys Val Gly Asn Pro Glu Val Glu
1580 1585 1590

Glu Glu Glu Val Ile Val Asn Pro Val Arg Asn Phe Lys Val Thr
1595 1600 1605

70

```
Asp Lys Thr Lys Lys Asn Val Thr Val Ser Trp Glu Glu Pro Glu
    1610                1615            1620

Lys Thr Tyr Gly Leu Glu Gly Tyr Val Leu Tyr Lys Asp Gly Lys
    1625            1630                1635

Lys Val Lys Glu Ile Gly Ala Asp Lys Thr Glu Phe Thr Phe Lys
    1640            1645                1650

Gly Leu Asn Arg His Thr Ile Tyr Asn Phe Lys Ile Ala Ala Lys
    1655            1660                1665

Tyr Ser Asn Gly Glu Leu Ser Thr Lys Glu Ser Ile Thr Val Arg
    1670            1675                1680

Thr Ala Arg
    1685
```

<210> 9
<211> 4086
<212> DNA
<213> Streptomyces coelicolor A3(2)

<220>
<221> CDS
<222> (1)..(4086)

<400> 9

```
atg gca gcg atc aca ccg aag cga tcg gtc aga gtg gac acc tcg aca          48
Met Ala Ala Ile Thr Pro Lys Arg Ser Val Arg Val Asp Thr Ser Thr
1               5                   10                  15

ggc tca tca tct ccc cca acc gga cgc gca cgg gtc cgg cgg cac ggc          96
Gly Ser Ser Ser Pro Pro Thr Gly Arg Ala Arg Val Arg Arg His Gly
                20                  25                  30

gcc gtc gtc gcg gcg ctg ggc ctc acc gcg ggc ctg ctg tcg gcc gcc         144
Ala Val Val Ala Ala Leu Gly Leu Thr Ala Gly Leu Leu Ser Ala Ala
            35                  40                  45

gcc ctg ccc gcg ggc gcg gcc ccg ccc cgc ccc gct gcc gct gcg gcg         192
Ala Leu Pro Ala Gly Ala Ala Pro Pro Arg Pro Ala Ala Ala Ala Ala
        50                  55                  60

ccc gcg ggg gca ccg acg ccg gtg gaa ctg agc cgg ggc ggg ctg acc         240
Pro Ala Gly Ala Pro Thr Pro Val Glu Leu Ser Arg Gly Gly Leu Thr
65                  70                  75                  80

gtc acc gtg gcg aag gag ttc ccc cag gtg atc tcc tac cgg ctg ggc         288
Val Thr Val Ala Lys Glu Phe Pro Gln Val Ile Ser Tyr Arg Leu Gly
                85                  90                  95

cgg cgc gga ctc gac ggg cgg gca acg gcg ctg gac ggc ttc acc gtc         336
Arg Arg Gly Leu Asp Gly Arg Ala Thr Ala Leu Asp Gly Phe Thr Val
                100                 105                 110

aac ggc gag agc cac cgc gcc acc acc acc gtg aag gcg aag ggc agc         384
Asn Gly Glu Ser His Arg Ala Thr Thr Thr Val Lys Ala Lys Gly Ser
                115                 120                 125

agg gcg gtc tac acc tcc acg ttc gag gac ctg ccc ggt ctc acg atc         432
Arg Ala Val Tyr Thr Ser Thr Phe Glu Asp Leu Pro Gly Leu Thr Ile
        130                 135                 140

acc tcc agc atc acg gtc acc aag gag acg acg gtc gtc ttc gcc gtc         480
Thr Ser Ser Ile Thr Val Thr Lys Glu Thr Thr Val Val Phe Ala Val
145                 150                 155                 160
```

```
gag aag atc tcg ggc gag gcc gcg ccg ggc gtg cgc acc ctc gcg atc    528
Glu Lys Ile Ser Gly Glu Ala Ala Pro Gly Val Arg Thr Leu Ala Ile
            165                 170                 175

ccc ggc cag tcc ctc gtc tcc gtc gac tcc gcc gag ccg ggc gcc aac    576
Pro Gly Gln Ser Leu Val Ser Val Asp Ser Ala Glu Pro Gly Ala Asn
            180                 185                 190

ctc gcc cgg acg aag atc tcc acc gac tcg acg acg acc gcc gac cgc    624
Leu Ala Arg Thr Lys Ile Ser Thr Asp Ser Thr Thr Thr Ala Asp Arg
            195                 200                 205

ttc gtc ccc gtc acc ggc gac acc gcc ccg gac aag ggg ccc gtc ggc    672
Phe Val Pro Val Thr Gly Asp Thr Ala Pro Asp Lys Gly Pro Val Gly
            210                 215                 220

acc ccg tac gcg ttc gtc ggc aac gcg cag ttg tcg gcg ggc atc atc    720
Thr Pro Tyr Ala Phe Val Gly Asn Ala Gln Leu Ser Ala Gly Ile Ile
225                 230                 235                 240

acc aac gcg acc gag gac tcg ccg cag gac gac aac acc gac tgg aac    768
Thr Asn Ala Thr Glu Asp Ser Pro Gln Asp Asp Asn Thr Asp Trp Asn
                245                 250                 255

acc cgc ctg cag tcc cgc atc gtc gac gag ggc gag gga cgg cgc cgg    816
Thr Arg Leu Gln Ser Arg Ile Val Asp Glu Gly Glu Gly Arg Arg Arg
            260                 265                 270

gcg gag ctg tcg gcc ggc acg tac acc tac cac ccc gag ggc gcc acc    864
Ala Glu Leu Ser Ala Gly Thr Tyr Thr Tyr His Pro Glu Gly Ala Thr
            275                 280                 285

gat cca cgg gtc gac acc tac gag ctg ccc cgg gcg acg gtg gtc ctc    912
Asp Pro Arg Val Asp Thr Tyr Glu Leu Pro Arg Ala Thr Val Val Leu
            290                 295                 300

gcc gcc gac gcc aac cgg gac ggc acg gtc gac tgg cag gac ggc gcc    960
Ala Ala Asp Ala Asn Arg Asp Gly Thr Val Asp Trp Gln Asp Gly Ala
305                 310                 315                 320

atc gcc cac cgg gag cac atg cgc cgc ccg ctc ggc gcc gac cgg gtg    1008
Ile Ala His Arg Glu His Met Arg Arg Pro Leu Gly Ala Asp Arg Val
                325                 330                 335

ccc gaa cgc gtg gtc cag cgc atc ccg ttc aac ttc gcg agc cag gcc    1056
Pro Glu Arg Val Val Gln Arg Ile Pro Phe Asn Phe Ala Ser Gln Ala
            340                 345                 350

acc aac ccg ttc ctg aag acg ctg gac aac acc aag cgc atc tcc atg    1104
Thr Asn Pro Phe Leu Lys Thr Leu Asp Asn Thr Lys Arg Ile Ser Met
            355                 360                 365

gcc acc gac gac ctc ggg cag tgg gtg ctg gag aag ggg tac gcg agc    1152
Ala Thr Asp Asp Leu Gly Gln Trp Val Leu Glu Lys Gly Tyr Ala Ser
            370                 375                 380

gag ggc cac gac tcc gcc cac ccc gac tac ggc ggc aac gag aac gtc    1200
Glu Gly His Asp Ser Ala His Pro Asp Tyr Gly Gly Asn Glu Asn Val
385                 390                 395                 400

cgc gcg ggc ggc tgg aag gac ctg aac cgc ctc acc cgg acg gga gcc    1248
Arg Ala Gly Gly Trp Lys Asp Leu Asn Arg Leu Thr Arg Thr Gly Ala
                405                 410                 415

ggc tac aac gcg gac ttc gcc gtc cac gtc aac gcc acg gag gcc tac    1296
Gly Tyr Asn Ala Asp Phe Ala Val His Val Asn Ala Thr Glu Ala Tyr
            420                 425                 430

gcc cag gcc agg acc ttc acc gag gac atg gtc gcg ggc cag gcc gac    1344
Ala Gln Ala Arg Thr Phe Thr Glu Asp Met Val Ala Gly Gln Ala Asp
            435                 440                 445

ggc tgg gac tgg ctc aac cag gcc tac cac atc gac cag cgc aag gac    1392
Gly Trp Asp Trp Leu Asn Gln Ala Tyr His Ile Asp Gln Arg Lys Asp
            450                 455                 460
```

```
ctg ggc acc ggc gcc gtc ctc gac cgc ttc aag cag ctc cgc aag gaa       1440
Leu Gly Thr Gly Ala Val Leu Asp Arg Phe Lys Gln Leu Arg Lys Glu
465             470             475             480

gca ccg ggc atc aga acc gtc tac atc gac gcc tac tac tcc agc ggc       1488
Ala Pro Gly Ile Arg Thr Val Tyr Ile Asp Ala Tyr Tyr Ser Ser Gly
                485             490             495

tgg ctg gcc gac ggt ctg gcc gcc ggg ctg cgt gag atg ggc ttc gag       1536
Trp Leu Ala Asp Gly Leu Ala Ala Gly Leu Arg Glu Met Gly Phe Glu
            500             505             510

gtc gcc acc gag tgg gcg tac aag ttc gag ggc acc tcg gtg tgg tcg       1584
Val Ala Thr Glu Trp Ala Tyr Lys Phe Glu Gly Thr Ser Val Trp Ser
            515             520             525

cac tgg gcg gcc gac aag aac tac ggc ggc gcc acg aac aag ggc atc       1632
His Trp Ala Ala Asp Lys Asn Tyr Gly Gly Ala Thr Asn Lys Gly Ile
            530             535             540

aac tcg gac atc gtc cgg ttc atc gcc aac gcc gac cgc gac gtg tgg       1680
Asn Ser Asp Ile Val Arg Phe Ile Ala Asn Ala Asp Arg Asp Val Trp
545             550             555             560

aac gtg gac ccg ctg ctc ggc ggc gcg agc gtc gtc gag ttc gag ggc       1728
Asn Val Asp Pro Leu Leu Gly Gly Ala Ser Val Val Glu Phe Glu Gly
            565             570             575

tgg acc ggc cag gac gac tgg aac gcc ttc tac cgc aac atc tgg acc       1776
Trp Thr Gly Gln Asp Asp Trp Asn Ala Phe Tyr Arg Asn Ile Trp Thr
            580             585             590

gac aac ctg ccg acc aag ttc ctc cag cac ttc cag gtg ctg gac tgg       1824
Asp Asn Leu Pro Thr Lys Phe Leu Gln His Phe Gln Val Leu Asp Trp
            595             600             605

gac cgc ggc agg tcc gcg agg ctc acc ggc ggg gtg gac gtg aag tcc       1872
Asp Arg Gly Arg Ser Ala Arg Leu Thr Gly Gly Val Asp Val Lys Ser
            610             615             620

gtc gac ggc gag cgg cgg atc tcc atg gac ggc acc gag gtc ctc aag       1920
Val Asp Gly Glu Arg Arg Ile Ser Met Asp Gly Thr Glu Val Leu Lys
625             630             635             640

ggc gac acc tac ctg ctg ccc tgg cag aac gcc ggg aag gac gac ggc       1968
Gly Asp Thr Tyr Leu Leu Pro Trp Gln Asn Ala Gly Lys Asp Asp Gly
            645             650             655

acc tcg tcg ccc cgc gac gcc gac aag atg tac ttc tac agc gcc tcc       2016
Thr Ser Ser Pro Arg Asp Ala Asp Lys Met Tyr Phe Tyr Ser Ala Ser
            660             665             670

ggc ggc gag cac acc ttc gaa ctg acc gga cag ttc gcg ggc acc gag       2064
Gly Gly Glu His Thr Phe Glu Leu Thr Gly Gln Phe Ala Gly Thr Glu
            675             680             685

gac ttc acc ctc tac gaa ctc acc gac cag ggc cgg gcg gag aag gcc       2112
Asp Phe Thr Leu Tyr Glu Leu Thr Asp Gln Gly Arg Ala Glu Lys Ala
            690             695             700

cgg gtg acg gcc cac gag ggg cgg gtg acc ctc acc gcc gag aag ggg       2160
Arg Val Thr Ala His Glu Gly Arg Val Thr Leu Thr Ala Glu Lys Gly
705             710             715             720

cag ccc tac gtc ctc gtg ccg aac ggc ggc agg gca ccg cac cgc gac       2208
Gln Pro Tyr Val Leu Val Pro Asn Gly Gly Arg Ala Pro His Arg Asp
            725             730             735

gcc cac tac ggc gag ttc acc ggg ctg tcc gac ccc ggc ttc aac ggc       2256
Ala His Tyr Gly Glu Phe Thr Gly Leu Ser Asp Pro Gly Phe Asn Gly
            740             745             750

ggg gac ctc gac gcc tgg aac gcg agc ggc ggc gcg gag atc gtc cgg       2304
Gly Asp Leu Asp Ala Trp Asn Ala Ser Gly Gly Ala Glu Ile Val Arg
            755             760             765
```

74

```
gcc ggc aac ggg gac aac gtg gtc cgc ctg ggc gag gac gcc tcg ggc    2352
Ala Gly Asn Gly Asp Asn Val Val Arg Leu Gly Glu Asp Ala Ser Gly
    770              775              780

atc gcg cag cgg gtc cgc ggc ctg acc ccg ggc gag cgg tac acg ctc    2400
Ile Ala Gln Arg Val Arg Gly Leu Thr Pro Gly Glu Arg Tyr Thr Leu
785              790              795              800

ggc gcg gac gtc ggg atc ggc ccc ggc gag cgc cgg gag acg acg ctg    2448
Gly Ala Asp Val Gly Ile Gly Pro Gly Glu Arg Arg Glu Thr Thr Leu
                805              810              815

cgg gtg cgc ggc ggc aag gac agc gag gcc agg acc ttc gac atc acg    2496
Arg Val Arg Gly Gly Lys Asp Ser Glu Ala Arg Thr Phe Asp Ile Thr
            820              825              830

ccg gcg cgg aac agg atg gcg tcc gac gag aag cga gac acg tac tcc    2544
Pro Ala Arg Asn Arg Met Ala Ser Asp Glu Lys Arg Asp Thr Tyr Ser
        835              840              845

cag cgg gcc tcg gtc tcc ttc acc gcg ccg cgc gac ggc tcg gtc acc    2592
Gln Arg Ala Ser Val Ser Phe Thr Ala Pro Arg Asp Gly Ser Val Thr
    850              855              860

gtg gag ctc ggg gcg gtc gcc ggt ggc gcc ccg gtg gtc ctg gac gac    2640
Val Glu Leu Gly Ala Val Ala Gly Gly Ala Pro Val Val Leu Asp Asp
865              870              875              880

gta cgg gtc atg gtg gac acc acc gct ccg ctc ccc cgc tcc cag gac    2688
Val Arg Val Met Val Asp Thr Thr Ala Pro Leu Pro Arg Ser Gln Asp
                885              890              895

ggc acg gtc gtc gcc cac gac gac ttc gag ggc aac cgg ccc ggc tgg    2736
Gly Thr Val Val Ala His Asp Asp Phe Glu Gly Asn Arg Pro Gly Trp
            900              905              910

gga ccg ttc gtc aag ggc gac gcc ggc ggc gtc acc gac ccc cgc acc    2784
Gly Pro Phe Val Lys Gly Asp Ala Gly Gly Val Thr Asp Pro Arg Thr
        915              920              925

agc atc agc gac ctg cac gcg ccc tac agc cag aag gag tgg aag aac    2832
Ser Ile Ser Asp Leu His Ala Pro Tyr Ser Gln Lys Glu Trp Lys Asn
    930              935              940

acc tac tcg ccc tac gac acc ggc gcg ctg aag ggc agg gcc gtc gac    2880
Thr Tyr Ser Pro Tyr Asp Thr Gly Ala Leu Lys Gly Arg Ala Val Asp
945              950              955              960

gac gtg ctc gcg ggc cgg cac tcg ctg aag tcc cac gcc gag aac acc    2928
Asp Val Leu Ala Gly Arg His Ser Leu Lys Ser His Ala Glu Asn Thr
                965              970              975

ggg ctg gtc cac cgc acg acc cct gcc acc gtg ccg ttc gag gag ggc    2976
Gly Leu Val His Arg Thr Thr Pro Ala Thr Val Pro Phe Glu Glu Gly
            980              985              990

cac cgg tac cgg gtc tcc ttc tcg  tac cag acc aac gtc   gag ggc cag    3024
His Arg Tyr Arg Val Ser Phe Ser  Tyr Gln Thr Asn Val   Glu Gly Gln
        995              1000            1005

tgg gcc  tgg gtc acc ggc gcg  gac cgg gtc gcc gac  ggg acg acg    3069
Trp Ala  Trp Val Thr Gly Ala  Asp Arg Val Ala Asp  Gly Thr Thr
    1010            1015            1020

acc tcg  cgg gac atc acc cgt  gac gtc ctg gca ccc  gcc ctg gac    3114
Thr Ser  Arg Asp Ile Thr Arg  Asp Val Leu Ala Pro  Ala Leu Asp
    1025            1030            1035

acg gcg  gcc tac tcc cgc gag  ttc gtc gcc ggc tgc  ggg gac acc    3159
Thr Ala  Ala Tyr Ser Arg Glu  Phe Val Ala Gly Cys  Gly Asp Thr
    1040            1045            1050

tgg gtc  ggg ctg cgc aga ctc  ggc agc gcc cgg ggc  acc gat ctc    3204
Trp Val  Gly Leu Arg Arg Leu  Gly Ser Ala Arg Gly  Thr Asp Leu
    1055            1060            1065
```

```
gtc ctc gac gac ttc acg gtg  acc gac ctc ggc gag  gcg gac acc      3249
Val Leu Asp Asp Phe Thr Val  Thr Asp Leu Gly Glu  Ala Asp Thr
    1070            1075           1080

ggg gcc gcc tgc gcc gct gtc  acg gcc ccg tcc ggc  gcc gaa ctg      3294
Gly Ala Ala Cys Ala Ala Val  Thr Ala Pro Ser Gly  Ala Glu Leu
    1085            1090           1095

agc ccc ggg gtc ccc ggt gag  tac gtc acg gcg ttc  acc aac cac      3339
Ser Pro Gly Val Pro Gly Glu  Tyr Val Thr Ala Phe  Thr Asn His
    1100            1105           1110

gag tcc gcc ggc gcc gag aac  gtg ggc atc gcc ctc  cag ggt ctg      3384
Glu Ser Ala Gly Ala Glu Asn  Val Gly Ile Ala Leu  Gln Gly Leu
    1115            1120           1125

ccc gag ggc tgg aag gcc gag  gtg aag gag aag gac  ggc aat ctc      3429
Pro Glu Gly Trp Lys Ala Glu  Val Lys Glu Lys Asp  Gly Asn Leu
    1130            1135           1140

ttc gag cgc gtg cag ccg ggc  gcg acc gtg cgc acc  acc tgg ctc      3474
Phe Glu Arg Val Gln Pro Gly  Ala Thr Val Arg Thr  Thr Trp Leu
    1145            1150           1155

ctc acc ccg ccc gcc ggc acg  gcc ggc acc tcc gcc  acg tgg cag      3519
Leu Thr Pro Pro Ala Gly Thr  Ala Gly Thr Ser Ala  Thr Trp Gln
    1160            1165           1170

gtg acg gcc gcc tac gca cac  gag gga gcc acc agg  acg gtc tcc      3564
Val Thr Ala Ala Tyr Ala His  Glu Gly Ala Thr Arg  Thr Val Ser
    1175            1180           1185

acc ggg gcc cgc gcc gcg gtg  acc gac gaa ccg gta  ctg gcc ccg      3609
Thr Gly Ala Arg Ala Ala Val  Thr Asp Glu Pro Val  Leu Ala Pro
    1190            1195           1200

gct tcg acg acc gcg acc gcc  gac tcg gag aac acc  tcg tcg ggc      3654
Ala Ser Thr Thr Ala Thr Ala  Asp Ser Glu Asn Thr  Ser Ser Gly
    1205            1210           1215

gcc tcc gaa ggg ccg gtg tcc  aac gtc ctc gac ggt  gac gcc ggc      3699
Ala Ser Glu Gly Pro Val Ser  Asn Val Leu Asp Gly  Asp Ala Gly
    1220            1225           1230

acc atc tgg cac acc gac tac  acc acc tcc cag gcg  ccg tat ccg      3744
Thr Ile Trp His Thr Asp Tyr  Thr Thr Ser Gln Ala  Pro Tyr Pro
    1235            1240           1245

cac tgg gtg acg ctg aag ctc  ggc ggc gcc gcc gac  gtc gac ggg      3789
His Trp Val Thr Leu Lys Leu  Gly Gly Ala Ala Asp  Val Asp Gly
    1250            1255           1260

ttc ggg tac ctg ggc cga cag  agc ggg ggc ccg aac  gga cgc gtc      3834
Phe Gly Tyr Leu Gly Arg Gln  Ser Gly Gly Pro Asn  Gly Arg Val
    1265            1270           1275

gcc gac tac gag gtc gcc gtg  tcg gac gac ggc gag  gcg tgg acg      3879
Ala Asp Tyr Glu Val Ala Val  Ser Asp Asp Gly Glu  Ala Trp Thr
    1280            1285           1290

acg gtg gcc acc ggc acc ctg  aag gac gtc ccg cgg  acc cag cgc      3924
Thr Val Ala Thr Gly Thr Leu  Lys Asp Val Pro Arg  Thr Gln Arg
    1295            1300           1305

gtc tcc ttc gac cgg gtc cgc  gcc tcc tac gtg cgc  ttc acc gcc      3969
Val Ser Phe Asp Arg Val Arg  Ala Ser Tyr Val Arg  Phe Thr Ala
    1310            1315           1320

ctc gac gcg ctc aac ggg cag  ccc tac gcg gcc gcc  gcg gag atg      4014
Leu Asp Ala Leu Asn Gly Gln  Pro Tyr Ala Ala Ala  Ala Glu Met
    1325            1330           1335

cgc gtg tac ggc gtg ccc gtg  gac ctg ccc acg ggc  tac ccg ccg      4059
Arg Val Tyr Gly Val Pro Val  Asp Leu Pro Thr Gly  Tyr Pro Pro
    1340            1345           1350
```

```
              ggc gaa  cgc ccc gcc gac gcc  cgc tga                        4086
              Gly Glu  Arg Pro Ala Asp Ala  Arg
                  1355                1360
```

<210> 10
<211> 1361
<212> PRT
<213> Streptomyces coelicolor A3(2)

<400> 10

```
Met Ala Ala Ile Thr Pro Lys Arg Ser Val Arg Val Asp Thr Ser Thr
1               5               10              15

Gly Ser Ser Ser Pro Pro Thr Gly Arg Ala Arg Val Arg Arg His Gly
            20              25                      30

Ala Val Val Ala Ala Leu Gly Leu Thr Ala Gly Leu Leu Ser Ala Ala
        35              40              45

Ala Leu Pro Ala Gly Ala Ala Pro Pro Arg Pro Ala Ala Ala Ala Ala
    50              55              60

Pro Ala Gly Ala Pro Thr Pro Val Glu Leu Ser Arg Gly Gly Leu Thr
65              70              75              80

Val Thr Val Ala Lys Glu Phe Pro Gln Val Ile Ser Tyr Arg Leu Gly
            85              90              95

Arg Arg Gly Leu Asp Gly Arg Ala Thr Ala Leu Asp Gly Phe Thr Val
        100             105             110

Asn Gly Glu Ser His Arg Ala Thr Thr Thr Val Lys Ala Lys Gly Ser
    115             120             125

Arg Ala Val Tyr Thr Ser Thr Phe Glu Asp Leu Pro Gly Leu Thr Ile
    130             135             140

Thr Ser Ser Ile Thr Val Thr Lys Glu Thr Thr Val Val Phe Ala Val
145             150             155             160

Glu Lys Ile Ser Gly Glu Ala Ala Pro Gly Val Arg Thr Leu Ala Ile
            165             170             175

Pro Gly Gln Ser Leu Val Ser Val Asp Ser Ala Glu Pro Gly Ala Asn
        180             185             190

Leu Ala Arg Thr Lys Ile Ser Thr Asp Ser Thr Thr Thr Ala Asp Arg
    195             200             205

Phe Val Pro Val Thr Gly Asp Thr Ala Pro Asp Lys Gly Pro Val Gly
    210             215             220

Thr Pro Tyr Ala Phe Val Gly Asn Ala Gln Leu Ser Ala Gly Ile Ile
225             230             235             240

Thr Asn Ala Thr Glu Asp Ser Pro Gln Asp Asp Asn Thr Asp Trp Asn
            245             250             255
```

Thr Arg Leu Gln Ser Arg Ile Val Asp Glu Gly Glu Gly Arg Arg Arg
        260           265           270

Ala Glu Leu Ser Ala Gly Thr Tyr Thr Tyr His Pro Glu Gly Ala Thr
        275           280           285

Asp Pro Arg Val Asp Thr Tyr Glu Leu Pro Arg Ala Thr Val Val Leu
        290           295           300

Ala Ala Asp Ala Asn Arg Asp Gly Thr Val Asp Trp Gln Asp Gly Ala
305           310           315           320

Ile Ala His Arg Glu His Met Arg Arg Pro Leu Gly Ala Asp Arg Val
                325           330           335

Pro Glu Arg Val Val Gln Arg Ile Pro Phe Asn Phe Ala Ser Gln Ala
        340           345           350

Thr Asn Pro Phe Leu Lys Thr Leu Asp Asn Thr Lys Arg Ile Ser Met
        355           360           365

Ala Thr Asp Asp Leu Gly Gln Trp Val Leu Glu Lys Gly Tyr Ala Ser
        370           375           380

Glu Gly His Asp Ser Ala His Pro Asp Tyr Gly Gly Asn Glu Asn Val
385           390           395           400

Arg Ala Gly Gly Trp Lys Asp Leu Asn Arg Leu Thr Arg Thr Gly Ala
        405           410           415

Gly Tyr Asn Ala Asp Phe Ala Val His Val Asn Ala Thr Glu Ala Tyr
        420           425           430

Ala Gln Ala Arg Thr Phe Thr Glu Asp Met Val Ala Gly Gln Ala Asp
        435           440           445

Gly Trp Asp Trp Leu Asn Gln Ala Tyr His Ile Asp Gln Arg Lys Asp
        450           455           460

Leu Gly Thr Gly Ala Val Leu Asp Arg Phe Lys Gln Leu Arg Lys Glu
465           470           475           480

Ala Pro Gly Ile Arg Thr Val Tyr Ile Asp Ala Tyr Tyr Ser Ser Gly
        485           490           495

Trp Leu Ala Asp Gly Leu Ala Ala Gly Leu Arg Glu Met Gly Phe Glu
        500           505           510

Val Ala Thr Glu Trp Ala Tyr Lys Phe Glu Gly Thr Ser Val Trp Ser
        515           520           525

His Trp Ala Ala Asp Lys Asn Tyr Gly Gly Ala Thr Asn Lys Gly Ile
        530           535           540

Asn Ser Asp Ile Val Arg Phe Ile Ala Asn Ala Asp Arg Asp Val Trp
545           550           555           560

```
Asn Val Asp Pro Leu Leu Gly Gly Ala Ser Val Val Glu Phe Glu Gly
            565                 570                 575

Trp Thr Gly Gln Asp Asp Trp Asn Ala Phe Tyr Arg Asn Ile Trp Thr
            580                 585                 590

Asp Asn Leu Pro Thr Lys Phe Leu Gln His Phe Gln Val Leu Asp Trp
            595                 600                 605

Asp Arg Gly Arg Ser Ala Arg Leu Thr Gly Gly Val Asp Val Lys Ser
        610             615             620

Val Asp Gly Glu Arg Arg Ile Ser Met Asp Gly Thr Glu Val Leu Lys
    625                 630                 635                 640

Gly Asp Thr Tyr Leu Leu Pro Trp Gln Asn Ala Gly Lys Asp Asp Gly
            645                 650                 655

Thr Ser Ser Pro Arg Asp Ala Asp Lys Met Tyr Phe Tyr Ser Ala Ser
            660                 665                 670

Gly Gly Glu His Thr Phe Glu Leu Thr Gly Gln Phe Ala Gly Thr Glu
        675             680             685

Asp Phe Thr Leu Tyr Glu Leu Thr Asp Gln Gly Arg Ala Glu Lys Ala
    690             695             700

Arg Val Thr Ala His Glu Gly Arg Val Thr Leu Thr Ala Glu Lys Gly
705             710             715             720

Gln Pro Tyr Val Leu Val Pro Asn Gly Gly Arg Ala Pro His Arg Asp
            725             730             735

Ala His Tyr Gly Glu Phe Thr Gly Leu Ser Asp Pro Gly Phe Asn Gly
            740             745             750

Gly Asp Leu Asp Ala Trp Asn Ala Ser Gly Gly Ala Glu Ile Val Arg
        755             760             765

Ala Gly Asn Gly Asp Asn Val Val Arg Leu Gly Glu Asp Ala Ser Gly
        770             775             780

Ile Ala Gln Arg Val Arg Gly Leu Thr Pro Gly Glu Arg Tyr Thr Leu
785             790             795             800

Gly Ala Asp Val Gly Ile Gly Pro Gly Glu Arg Arg Glu Thr Thr Leu
            805             810             815

Arg Val Arg Gly Gly Lys Asp Ser Glu Ala Arg Thr Phe Asp Ile Thr
        820             825             830

Pro Ala Arg Asn Arg Met Ala Ser Asp Glu Lys Arg Asp Thr Tyr Ser
        835             840             845

Gln Arg Ala Ser Val Ser Phe Thr Ala Pro Arg Asp Gly Ser Val Thr
    850             855             860
```

Val Glu Leu Gly Ala Val Ala Gly Gly Ala Pro Val Val Leu Asp Asp
865                 870             875             880

Val Arg Val Met Val Asp Thr Thr Ala Pro Leu Pro Arg Ser Gln Asp
                885             890             895

Gly Thr Val Val Ala His Asp Asp Phe Glu Gly Asn Arg Pro Gly Trp
            900             905             910

Gly Pro Phe Val Lys Gly Asp Ala Gly Gly Val Thr Asp Pro Arg Thr
        915             920             925

Ser Ile Ser Asp Leu His Ala Pro Tyr Ser Gln Lys Glu Trp Lys Asn
    930             935             940

Thr Tyr Ser Pro Tyr Asp Thr Gly Ala Leu Lys Gly Arg Ala Val Asp
945             950             955             960

Asp Val Leu Ala Gly Arg His Ser Leu Lys Ser His Ala Glu Asn Thr
            965             970             975

Gly Leu Val His Arg Thr Thr Pro Ala Thr Val Pro Phe Glu Glu Gly
            980             985             990

His Arg Tyr Arg Val Ser Phe Ser Tyr Gln Thr Asn Val Glu Gly Gln
        995             1000            1005

Trp Ala Trp Val Thr Gly Ala Asp Arg Val Ala Asp Gly Thr Thr
    1010            1015            1020

Thr Ser Arg Asp Ile Thr Arg Asp Val Leu Ala Pro Ala Leu Asp
    1025            1030            1035

Thr Ala Ala Tyr Ser Arg Glu Phe Val Ala Gly Cys Gly Asp Thr
    1040            1045            1050

Trp Val Gly Leu Arg Arg Leu Gly Ser Ala Arg Gly Thr Asp Leu
    1055            1060            1065

Val Leu Asp Asp Phe Thr Val Thr Asp Leu Gly Glu Ala Asp Thr
    1070            1075            1080

Gly Ala Ala Cys Ala Ala Val Thr Ala Pro Ser Gly Ala Glu Leu
    1085            1090            1095

Ser Pro Gly Val Pro Gly Glu Tyr Val Thr Ala Phe Thr Asn His
    1100            1105            1110

Glu Ser Ala Gly Ala Glu Asn Val Gly Ile Ala Leu Gln Gly Leu
    1115            1120            1125

Pro Glu Gly Trp Lys Ala Glu Val Lys Glu Lys Asp Gly Asn Leu
    1130            1135            1140

Phe Glu Arg Val Gln Pro Gly Ala Thr Val Arg Thr Thr Trp Leu
    1145            1150            1155

```
Leu Thr Pro Pro Ala Gly Thr Ala Gly Thr Ser Ala Thr Trp Gln
    1160          1165          1170

Val Thr Ala Ala Tyr Ala His Glu Gly Ala Thr Arg Thr Val Ser
    1175          1180          1185

Thr Gly Ala Arg Ala Ala Val Thr Asp Glu Pro Val Leu Ala Pro
    1190          1195          1200

Ala Ser Thr Thr Ala Thr Ala Asp Ser Glu Asn Thr Ser Ser Gly
    1205          1210          1215

Ala Ser Glu Gly Pro Val Ser Asn Val Leu Asp Gly Asp Ala Gly
    1220          1225          1230

Thr Ile Trp His Thr Asp Tyr Thr Thr Ser Gln Ala Pro Tyr Pro
    1235          1240          1245

His Trp Val Thr Leu Lys Leu Gly Gly Ala Ala Asp Val Asp Gly
    1250          1255          1260

Phe Gly Tyr Leu Gly Arg Gln Ser Gly Gly Pro Asn Gly Arg Val
    1265          1270          1275

Ala Asp Tyr Glu Val Ala Val Ser Asp Asp Gly Glu Ala Trp Thr
    1280          1285          1290

Thr Val Ala Thr Gly Thr Leu Lys Asp Val Pro Arg Thr Gln Arg
    1295          1300          1305

Val Ser Phe Asp Arg Val Arg Ala Ser Tyr Val Arg Phe Thr Ala
    1310          1315          1320

Leu Asp Ala Leu Asn Gly Gln Pro Tyr Ala Ala Ala Ala Glu Met
    1325          1330          1335

Arg Val Tyr Gly Val Pro Val Asp Leu Pro Thr Gly Tyr Pro Pro
    1340          1345          1350

Gly Glu Arg Pro Ala Asp Ala Arg
    1355          1360
```

<210> 11
<211> 3975
<212> DNA
<213> Enterococcus faecalis V583

<220>
<221> CDS
<222> (1).. (3975)

<400> 11

82

```
atg aaa cat gga aaa ata aaa cga ttt agt aca ttg aca cta ttg gca        48
Met Lys His Gly Lys Ile Lys Arg Phe Ser Thr Leu Thr Leu Leu Ala
1               5               10              15

agc gca acg att tta gta cca tta agt acg tct gca gaa gaa aca aca        96
Ser Ala Thr Ile Leu Val Pro Leu Ser Thr Ser Ala Glu Glu Thr Thr
                20              25              30

aac agc agc act gaa aca agc tca tca atg gtg gaa cct acc gca aca       144
Asn Ser Ser Thr Glu Thr Ser Ser Ser Met Val Glu Pro Thr Ala Thr
```

```
                    35                    40                    45
gaa gaa aaa ttg tgg caa tct gat ttt cca gga ggg aaa act ggt gag    192
Glu Glu Lys Leu Trp Gln Ser Asp Phe Pro Gly Gly Lys Thr Gly Glu
        50                    55                    60

tgg caa gat gtg att ggt aaa aca aat cga gaa tta gca gga gag tca    240
Trp Gln Asp Val Ile Gly Lys Thr Asn Arg Glu Leu Ala Gly Glu Ser
65                    70                    75                    80

ttg gcg att tca cga gat gca gca gca ggt aat aat gcc gta tct tta    288
Leu Ala Ile Ser Arg Asp Ala Ala Ala Gly Asn Asn Ala Val Ser Leu
                    85                    90                    95

aat tta gat tcc cct aaa tta gct gat ggt gaa gta gaa aca aag ttt    336
Asn Leu Asp Ser Pro Lys Leu Ala Asp Gly Glu Val Glu Thr Lys Phe
                    100                   105                   110

aaa tac act gct gga agc ggt cga acg ggt gtg att ata cgt ggt aat    384
Lys Tyr Thr Ala Gly Ser Gly Arg Thr Gly Val Ile Ile Arg Gly Asn
                    115                   120                   125

acc aaa gat agc tgg gtt ttt gta ggc tat aac gcg aat ggc aaa tgg    432
Thr Lys Asp Ser Trp Val Phe Val Gly Tyr Asn Ala Asn Gly Lys Trp
                    130                   135                   140

tta gtt gaa agc cct aat tcg tgg aat gat tca att tct ggg cca acg    480
Leu Val Glu Ser Pro Asn Ser Trp Asn Asp Ser Ile Ser Gly Pro Thr
145                   150                   155                   160

tta aat gaa gat acg aat tat ttg ttg aaa gta cgt tat gtt ggt gaa    528
Leu Asn Glu Asp Thr Asn Tyr Leu Leu Lys Val Arg Tyr Val Gly Glu
                    165                   170                   175

aaa att act atc tgg ctt aac acc acg ttg att tac gaa gga gaa cct    576
Lys Ile Thr Ile Trp Leu Asn Thr Thr Leu Ile Tyr Glu Gly Glu Pro
                    180                   185                   190

gtt ttg gcc aat gga gac aaa att cca aca gaa gcc gga cat gtg ggt    624
Val Leu Ala Asn Gly Asp Lys Ile Pro Thr Glu Ala Gly His Val Gly
                    195                   200                   205

gtc cgt ttg tgg tac gac aaa aaa att gtc aat tat gac tat ttt aaa    672
Val Arg Leu Trp Tyr Asp Lys Lys Ile Val Asn Tyr Asp Tyr Phe Lys
210                   215                   220

aat ggc ccc gta gat agc att cca gaa att gtg cca gaa gtg aca caa    720
Asn Gly Pro Val Asp Ser Ile Pro Glu Ile Val Pro Glu Val Thr Gln
225                   230                   235                   240

att gcg cca gtc aaa gtt ttt aca aaa att ggt gtc gca cca aaa tta    768
Ile Ala Pro Val Lys Val Phe Thr Lys Ile Gly Val Ala Pro Lys Leu
                    245                   250                   255

ccg aaa caa gta aaa gtg acc tat aat act ggt aaa gaa gcc aat gaa    816
Pro Lys Gln Val Lys Val Thr Tyr Asn Thr Gly Lys Glu Ala Asn Glu
                    260                   265                   270

gca gtc cgt tgg aat gaa atc gat cct gat gca tat aaa gaa cca gga    864
Ala Val Arg Trp Asn Glu Ile Asp Pro Asp Ala Tyr Lys Glu Pro Gly
                    275                   280                   285

act ttt gaa gtc gac ggt act ttg gaa aat aca aac atc aaa gca aaa    912
Thr Phe Glu Val Asp Gly Thr Leu Glu Asn Thr Asn Ile Lys Ala Lys
                    290                   295                   300

gcc agc att gtt gtt gct aaa gac aat gaa gct gaa aaa ggc gac aaa    960
Ala Ser Ile Val Val Ala Lys Asp Asn Glu Ala Glu Lys Gly Asp Lys
305                   310                   315                   320

atc tct tcc gca gat tta aca gcc gtg gtt gat cca caa ttt cca cga    1008
Ile Ser Ser Ala Asp Leu Thr Ala Val Val Asp Pro Gln Phe Pro Arg
                    325                   330                   335

att att cgc tac gaa gac cct cag agt aat caa gtg att ttt aat ggc    1056
Ile Ile Arg Tyr Glu Asp Pro Gln Ser Asn Gln Val Ile Phe Asn Gly
```

```
                     340                   345                   350
caa cac gag aaa att gac caa gta atg att gat ggc aaa gca tat aaa      1104
Gln His Glu Lys Ile Asp Gln Val Met Ile Asp Gly Lys Ala Tyr Lys
        355                   360                   365

gca act gct gaa aaa cag aag agt gaa gca aat caa gcc gtt tat aac      1152
Ala Thr Ala Glu Lys Gln Lys Ser Glu Ala Asn Gln Ala Val Tyr Asn
    370                   375                   380

gta gct gtc cca gaa att ggt ttg cgt ttc aca acg aca ttg act gtt      1200
Val Ala Val Pro Glu Ile Gly Leu Arg Phe Thr Thr Thr Leu Thr Val
385                   390                   395                   400

tcc gaa ggc caa gaa tta gct atg aaa ctc tca gat att cgt gaa gaa      1248
Ser Glu Gly Gln Glu Leu Ala Met Lys Leu Ser Asp Ile Arg Glu Glu
                  405                   410                   415

gga acc aaa ata cac aca att tca att cca aat caa ggc ttg att tct      1296
Gly Thr Lys Ile His Thr Ile Ser Ile Pro Asn Gln Gly Leu Ile Ser
                  420                   425                   430

gtc aat agt aca gat gaa ggg gcg act ttt gct ggc gtt gtg atg aat      1344
Val Asn Ser Thr Asp Glu Gly Ala Thr Phe Ala Gly Val Val Met Asn
                  435                   440                   445

act ggg aca aat gca aat aac gga aat aaa aat ggt gat act atc caa      1392
Thr Gly Thr Asn Ala Asn Asn Gly Asn Lys Asn Gly Asp Thr Ile Gln
        450                   455                   460

gat tta act aca aca agc caa gaa gaa acg aaa aaa tat atg tat ggt      1440
Asp Leu Thr Thr Thr Ser Gln Glu Glu Thr Lys Lys Tyr Met Tyr Gly
465                   470                   475                   480

ttc tta aat acg gcg aat tat gct gca agt ttt tgg acg aac gcc tat      1488
Phe Leu Asn Thr Ala Asn Tyr Ala Ala Ser Phe Trp Thr Asn Ala Tyr
                  485                   490                   495

gga gac ggc tct gtc gat ggt agt gac aac aat cga atc cat aaa caa      1536
Gly Asp Gly Ser Val Asp Gly Ser Asp Asn Asn Arg Ile His Lys Gln
                  500                   505                   510

aca aaa gaa gcg gcg act ggt ttt gta aca acc ttg tca agt ggg gca      1584
Thr Lys Glu Ala Ala Thr Gly Phe Val Thr Thr Leu Ser Ser Gly Ala
        515                   520                   525

tgg acc tat cga cca ttt gat gca ccg gaa gat tac aca act gga gaa      1632
Trp Thr Tyr Arg Pro Phe Asp Ala Pro Glu Asp Tyr Thr Thr Gly Glu
        530                   535                   540

acg cca gaa gtg aaa gtt aaa ttc tca aaa gat agc aac gac gac aat      1680
Thr Pro Glu Val Lys Val Lys Phe Ser Lys Asp Ser Asn Asp Asp Asn
545                   550                   555                   560

cgg gtg gat tgg caa gat gcg gca att ggg ttc cgt tca att atg aat      1728
Arg Val Asp Trp Gln Asp Ala Ala Ile Gly Phe Arg Ser Ile Met Asn
                  565                   570                   575

aac cca atg ggt gcg gaa aaa gtc cct gaa tta gtc aac caa cgg att      1776
Asn Pro Met Gly Ala Glu Lys Val Pro Glu Leu Val Asn Gln Arg Ile
                  580                   585                   590

cct ttt aac ttt gct agt cag gcg aca aac cca ttc tta gtg acg tta      1824
Pro Phe Asn Phe Ala Ser Gln Ala Thr Asn Pro Phe Leu Val Thr Leu
                  595                   600                   605

gac gaa tca aaa cgt att tac aat tta aca gat gga tta gga caa atg      1872
Asp Glu Ser Lys Arg Ile Tyr Asn Leu Thr Asp Gly Leu Gly Gln Met
        610                   615                   620

aat tta cta aaa ggg tat caa aat gaa gga cat gat tct gcg cat cca      1920
Asn Leu Leu Lys Gly Tyr Gln Asn Glu Gly His Asp Ser Ala His Pro
625                   630                   635                   640

gat tac ggt gct att ggt cag cga cct ggt ggg gaa caa gcg ttg aat      1968
Asp Tyr Gly Ala Ile Gly Gln Arg Pro Gly Gly Glu Gln Ala Leu Asn
```

645 650 655

```
caa tta att gat gaa gga cat aaa tta aat gcc gtt ttc ggt gtg cat      2016
Gln Leu Ile Asp Glu Gly His Lys Leu Asn Ala Val Phe Gly Val His
            660                 665                 670

att aat gac acc gag tct tac cca gaa gca aaa gga ttt aat gag gaa      2064
Ile Asn Asp Thr Glu Ser Tyr Pro Glu Ala Lys Gly Phe Asn Glu Glu
            675                 680                 685

tta gtt gat cca acg aag cgt ggc tgg gat tgg tta gat ccg tct tat      2112
Leu Val Asp Pro Thr Lys Arg Gly Trp Asp Trp Leu Asp Pro Ser Tyr
            690                 695                 700

ttt att aaa caa aga ccc gat aca ttg agt ggt cgt cgc tat gag cgc      2160
Phe Ile Lys Gln Arg Pro Asp Thr Leu Ser Gly Arg Arg Tyr Glu Arg
705                 710                 715                 720

ttt aaa gaa tta aaa caa aaa gca ccg aat cta gat tat att tat gtt      2208
Phe Lys Glu Leu Lys Gln Lys Ala Pro Asn Leu Asp Tyr Ile Tyr Val
                725                 730                 735

gat gtt tgg ggc aac caa ggc gaa tca ggt tgg gca agt cgt caa cta      2256
Asp Val Trp Gly Asn Gln Gly Glu Ser Gly Trp Ala Ser Arg Gln Leu
            740                 745                 750

agt aaa gaa att aat tca ctc ggt tgg ttt aca acc aat gaa ttt ccg      2304
Ser Lys Glu Ile Asn Ser Leu Gly Trp Phe Thr Thr Asn Glu Phe Pro
            755                 760                 765

aat gct tta gag tat gac tcg gtt tgg aac cat tgg tct gca gaa aaa      2352
Asn Ala Leu Glu Tyr Asp Ser Val Trp Asn His Trp Ser Ala Glu Lys
770                 775                 780

gat tac ggc ggt aca aca acg aaa ggc ttt aac agt aca atc gtt cgt      2400
Asp Tyr Gly Gly Thr Thr Thr Lys Gly Phe Asn Ser Thr Ile Val Arg
785                 790                 795                 800

ttt att cgg aat cat caa aaa gac act tgg att att tcc gac aac cct      2448
Phe Ile Arg Asn His Gln Lys Asp Thr Trp Ile Ile Ser Asp Asn Pro
                805                 810                 815

ttg tta ggt gga gct gaa ttt gaa gcc tat gaa ggt tgg gtg ggt aaa      2496
Leu Leu Gly Gly Ala Glu Phe Glu Ala Tyr Glu Gly Trp Val Gly Lys
            820                 825                 830

acc aat ttt aat acc tat cgc caa aaa act ttt gcc att aat gtc cca      2544
Thr Asn Phe Asn Thr Tyr Arg Gln Lys Thr Phe Ala Ile Asn Val Pro
            835                 840                 845

act aag ttc tta caa cat tac caa att aca aac tgg gaa act aca aca      2592
Thr Lys Phe Leu Gln His Tyr Gln Ile Thr Asn Trp Glu Thr Thr Thr
            850                 855                 860

gca gca gat ggt caa atc tat ggc aca att aaa tta gcg aac ggt gct      2640
Ala Ala Asp Gly Gln Ile Tyr Gly Thr Ile Lys Leu Ala Asn Gly Ala
865                 870                 875                 880

gaa aaa gtg acc gtt act caa gca gat gct aat tcg cca aga agc att      2688
Glu Lys Val Thr Val Thr Gln Ala Asp Ala Asn Ser Pro Arg Ser Ile
                885                 890                 895

acg tta aat gag aca gaa gtt cta aaa ggt gat gcg tat cta ctg cct      2736
Thr Leu Asn Glu Thr Glu Val Leu Lys Gly Asp Ala Tyr Leu Leu Pro
            900                 905                 910

tgg aat gtc aat ggt caa gac aaa cta tat cac tgg aat cca aaa ggc      2784
Trp Asn Val Asn Gly Gln Asp Lys Leu Tyr His Trp Asn Pro Lys Gly
            915                 920                 925

ggt acc agc act tgg tca ttg gat aag aaa atg caa gga aaa acg aat      2832
Gly Thr Ser Thr Trp Ser Leu Asp Lys Lys Met Gln Gly Lys Thr Asn
930                 935                 940

tta cat tta tat gaa tta aca gat caa ggg cgt att gac aaa ggc gca      2880
Leu His Leu Tyr Glu Leu Thr Asp Gln Gly Arg Ile Asp Lys Gly Ala
```

```
            945                    950                   955                   960
      att gcc act aca aat aac caa gtg acc atc caa gcc gag gct aat aca      2928
      Ile Ala Thr Thr Asn Asn Gln Val Thr Ile Gln Ala Glu Ala Asn Thr
                      965                   970                   975

      ccg tat gtc att gct gaa cct gac agt att gaa ccg atg aca ttt gga      2976
      Pro Tyr Val Ile Ala Glu Pro Asp Ser Ile Glu Pro Met Thr Phe Gly
                      980                   985                   990

      aca gga aca cca ttt aaa gat cct  gga ttt aat gaa gcc  aat acc tta    3024
      Thr Gly Thr Pro Phe Lys Asp Pro Gly Phe Asn Glu Ala  Asn Thr Leu
                995                  1000                  1005

      aaa aat  aac tgg aaa gtt ttc  cga ggt gat gga gag  gtt aaa aaa       3069
      Lys Asn Asn Trp Lys Val Phe Arg Gly Asp Gly Glu Val Lys Lys
          1010                 1015                 1020

      gat gcc  aat ggt gat tat gtc  ttt agt tca gaa aaa  gaa aga acc       3114
      Asp Ala Asn Gly Asp Tyr Val Phe Ser Ser Glu Lys Glu Arg Thr
          1025                 1030                 1035

      gaa atc  aaa caa gat atc aat  ctt cct aaa cca gga  aaa tat agt       3159
      Glu Ile Lys Gln Asp Ile Asn Leu Pro Lys Pro Gly Lys Tyr Ser
          1040                 1045                 1050

      ttg tat  cta aac aca gaa aca  cat gat cgt aaa gcc  aca gta act       3204
      Leu Tyr Leu Asn Thr Glu Thr His Asp Arg Lys Ala Thr Val Thr
          1055                 1060                 1065

      gtt aaa  att ggt ggt aag aaa  tat acg cgg aca gtg  aat aat tcg       3249
      Val Lys Ile Gly Gly Lys Lys Tyr Thr Arg Thr Val Asn Asn Ser
          1070                 1075                 1080

      gtt gcc  caa aac tac att cag  gca gat att aac cat  aca agc agg       3294
      Val Ala Gln Asn Tyr Ile Gln Ala Asp Ile Asn His Thr Ser Arg
          1085                 1090                 1095

      aaa aat  ccg cag tat atg caa  aat atg cga att gat  ttt gaa atc       3339
      Lys Asn Pro Gln Tyr Met Gln Asn Met Arg Ile Asp Phe Glu Ile
          1100                 1105                 1110

      cca gat  aat gcc aaa aaa ggc  tcg gtg aca tta gcg  gtt gat aaa       3384
      Pro Asp Asn Ala Lys Lys Gly Ser Val Thr Leu Ala Val Asp Lys
          1115                 1120                 1125

      ggc aat  tcc gtt aca aaa ttt  gat gat tta cga att  gtt gag cgt       3429
      Gly Asn Ser Val Thr Lys Phe Asp Asp Leu Arg Ile Val Glu Arg
          1130                 1135                 1140

      caa acg  gat atc atg aac cca  gac aaa caa aca gtt  att aag caa       3474
      Gln Thr Asp Ile Met Asn Pro Asp Lys Gln Thr Val Ile Lys Gln
          1145                 1150                 1155

      gat ttt  gaa gac aca caa gca  gtt ggg tta tat ccg  ttt gtt aaa       3519
      Asp Phe Glu Asp Thr Gln Ala Val Gly Leu Tyr Pro Phe Val Lys
          1160                 1165                 1170

      ggc tca  gct ggt ggt gta gaa  gat cca cgg att cat  tta tca gaa       3564
      Gly Ser Ala Gly Gly Val Glu Asp Pro Arg Ile His Leu Ser Glu
          1175                 1180                 1185

      aga aat  gaa cct tac aca caa  tat ggt tgg aat gga  aac ctt gtt       3609
      Arg Asn Glu Pro Tyr Thr Gln Tyr Gly Trp Asn Gly Asn Leu Val
          1190                 1195                 1200

      tca gat  gta tta gaa ggc aac  tgg tcc ttg aaa gcc  cat aaa caa       3654
      Ser Asp Val Leu Glu Gly Asn Trp Ser Leu Lys Ala His Lys Gln
          1205                 1210                 1215

      gga gca  gga ttg atg ctt caa  aca att ccg caa aat  att aaa ttt       3699
      Gly Ala Gly Leu Met Leu Gln Thr Ile Pro Gln Asn Ile Lys Phe
          1220                 1225                 1230

      gaa ccg  aac aag aaa tat acg  gtc caa ttt gat tat  caa act gat       3744
      Glu Pro Asn Lys Lys Tyr Thr Val Gln Phe Asp Tyr Gln Thr Asp
```

```
                    1235                  1240                 1245
     ggt gaa aat gtc ttt act gct ggg acc att aat ggg gag ttg aaa    3789
     Gly Glu Asn Val Phe Thr Ala Gly Thr Ile Asn Gly Glu Leu Lys
         1250                 1255                 1260

     aat aac aat gac ttt aag cca gtc ggt gag tta act tcg aca gca    3834
     Asn Asn Asn Asp Phe Lys Pro Val Gly Glu Leu Thr Ser Thr Ala
         1265                 1270                 1275

     gca gat ggt caa acc aag cat tat gaa gca gaa ata att ggg gat    3879
     Ala Asp Gly Gln Thr Lys His Tyr Glu Ala Glu Ile Ile Gly Asp
         1280                 1285                 1290

     gct tca gga aac act acg ttt ggt att ttt aca aca ggt gcc gat    3924
     Ala Ser Gly Asn Thr Thr Phe Gly Ile Phe Thr Thr Gly Ala Asp
         1295                 1300                 1305

     aaa gat ttc att atg gat aac ttt acg gtc aca gtg gaa tca aaa    3969
     Lys Asp Phe Ile Met Asp Asn Phe Thr Val Thr Val Glu Ser Lys
         1310                 1315                 1320

     aaa taa                                                        3975
     Lys
```

<210> 12
<211> 1324
<212> PRT
<213> Enterococcus faecalis V583

<400> 12

```
Met Lys His Gly Lys Ile Lys Arg Phe Ser Thr Leu Thr Leu Leu Ala
1               5               10              15

Ser Ala Thr Ile Leu Val Pro Leu Ser Thr Ser Ala Glu Glu Thr Thr
            20              25              30

Asn Ser Ser Thr Glu Thr Ser Ser Ser Met Val Glu Pro Thr Ala Thr
        35              40              45

Glu Glu Lys Leu Trp Gln Ser Asp Phe Pro Gly Gly Lys Thr Gly Glu
    50              55              60

Trp Gln Asp Val Ile Gly Lys Thr Asn Arg Glu Leu Ala Gly Glu Ser
65              70              75              80

Leu Ala Ile Ser Arg Asp Ala Ala Ala Gly Asn Asn Ala Val Ser Leu
            85              90              95

Asn Leu Asp Ser Pro Lys Leu Ala Asp Gly Glu Val Glu Thr Lys Phe
            100             105             110

Lys Tyr Thr Ala Gly Ser Gly Arg Thr Gly Val Ile Ile Arg Gly Asn
    115             120             125

Thr Lys Asp Ser Trp Val Phe Val Gly Tyr Asn Ala Asn Gly Lys Trp
    130             135             140

Leu Val Glu Ser Pro Asn Ser Trp Asn Asp Ser Ile Ser Gly Pro Thr
145             150             155             160

Leu Asn Glu Asp Thr Asn Tyr Leu Leu Lys Val Arg Tyr Val Gly Glu
            165             170             175
```

89

Lys Ile Thr Ile Trp Leu Asn Thr Thr Leu Ile Tyr Glu Gly Glu Pro
180 185 190

Val Leu Ala Asn Gly Asp Lys Ile Pro Thr Glu Ala Gly His Val Gly
195 200 205

Val Arg Leu Trp Tyr Asp Lys Lys Ile Val Asn Tyr Asp Tyr Phe Lys
210 215 220

Asn Gly Pro Val Asp Ser Ile Pro Glu Ile Val Pro Glu Val Thr Gln
225 230 235 240

Ile Ala Pro Val Lys Val Phe Thr Lys Ile Gly Val Ala Pro Lys Leu
245 250 255

Pro Lys Gln Val Lys Val Thr Tyr Asn Thr Gly Lys Glu Ala Asn Glu
260 265 270

Ala Val Arg Trp Asn Glu Ile Asp Pro Asp Ala Tyr Lys Glu Pro Gly
275 280 285

Thr Phe Glu Val Asp Gly Thr Leu Glu Asn Thr Asn Ile Lys Ala Lys
290 295 300

Ala Ser Ile Val Val Ala Lys Asp Asn Glu Ala Glu Lys Gly Asp Lys
305 310 315 320

Ile Ser Ser Ala Asp Leu Thr Ala Val Val Asp Pro Gln Phe Pro Arg
325 330 335

Ile Ile Arg Tyr Glu Asp Pro Gln Ser Asn Gln Val Ile Phe Asn Gly
340 345 350

Gln His Glu Lys Ile Asp Gln Val Met Ile Asp Gly Lys Ala Tyr Lys
355 360 365

Ala Thr Ala Glu Lys Gln Lys Ser Glu Ala Asn Gln Ala Val Tyr Asn
370 375 380

Val Ala Val Pro Glu Ile Gly Leu Arg Phe Thr Thr Thr Leu Thr Val
385 390 395 400

Ser Glu Gly Gln Glu Leu Ala Met Lys Leu Ser Asp Ile Arg Glu Glu
405 410 415

Gly Thr Lys Ile His Thr Ile Ser Ile Pro Asn Gln Gly Leu Ile Ser
420 425 430

Val Asn Ser Thr Asp Glu Gly Ala Thr Phe Ala Gly Val Val Met Asn
435 440 445

Thr Gly Thr Asn Ala Asn Asn Gly Asn Lys Asn Gly Asp Thr Ile Gln
450 455 460

Asp Leu Thr Thr Thr Ser Gln Glu Glu Thr Lys Lys Tyr Met Tyr Gly
465 470 475 480

```
Phe Leu Asn Thr Ala Asn Tyr Ala Ala Ser Phe Trp Thr Asn Ala Tyr
            485             490                 495

Gly Asp Gly Ser Val Asp Gly Ser Asp Asn Asn Arg Ile His Lys Gln
            500             505             510

Thr Lys Glu Ala Ala Thr Gly Phe Val Thr Thr Leu Ser Ser Gly Ala
        515             520             525

Trp Thr Tyr Arg Pro Phe Asp Ala Pro Glu Asp Tyr Thr Thr Gly Glu
    530             535             540

Thr Pro Glu Val Lys Val Lys Phe Ser Lys Asp Ser Asn Asp Asp Asn
545             550             555             560

Arg Val Asp Trp Gln Asp Ala Ala Ile Gly Phe Arg Ser Ile Met Asn
            565             570             575

Asn Pro Met Gly Ala Glu Lys Val Pro Glu Leu Val Asn Gln Arg Ile
            580             585             590

Pro Phe Asn Phe Ala Ser Gln Ala Thr Asn Pro Phe Leu Val Thr Leu
        595             600             605

Asp Glu Ser Lys Arg Ile Tyr Asn Leu Thr Asp Gly Leu Gly Gln Met
    610             615             620

Asn Leu Leu Lys Gly Tyr Gln Asn Glu Gly His Asp Ser Ala His Pro
625             630             635             640

Asp Tyr Gly Ala Ile Gly Gln Arg Pro Gly Gly Glu Gln Ala Leu Asn
            645             650             655

Gln Leu Ile Asp Glu Gly His Lys Leu Asn Ala Val Phe Gly Val His
            660             665             670

Ile Asn Asp Thr Glu Ser Tyr Pro Glu Ala Lys Gly Phe Asn Glu Glu
            675             680             685

Leu Val Asp Pro Thr Lys Arg Gly Trp Asp Trp Leu Asp Pro Ser Tyr
    690             695             700

Phe Ile Lys Gln Arg Pro Asp Thr Leu Ser Gly Arg Arg Tyr Glu Arg
705             710             715             720

Phe Lys Glu Leu Lys Gln Lys Ala Pro Asn Leu Asp Tyr Ile Tyr Val
            725             730             735

Asp Val Trp Gly Asn Gln Gly Glu Ser Gly Trp Ala Ser Arg Gln Leu
            740             745             750

Ser Lys Glu Ile Asn Ser Leu Gly Trp Phe Thr Thr Asn Glu Phe Pro
        755             760             765

Asn Ala Leu Glu Tyr Asp Ser Val Trp Asn His Trp Ser Ala Glu Lys
    770             775             780
```

Asp Tyr Gly Gly Thr Thr Thr Lys Gly Phe Asn Ser Thr Ile Val Arg
785                790           795               800

Phe Ile Arg Asn His Gln Lys Asp Thr Trp Ile Ile Ser Asp Asn Pro
              805               810               815

Leu Leu Gly Gly Ala Glu Phe Glu Ala Tyr Glu Gly Trp Val Gly Lys
              820               825               830

Thr Asn Phe Asn Thr Tyr Arg Gln Lys Thr Phe Ala Ile Asn Val Pro
          835               840               845

Thr Lys Phe Leu Gln His Tyr Gln Ile Thr Asn Trp Glu Thr Thr Thr
    850               855               860

Ala Ala Asp Gly Gln Ile Tyr Gly Thr Ile Lys Leu Ala Asn Gly Ala
865               870               875               880

Glu Lys Val Thr Val Thr Gln Ala Asp Ala Asn Ser Pro Arg Ser Ile
              885               890               895

Thr Leu Asn Glu Thr Glu Val Leu Lys Gly Asp Ala Tyr Leu Leu Pro
          900               905               910

Trp Asn Val Asn Gly Gln Asp Lys Leu Tyr His Trp Asn Pro Lys Gly
    915               920               925

Gly Thr Ser Thr Trp Ser Leu Asp Lys Lys Met Gln Gly Lys Thr Asn
    930               935               940

Leu His Leu Tyr Glu Leu Thr Asp Gln Gly Arg Ile Asp Lys Gly Ala
945               950               955               960

Ile Ala Thr Thr Asn Asn Gln Val Thr Ile Gln Ala Glu Ala Asn Thr
              965               970               975

Pro Tyr Val Ile Ala Glu Pro Asp Ser Ile Glu Pro Met Thr Phe Gly
              980               985               990

Thr Gly Thr Pro Phe Lys Asp Pro Gly Phe Asn Glu Ala Asn Thr Leu
    995               1000              1005

Lys Asn Asn Trp Lys Val Phe Arg Gly Asp Gly Glu Val Lys Lys
    1010              1015              1020

Asp Ala Asn Gly Asp Tyr Val Phe Ser Ser Glu Lys Glu Arg Thr
    1025              1030              1035

Glu Ile Lys Gln Asp Ile Asn Leu Pro Lys Pro Gly Lys Tyr Ser
    1040              1045              1050

Leu Tyr Leu Asn Thr Glu Thr His Asp Arg Lys Ala Thr Val Thr
    1055              1060              1065

Val Lys Ile Gly Gly Lys Lys Tyr Thr Arg Thr Val Asn Asn Ser
    1070              1075              1080

Val Ala Gln Asn Tyr Ile Gln Ala Asp Ile Asn His Thr Ser Arg
1085 1090 1095

Lys Asn Pro Gln Tyr Met Gln Asn Met Arg Ile Asp Phe Glu Ile
1100 1105 1110

Pro Asp Asn Ala Lys Lys Gly Ser Val Thr Leu Ala Val Asp Lys
1115 1120 1125

Gly Asn Ser Val Thr Lys Phe Asp Asp Leu Arg Ile Val Glu Arg
1130 1135 1140

Gln Thr Asp Ile Met Asn Pro Asp Lys Gln Thr Val Ile Lys Gln
1145 1150 1155

Asp Phe Glu Asp Thr Gln Ala Val Gly Leu Tyr Pro Phe Val Lys
1160 1165 1170

Gly Ser Ala Gly Gly Val Glu Asp Pro Arg Ile His Leu Ser Glu
1175 1180 1185

Arg Asn Glu Pro Tyr Thr Gln Tyr Gly Trp Asn Gly Asn Leu Val
1190 1195 1200

Ser Asp Val Leu Glu Gly Asn Trp Ser Leu Lys Ala His Lys Gln
1205 1210 1215

Gly Ala Gly Leu Met Leu Gln Thr Ile Pro Gln Asn Ile Lys Phe
1220 1225 1230

Glu Pro Asn Lys Lys Tyr Thr Val Gln Phe Asp Tyr Gln Thr Asp
1235 1240 1245

Gly Glu Asn Val Phe Thr Ala Gly Thr Ile Asn Gly Glu Leu Lys
1250 1255 1260

Asn Asn Asn Asp Phe Lys Pro Val Gly Glu Leu Thr Ser Thr Ala
1265 1270 1275

Ala Asp Gly Gln Thr Lys His Tyr Glu Ala Glu Ile Ile Gly Asp
1280 1285 1290

Ala Ser Gly Asn Thr Thr Phe Gly Ile Phe Thr Thr Gly Ala Asp
1295 1300 1305

Lys Asp Phe Ile Met Asp Asn Phe Thr Val Thr Val Glu Ser Lys
1310 1315 1320

Lys

<210> 13
<211> 26
<212> DNA
<213> Artificial

<220>
<223> Primer

<400> 13
ttaacctcca tgggcagcgg gggagg 26

<210> 14
<211> 30
<212> DNA
<213> Artificial

<220>
<223> Primer

<400> 14
aacctgcggc cgccagttgc tcgcgattgc 30

<210> 15
<211> 20
<212> DNA
<213> Artificial

<220>
<223> Primer

<400> 15
tgcgattcat cgcctagcag 20

<210> 16
<211> 8
<212> PRT
<213> Homo sapiens

<220>
<223> Primer

<400> 16

```
        Ala Ser Thr Thr Thr Asn Tyr Thr
        1               5
```

<210> 17
<211> 12
<212> PRT
<213> Homo sapiens

<220>
<223> Primer

<400> 17

```
        Phe Arg Lys Lys Trp Asn Lys Trp Ala Leu Ser Arg
        1               5                   10
```

<210> 18
<211> 9

<212> PRT
<213> Digitalis purpurea

<400> 18

```
Arg Pro Pro Gly Phe Ser Pro Phe Arg
1               5
```

SEQUENCE LISTING

<110> Ajinomoto Co., Inc.

<120> Transglycosylation method and method for producing glycoprotein

<130> PAMA-05004-EP

<160> 18

<170> Patent In version 3.1

<210> 1
<211> 5901
<212> DNA
<213> Bifidobacterium lomgum JCM1217

<220>
<221> CDS
<222> (1).. (5901)

<400> 1

```
atg aaa aag aag aag act ata tcg gct gcg ctg gca aca gcg tta gcc        48
Met Lys Lys Lys Lys Thr Ile Ser Ala Ala Leu Ala Thr Ala Leu Ala
1               5               10              15

tta acc tgc atg ggc agc ggg gga ggt act gcg ttc gca gtg ccc ctg        96
Leu Thr Cys Met Gly Ser Gly Gly Gly Thr Ala Phe Ala Val Pro Leu
            20              25              30

tct gat gct gac ttg cag act ttg gca agt cag att cag caa atc aac       144
Ser Asp Ala Asp Leu Gln Thr Leu Ala Ser Gln Ile Gln Gln Ile Asn
        35              40              45

gat act tct gat tct gca act gct tcc gag act cct tcc gca caa gcc       192
Asp Thr Ser Asp Ser Ala Thr Ala Ser Glu Thr Pro Ser Ala Gln Ala
        50              55              60

gat gcg gtt gaa ggc tgg act att gat tcc aac atc gct cag ggc ggc       240
Asp Ala Val Glu Gly Trp Thr Ile Asp Ser Asn Ile Ala Gln Gly Gly
65              70              75              80

gaa atc ctg gag atg gca aac ggt tgg ctg cac ctc aag tcc act gcc       288
Glu Ile Leu Glu Met Ala Asn Gly Trp Leu His Leu Lys Ser Thr Ala
                85              90              95

tct aac ggt aat gcg gca gcg aac ccc agc tcc agc aac aac tgg ccg       336
Ser Asn Gly Asn Ala Ala Ala Asn Pro Ser Ser Ser Asn Asn Trp Pro
            100             105             110

gca gta gcc gta tgg ggc aca gat tac gac ttc tcc aag gcc ggc tcc       384
Ala Val Ala Val Trp Gly Thr Asp Tyr Asp Phe Ser Lys Ala Gly Ser
            115             120             125

ttc cac gcc acc atc aaa tcc ccg cag gaa ggc tcc gcc aac cgc ttc       432
Phe His Ala Thr Ile Lys Ser Pro Gln Glu Gly Ser Ala Asn Arg Phe
        130             135             140

ggc ttc tac ctg ggc tac aac gac ccg ggc agc ggc ctg ttc atc ggc       480
Gly Phe Tyr Leu Gly Tyr Asn Asp Pro Gly Ser Gly Leu Phe Ile Gly
145             150             155             160

tac gat tcg ggc ggc tgg ttc tgg cag acc tac acc ggt ggc ggt agc       528
Tyr Asp Ser Gly Gly Trp Phe Trp Gln Thr Tyr Thr Gly Gly Gly Ser
            165             170             175

ggc agc tgg tac agc ggt gct cgt atc gct gct ccg agc gcc aac gaa       576
Gly Ser Trp Tyr Ser Gly Ala Arg Ile Ala Ala Pro Ser Ala Asn Glu
            180             185             190

gag cac gac att cgg gtc tcc tgg acc gac gcc aag gtc gcc aca ctg       624
Glu His Asp Ile Arg Val Ser Trp Thr Asp Ala Lys Val Ala Thr Leu
            195             200             205
```

```
acc gtg gat ggc cag aag gca ttc gat gtc gat tac tcc gca atg acg       672
Thr Val Asp Gly Gln Lys Ala Phe Asp Val Asp Tyr Ser Ala Met Thr
    210             215             220

aac ctc tcc aac aag ctt gcc atc aag gcc ggc tcc tgg aag gag ctg       720
Asn Leu Ser Asn Lys Leu Ala Ile Lys Ala Gly Ser Trp Lys Glu Leu
225             230             235             240

aac gag gtc acc gac gtg tac atc aag gac ttc ccg gag gtt gtc gaa       768
Asn Glu Val Thr Asp Val Tyr Ile Lys Asp Phe Pro Glu Val Val Glu
            245             250             255

gcc gcc aag cac gcg gtt tcc ggc aag gtt gtg gac gct gga ggc gct       816
Ala Ala Lys His Ala Val Ser Gly Lys Val Val Asp Ala Gly Gly Ala
        260             265             270

gcc att gaa ggc gca aca gtg cgt ttg gat aag acc aag gtg aag acc       864
Ala Ile Glu Gly Ala Thr Val Arg Leu Asp Lys Thr Lys Val Lys Thr
    275             280             285

ggt gca gat ggc acc ttc tcc ttc gcc gac att gaa gaa ggc gaa cac       912
Gly Ala Asp Gly Thr Phe Ser Phe Ala Asp Ile Glu Glu Gly Glu His
    290             295             300

acg ctt tcg att gcc aag gaa ggc tat gag gac gtc tcc cag cag gtg       960
Thr Leu Ser Ile Ala Lys Glu Gly Tyr Glu Asp Val Ser Gln Gln Val
305             310             315             320

acg gtc tcc ggc gcg gat ctt gcc atc gat ccc atc acc ctg aac aaa      1008
Thr Val Ser Gly Ala Asp Leu Ala Ile Asp Pro Ile Thr Leu Asn Lys
            325             330             335

acc gtt cag gtc gcc tcc gaa acg ctg aag acc aag aag atg gaa gtt      1056
Thr Val Gln Val Ala Ser Glu Thr Leu Lys Thr Lys Lys Met Glu Val
            340             345             350

cag att aag aag aac ttc ccc tct gtg ctg cag tac acg atg acc gac      1104
Gln Ile Lys Lys Asn Phe Pro Ser Val Leu Gln Tyr Thr Met Thr Asp
        355             360             365

ggc aag gtg atg tac ggc cag tcc aag gat gtg cgc acc gtc gaa atc      1152
Gly Lys Val Met Tyr Gly Gln Ser Lys Asp Val Arg Thr Val Glu Ile
    370             375             380

aac ggc acc aac atc gaa ctg ggc gat gac gac gtg acc ttc aag aag      1200
Asn Gly Thr Asn Ile Glu Leu Gly Asp Asp Asp Val Thr Phe Lys Lys
385             390             395             400

gtt tcc gac acc gag gcc acc tac acg ctg aag gtc aag gat gag gcc      1248
Val Ser Asp Thr Glu Ala Thr Tyr Thr Leu Lys Val Lys Asp Glu Ala
            405             410             415

aag aag att gac gcg gtg atc acc gtt cag atc acg gtc aag gcc aac      1296
Lys Lys Ile Asp Ala Val Ile Thr Val Gln Ile Thr Val Lys Ala Asn
            420             425             430

cag ctg cac ctc aac gtc acc aag atc aag aac aac ctg tcc gaa ggc      1344
Gln Leu His Leu Asn Val Thr Lys Ile Lys Asn Asn Leu Ser Glu Gly
        435             440             445

att cct gag ggc aac ggt gtg gag gag aac gcc atc cag acg ctg tcc      1392
Ile Pro Glu Gly Asn Gly Val Glu Glu Asn Ala Ile Gln Thr Leu Ser
    450             455             460

ttc ccg aac cag agt ctc gtt tcc gtg cgt tcc agc cag gaa aat gcc      1440
Phe Pro Asn Gln Ser Leu Val Ser Val Arg Ser Ser Gln Glu Asn Ala
465             470             475             480

caa ttc act ggt gct cgt atg tct tcc aac acg cag aag cct ggc gat      1488
Gln Phe Thr Gly Ala Arg Met Ser Ser Asn Thr Gln Lys Pro Gly Asp
            485             490             495

acc aac ttc gca gtg acc gaa gat act aac gtc acc gat agc gac tac      1536
Thr Asn Phe Ala Val Thr Glu Asp Thr Asn Val Thr Asp Ser Asp Tyr
            500             505             510
```

97

```
acc tac ggc ttc atc tcc ggt gct ggc ctg agt gcc ggc ctg tgg agc      1584
Thr Tyr Gly Phe Ile Ser Gly Ala Gly Leu Ser Ala Gly Leu Trp Ser
        515             520             525

aac tcc gag cac gat ggc acc tat gtg gcg gct cct gtg cgc ggc ggc      1632
Asn Ser Glu His Asp Gly Thr Tyr Val Ala Ala Pro Val Arg Gly Gly
    530             535             540

agc cag aac acg cgt gtc tac gcc acc acc cag cag act ggt gac gcc      1680
Ser Gln Asn Thr Arg Val Tyr Ala Thr Thr Gln Gln Thr Gly Asp Ala
545             550             555             560

acc tcc ctg ggc ctg gcc agc gct ccg tgg tac tac cac cgc acg gtc      1728
Thr Ser Leu Gly Leu Ala Ser Ala Pro Trp Tyr Tyr His Arg Thr Val
        565             570             575

acc gat tcc aag ggc aag aag tac acc gtg gcc gaa acc gct ctg ccg      1776
Thr Asp Ser Lys Gly Lys Lys Tyr Thr Val Ala Glu Thr Ala Leu Pro
        580             585             590

cag atg gcc gtg gcc atc gcc ggc gac gag aac gaa gac ggt gcc gtc      1824
Gln Met Ala Val Ala Ile Ala Gly Asp Glu Asn Glu Asp Gly Ala Val
        595             600             605

aac tgg cag gat ggc gca atc gcc tac cgc gac atc atg aac aac ccg      1872
Asn Trp Gln Asp Gly Ala Ile Ala Tyr Arg Asp Ile Met Asn Asn Pro
    610             615             620

tac aag tcc gag gaa gtt ccc gaa ctg gtg gca tgg cgt atc gcc atg      1920
Tyr Lys Ser Glu Glu Val Pro Glu Leu Val Ala Trp Arg Ile Ala Met
625             630             635             640

aac ttc ggc tcc cag gcg cag aac ccg ttc ctc acc acg ctt gac aac      1968
Asn Phe Gly Ser Gln Ala Gln Asn Pro Phe Leu Thr Thr Leu Asp Asn
        645             650             655

gtc aag aag gtg gcc ttg aac acc gac ggc ctc ggc cag tcc gtg ctg      2016
Val Lys Lys Val Ala Leu Asn Thr Asp Gly Leu Gly Gln Ser Val Leu
        660             665             670

ctc aag ggc tac ggc aat gaa ggc cac gac tcc ggc cac ccg gac tac      2064
Leu Lys Gly Tyr Gly Asn Glu Gly His Asp Ser Gly His Pro Asp Tyr
        675             680             685

ggc gat atc ggc cag cgt ctc ggc ggc gcc gac gac atg aac acc atg      2112
Gly Asp Ile Gly Gln Arg Leu Gly Gly Ala Asp Asp Met Asn Thr Met
        690             695             700

atg gaa gag ggc tcc aag tat ggc gct cgc ttc ggt gtg cac gtc aac      2160
Met Glu Glu Gly Ser Lys Tyr Gly Ala Arg Phe Gly Val His Val Asn
705             710             715             720

gcc tcc gaa atg tat ccg gaa gcc aag gcc ttc agc gag gac atg gtg      2208
Ala Ser Glu Met Tyr Pro Glu Ala Lys Ala Phe Ser Glu Asp Met Val
        725             730             735

cgc cgc aac tct gca ggc ggc ctg agc tac ggc tgg aac tgg ctt gat      2256
Arg Arg Asn Ser Ala Gly Gly Leu Ser Tyr Gly Trp Asn Trp Leu Asp
        740             745             750

cag ggt gtc ggc atc gac ggc atc tac gat ctg gca tcc ggt tct cgt      2304
Gln Gly Val Gly Ile Asp Gly Ile Tyr Asp Leu Ala Ser Gly Ser Arg
        755             760             765

gta agc cgt ttc gct gac ctc agc aag gaa gtc ggc gac aac atg gac      2352
Val Ser Arg Phe Ala Asp Leu Ser Lys Glu Val Gly Asp Asn Met Asp
        770             775             780

ttc atc tac ctc gat gtg tgg ggc aac ctg act tct tcc ggt tcg gaa      2400
Phe Ile Tyr Leu Asp Val Trp Gly Asn Leu Thr Ser Ser Gly Ser Glu
785             790             795             800

gat tct tgg gaa acc cgc aag atg agc aag atg atc aac gac aac ggc      2448
Asp Ser Trp Glu Thr Arg Lys Met Ser Lys Met Ile Asn Asp Asn Gly
            805             810             815
```

```
tgg cgt atg acc acc gaa tgg ggt tcc ggc aac gag tac gac tcc acc      2496
Trp Arg Met Thr Thr Glu Trp Gly Ser Gly Asn Glu Tyr Asp Ser Thr
            820               825               830

ttc cag cac tgg gca gct gat ctg acc tac ggc ggc tac acc tcc aag      2544
Phe Gln His Trp Ala Ala Asp Leu Thr Tyr Gly Gly Tyr Thr Ser Lys
        835               840               845

ggc gag aac tcc gaa gtg atg cgc ttc ctg cgc aac cac cag aag gac      2592
Gly Glu Asn Ser Glu Val Met Arg Phe Leu Arg Asn His Gln Lys Asp
    850               855               860

agc tgg gtt ggc gac tac ccg caa tac ggc ggc gct gcc aac gcc ccg      2640
Ser Trp Val Gly Asp Tyr Pro Gln Tyr Gly Gly Ala Ala Asn Ala Pro
865               870               875               880

ctg ctc ggc ggc tac aac atg aag gac ttc gaa ggc tgg cag ggc cgc      2688
Leu Leu Gly Gly Tyr Asn Met Lys Asp Phe Glu Gly Trp Gln Gly Arg
                885               890               895

aac gac tat gcc gcc tac atc aag aac ctg tac acc cat gat gtg tcc      2736
Asn Asp Tyr Ala Ala Tyr Ile Lys Asn Leu Tyr Thr His Asp Val Ser
                900               905               910

act aag ttc atc cag cac ttc aag gtg acc cgc tgg gtc aac aac ccg      2784
Thr Lys Phe Ile Gln His Phe Lys Val Thr Arg Trp Val Asn Asn Pro
            915               920               925

ctg ctg acc gcc gac aat ggc aat gcc gct gcc gtg tcc gac ccg aac      2832
Leu Leu Thr Ala Asp Asn Gly Asn Ala Ala Ala Val Ser Asp Pro Asn
        930               935               940

acg aac aac ggc aac gag cag att acc ctg aag gat tcc aac ggc aac      2880
Thr Asn Asn Gly Asn Glu Gln Ile Thr Leu Lys Asp Ser Asn Gly Asn
945               950               955               960

gtt gta gta gtc tcc cgt ggt tcc aac gac acc tct agc gca gcc tac      2928
Val Val Val Val Ser Arg Gly Ser Asn Asp Thr Ser Ser Ala Ala Tyr
                965               970               975

cgc cag cgc acc atc acc ttc aac ggc gtg aag gtc gca tcc ggt gtg      2976
Arg Gln Arg Thr Ile Thr Phe Asn Gly Val Lys Val Ala Ser Gly Val
            980               985               990

gtc tcc gca ggc gat ggc agc gcc  act ggc gac gag tcc  tac ctg ctg    3024
Val Ser Ala Gly Asp Gly Ser Ala  Thr Gly Asp Glu Ser  Tyr Leu Leu
            995               1000              1005

ccg tgg  atg tgg gat tcc ttc  acc ggc aag ctg gtc  aag gat tcc       3069
Pro Trp  Met Trp Asp Ser Phe  Thr Gly Lys Leu Val  Lys Asp Ser
    1010              1015              1020

gag cag  aag ctc tac cac tgg  aac acc aag ggc ggc  acc acc acc       3114
Glu Gln  Lys Leu Tyr His Trp  Asn Thr Lys Gly Gly  Thr Thr Thr
    1025              1030              1035

tgg acg  ctg ccg gac agc tgg  aag aac ctc tcc agc  gta aag gtg       3159
Trp Thr  Leu Pro Asp Ser Trp  Lys Asn Leu Ser Ser  Val Lys Val
    1040              1045              1050

tac cag  ctc acc gat cag ggc  aag acc aac gag cag  acc gtt gcc       3204
Tyr Gln  Leu Thr Asp Gln Gly  Lys Thr Asn Glu Gln  Thr Val Ala
    1055              1060              1065

gtc tcc  ggc ggc aag gtg acg  ctt acc gct gat gcc  gaa acc ccg       3249
Val Ser  Gly Gly Lys Val Thr  Leu Thr Ala Asp Ala  Glu Thr Pro
    1070              1075              1080

tac gtg  gtg tac aag ggc gaa  gcc aag cag atc cag  gtc aac tgg       3294
Tyr Val  Val Tyr Lys Gly Glu  Ala Lys Gln Ile Gln  Val Asn Trp
    1085              1090              1095

agc gaa  ggc atg cat gtg gta  gac gcc ggc ttc aac  ggc ggc tcc       3339
Ser Glu  Gly Met His Val Val  Asp Ala Gly Phe Asn  Gly Gly Ser
    1100              1105              1110
```

EP 1 767 645 B1

```
aac acc ctc acc gac aac tgg acc gtc agc ggc tcc ggc aag gcc     3384
Asn Thr Leu Thr Asp Asn Trp Thr Val Ser Gly Ser Gly Lys Ala
    1115                1120                1125

gaa gtt gaa ggc gac aac aac gcc atg ctg cgc ctg acc ggc aag     3429
Glu Val Glu Gly Asp Asn Asn Ala Met Leu Arg Leu Thr Gly Lys
    1130                1135                1140

gtc gat gtc tcc cag cgt ctg acc gat ctc aag gct ggc cag aag     3474
Val Asp Val Ser Gln Arg Leu Thr Asp Leu Lys Ala Gly Gln Lys
    1145                1150                1155

tac gcg ctg tat gtt ggc gtc gac aac cgc tcc acc ggc gat gca     3519
Tyr Ala Leu Tyr Val Gly Val Asp Asn Arg Ser Thr Gly Asp Ala
    1160                1165                1170

tcc gtc acc gta acc agc ggc ggc aag gtg ctg gcc acc aac tcc     3564
Ser Val Thr Val Thr Ser Gly Gly Lys Val Leu Ala Thr Asn Ser
    1175                1180                1185

acc ggc aag tcc atc gcc aag aac tac atc aag gca tac ggc cac     3609
Thr Gly Lys Ser Ile Ala Lys Asn Tyr Ile Lys Ala Tyr Gly His
    1190                1195                1200

aac acg aac agc aat acg gaa aat ggc tcc agc tac ttc cag aac     3654
Asn Thr Asn Ser Asn Thr Glu Asn Gly Ser Ser Tyr Phe Gln Asn
    1205                1210                1215

atg tac gtg ttc ttc acc gcg cct gag aac ggc gat gcc acg gta     3699
Met Tyr Val Phe Phe Thr Ala Pro Glu Asn Gly Asp Ala Thr Val
    1220                1225                1230

acc ctg tct cac aag agc acc gac gga gca cac acc tac ttc gac     3744
Thr Leu Ser His Lys Ser Thr Asp Gly Ala His Thr Tyr Phe Asp
    1235                1240                1245

gat gtg cgc atc gtg gag aac cag tac tcc ggc atc acc tat gag     3789
Asp Val Arg Ile Val Glu Asn Gln Tyr Ser Gly Ile Thr Tyr Glu
    1250                1255                1260

aag gac ggc acg ctg aag tcc ctc acc aac gga ttc gaa aac aac     3834
Lys Asp Gly Thr Leu Lys Ser Leu Thr Asn Gly Phe Glu Asn Asn
    1265                1270                1275

gcc cag ggc atc tgg ccg ttc gtg gtc tcc ggt tcc gaa ggc gtt     3879
Ala Gln Gly Ile Trp Pro Phe Val Val Ser Gly Ser Glu Gly Val
    1280                1285                1290

gag gac aac cgc atc cac ctc tcc gag ctg cat gct ccg ttc acg     3924
Glu Asp Asn Arg Ile His Leu Ser Glu Leu His Ala Pro Phe Thr
    1295                1300                1305

cgg gcc ggt tgg gat gtc aag aag atg gac gat gtg ctc gat ggc     3969
Arg Ala Gly Trp Asp Val Lys Lys Met Asp Asp Val Leu Asp Gly
    1310                1315                1320

act tgg tct gtg aag gtt aac ggc ctg acc cag aag ggc acg ctg     4014
Thr Trp Ser Val Lys Val Asn Gly Leu Thr Gln Lys Gly Thr Leu
    1325                1330                1335

gtc tac cag acg atc ccg cag aac gtg aag ttc gag gcg ggt gcc     4059
Val Tyr Gln Thr Ile Pro Gln Asn Val Lys Phe Glu Ala Gly Ala
    1340                1345                1350

aag tac aag gtg agc ttc gac tac cag tcc ggt tcc gat gac atc     4104
Lys Tyr Lys Val Ser Phe Asp Tyr Gln Ser Gly Ser Asp Asp Ile
    1355                1360                1365

tac gcc atc gct gtg ggc cag ggt gaa tac tct gcc ggc agc gtg     4149
Tyr Ala Ile Ala Val Gly Gln Gly Glu Tyr Ser Ala Gly Ser Val
    1370                1375                1380

aag ctg acc aac ctg aag aag gct ctg ggt gag acc ggc aag gcc     4194
Lys Leu Thr Asn Leu Lys Lys Ala Leu Gly Glu Thr Gly Lys Ala
    1385                1390                1395
```

100

```
gag ttc  gag ctg acc ggt ggc  gtc aac ggc gat tcc  tgg ttc ggt      4239
Glu Phe  Glu Leu Thr Gly Gly  Val Asn Gly Asp Ser  Trp Phe Gly
    1400              1405              1410

att tac  tcg acc gca acc gca  cct gat ctg cag ggt  tcc acc ggc      4284
Ile Tyr  Ser Thr Ala Thr Ala  Pro Asp Leu Gln Gly  Ser Thr Gly
    1415              1420              1425

aat gca  cag gac ttc ggc gga  tac aag gac ttc gtg  ctc gac aac      4329
Asn Ala  Gln Asp Phe Gly Gly  Tyr Lys Asp Phe Val  Leu Asp Asn
    1430              1435              1440

ctg aag  atc gag cgc atc gag  tcc cag acc cgc acc  aag gcc gaa      4374
Leu Lys  Ile Glu Arg Ile Glu  Ser Gln Thr Arg Thr  Lys Ala Glu
    1445              1450              1455

gcg cag  gac aag gtc aag gaa  atc cgc ggc aag tac  gat tcc aag      4419
Ala Gln  Asp Lys Val Lys Glu  Ile Arg Gly Lys Tyr  Asp Ser Lys
    1460              1465              1470

cgt gct  gag ctc tcc gat gcc  gca tgg cag cag tat  cag gac acc      4464
Arg Ala  Glu Leu Ser Asp Ala  Ala Trp Gln Gln Tyr  Gln Asp Thr
    1475              1480              1485

ttg gtc  aag gct cgc gtg ctc  atc aac aag aat ggc  gca acc gct      4509
Leu Val  Lys Ala Arg Val Leu  Ile Asn Lys Asn Gly  Ala Thr Ala
    1490              1495              1500

gag gac  ttc acc aag gca tac  gac att ctc gtg gcc  ctc gac gag      4554
Glu Asp  Phe Thr Lys Ala Tyr  Asp Ile Leu Val Ala  Leu Asp Glu
    1505              1510              1515

tac atg  aag acc gct ccc ggc  aac gag agc agc gac  aag tac gac      4599
Tyr Met  Lys Thr Ala Pro Gly  Asn Glu Ser Ser Asp  Lys Tyr Asp
    1520              1525              1530

gtg gca  gct gac ggc tcc gat  gag ctg ggt ggc tac  acc gtg gcc      4644
Val Ala  Ala Asp Gly Ser Asp  Glu Leu Gly Gly Tyr  Thr Val Ala
    1535              1540              1545

acg ggc  agc gaa gag cct acc  gca ggc ctg ccg agc  gaa ggc ccg      4689
Thr Gly  Ser Glu Glu Pro Thr  Ala Gly Leu Pro Ser  Glu Gly Pro
    1550              1555              1560

gcc gat  ctg gca cag gat ggc  aac gac agc acc cac  tgg cac acc      4734
Ala Asp  Leu Ala Gln Asp Gly  Asn Asp Ser Thr His  Trp His Thr
    1565              1570              1575

agc tgg  agc gag aac gca gtc  ggc aac ggc acc gca  tgg tat cag      4779
Ser Trp  Ser Glu Asn Ala Val  Gly Asn Gly Thr Ala  Trp Tyr Gln
    1580              1585              1590

ttc aac  ctc aac gaa ccg acc  acc atc aac ggc ctg  cgc tac ctg      4824
Phe Asn  Leu Asn Glu Pro Thr  Thr Ile Asn Gly Leu  Arg Tyr Leu
    1595              1600              1605

ccg cgc  tcc gga ggt atg aac  gcc aac ggc aag atc  aag ggc tac      4869
Pro Arg  Ser Gly Gly Met Asn  Ala Asn Gly Lys Ile  Lys Gly Tyr
    1610              1615              1620

aag atc  acg ctc act ctg gcg  gat ggc acc acc aag  gat gtc gtc      4914
Lys Ile  Thr Leu Thr Leu Ala  Asp Gly Thr Thr Lys  Asp Val Val
    1625              1630              1635

acc gat  gct gag ttc tcc acc  acc acc atg tgg cag  aag gcc agc      4959
Thr Asp  Ala Glu Phe Ser Thr  Thr Thr Met Trp Gln  Lys Ala Ser
    1640              1645              1650

ttc gac  gcc gtc gag aat gtg  acc gcc gta cgc tcg  acc gtc ctg      5004
Phe Asp  Ala Val Glu Asn Val  Thr Ala Val Arg Ser  Thr Val Leu
    1655              1660              1665

tct tcc  gca ggc cag agc gac  tcc cag gcc aac aag  ttc gca tcc      5049
Ser Ser  Ala Gly Gln Ser Asp  Ser Gln Ala Asn Lys  Phe Ala Ser
    1670              1675              1680
```

```
gct gcc  gaa ctg cgt ttg acc  acg gac cgc gag gtt  gag gaa gag      5094
Ala Ala  Glu Leu Arg Leu Thr  Thr Asp Arg Glu Val  Glu Glu Glu
    1685              1690              1695

act gtc  gct ccg gac aag acc  gac ctc aac gac acc  atc gcc aag      5139
Thr Val  Ala Pro Asp Lys Thr  Asp Leu Asn Asp Thr  Ile Ala Lys
    1700              1705              1710

gct aac  ggt ctt aag gaa tcc  gac tac acg gct gaa  agc tgg act      5184
Ala Asn  Gly Leu Lys Glu Ser  Asp Tyr Thr Ala Glu  Ser Trp Thr
    1715              1720              1725

gct ctg  gtc aag gcc cgc gaa  gct gca cag gcc gtg  gcg gat aac      5229
Ala Leu  Val Lys Ala Arg Glu  Ala Ala Gln Ala Val  Ala Asp Asn
    1730              1735              1740

gat aag  gcc acc gct tac gat  gtg gct ctg gcg ctg  acg aac ctc      5274
Asp Lys  Ala Thr Ala Tyr Asp  Val Ala Leu Ala Leu  Thr Asn Leu
    1745              1750              1755

gaa tcc  gct atc gct ggt ctc  gag aag acc ggt gag  gag cct ggc      5319
Glu Ser  Ala Ile Ala Gly Leu  Glu Lys Thr Gly Glu  Glu Pro Gly
    1760              1765              1770

cca ggc  ccg gtt gag gtg aac  aag acc gac ctg cag  act gca gtg      5364
Pro Gly  Pro Val Glu Val Asn  Lys Thr Asp Leu Gln  Thr Ala Val
    1775              1780              1785

aac aag  gca agc aag ctc gag  aag gcc gat tac acg  acc aac tcg      5409
Asn Lys  Ala Ser Lys Leu Glu  Lys Ala Asp Tyr Thr  Thr Asn Ser
    1790              1795              1800

tgg gaa  gct ttc gcc gag gca  ctg aag gct gca cag  cag gtg ctc      5454
Trp Glu  Ala Phe Ala Glu Ala  Leu Lys Ala Ala Gln  Gln Val Leu
    1805              1810              1815

gac aac  aag aac gcc acc cag  cag gat gtg gat acc  gca ctg agc      5499
Asp Asn  Lys Asn Ala Thr Gln  Gln Asp Val Asp Thr  Ala Leu Ser
    1820              1825              1830

gct ctt  cag gac gcc atc tcc  aag ctg gaa gct gcc  acc gag ccg      5544
Ala Leu  Gln Asp Ala Ile Ser  Lys Leu Glu Ala Ala  Thr Glu Pro
    1835              1840              1845

aag ccg  aat ccg gaa ccg ggc  gtg gtg gac aag gct  gct ctg aac      5589
Lys Pro  Asn Pro Glu Pro Gly  Val Val Asp Lys Ala  Ala Leu Asn
    1850              1855              1860

gcg acc  atc aac aag gcc gcc  gcc atc aac ctg ggt  ctc tac acc      5634
Ala Thr  Ile Asn Lys Ala Ala  Ala Ile Asn Leu Gly  Leu Tyr Thr
    1865              1870              1875

gac gac  tcc gcc aac gct ctg  cgc gcc gcg ctg aag  aag gcc cgt      5679
Asp Asp  Ser Ala Asn Ala Leu  Arg Ala Ala Leu Lys  Lys Ala Arg
    1880              1885              1890

gag gtc  tcc gac aac agc aac  gcc acg cag aag cag  gtc gac gca      5724
Glu Val  Ser Asp Asn Ser Asn  Ala Thr Gln Lys Gln  Val Asp Ala
    1895              1900              1905

gct cgt  gaa gcc ctg gag aag  gca att gcc gct ctg  gtg aag cgt      5769
Ala Arg  Glu Ala Leu Glu Lys  Ala Ile Ala Ala Leu  Val Lys Arg
    1910              1915              1920

ccc gct  gcc aag ggt gat ggc  aac gtt gtt tcc aac  acg ggc tcc      5814
Pro Ala  Ala Lys Gly Asp Gly  Asn Val Val Ser Asn  Thr Gly Ser
    1925              1930              1935

gat gtc  gcc acg atc gct ctg  gct gga ctg ctg ctg  gca ggt gct      5859
Asp Val  Ala Thr Ile Ala Leu  Ala Gly Leu Leu Leu  Ala Gly Ala
    1940              1945              1950

ggc gcc  gcc atc gcc tac cgt  cgc aat cgc gag caa  ctg tga          5901
Gly Ala  Ala Ile Ala Tyr Arg  Arg Asn Arg Glu Gln  Leu
    1955              1960              1965
```

<210> 2
<211> 1966
<212> PRT
<213> Bifidobacterium lomgum JCM1217

<400> 2

```
Met Lys Lys Lys Thr Ile Ser Ala Ala Leu Ala Thr Ala Leu Ala
1               5               10              15

Leu Thr Cys Met Gly Ser Gly Gly Thr Ala Phe Ala Val Pro Leu
            20              25              30   .

Ser Asp Ala Asp Leu Gln Thr Leu Ala Ser Gln Ile Gln Gln Ile Asn
        35              40              45

Asp Thr Ser Asp Ser Ala Thr Ala Ser Glu Thr Pro Ser Ala Gln Ala
        50              55              60

Asp Ala Val Glu Gly Trp Thr Ile Asp Ser Asn Ile Ala Gln Gly Gly
65              70              75              80

Glu Ile Leu Glu Met Ala Asn Gly Trp Leu His Leu Lys Ser Thr Ala
            85              90              95

Ser Asn Gly Asn Ala Ala Ala Asn Pro Ser Ser Ser Asn Asn Trp Pro
        100             105             110

Ala Val Ala Val Trp Gly Thr Asp Tyr Asp Phe Ser Lys Ala Gly Ser
        115             120             125

Phe His Ala Thr Ile Lys Ser Pro Gln Glu Gly Ser Ala Asn Arg Phe
    130             135             140

Gly Phe Tyr Leu Gly Tyr Asn Asp Pro Gly Ser Gly Leu Phe Ile Gly
145             150             155             160

Tyr Asp Ser Gly Gly Trp Phe Trp Gln Thr Tyr Thr Gly Gly Gly Ser
            165             170             175

Gly Ser Trp Tyr Ser Gly Ala Arg Ile Ala Ala Pro Ser Ala Asn Glu
            180             185             190

Glu His Asp Ile Arg Val Ser Trp Thr Asp Ala Lys Val Ala Thr Leu
    195             200             205

Thr Val Asp Gly Gln Lys Ala Phe Asp Val Asp Tyr Ser Ala Met Thr
    210             215             220

Asn Leu Ser Asn Lys Leu Ala Ile Lys Ala Gly Ser Trp Lys Glu Leu
225             230             235             240

Asn Glu Val Thr Asp Val Tyr Ile Lys Asp Phe Pro Glu Val Val Glu
            245             250             255

Ala Ala Lys His Ala Val Ser Gly Lys Val Val Asp Ala Gly Gly Ala
            260             265             270
```

Ala Ile Glu Gly Ala Thr Val Arg Leu Asp Lys Thr Lys Val Lys Thr
275 280 285

Gly Ala Asp Gly Thr Phe Ser Phe Ala Asp Ile Glu Glu Gly Glu His
290 295 300

Thr Leu Ser Ile Ala Lys Glu Gly Tyr Glu Asp Val Ser Gln Gln Val
305 310 315 320

Thr Val Ser Gly Ala Asp Leu Ala Ile Asp Pro Ile Thr Leu Asn Lys
325 330 335

Thr Val Gln Val Ala Ser Glu Thr Leu Lys Thr Lys Lys Met Glu Val
340 345 350

Gln Ile Lys Lys Asn Phe Pro Ser Val Leu Gln Tyr Thr Met Thr Asp
355 360 365

Gly Lys Val Met Tyr Gly Gln Ser Lys Asp Val Arg Thr Val Glu Ile
370 375 380

Asn Gly Thr Asn Ile Glu Leu Gly Asp Asp Asp Val Thr Phe Lys Lys
385 390 395 400

Val Ser Asp Thr Glu Ala Thr Tyr Thr Leu Lys Val Lys Asp Glu Ala
405 410 415

Lys Lys Ile Asp Ala Val Ile Thr Val Gln Ile Thr Val Lys Ala Asn
420 425 430

Gln Leu His Leu Asn Val Thr Lys Ile Lys Asn Asn Leu Ser Glu Gly
435 440 445

Ile Pro Glu Gly Asn Gly Val Glu Glu Asn Ala Ile Gln Thr Leu Ser
450 455 460

Phe Pro Asn Gln Ser Leu Val Ser Val Arg Ser Ser Gln Glu Asn Ala
465 470 475 480

Gln Phe Thr Gly Ala Arg Met Ser Ser Asn Thr Gln Lys Pro Gly Asp
485 490 495

Thr Asn Phe Ala Val Thr Glu Asp Thr Asn Val Thr Asp Ser Asp Tyr
500 505 510

Thr Tyr Gly Phe Ile Ser Gly Ala Gly Leu Ser Ala Gly Leu Trp Ser
515 520 525

Asn Ser Glu His Asp Gly Thr Tyr Val Ala Ala Pro Val Arg Gly Gly
530 535 540

Ser Gln Asn Thr Arg Val Tyr Ala Thr Thr Gln Gln Thr Gly Asp Ala
545 550 555 560

Thr Ser Leu Gly Leu Ala Ser Ala Pro Trp Tyr Tyr His Arg Thr Val
565 570 575

Thr Asp Ser Lys Gly Lys Lys Tyr Thr Val Ala Glu Thr Ala Leu Pro
            580                 585                 590

Gln Met Ala Val Ala Ile Ala Gly Asp Glu Asn Glu Asp Gly Ala Val
            595                 600                 605

Asn Trp Gln Asp Gly Ala Ile Ala Tyr Arg Asp Ile Met Asn Asn Pro
            610                 615                 620

Tyr Lys Ser Glu Glu Val Pro Glu Leu Val Ala Trp Arg Ile Ala Met
625                 630                 635                 640

Asn Phe Gly Ser Gln Ala Gln Asn Pro Phe Leu Thr Thr Leu Asp Asn
            645                 650                 655

Val Lys Lys Val Ala Leu Asn Thr Asp Gly Leu Gly Gln Ser Val Leu
            660                 665                 670

Leu Lys Gly Tyr Gly Asn Glu Gly His Asp Ser Gly His Pro Asp Tyr
            675                 680                 685

Gly Asp Ile Gly Gln Arg Leu Gly Gly Ala Asp Asp Met Asn Thr Met
            690                 695                 700

Met Glu Glu Gly Ser Lys Tyr Gly Ala Arg Phe Gly Val His Val Asn
705                 710                 715                 720

Ala Ser Glu Met Tyr Pro Glu Ala Lys Ala Phe Ser Glu Asp Met Val
            725                 730                 735

Arg Arg Asn Ser Ala Gly Gly Leu Ser Tyr Gly Trp Asn Trp Leu Asp
            740                 745                 750

Gln Gly Val Gly Ile Asp Gly Ile Tyr Asp Leu Ala Ser Gly Ser Arg
            755                 760                 765

Val Ser Arg Phe Ala Asp Leu Ser Lys Glu Val Gly Asp Asn Met Asp
            770                 775                 780

Phe Ile Tyr Leu Asp Val Trp Gly Asn Leu Thr Ser Ser Gly Ser Glu
785                 790                 795                 800

Asp Ser Trp Glu Thr Arg Lys Met Ser Lys Met Ile Asn Asp Asn Gly
            805                 810                 815

Trp Arg Met Thr Thr Glu Trp Gly Ser Gly Asn Glu Tyr Asp Ser Thr
            820                 825                 830

Phe Gln His Trp Ala Ala Asp Leu Thr Tyr Gly Gly Tyr Thr Ser Lys
            835                 840                 845

Gly Glu Asn Ser Glu Val Met Arg Phe Leu Arg Asn His Gln Lys Asp
            850                 855                 860

Ser Trp Val Gly Asp Tyr Pro Gln Tyr Gly Gly Ala Ala Asn Ala Pro
865                 870                 875                 880

Leu Leu Gly Gly Tyr Asn Met Lys Asp Phe Glu Gly Trp Gln Gly Arg
885 890 895

Asn Asp Tyr Ala Ala Tyr Ile Lys Asn Leu Tyr Thr His Asp Val Ser
900 905 910

Thr Lys Phe Ile Gln His Phe Lys Val Thr Arg Trp Val Asn Asn Pro
915 920 925

Leu Leu Thr Ala Asp Asn Gly Asn Ala Ala Ala Val Ser Asp Pro Asn
930 935 940

Thr Asn Asn Gly Asn Glu Gln Ile Thr Leu Lys Asp Ser Asn Gly Asn
945 950 955 960

Val Val Val Val Ser Arg Gly Ser Asn Asp Thr Ser Ser Ala Ala Tyr
965 970 975

Arg Gln Arg Thr Ile Thr Phe Asn Gly Val Lys Val Ala Ser Gly Val
980 985 990

Val Ser Ala Gly Asp Gly Ser Ala Thr Gly Asp Glu Ser Tyr Leu Leu
995 1000 1005

Pro Trp Met Trp Asp Ser Phe Thr Gly Lys Leu Val Lys Asp Ser
1010 1015 1020

Glu Gln Lys Leu Tyr His Trp Asn Thr Lys Gly Gly Thr Thr Thr
1025 1030 1035

Trp Thr Leu Pro Asp Ser Trp Lys Asn Leu Ser Ser Val Lys Val
1040 1045 1050

Tyr Gln Leu Thr Asp Gln Gly Lys Thr Asn Glu Gln Thr Val Ala
1055 1060 1065

Val Ser Gly Gly Lys Val Thr Leu Thr Ala Asp Ala Glu Thr Pro
1070 1075 1080

Tyr Val Val Tyr Lys Gly Glu Ala Lys Gln Ile Gln Val Asn Trp
1085 1090 1095

Ser Glu Gly Met His Val Val Asp Ala Gly Phe Asn Gly Gly Ser
1100 1105 1110

Asn Thr Leu Thr Asp Asn Trp Thr Val Ser Gly Ser Gly Lys Ala
1115 1120 1125

Glu Val Glu Gly Asp Asn Asn Ala Met Leu Arg Leu Thr Gly Lys
1130 1135 1140

Val Asp Val Ser Gln Arg Leu Thr Asp Leu Lys Ala Gly Gln Lys
1145 1150 1155

Tyr Ala Leu Tyr Val Gly Val Asp Asn Arg Ser Thr Gly Asp Ala
1160 1165 1170

```
Ser Val  Thr Val Thr Ser Gly  Gly Lys Val Leu Ala  Thr Asn Ser
    1175             1180              1185

Thr Gly  Lys Ser Ile Ala Lys  Asn Tyr Ile Lys Ala  Tyr Gly His
    1190             1195              1200

Asn Thr  Asn Ser Asn Thr Glu  Asn Gly Ser Ser Tyr  Phe Gln Asn
    1205             1210              1215

Met Tyr  Val Phe Phe Thr Ala  Pro Glu Asn Gly Asp  Ala Thr Val
    1220             1225              1230

Thr Leu  Ser His Lys Ser Thr  Asp Gly Ala His Thr  Tyr Phe Asp
    1235             1240              1245

Asp Val  Arg Ile Val Glu Asn  Gln Tyr Ser Gly Ile  Thr Tyr Glu
    1250             1255              1260

Lys Asp  Gly Thr Leu Lys Ser  Leu Thr Asn Gly Phe  Glu Asn Asn
    1265             1270              1275

Ala Gln  Gly Ile Trp Pro Phe  Val Val Ser Gly Ser  Glu Gly Val
    1280             1285              1290

Glu Asp  Asn Arg Ile His Leu  Ser Glu Leu His Ala  Pro Phe Thr
    1295             1300              1305

Arg Ala  Gly Trp Asp Val Lys  Lys Met Asp Asp Val  Leu Asp Gly
    1310             1315              1320

Thr Trp  Ser Val Lys Val Asn  Gly Leu Thr Gln Lys  Gly Thr Leu
    1325             1330              1335

Val Tyr  Gln Thr Ile Pro Gln  Asn Val Lys Phe Glu  Ala Gly Ala
    1340             1345              1350

Lys Tyr  Lys Val Ser Phe Asp  Tyr Gln Ser Gly Ser  Asp Asp Ile
    1355             1360              1365

Tyr Ala  Ile Ala Val Gly Gln  Gly Glu Tyr Ser Ala  Gly Ser Val
    1370             1375              1380

Lys Leu  Thr Asn Leu Lys Lys  Ala Leu Gly Glu Thr  Gly Lys Ala
    1385             1390              1395

Glu Phe  Glu Leu Thr Gly Gly  Val Asn Gly Asp Ser  Trp Phe Gly
    1400             1405              1410

Ile Tyr  Ser Thr Ala Thr Ala  Pro Asp Leu Gln Gly  Ser Thr Gly
    1415             1420              1425

Asn Ala  Gln Asp Phe Gly Gly  Tyr Lys Asp Phe Val  Leu Asp Asn
    1430             1435              1440

Leu Lys  Ile Glu Arg Ile Glu  Ser Gln Thr Arg Thr  Lys Ala Glu
    1445             1450              1455
```

Ala Gln Asp Lys Val Lys Glu Ile Arg Gly Lys Tyr Asp Ser Lys
1460             1465             1470

Arg Ala Glu Leu Ser Asp Ala Ala Trp Gln Gln Tyr Gln Asp Thr
1475             1480             1485

Leu Val Lys Ala Arg Val Leu Ile Asn Lys Asn Gly Ala Thr Ala
1490             1495             1500

Glu Asp Phe Thr Lys Ala Tyr Asp Ile Leu Val Ala Leu Asp Glu
1505             1510             1515

Tyr Met Lys Thr Ala Pro Gly Asn Glu Ser Ser Asp Lys Tyr Asp
1520             1525             1530

Val Ala Ala Asp Gly Ser Asp Glu Leu Gly Gly Tyr Thr Val Ala
1535             1540             1545

Thr Gly Ser Glu Glu Pro Thr Ala Gly Leu Pro Ser Glu Gly Pro
1550             1555             1560

Ala Asp Leu Ala Gln Asp Gly Asn Asp Ser Thr His Trp His Thr
1565             1570             1575

Ser Trp Ser Glu Asn Ala Val Gly Asn Gly Thr Ala Trp Tyr Gln
1580             1585             1590

Phe Asn Leu Asn Glu Pro Thr Thr Ile Asn Gly Leu Arg Tyr Leu
1595             1600             1605

Pro Arg Ser Gly Gly Met Asn Ala Asn Gly Lys Ile Lys Gly Tyr
1610             1615             1620

Lys Ile Thr Leu Thr Leu Ala Asp Gly Thr Thr Lys Asp Val Val
1625             1630             1635

Thr Asp Ala Glu Phe Ser Thr Thr Thr Met Trp Gln Lys Ala Ser
1640             1645             1650

Phe Asp Ala Val Glu Asn Val Thr Ala Val Arg Ser Thr Val Leu
1655             1660             1665

Ser Ser Ala Gly Gln Ser Asp Ser Gln Ala Asn Lys Phe Ala Ser
1670             1675             1680

Ala Ala Glu Leu Arg Leu Thr Thr Asp Arg Glu Val Glu Glu Glu
1685             1690             1695

Thr Val Ala Pro Asp Lys Thr Asp Leu Asn Asp Thr Ile Ala Lys
1700             1705             1710

Ala Asn Gly Leu Lys Glu Ser Asp Tyr Thr Ala Glu Ser Trp Thr
1715             1720             1725

Ala Leu Val Lys Ala Arg Glu Ala Ala Gln Ala Val Ala Asp Asn
1730             1735             1740

```
Asp Lys  Ala Thr Ala Tyr Asp  Val Ala Leu Ala Leu  Thr Asn Leu
    1745             1750             1755

Glu Ser  Ala Ile Ala Gly Leu  Glu Lys Thr Gly Glu  Glu Pro Gly
    1760             1765             1770

Pro Gly  Pro Val Glu Val Asn  Lys Thr Asp Leu Gln  Thr Ala Val
    1775             1780             1785

Asn Lys  Ala Ser Lys Leu Glu  Lys Ala Asp Tyr Thr  Thr Asn Ser
    1790             1795             1800

Trp Glu  Ala Phe Ala Glu Ala  Leu Lys Ala Ala Gln  Gln Val Leu
    1805             1810             1815

Asp Asn  Lys Asn Ala Thr Gln  Gln Asp Val Asp Thr  Ala Leu Ser
    1820             1825             1830

Ala Leu  Gln Asp Ala Ile Ser  Lys Leu Glu Ala Ala  Thr Glu Pro
    1835             1840             1845

Lys Pro  Asn Pro Glu Pro Gly  Val Val Asp Lys Ala  Ala Leu Asn
    1850             1855             1860

Ala Thr  Ile Asn Lys Ala Ala  Ala Ile Asn Leu Gly  Leu Tyr Thr
    1865             1870             1875

Asp Asp  Ser Ala Asn Ala Leu  Arg Ala Ala Leu Lys  Lys Ala Arg
    1880             1885             1890

Glu Val  Ser Asp Asn Ser Asn  Ala Thr Gln Lys Gln  Val Asp Ala
    1895             1900             1905

Ala Arg  Glu Ala Leu Glu Lys  Ala Ile Ala Ala Leu  Val Lys Arg
    1910             1915             1920

Pro Ala  Ala Lys Gly Asp Gly  Asn Val Val Ser Asn  Thr Gly Ser
    1925             1930             1935

Asp Val  Ala Thr Ile Ala Leu  Ala Gly Leu Leu Leu  Ala Gly Ala
    1940             1945             1950

Gly Ala  Ala Ile Ala Tyr Arg  Arg Asn Arg Glu Gln  Leu
    1955             1960             1965
```

<210> 3

<211> 5304

<212> DNA

<213> Streptococcus pneumoniae R6

<220>

<221> CDS

<222> (1).. (5304)

<400> 3

```
atg aat aaa gga tta ttt gaa aaa cgt tgt aaa tat agt att cgg aaa    48
Met Asn Lys Gly Leu Phe Glu Lys Arg Cys Lys Tyr Ser Ile Arg Lys
1               5                   10                  15

ttt tca tta ggt gtt gct tct gtt atg att gga gct aca ttc ttt ggg    96
Phe Ser Leu Gly Val Ala Ser Val Met Ile Gly Ala Thr Phe Phe Gly
```

```
                    20              25              30

aca agt ccg gtt ctt gca gat agc gtg cag tct ggt tcc acg gcg aac    144
Thr Ser Pro Val Leu Ala Asp Ser Val Gln Ser Gly Ser Thr Ala Asn
        35              40              45

tta cca gct gat tta gct act gct ctt gca aca gca aaa gag aat gat    192
Leu Pro Ala Asp Leu Ala Thr Ala Leu Ala Thr Ala Lys Glu Asn Asp
    50              55              60

ggg cat gat ttt gaa gcg cct aag gtg gga gaa gac caa ggt tct cca    240
Gly His Asp Phe Glu Ala Pro Lys Val Gly Glu Asp Gln Gly Ser Pro
65              70              75              80

gaa gtt aca gat gga cct aag aca gaa gaa gaa cta tta gca ctt gaa    288
Glu Val Thr Asp Gly Pro Lys Thr Glu Glu Glu Leu Leu Ala Leu Glu
                85              90              95

aaa gaa aaa ccg gct gaa gaa aaa cca aaa gag gat aaa cct gca gct    336
Lys Glu Lys Pro Ala Glu Glu Lys Pro Lys Glu Asp Lys Pro Ala Ala
            100             105             110

gct aaa cct gaa aca cct aag acg gta acc cct gaa tgg caa acg gta    384
Ala Lys Pro Glu Thr Pro Lys Thr Val Thr Pro Glu Trp Gln Thr Val
        115             120             125

gag aaa aaa gaa caa cag gga aca gtc act atc cga gaa gaa aaa ggt    432
Glu Lys Lys Glu Gln Gln Gly Thr Val Thr Ile Arg Glu Glu Lys Gly
    130             135             140

gtc cgc tac aac caa tta tcc tca act gct caa aat gat aac gca ggt    480
Val Arg Tyr Asn Gln Leu Ser Ser Thr Ala Gln Asn Asp Asn Ala Gly
145             150             155             160

aaa cca gcc ctg ttt gaa aag aag ggc ttg acc gtt gat gcc aat gga    528
Lys Pro Ala Leu Phe Glu Lys Lys Gly Leu Thr Val Asp Ala Asn Gly
                165             170             175

aat gca act gtt gat tta acc ttc aaa gat gat tct gaa aag ggc aaa    576
Asn Ala Thr Val Asp Leu Thr Phe Lys Asp Asp Ser Glu Lys Gly Lys
            180             185             190

tca cgc ttt ggt gtc ttc ttg aaa ttt aaa gat acc aag aat aat gtt    624
Ser Arg Phe Gly Val Phe Leu Lys Phe Lys Asp Thr Lys Asn Asn Val
        195             200             205

ttt gtc ggt tac gac aag gat ggc tgg ttc tgg gag tat aaa tct cca    672
Phe Val Gly Tyr Asp Lys Asp Gly Trp Phe Trp Glu Tyr Lys Ser Pro
    210             215             220

aca act agc act tgg tat aga ggt agt cgt gtt gct gct cct gaa aca    720
Thr Thr Ser Thr Trp Tyr Arg Gly Ser Arg Val Ala Ala Pro Glu Thr
225             230             235             240

gga tca aca aac cgt ctc tct atc act ctc aag tca gac ggt cag cta    768
Gly Ser Thr Asn Arg Leu Ser Ile Thr Leu Lys Ser Asp Gly Gln Leu
                245             250             255

aat gcc agc aat aac gat gtc aat ctc ttt gac aca gtg act cta cca    816
Asn Ala Ser Asn Asn Asp Val Asn Leu Phe Asp Thr Val Thr Leu Pro
            260             265             270

gct gcg gtc aat gac cat ctt aaa aat gag aag aag att ctt ctc aag    864
Ala Ala Val Asn Asp His Leu Lys Asn Glu Lys Lys Ile Leu Leu Lys
        275             280             285

gcg ggc tct tat gac gat gag cga aca gtt gtt agc gtt aaa acg gat    912
Ala Gly Ser Tyr Asp Asp Glu Arg Thr Val Val Ser Val Lys Thr Asp
    290             295             300

aac caa gag ggg gta aaa aca gag gat acc cct gct gaa aaa gaa aca    960
Asn Gln Glu Gly Val Lys Thr Glu Asp Thr Pro Ala Glu Lys Glu Thr
305             310             315             320

ggt cct gaa gtt gat gat agc aag gtg act tat gac acg att cag tct    1008
Gly Pro Glu Val Asp Asp Ser Lys Val Thr Tyr Asp Thr Ile Gln Ser
```

112

```
                          325                 330                 335
aag gtt ctc aaa gca gtg att gac caa gcc ttc cct cgt gtc aag gaa   1056
Lys Val Leu Lys Ala Val Ile Asp Gln Ala Phe Pro Arg Val Lys Glu
            340                 345                 350

tac agc ttg aat gga cat act ttg cca gga cag gtt caa cag ttc aac   1104
Tyr Ser Leu Asn Gly His Thr Leu Pro Gly Gln Val Gln Gln Phe Asn
            355                 360                 365

caa gtc ttt atc aat aac cac cga atc acc cct gaa gtc act tat aag   1152
Gln Val Phe Ile Asn Asn His Arg Ile Thr Pro Glu Val Thr Tyr Lys
            370                 375                 380

aaa atc aat gag aca aca gca gag tac ttg atg aag ctt cgc gat gat   1200
Lys Ile Asn Glu Thr Thr Ala Glu Tyr Leu Met Lys Leu Arg Asp Asp
385                 390                 395                 400

gct cac tta atc aat gcg gaa atg aca gta cgc ttg caa gtt gtg gac   1248
Ala His Leu Ile Asn Ala Glu Met Thr Val Arg Leu Gln Val Val Asp
                405                 410                 415

aat caa ttg cac ttt gat gtg acc aag att gtc aac cac aat caa gtc   1296
Asn Gln Leu His Phe Asp Val Thr Lys Ile Val Asn His Asn Gln Val
                420                 425                 430

act cca ggt caa aag att gat gac gaa aga aaa cta ctt tct tct att   1344
Thr Pro Gly Gln Lys Ile Asp Asp Glu Arg Lys Leu Leu Ser Ser Ile
            435                 440                 445

agt ttc ctc ggc aat gct tta gtc tct gtt tct agt gat caa act ggt   1392
Ser Phe Leu Gly Asn Ala Leu Val Ser Val Ser Ser Asp Gln Thr Gly
            450                 455                 460

gct aag ttt gat ggg gca acc atg tca aac aat acg cat gtc agc gga   1440
Ala Lys Phe Asp Gly Ala Thr Met Ser Asn Asn Thr His Val Ser Gly
465                 470                 475                 480

gat gat cat atc gat gta acc aat cca atg aaa gat cta gcc aag ggt   1488
Asp Asp His Ile Asp Val Thr Asn Pro Met Lys Asp Leu Ala Lys Gly
                485                 490                 495

tac atg tat gga ttt gtt tct aca gat aag ctt gct gct ggt gtt tgg   1536
Tyr Met Tyr Gly Phe Val Ser Thr Asp Lys Leu Ala Ala Gly Val Trp
            500                 505                 510

agt aac tct caa aac agc tat ggt ggt ggt tcg aat gac tgg act cgt   1584
Ser Asn Ser Gln Asn Ser Tyr Gly Gly Gly Ser Asn Asp Trp Thr Arg
            515                 520                 525

ttg aca gcc tat aaa gaa aca gtc gga aat gcc aac tat gta gga atc   1632
Leu Thr Ala Tyr Lys Glu Thr Val Gly Asn Ala Asn Tyr Val Gly Ile
            530                 535                 540

cac agc tct gaa tgg caa tgg gaa aaa gct tat aag ggc att gtt ttc   1680
His Ser Ser Glu Trp Gln Trp Glu Lys Ala Tyr Lys Gly Ile Val Phe
545                 550                 555                 560

cca gaa tac acg aag gaa ctt cca agt gct aag gtt gtt atc act gaa   1728
Pro Glu Tyr Thr Lys Glu Leu Pro Ser Ala Lys Val Val Ile Thr Glu
                565                 570                 575

gat gcc aat gca gac aag aaa gtc gat tgg cag gat ggt gcc att gct   1776
Asp Ala Asn Ala Asp Lys Lys Val Asp Trp Gln Asp Gly Ala Ile Ala
                580                 585                 590

tat cgt agc att atg aac aat cct caa ggt tgg aaa aaa gtt aag gat   1824
Tyr Arg Ser Ile Met Asn Asn Pro Gln Gly Trp Lys Lys Val Lys Asp
            595                 600                 605

atc aca gct tac cgt atc gcg atg aac ttt ggt tct caa gca caa aac   1872
Ile Thr Ala Tyr Arg Ile Ala Met Asn Phe Gly Ser Gln Ala Gln Asn
            610                 615                 620

cca ttc ctt atg acc ttg gat ggt atc aag aaa atc aat ctc cac aca   1920
Pro Phe Leu Met Thr Leu Asp Gly Ile Lys Lys Ile Asn Leu His Thr
```

113

|   |   | 625 |   |   |   |   | 630 |   |   |   |   | 635 |   |   |   |   | 640 |   |   |   |   |   |
|---|---|---|---|---|---|---|---|---|---|---|---|---|---|---|---|---|---|---|---|---|---|---|

gat ggt ctt ggg caa ggt gtt ctc ctt aaa gga tat ggt agc gaa ggc    1968
Asp Gly Leu Gly Gln Gly Val Leu Leu Lys Gly Tyr Gly Ser Glu Gly
                645                     650                 655

cat gac tct ggt cac ttg aac tat gct gat att ggt aag cgt atc ggt    2016
His Asp Ser Gly His Leu Asn Tyr Ala Asp Ile Gly Lys Arg Ile Gly
                660                     665                 670

ggt gtc gaa gac ttc aag acc cta att gag aag gct aag aaa tat gga    2064
Gly Val Glu Asp Phe Lys Thr Leu Ile Glu Lys Ala Lys Lys Tyr Gly
                675                     680                 685

gct cat cta ggt atc cac gtt aac gct tca gaa act tat cct gag tct    2112
Ala His Leu Gly Ile His Val Asn Ala Ser Glu Thr Tyr Pro Glu Ser
            690                     695                 700

aaa tac ttc aat gaa aaa att ctc cgt aag aat cca gat gga agc tat    2160
Lys Tyr Phe Asn Glu Lys Ile Leu Arg Lys Asn Pro Asp Gly Ser Tyr
705                     710                     715                 720

agc tat ggt tgg aac tgg cta gat caa ggt atc aac att gat gct gcc    2208
Ser Tyr Gly Trp Asn Trp Leu Asp Gln Gly Ile Asn Ile Asp Ala Ala
                725                     730                 735

tat gac cta gct cat ggt cgt ttg gca cgt tgg gaa gat ttg aag aaa    2256
Tyr Asp Leu Ala His Gly Arg Leu Ala Arg Trp Glu Asp Leu Lys Lys
            740                     745                 750

aaa ctt ggt gac ggt ctc gac ttt atc tat gtg gac gtt tgg ggt aat    2304
Lys Leu Gly Asp Gly Leu Asp Phe Ile Tyr Val Asp Val Trp Gly Asn
                755                     760                 765

ggt caa tca ggt gat aac ggt gcc tgg gct acc cac gtt ctt gct aaa    2352
Gly Gln Ser Gly Asp Asn Gly Ala Trp Ala Thr His Val Leu Ala Lys
            770                     775                 780

gaa att aac aaa caa ggc tgg cgc ttt gcg atc gag tgg ggc cat ggt    2400
Glu Ile Asn Lys Gln Gly Trp Arg Phe Ala Ile Glu Trp Gly His Gly
785                     790                     795                 800

ggt gag tac gac tct acc ttc cat cac tgg gca gct gac ttg acc tac    2448
Gly Glu Tyr Asp Ser Thr Phe His His Trp Ala Ala Asp Leu Thr Tyr
                805                     810                 815

ggt ggc tac acc aat aaa ggt atc aac agt gcc atc acc cgc ttt ata    2496
Gly Gly Tyr Thr Asn Lys Gly Ile Asn Ser Ala Ile Thr Arg Phe Ile
                820                     825                 830

cgt aac cac caa aaa gat gct tgg gta ggg gac tac aga agt tat ggt    2544
Arg Asn His Gln Lys Asp Ala Trp Val Gly Asp Tyr Arg Ser Tyr Gly
            835                     840                 845

ggt gca gcc aac tat cca ctg cta ggt ggc tac agc atg aaa gac ttt    2592
Gly Ala Ala Asn Tyr Pro Leu Leu Gly Gly Tyr Ser Met Lys Asp Phe
           850                     855                 860

gaa ggc tgg caa gga aga agt gac tac aat ggc tat gta act aac tta    2640
Glu Gly Trp Gln Gly Arg Ser Asp Tyr Asn Gly Tyr Val Thr Asn Leu
865                     870                     875                 880

ttt gcc cat gac gtc atg acc aag tac ttc caa cac ttc act gta agt    2688
Phe Ala His Asp Val Met Thr Lys Tyr Phe Gln His Phe Thr Val Ser
                885                     890                 895

aaa tgg gaa aat ggt aca ccg gtg act atg acc gat aac ggt agc acc    2736
Lys Trp Glu Asn Gly Thr Pro Val Thr Met Thr Asp Asn Gly Ser Thr
            900                     905                 910

tat aaa tgg act cca gaa atg cga gtg gaa ttg gta gat gct gac aat    2784
Tyr Lys Trp Thr Pro Glu Met Arg Val Glu Leu Val Asp Ala Asp Asn
                915                     920                 925

aat aaa gta gtt gta act cgt aag tca aat gat gtc aat agt cca caa    2832
Asn Lys Val Val Val Thr Arg Lys Ser Asn Asp Val Asn Ser Pro Gln

```
                930             935             940
tat cgc gaa cgt aca gta act ctc aac gga cgt gtc atc caa gat ggt    2880
Tyr Arg Glu Arg Thr Val Thr Leu Asn Gly Arg Val Ile Gln Asp Gly
945             950             955             960

tca gct tac ttg act cct tgg aac tgg gat gca aat ggt aag aaa ctt    2928
Ser Ala Tyr Leu Thr Pro Trp Asn Trp Asp Ala Asn Gly Lys Lys Leu
                965             970             975

tct act gat aag gaa aag atg tac tac ttc aat acg cag gcc ggt gca    2976
Ser Thr Asp Lys Glu Lys Met Tyr Tyr Phe Asn Thr Gln Ala Gly Ala
                980             985             990

aca act tgg acc ctt cca agc gat  tgg gca aag agc aag  gtt tac ctt  3024
Thr Thr Trp Thr Leu Pro Ser Asp  Trp Ala Lys Ser Lys  Val Tyr Leu
                995             1000            1005

tac aag  cta act gac caa ggt  aag aca gaa gag caa  gaa cta act      3069
Tyr Lys  Leu Thr Asp Gln Gly  Lys Thr Glu Glu Gln  Glu Leu Thr
        1010            1015            1020

gta aaa  gat ggt aaa att acc  cta gat ctt cta gca  aat caa cca      3114
Val Lys  Asp Gly Lys Ile Thr  Leu Asp Leu Leu Ala  Asn Gln Pro
        1025            1030            1035

tac gtt  ctc tat cgt tcg aaa  caa acc aat cct gaa  atg tca tgg      3159
Tyr Val  Leu Tyr Arg Ser Lys  Gln Thr Asn Pro Glu  Met Ser Trp
        1040            1045            1050

agt gaa  ggc atg cac atc tat  gac caa gga ttt aac  agt ggt acc      3204
Ser Glu  Gly Met His Ile Tyr  Asp Gln Gly Phe Asn  Ser Gly Thr
        1055            1060            1065

ttg aaa  cat tgg acc att tca  ggc gat gct tct aag  gca gaa att      3249
Leu Lys  His Trp Thr Ile Ser  Gly Asp Ala Ser Lys  Ala Glu Ile
        1070            1075            1080

gtc aag  tct caa ggg gca aac  gat atg ctt cgt att  caa gga aac      3294
Val Lys  Ser Gln Gly Ala Asn  Asp Met Leu Arg Ile  Gln Gly Asn
        1085            1090            1095

aaa gaa  aaa gtt agt ctc act  cag aaa tta act ggc  ttg aaa cca      3339
Lys Glu  Lys Val Ser Leu Thr  Gln Lys Leu Thr Gly  Leu Lys Pro
        1100            1105            1110

aat acc  aag tat gcc gtt tat  gtc ggt gtc gat aac  cgt agt aat      3384
Asn Thr  Lys Tyr Ala Val Tyr  Val Gly Val Asp Asn  Arg Ser Asn
        1115            1120            1125

gcc aag  gcg agc atc act gta  aat act ggt gaa aaa  gaa gtg act      3429
Ala Lys  Ala Ser Ile Thr Val  Asn Thr Gly Glu Lys  Glu Val Thr
        1130            1135            1140

act tat  acc aat aag tct ctc  gcc ctc aac tat gta  aaa gcc tat      3474
Thr Tyr  Thr Asn Lys Ser Leu  Ala Leu Asn Tyr Val  Lys Ala Tyr
        1145            1150            1155

gcc cac  aat aca cgt cgt aac  aat gct aca gtt gac  gat aca agt      3519
Ala His  Asn Thr Arg Arg Asn  Asn Ala Thr Val Asp  Asp Thr Ser
        1160            1165            1170

tac ttc  caa aac atg tac gcc  ttc ttt aca act gga  tcg gac gta      3564
Tyr Phe  Gln Asn Met Tyr Ala  Phe Phe Thr Thr Gly  Ser Asp Val
        1175            1180            1185

tca aat  gtt act ctg aca ttg  agt cgt gaa gct ggt  gat gaa gca      3609
Ser Asn  Val Thr Leu Thr Leu  Ser Arg Glu Ala Gly  Asp Glu Ala
        1190            1195            1200

act tac  ttt gat gaa att cgt  acc ttt gaa aac aat  tca agc atg      3654
Thr Tyr  Phe Asp Glu Ile Arg  Thr Phe Glu Asn Asn  Ser Ser Met
        1205            1210            1215

tac gga  gac aag cat gat aca  ggt aaa ggc acc ttc  aag caa gac      3699
Tyr Gly  Asp Lys His Asp Thr  Gly Lys Gly Thr Phe  Lys Gln Asp
```

```
            1220                    1225                    1230

        ttt gaa  aat gtt gct cag ggt  atc ttc cca ttt gta  gtg ggt ggt    3744
        Phe Glu  Asn Val Ala Gln Gly  Ile Phe Pro Phe Val  Val Gly Gly
            1235                    1240                    1245

        gtc gaa  ggt gtc gaa gac aac  cgc act cac ttg tct  gaa aaa cac    3789
        Val Glu  Gly Val Glu Asp Asn  Arg Thr His Leu Ser  Glu Lys His
            1250                    1255                    1260

        gat cca  tat aca caa cgt ggt  tgg aat ggt aag aaa  gtc gat gat    3834
        Asp Pro  Tyr Thr Gln Arg Gly  Trp Asn Gly Lys Lys  Val Asp Asp
            1265                    1270                    1275

        gtt atc  gaa gga aat tgg tca  ctc aag aca aat gga  cta gtg agc    3879
        Val Ile  Glu Gly Asn Trp Ser  Leu Lys Thr Asn Gly  Leu Val Ser
            1280                    1285                    1290

        cgt cgt  aac ttg gtt tac caa  act att ccg caa aac  ttc cgt ttt    3924
        Arg Arg  Asn Leu Val Tyr Gln  Thr Ile Pro Gln Asn  Phe Arg Phe
            1295                    1300                    1305

        gaa gca  ggt aag acc tac cgt  gta acc ttt gaa tac  gaa gca ggt    3969
        Glu Ala  Gly Lys Thr Tyr Arg  Val Thr Phe Glu Tyr  Glu Ala Gly
            1310                    1315                    1320

        tca gac  aat acc tat gct ttt  gta gtc ggt aag gga  gaa ttc cag    4014
        Ser Asp  Asn Thr Tyr Ala Phe  Val Val Gly Lys Gly  Glu Phe Gln
            1325                    1330                    1335

        tca ggt  cgt cgt ggt act caa  gca agc aac ttg gaa  atg cat gaa    4059
        Ser Gly  Arg Arg Gly Thr Gln  Ala Ser Asn Leu Glu  Met His Glu
            1340                    1345                    1350

        ttg cca  aat act tgg aca gat  tct aag aaa gcc aag  aag gca acc    4104
        Leu Pro  Asn Thr Trp Thr Asp  Ser Lys Lys Ala Lys  Lys Ala Thr
            1355                    1360                    1365

        ttc ctc  gtg aca ggt gca gaa  aca ggg gat act tgg  gta ggt atc    4149
        Phe Leu  Val Thr Gly Ala Glu  Thr Gly Asp Thr Trp  Val Gly Ile
            1370                    1375                    1380

        tac tca  act gga aat gca agt  aat act cgt ggt gat  tct ggt gga    4194
        Tyr Ser  Thr Gly Asn Ala Ser  Asn Thr Arg Gly Asp  Ser Gly Gly
            1385                    1390                    1395

        aat gcc  aac ttc cgt ggt tat  aac gac ttc atg atg  gat aat ctt    4239
        Asn Ala  Asn Phe Arg Gly Tyr  Asn Asp Phe Met Met  Asp Asn Leu
            1400                    1405                    1410

        caa atc  gaa gaa att acc cta  aca ggt aag atg ttg  aca gaa aat    4284
        Gln Ile  Glu Glu Ile Thr Leu  Thr Gly Lys Met Leu  Thr Glu Asn
            1415                    1420                    1425

        gct ctg  aag aac tac ttg cca  acg gtt gcc atg act  aac tac acc    4329
        Ala Leu  Lys Asn Tyr Leu Pro  Thr Val Ala Met Thr  Asn Tyr Thr
            1430                    1435                    1440

        aaa gag  tct atg gat gct ttg  aaa gag gcg gtc ttt  aac ctc agt    4374
        Lys Glu  Ser Met Asp Ala Leu  Lys Glu Ala Val Phe  Asn Leu Ser
            1445                    1450                    1455

        cag gcc  gat gat gat atc agt  gtg gaa gaa gcg cgt  gca gag att    4419
        Gln Ala  Asp Asp Asp Ile Ser  Val Glu Glu Ala Arg  Ala Glu Ile
            1460                    1465                    1470

        gcc aag  att gaa gcc ttg aag  aat gct ttg gtt cag  aag aaa acg    4464
        Ala Lys  Ile Glu Ala Leu Lys  Asn Ala Leu Val Gln  Lys Lys Thr
            1475                    1480                    1485

        gct ttg  gta gca gat gac ttt  gca agt ctt aca gct  cct gct cag    4509
        Ala Leu  Val Ala Asp Asp Phe  Ala Ser Leu Thr Ala  Pro Ala Gln
            1490                    1495                    1500

        gct caa  gaa ggt ctt gca aat  gcc ttt gat gga aac  tta tct agt    4554
        Ala Gln  Glu Gly Leu Ala Asn  Ala Phe Asp Gly Asn  Leu Ser Ser
```

```
                1505              1510              1515

tta tgg  cat aca tca tgg ggc  gga gga gat gta ggc  aag cct gca          4599
Leu Trp  His Thr Ser Trp Gly  Gly Gly Asp Val Gly  Lys Pro Ala
         1520              1525              1530

acc atg  gtc ttg aaa gaa gca  act gaa atc act gga  ctt cgt tat          4644
Thr Met  Val Leu Lys Glu Ala  Thr Glu Ile Thr Gly  Leu Arg Tyr
         1535              1540              1545

gtt cca  cgt gga tca ggt tca  aat ggt aac ttg cgt  gat gtg aaa          4689
Val Pro  Arg Gly Ser Gly Ser  Asn Gly Asn Leu Arg  Asp Val Lys
         1550              1555              1560

ctt gtt  gtg aca gat gag tct  ggc aag gag cat acc  ttt act gca          4734
Leu Val  Val Thr Asp Glu Ser  Gly Lys Glu His Thr  Phe Thr Ala
         1565              1570              1575

act gat  tgg cca gat aac aat  aag cca aaa gac att  gat ttt ggt          4779
Thr Asp  Trp Pro Asp Asn Asn  Lys Pro Lys Asp Ile  Asp Phe Gly
         1580              1585              1590

aag aca  att aag gct aag aaa  att gtc ctt aca ggt  act aag act          4824
Lys Thr  Ile Lys Ala Lys Lys  Ile Val Leu Thr Gly  Thr Lys Thr
         1595              1600              1605

tac gga  gat ggt ggc gat aaa  tac caa tct gca gcg  gaa ctc atc          4869
Tyr Gly  Asp Gly Gly Asp Lys  Tyr Gln Ser Ala Ala  Glu Leu Ile
         1610              1615              1620

ttt act  cgt cca cag gta gca  gaa aca cct ctt gac  ttg tca ggc          4914
Phe Thr  Arg Pro Gln Val Ala  Glu Thr Pro Leu Asp  Leu Ser Gly
         1625              1630              1635

tat gaa  gca gct ttg gct aag  gct cag aaa tta aca  gac aaa gac          4959
Tyr Glu  Ala Ala Leu Ala Lys  Ala Gln Lys Leu Thr  Asp Lys Asp
         1640              1645              1650

aat caa  gag gaa gta gct agc  gtt cag gca agc atg  aaa tat gcg          5004
Asn Gln  Glu Glu Val Ala Ser  Val Gln Ala Ser Met  Lys Tyr Ala
         1655              1660              1665

acg gat  aac cat ctc ttg acg  gaa aga atg gtg gaa  tac ttt gca          5049
Thr Asp  Asn His Leu Leu Thr  Glu Arg Met Val Glu  Tyr Phe Ala
         1670              1675              1680

gat tat  ctc aac caa tta aaa  gat tct gct acg aaa  cca gat gct          5094
Asp Tyr  Leu Asn Gln Leu Lys  Asp Ser Ala Thr Lys  Pro Asp Ala
         1685              1690              1695

cca act  gta gag aaa cct gag  ttt aaa ctt agc tct  gta gct tcc          5139
Pro Thr  Val Glu Lys Pro Glu  Phe Lys Leu Ser Ser  Val Ala Ser
         1700              1705              1710

gat caa  ggt aag acg cca gat  tat aag caa gaa ata  gct aga cca          5184
Asp Gln  Gly Lys Thr Pro Asp  Tyr Lys Gln Glu Ile  Ala Arg Pro
         1715              1720              1725

gaa aca  cct gaa caa atc ttg  cca gca aca ggt gag  agt caa ttt          5229
Glu Thr  Pro Glu Gln Ile Leu  Pro Ala Thr Gly Glu  Ser Gln Phe
         1730              1735              1740

gac aca  gcc ctc ttc cta gca  agt gtt agc cta gcc  cta tct gct          5274
Asp Thr  Ala Leu Phe Leu Ala  Ser Val Ser Leu Ala  Leu Ser Ala
         1745              1750              1755

ctc ttt  gta gta aaa acg aag  aaa gac tag                              5304
Leu Phe  Val Val Lys Thr Lys  Lys Asp
         1760              1765
```

<210> 4

117

<211> 1767
<212> PRT
<213> Streptococcus pneumoniae R6

<400> 4

```
Met Asn Lys Gly Leu Phe Glu Lys Arg Cys Lys Tyr Ser Ile Arg Lys
1             5               10              15

Phe Ser Leu Gly Val Ala Ser Val Met Ile Gly Ala Thr Phe Phe Gly
            20              25              30

Thr Ser Pro Val Leu Ala Asp Ser Val Gln Ser Gly Ser Thr Ala Asn
        35              40              45

Leu Pro Ala Asp Leu Ala Thr Ala Leu Ala Thr Ala Lys Glu Asn Asp
        50              55              60

Gly His Asp Phe Glu Ala Pro Lys Val Gly Glu Asp Gln Gly Ser Pro
65              70              75              80

Glu Val Thr Asp Gly Pro Lys Thr Glu Glu Glu Leu Leu Ala Leu Glu
            85              90              95

Lys Glu Lys Pro Ala Glu Glu Lys Pro Lys Glu Asp Lys Pro Ala Ala
            100             105             110

Ala Lys Pro Glu Thr Pro Lys Thr Val Thr Pro Glu Trp Gln Thr Val
        115             120             125

Glu Lys Lys Glu Gln Gln Gly Thr Val Thr Ile Arg Glu Glu Lys Gly
        130             135             140

Val Arg Tyr Asn Gln Leu Ser Ser Thr Ala Gln Asn Asp Asn Ala Gly
145             150             155             160

Lys Pro Ala Leu Phe Glu Lys Lys Gly Leu Thr Val Asp Ala Asn Gly
            165             170             175

Asn Ala Thr Val Asp Leu Thr Phe Lys Asp Asp Ser Glu Lys Gly Lys
        180             185             190

Ser Arg Phe Gly Val Phe Leu Lys Phe Lys Asp Thr Lys Asn Asn Val
        195             200             205

Phe Val Gly Tyr Asp Lys Asp Gly Trp Phe Trp Glu Tyr Lys Ser Pro
    210             215             220

Thr Thr Ser Thr Trp Tyr Arg Gly Ser Arg Val Ala Ala Pro Glu Thr
225             230             235             240

Gly Ser Thr Asn Arg Leu Ser Ile Thr Leu Lys Ser Asp Gly Gln Leu
            245             250             255

Asn Ala Ser Asn Asn Asp Val Asn Leu Phe Asp Thr Val Thr Leu Pro
        260             265             270

Ala Ala Val Asn Asp His Leu Lys Asn Glu Lys Lys Ile Leu Leu Lys
        275             280             285

Ala Gly Ser Tyr Asp Asp Glu Arg Thr Val Val Ser Val Lys Thr Asp
    290             295             300
```

Asn Gln Glu Gly Val Lys Thr Glu Asp Thr Pro Ala Glu Lys Glu Thr
305                 310             315             320

Gly Pro Glu Val Asp Asp Ser Lys Val Thr Tyr Asp Thr Ile Gln Ser
                325             330             335

Lys Val Leu Lys Ala Val Ile Asp Gln Ala Phe Pro Arg Val Lys Glu
            340             345             350

Tyr Ser Leu Asn Gly His Thr Leu Pro Gly Gln Val Gln Gln Phe Asn
            355             360             365

Gln Val Phe Ile Asn Asn His Arg Ile Thr Pro Glu Val Thr Tyr Lys
        370             375             380

Lys Ile Asn Glu Thr Thr Ala Glu Tyr Leu Met Lys Leu Arg Asp Asp
385             390             395             400

Ala His Leu Ile Asn Ala Glu Met Thr Val Arg Leu Gln Val Val Asp
            405             410             415

Asn Gln Leu His Phe Asp Val Thr Lys Ile Val Asn His Asn Gln Val
            420             425             430

Thr Pro Gly Gln Lys Ile Asp Asp Glu Arg Lys Leu Leu Ser Ser Ile
        435             440             445

Ser Phe Leu Gly Asn Ala Leu Val Ser Val Ser Ser Asp Gln Thr Gly
    450             455             460

Ala Lys Phe Asp Gly Ala Thr Met Ser Asn Asn Thr His Val Ser Gly
465             470             475             480

Asp Asp His Ile Asp Val Thr Asn Pro Met Lys Asp Leu Ala Lys Gly
            485             490             495

Tyr Met Tyr Gly Phe Val Ser Thr Asp Lys Leu Ala Ala Gly Val Trp
            500             505             510

Ser Asn Ser Gln Asn Ser Tyr Gly Gly Gly Ser Asn Asp Trp Thr Arg
    515             520             525

Leu Thr Ala Tyr Lys Glu Thr Val Gly Asn Ala Asn Tyr Val Gly Ile
    530             535             540

His Ser Ser Glu Trp Gln Trp Glu Lys Ala Tyr Lys Gly Ile Val Phe
545             550             555             560

Pro Glu Tyr Thr Lys Glu Leu Pro Ser Ala Lys Val Val Ile Thr Glu
            565             570             575

Asp Ala Asn Ala Asp Lys Lys Val Asp Trp Gln Asp Gly Ala Ile Ala
            580             585             590

Tyr Arg Ser Ile Met Asn Asn Pro Gln Gly Trp Lys Lys Val Lys Asp
    595             600             605

Ile Thr Ala Tyr Arg Ile Ala Met Asn Phe Gly Ser Gln Ala Gln Asn
610             615                 620

Pro Phe Leu Met Thr Leu Asp Gly Ile Lys Lys Ile Asn Leu His Thr
625             630                 635                 640

Asp Gly Leu Gly Gln Gly Val Leu Leu Lys Gly Tyr Gly Ser Glu Gly
                645                 650                 655

His Asp Ser Gly His Leu Asn Tyr Ala Asp Ile Gly Lys Arg Ile Gly
            660                 665                 670

Gly Val Glu Asp Phe Lys Thr Leu Ile Glu Lys Ala Lys Lys Tyr Gly
        675                 680                 685

Ala His Leu Gly Ile His Val Asn Ala Ser Glu Thr Tyr Pro Glu Ser
    690                 695                 700

Lys Tyr Phe Asn Glu Lys Ile Leu Arg Lys Asn Pro Asp Gly Ser Tyr
705                 710                 715                 720

Ser Tyr Gly Trp Asn Trp Leu Asp Gln Gly Ile Asn Ile Asp Ala Ala
                725                 730                 735

Tyr Asp Leu Ala His Gly Arg Leu Ala Arg Trp Glu Asp Leu Lys Lys
        740                 745                 750

Lys Leu Gly Asp Gly Leu Asp Phe Ile Tyr Val Asp Val Trp Gly Asn
        755                 760                 765

Gly Gln Ser Gly Asp Asn Gly Ala Trp Ala Thr His Val Leu Ala Lys
    770                 775                 780

Glu Ile Asn Lys Gln Gly Trp Arg Phe Ala Ile Glu Trp Gly His Gly
785                 790                 795                 800

Gly Glu Tyr Asp Ser Thr Phe His His Trp Ala Ala Asp Leu Thr Tyr
                805                 810                 815

Gly Gly Tyr Thr Asn Lys Gly Ile Asn Ser Ala Ile Thr Arg Phe Ile
            820                 825                 830

Arg Asn His Gln Lys Asp Ala Trp Val Gly Asp Tyr Arg Ser Tyr Gly
        835                 840                 845

Gly Ala Ala Asn Tyr Pro Leu Leu Gly Gly Tyr Ser Met Lys Asp Phe
    850                 855                 860

Glu Gly Trp Gln Gly Arg Ser Asp Tyr Asn Gly Tyr Val Thr Asn Leu
865                 870                 875                 880

Phe Ala His Asp Val Met Thr Lys Tyr Phe Gln His Phe Thr Val Ser
            885                 890                 895

Lys Trp Glu Asn Gly Thr Pro Val Thr Met Thr Asp Asn Gly Ser Thr
            900                 905                 910

Tyr Lys Trp Thr Pro Glu Met Arg Val Glu Leu Val Asp Ala Asp Asn
    915              920              925

Asn Lys Val Val Val Thr Arg Lys Ser Asn Asp Val Asn Ser Pro Gln
    930              935              940

Tyr Arg Glu Arg Thr Val Thr Leu Asn Gly Arg Val Ile Gln Asp Gly
945              950              955              960

Ser Ala Tyr Leu Thr Pro Trp Asn Trp Asp Ala Asn Gly Lys Lys Leu
            965              970              975

Ser Thr Asp Lys Glu Lys Met Tyr Tyr Phe Asn Thr Gln Ala Gly Ala
            980              985              990

Thr Thr Trp Thr Leu Pro Ser Asp Trp Ala Lys Ser Lys Val Tyr Leu
    995              1000             1005

Tyr Lys Leu Thr Asp Gln Gly Lys Thr Glu Glu Gln Glu Leu Thr
    1010             1015             1020

Val Lys Asp Gly Lys Ile Thr Leu Asp Leu Leu Ala Asn Gln Pro
    1025             1030             1035

Tyr Val Leu Tyr Arg Ser Lys Gln Thr Asn Pro Glu Met Ser Trp
    1040             1045             1050

Ser Glu Gly Met His Ile Tyr Asp Gln Gly Phe Asn Ser Gly Thr
    1055             1060             1065

Leu Lys His Trp Thr Ile Ser Gly Asp Ala Ser Lys Ala Glu Ile
    1070             1075             1080

Val Lys Ser Gln Gly Ala Asn Asp Met Leu Arg Ile Gln Gly Asn
    1085             1090             1095

Lys Glu Lys Val Ser Leu Thr Gln Lys Leu Thr Gly Leu Lys Pro
    1100             1105             1110

Asn Thr Lys Tyr Ala Val Tyr Val Gly Val Asp Asn Arg Ser Asn
    1115             1120             1125

Ala Lys Ala Ser Ile Thr Val Asn Thr Gly Glu Lys Glu Val Thr
    1130             1135             1140

Thr Tyr Thr Asn Lys Ser Leu Ala Leu Asn Tyr Val Lys Ala Tyr
    1145             1150             1155

Ala His Asn Thr Arg Arg Asn Asn Ala Thr Val Asp Asp Thr Ser
    1160             1165             1170

Tyr Phe Gln Asn Met Tyr Ala Phe Phe Thr Thr Gly Ser Asp Val
    1175             1180             1185

Ser Asn Val Thr Leu Thr Leu Ser Arg Glu Ala Gly Asp Glu Ala
    1190             1195             1200

```
Thr Tyr Phe Asp Glu Ile Arg  Thr Phe Glu Asn Asn  Ser Ser Met
    1205              1210              1215

Tyr Gly Asp Lys His Asp Thr  Gly Lys Gly Thr Phe  Lys Gln Asp
    1220              1225              1230

Phe Glu Asn Val Ala Gln Gly  Ile Phe Pro Phe Val  Val Gly Gly
    1235              1240              1245

Val Glu Gly Val Glu Asp Asn  Arg Thr His Leu Ser  Glu Lys His
    1250              1255              1260

Asp Pro Tyr Thr Gln Arg Gly  Trp Asn Gly Lys Lys  Val Asp Asp
    1265              1270              1275

Val Ile Glu Gly Asn Trp Ser  Leu Lys Thr Asn Gly  Leu Val Ser
    1280              1285              1290

Arg Arg Asn Leu Val Tyr Gln  Thr Ile Pro Gln Asn  Phe Arg Phe
    1295              1300              1305

Glu Ala Gly Lys Thr Tyr Arg  Val Thr Phe Glu Tyr  Glu Ala Gly
    1310              1315              1320

Ser Asp Asn Thr Tyr Ala Phe  Val Val Gly Lys Gly  Glu Phe Gln
    1325              1330              1335

Ser Gly Arg Arg Gly Thr Gln  Ala Ser Asn Leu Glu  Met His Glu
    1340              1345              1350

Leu Pro Asn Thr Trp Thr Asp  Ser Lys Lys Ala Lys  Lys Ala Thr
    1355              1360              1365

Phe Leu Val Thr Gly Ala Glu  Thr Gly Asp Thr Trp  Val Gly Ile
    1370              1375              1380

Tyr Ser Thr Gly Asn Ala Ser  Asn Thr Arg Gly Asp  Ser Gly Gly
    1385              1390              1395

Asn Ala Asn Phe Arg Gly Tyr  Asn Asp Phe Met Met  Asp Asn Leu
    1400              1405              1410

Gln Ile Glu Glu Ile Thr Leu  Thr Gly Lys Met Leu  Thr Glu Asn
    1415              1420              1425

Ala Leu Lys Asn Tyr Leu Pro  Thr Val Ala Met Thr  Asn Tyr Thr
    1430              1435              1440

Lys Glu Ser Met Asp Ala Leu  Lys Glu Ala Val Phe  Asn Leu Ser
    1445              1450              1455

Gln Ala Asp Asp Asp Ile Ser  Val Glu Glu Ala Arg  Ala Glu Ile
    1460              1465              1470

Ala Lys Ile Glu Ala Leu Lys  Asn Ala Leu Val Gln  Lys Lys Thr
    1475              1480              1485
```

```
Ala Leu  Val Ala Asp Asp Phe  Ala Ser Leu Thr Ala  Pro Ala Gln
    1490            1495                1500

Ala Gln  Glu Gly Leu Ala Asn  Ala Phe Asp Gly Asn  Leu Ser Ser
    1505            1510                1515

Leu Trp  His Thr Ser Trp Gly  Gly Gly Asp Val Gly  Lys Pro Ala
    1520            1525                1530

Thr Met  Val Leu Lys Glu Ala  Thr Glu Ile Thr Gly  Leu Arg Tyr
    1535            1540                1545

Val Pro  Arg Gly Ser Gly Ser  Asn Gly Asn Leu Arg  Asp Val Lys
    1550            1555                1560

Leu Val  Val Thr Asp Glu Ser  Gly Lys Glu His Thr  Phe Thr Ala
    1565            1570                1575

Thr Asp  Trp Pro Asp Asn Asn  Lys Pro Lys Asp Ile  Asp Phe Gly
    1580            1585                1590

Lys Thr  Ile Lys Ala Lys Lys  Ile Val Leu Thr Gly  Thr Lys Thr
    1595            1600                1605

Tyr Gly  Asp Gly Gly Asp Lys  Tyr Gln Ser Ala Ala  Glu Leu Ile
    1610            1615                1620

Phe Thr  Arg Pro Gln Val Ala  Glu Thr Pro Leu Asp  Leu Ser Gly
    1625            1630                1635

Tyr Glu  Ala Ala Leu Ala Lys  Ala Gln Lys Leu Thr  Asp Lys Asp
    1640            1645                1650

Asn Gln  Glu Glu Val Ala Ser  Val Gln Ala Ser Met  Lys Tyr Ala
    1655            1660                1665

Thr Asp  Asn His Leu Leu Thr  Glu Arg Met Val Glu  Tyr Phe Ala
    1670            1675                1680

Asp Tyr  Leu Asn Gln Leu Lys  Asp Ser Ala Thr Lys  Pro Asp Ala
    1685            1690                1695

Pro Thr  Val Glu Lys Pro Glu  Phe Lys Leu Ser Ser  Val Ala Ser
    1700            1705                1710

Asp Gln  Gly Lys Thr Pro Asp  Tyr Lys Gln Glu Ile  Ala Arg Pro
    1715            1720                1725

Glu Thr  Pro Glu Gln Ile Leu  Pro Ala Thr Gly Glu  Ser Gln Phe
    1730            1735                1740

Asp Thr  Ala Leu Phe Leu Ala  Ser Val Ser Leu Ala  Leu Ser Ala
    1745            1750                1755

Leu Phe  Val Val Lys Thr Lys  Lys Asp
    1760            1765
```

```
<210> 5
<211> 5901
<212> DNA
<213> Bifidobacterium longum NCC2705

<220>
<221> CDS
<222> (1).. (5901)

<400> 5
```

```
atg aaa aag aag aag act ata tcg gct gcg ctg gca aca gcg tta gcc    48
Met Lys Lys Lys Lys Thr Ile Ser Ala Ala Leu Ala Thr Ala Leu Ala
1               5                   10                  15

tta acc tgc atg ggc agc ggg gga ggt act gcg ttc gca gtg ccc ctg    96
Leu Thr Cys Met Gly Ser Gly Gly Gly Thr Ala Phe Ala Val Pro Leu
            20                  25                  30

tct gat gct gac ttg cag act ttg gca agt cag att cag caa atc aac   144
Ser Asp Ala Asp Leu Gln Thr Leu Ala Ser Gln Ile Gln Gln Ile Asn
        35                  40                  45

gat act tct gat tct gca act gct tcc gag act cct tcc gca caa gcc   192
Asp Thr Ser Asp Ser Ala Thr Ala Ser Glu Thr Pro Ser Ala Gln Ala
        50                  55                  60

gat gcg gtt gaa ggc tgg act att gat tcc aac atc gct cag ggc gac   240
Asp Ala Val Glu Gly Trp Thr Ile Asp Ser Asn Ile Ala Gln Gly Asp
65                  70                  75                  80

gaa atc ctg gag atg gca aac ggt tgg ctg cac ctc aag tcc act gcc   288
Glu Ile Leu Glu Met Ala Asn Gly Trp Leu His Leu Lys Ser Thr Ala
                85                  90                  95

tct aac ggt aat gcg gca gcg aac ccc agc tcc agc aac aac tgg ccg   336
Ser Asn Gly Asn Ala Ala Ala Asn Pro Ser Ser Ser Asn Asn Trp Pro
            100                 105                 110

gca gta gcc gta tgg ggc aca gat tac gac ttc tcc aag gcc ggc tcc   384
Ala Val Ala Val Trp Gly Thr Asp Tyr Asp Phe Ser Lys Ala Gly Ser
            115                 120                 125

ttc cac gcc acc atc aaa tcc ccg cag gaa ggc tcc gcc aac cgc ttc   432
Phe His Ala Thr Ile Lys Ser Pro Gln Glu Gly Ser Ala Asn Arg Phe
        130                 135                 140

ggc ttc tac ctg ggc tac aac gac ccg ggc agc ggc ctg ttc atc ggc   480
Gly Phe Tyr Leu Gly Tyr Asn Asp Pro Gly Ser Gly Leu Phe Ile Gly
145                 150                 155                 160

tac gat tcg gac ggc tgg ttc tgg cag acc tac acc ggt ggc ggt agc   528
Tyr Asp Ser Asp Gly Trp Phe Trp Gln Thr Tyr Thr Gly Gly Gly Ser
                165                 170                 175

ggc agc tgg tac agc ggt gct cgt atc gct gct ccg agc gcc aac gaa   576
Gly Ser Trp Tyr Ser Gly Ala Arg Ile Ala Ala Pro Ser Ala Asn Glu
            180                 185                 190

gag cac gac att cag gtc tcc tgg acc gac gcc aag gtc gcc aca ctg   624
Glu His Asp Ile Gln Val Ser Trp Thr Asp Ala Lys Val Ala Thr Leu
        195                 200                 205

acc gtg gat ggc cag aag gca ttc gat gtc gat tac tcc gca atg acg   672
Thr Val Asp Gly Gln Lys Ala Phe Asp Val Asp Tyr Ser Ala Met Thr
        210                 215                 220

aac ctc tcc aac aag ctt gcc atc aag gcc ggc tcc tgg aag ggg ctg   720
Asn Leu Ser Asn Lys Leu Ala Ile Lys Ala Gly Ser Trp Lys Gly Leu
225                 230                 235                 240

aac cag gtc acc gac gtg tac atc aag gac ttc ccg gag gtt gtc gaa   768
Asn Gln Val Thr Asp Val Tyr Ile Lys Asp Phe Pro Glu Val Val Glu
                245                 250                 255
```

```
gcc gcc aag cac gcg gtt tcc ggc aag gtt gtg gac gct gga ggc gct        816
Ala Ala Lys His Ala Val Ser Gly Lys Val Val Asp Ala Gly Gly Ala
            260             265             270

gcc att gaa ggc gca aca gtg cgt ttg gat aag acc aag gtg aag acc        864
Ala Ile Glu Gly Ala Thr Val Arg Leu Asp Lys Thr Lys Val Lys Thr
        275             280             285

ggt gca gat ggc acc ttc tcc ttc gcc gac att gaa gaa ggc gaa cac        912
Gly Ala Asp Gly Thr Phe Ser Phe Ala Asp Ile Glu Glu Gly Glu His
        290             295             300

acg ctt tcg att gcc aag gaa ggc tat gag gac gtc tcc cag cag gtg        960
Thr Leu Ser Ile Ala Lys Glu Gly Tyr Glu Asp Val Ser Gln Gln Val
305             310             315             320

gcg gtc tcc ggc gcg gac ctt gcc atc gat ccc atc acc ctg aac aaa       1008
Ala Val Ser Gly Ala Asp Leu Ala Ile Asp Pro Ile Thr Leu Asn Lys
            325             330             335

acc gtt cag gtc gcc tcc gaa acg ctg aag acc aag aag atg gaa gtt       1056
Thr Val Gln Val Ala Ser Glu Thr Leu Lys Thr Lys Lys Met Glu Val
            340             345             350

cag att aag aag aac ttc ccc tct gtg ctg cag tac acg atg acc gac       1104
Gln Ile Lys Lys Asn Phe Pro Ser Val Leu Gln Tyr Thr Met Thr Asp
            355             360             365

ggc aag gtg atg tac ggc caa acc aag gat gtg cgc acc gtt gag atc       1152
Gly Lys Val Met Tyr Gly Gln Thr Lys Asp Val Arg Thr Val Glu Ile
            370             375             380

aac ggc acc aac atc gaa ctg acc gac gat gac gtg acc ttc aag aag       1200
Asn Gly Thr Asn Ile Glu Leu Thr Asp Asp Asp Val Thr Phe Lys Lys
385             390             395             400

gtt tcc gat acc gaa gcc acc tac acg ctg aag gtc aag gat gag gcc       1248
Val Ser Asp Thr Glu Ala Thr Tyr Thr Leu Lys Val Lys Asp Glu Ala
            405             410             415

aag aag att gac gcg gtg atc acc gtt cag atc acg gtc aag gcc aac       1296
Lys Lys Ile Asp Ala Val Ile Thr Val Gln Ile Thr Val Lys Ala Asn
            420             425             430

cag ctg cac ctc aac gtc acc aag atc aag aac aac ctg tcc gaa ggc       1344
Gln Leu His Leu Asn Val Thr Lys Ile Lys Asn Asn Leu Ser Glu Gly
            435             440             445

att cct gag ggc aac ggt gtg gag gag aac gcc atc cag acg ctg tcc       1392
Ile Pro Glu Gly Asn Gly Val Glu Glu Asn Ala Ile Gln Thr Leu Ser
        450             455             460

ttc ccg aac cag agt ctc gtt tcc gtg cgt tcc agc cag gaa aat gcc       1440
Phe Pro Asn Gln Ser Leu Val Ser Val Arg Ser Ser Gln Glu Asn Ala
465             470             475             480

caa ttc act ggt gct cgt atg tct tcc aac acg cag aag cct ggc gat       1488
Gln Phe Thr Gly Ala Arg Met Ser Ser Asn Thr Gln Lys Pro Gly Asp
            485             490             495

acc aac ttc gca gtg acc gaa gat act aac gtc acc gat agc gac tac       1536
Thr Asn Phe Ala Val Thr Glu Asp Thr Asn Val Thr Asp Ser Asp Tyr
            500             505             510

acc tac ggc ttc atc tcc ggt gct ggc ctg agt gcc ggc ctg tgg agc       1584
Thr Tyr Gly Phe Ile Ser Gly Ala Gly Leu Ser Ala Gly Leu Trp Ser
            515             520             525

aac tcc gag cac gat ggc acc tat gtg gcg gct cct gtg cgc ggc ggc       1632
Asn Ser Glu His Asp Gly Thr Tyr Val Ala Ala Pro Val Arg Gly Gly
        530             535             540

agc cag aac acg cgt gtc tac gcc acc acc cag cag act ggt gac gcc       1680
Ser Gln Asn Thr Arg Val Tyr Ala Thr Thr Gln Gln Thr Gly Asp Ala
545             550             555             560
```

```
acc tcc ctg ggc ctg gcc agc gct ccg tgg tac tac cac cgc acg gtc    1728
Thr Ser Leu Gly Leu Ala Ser Ala Pro Trp Tyr Tyr His Arg Thr Val
            565             570                 575

acc gat tcc aag ggc aag aag tac acc gtg gcc gaa acc gct ctg ccg    1776
Thr Asp Ser Lys Gly Lys Lys Tyr Thr Val Ala Glu Thr Ala Leu Pro
            580             585             590

cag atg gcc gtg gcc atc gcc ggc gac gag aac ggt gac ggt gcc gtc    1824
Gln Met Ala Val Ala Ile Ala Gly Asp Glu Asn Gly Asp Gly Ala Val
            595             600             605

aac tgg cag gat ggc gca atc gcc tac cgc gac atc atg aac aac ccg    1872
Asn Trp Gln Asp Gly Ala Ile Ala Tyr Arg Asp Ile Met Asn Asn Pro
            610             615             620

tac aag tcc gag gaa gtt ccc gaa ctg gtg gca tgg cgt atc gcc atg    1920
Tyr Lys Ser Glu Glu Val Pro Glu Leu Val Ala Trp Arg Ile Ala Met
625             630             635             640

aac ttc ggc tcc cag gcg cag aac ccg ttc ctc acc acg ctt gac aac    1968
Asn Phe Gly Ser Gln Ala Gln Asn Pro Phe Leu Thr Thr Leu Asp Asn
            645             650             655

gtc aag aag gtg gcc ttg aac acc gac ggc ctc ggc cag tcc gtg ctg    2016
Val Lys Lys Val Ala Leu Asn Thr Asp Gly Leu Gly Gln Ser Val Leu
            660             665             670

ctc aag ggc tac ggc aat gaa ggc cac gac tcc ggc cac ccg gac tac    2064
Leu Lys Gly Tyr Gly Asn Glu Gly His Asp Ser Gly His Pro Asp Tyr
            675             680             685

ggc gat atc ggc cag cgt ctc ggc ggc gcc gac gac atg aac acc atg    2112
Gly Asp Ile Gly Gln Arg Leu Gly Gly Ala Asp Asp Met Asn Thr Met
            690             695             700

atg gaa gag ggc tcc aag tat ggc gct cgc ttc ggt gtg cac gtc aac    2160
Met Glu Glu Gly Ser Lys Tyr Gly Ala Arg Phe Gly Val His Val Asn
705             710             715             720

gcc tcc gaa atg tat ccg gaa gcc aag gcc ttc agc gag gac atg gtg    2208
Ala Ser Glu Met Tyr Pro Glu Ala Lys Ala Phe Ser Glu Asp Met Val
            725             730             735

cgc cgc aac tct gca ggc ggc ctg agc tac ggc tgg aac tgg ctt gat    2256
Arg Arg Asn Ser Ala Gly Gly Leu Ser Tyr Gly Trp Asn Trp Leu Asp
            740             745             750

cag ggt gtc ggc atc gac ggc atc tac gat ctg gca tcc ggt tct cgt    2304
Gln Gly Val Gly Ile Asp Gly Ile Tyr Asp Leu Ala Ser Gly Ser Arg
            755             760             765

gta agc cgt ttc gct gac ctc agc aag gaa gtc ggc gac aac atg gac    2352
Val Ser Arg Phe Ala Asp Leu Ser Lys Glu Val Gly Asp Asn Met Asp
            770             775             780

ttc atc tac ctc gat gtg tgg ggc aac ctg act tct tcc ggt tcg gaa    2400
Phe Ile Tyr Leu Asp Val Trp Gly Asn Leu Thr Ser Ser Gly Ser Glu
785             790             795             800

gat tct tgg gaa acc cgc aag atg agc aag atg atc aac gac aac ggc    2448
Asp Ser Trp Glu Thr Arg Lys Met Ser Lys Met Ile Asn Asp Asn Gly
            805             810             815

tgg cgt atg acc acc gaa tgg ggt tcc ggc aac gag tac gac tcc acc    2496
Trp Arg Met Thr Thr Glu Trp Gly Ser Gly Asn Glu Tyr Asp Ser Thr
            820             825             830

ttc cag cac tgg gca gct gat ctg acc tac ggc ggc tac acc tcc aag    2544
Phe Gln His Trp Ala Ala Asp Leu Thr Tyr Gly Gly Tyr Thr Ser Lys
            835             840             845

ggc gag aac tcc gaa gtg atg cgc ttc ctg cgc aac cac cag aag gac    2592
Gly Glu Asn Ser Glu Val Met Arg Phe Leu Arg Asn His Gln Lys Asp
            850             855             860
```

```
agc tgg gtt ggc gac tac ccg caa tac ggc ggc gct gcc aac gcc ccg     2640
Ser Trp Val Gly Asp Tyr Pro Gln Tyr Gly Gly Ala Ala Asn Ala Pro
865             870             875             880

ctg ctc ggc ggc tac aac atg aag gac ttc gaa ggc tgg cag ggc cgc     2688
Leu Leu Gly Gly Tyr Asn Met Lys Asp Phe Glu Gly Trp Gln Gly Arg
                885             890             895

aac gac tat gcc gcc tac atc aag aac ctg tac acc cat gat gtg tcc     2736
Asn Asp Tyr Ala Ala Tyr Ile Lys Asn Leu Tyr Thr His Asp Val Ser
                900             905             910

act aag ttc atc cag cac ttc aag gtg acc cgc tgg gtc aac aac ccg     2784
Thr Lys Phe Ile Gln His Phe Lys Val Thr Arg Trp Val Asn Asn Pro
                915             920             925

ctg ctg acc gcc gac aat ggc aat gcc gct gcc gtg tcc gac ccg aac     2832
Leu Leu Thr Ala Asp Asn Gly Asn Ala Ala Ala Val Ser Asp Pro Asn
    930             935             940

acg aac aac ggc aac gag cag att acc ctg aag gat tcc aac ggc aac     2880
Thr Asn Asn Gly Asn Glu Gln Ile Thr Leu Lys Asp Ser Asn Gly Asn
945             950             955             960

gtt gta gta gtc tcc cgt ggt tcc aac gac acc tct agc gca gcc tac     2928
Val Val Val Val Ser Arg Gly Ser Asn Asp Thr Ser Ser Ala Ala Tyr
                965             970             975

cgc cag cgc acc atc acc ttc aac ggc gtg aag gtc gca tcc ggt gtg     2976
Arg Gln Arg Thr Ile Thr Phe Asn Gly Val Lys Val Ala Ser Gly Val
                980             985             990

gtc tcc gca ggc gat ggc agc gcc act ggc gac gag tcc tac ctg ctg     3024
Val Ser Ala Gly Asp Gly Ser Ala Thr Gly Asp Glu Ser Tyr Leu Leu
        995             1000            1005

ccg tgg atg tgg gat tcc ttc acc ggc aag ctg gtc aag gat tcc        3069
Pro Trp Met Trp Asp Ser Phe Thr Gly Lys Leu Val Lys Asp Ser
    1010            1015            1020

gag cag aag ctc tac cac tgg aac acc aag ggc ggc acc acc acc        3114
Glu Gln Lys Leu Tyr His Trp Asn Thr Lys Gly Gly Thr Thr Thr
    1025            1030            1035

tgg acg ctg ccg gac agc tgg aag aac ctc tcc agc gta aag gtg        3159
Trp Thr Leu Pro Asp Ser Trp Lys Asn Leu Ser Ser Val Lys Val
    1040            1045            1050

tac cag ctc acc gat cag ggc aag acc aac gag cag acc gtt gcc        3204
Tyr Gln Leu Thr Asp Gln Gly Lys Thr Asn Glu Gln Thr Val Ala
    1055            1060            1065

gtc tcc ggc ggc aag gtg acg ctt acc gct gat gcc gaa acc ccg        3249
Val Ser Gly Gly Lys Val Thr Leu Thr Ala Asp Ala Glu Thr Pro
    1070            1075            1080

tac gtg gtg tac aag ggc gaa gcc aag cag atc cag gtc aac tgg        3294
Tyr Val Val Tyr Lys Gly Glu Ala Lys Gln Ile Gln Val Asn Trp
    1085            1090            1095

agc gaa ggc atg cat gtg gta gac gcc ggc ttc aac ggc ggc tcc        3339
Ser Glu Gly Met His Val Val Asp Ala Gly Phe Asn Gly Gly Ser
    1100            1105            1110

aac acc ctc acc gac aac tgg acc gtc gcc gga acc ggc aag gcc        3384
Asn Thr Leu Thr Asp Asn Trp Thr Val Ala Gly Thr Gly Lys Ala
    1115            1120            1125

gaa gtt gaa ggc gac aac aac gcc atg ctg cgc ctg acc ggc aag        3429
Glu Val Glu Gly Asp Asn Asn Ala Met Leu Arg Leu Thr Gly Lys
    1130            1135            1140

gtc gat gtc tcc cag cgt ctg acc gat ctc aag gct ggc cag aag        3474
Val Asp Val Ser Gln Arg Leu Thr Asp Leu Lys Ala Gly Gln Lys
    1145            1150            1155
```

```
tac gcg ctg tat gtt ggc gtc  gac aac cgc tcc acc  ggc gat gca      3519
Tyr Ala Leu Tyr Val Gly Val  Asp Asn Arg Ser Thr  Gly Asp Ala
    1160            1165                1170

tcc gtc acc gta acc agc ggc  ggc aag gtg ctg gcc  gcc aac tcc      3564
Ser Val Thr Val Thr Ser Gly  Gly Lys Val Leu Ala  Ala Asn Ser
    1175            1180                1185

acc ggc aag tcc atc gcc aag  aac tac atc aag gca  tac ggc cac      3609
Thr Gly Lys Ser Ile Ala Lys  Asn Tyr Ile Lys Ala  Tyr Gly His
    1190            1195                1200

aac acg aac agc aat acg gaa  aat ggc tcc agc tac  ttc cag aac      3654
Asn Thr Asn Ser Asn Thr Glu  Asn Gly Ser Ser Tyr  Phe Gln Asn
    1205            1210                1215

atg tac gtg ttc ttc acc gcg  cct gag aac ggc gat  gcc acg gta      3699
Met Tyr Val Phe Phe Thr Ala  Pro Glu Asn Gly Asp  Ala Thr Val
    1220            1225                1230

acc ctg tct cac aag agc acc  gac gga gca cac acc  tac ttc gac      3744
Thr Leu Ser His Lys Ser Thr  Asp Gly Ala His Thr  Tyr Phe Asp
    1235            1240                1245

gat gtg cgc atc gtg gag aac  cag tac tcc ggc atc  acc tat gag      3789
Asp Val Arg Ile Val Glu Asn  Gln Tyr Ser Gly Ile  Thr Tyr Glu
    1250            1255                1260

aag gac ggc acg ctg aag tcc  ctc acc aac gga ttc  gaa aac aac      3834
Lys Asp Gly Thr Leu Lys Ser  Leu Thr Asn Gly Phe  Glu Asn Asn
    1265            1270                1275

gcc cag ggc atc tgg ccg ttc  gtg gtc tcc ggt tcc  gaa ggc gtt      3879
Ala Gln Gly Ile Trp Pro Phe  Val Val Ser Gly Ser  Glu Gly Val
    1280            1285                1290

gag gac aac cgc atc cac ctc  tcc gag ctg cat gct  ccg ttc acg      3924
Glu Asp Asn Arg Ile His Leu  Ser Glu Leu His Ala  Pro Phe Thr
    1295            1300                1305

cag gcc ggt tgg gat gtc aag  aag atg gac gat gtg  ctc gat ggc      3969
Gln Ala Gly Trp Asp Val Lys  Lys Met Asp Asp Val  Leu Asp Gly
    1310            1315                1320

act tgg tct gtg aag gtt aac  ggc ctg acc cag aag  ggc acg ctg      4014
Thr Trp Ser Val Lys Val Asn  Gly Leu Thr Gln Lys  Gly Thr Leu
    1325            1330                1335

gtc tac cag acg atc ccg cag  aac gtg aag ttc gag  gcg ggt gcc      4059
Val Tyr Gln Thr Ile Pro Gln  Asn Val Lys Phe Glu  Ala Gly Ala
    1340            1345                1350

aag tac aag gtg agc ttc gac  tac cag tcc ggt tcc  gat gac atc      4104
Lys Tyr Lys Val Ser Phe Asp  Tyr Gln Ser Gly Ser  Asp Asp Ile
    1355            1360                1365

tac gcc atc gct gtg ggc cag  ggt gaa tac tct gcc  ggc agc gtg      4149
Tyr Ala Ile Ala Val Gly Gln  Gly Glu Tyr Ser Ala  Gly Ser Val
    1370            1375                1380

aag ctg acc aac ctg aag aag  gct ctg ggt gag acc  ggc aag gcc      4194
Lys Leu Thr Asn Leu Lys Lys  Ala Leu Gly Glu Thr  Gly Lys Ala
    1385            1390                1395

gag ttc gag ctg acc ggt ggc  gtc aac ggc gat tcc  tgg ttc ggt      4239
Glu Phe Glu Leu Thr Gly Gly  Val Asn Gly Asp Ser  Trp Phe Gly
    1400            1405                1410

att tac tcg acc gca acc gca  cct gat ctg cag ggt  tcc acc ggc      4284
Ile Tyr Ser Thr Ala Thr Ala  Pro Asp Leu Gln Gly  Ser Thr Gly
    1415            1420                1425

aat gca cag gac ttc ggc gga  tac aag gac ttc gtg  ctc gac aac      4329
Asn Ala Gln Asp Phe Gly Gly  Tyr Lys Asp Phe Val  Leu Asp Asn
    1430            1435                1440
```

130

EP 1 767 645 B1

```
ctg aag  atc gag cgc atc gag  tcc cag acc cgc acc  aag gcc gaa        4374
Leu Lys  Ile Glu Arg Ile Glu  Ser Gln Thr Arg Thr  Lys Ala Glu
    1445             1450               1455

gcg cag  gac aag gtc aag gaa  atc cgc ggc aag tac  gat tcc aag        4419
Ala Gln  Asp Lys Val Lys Glu  Ile Arg Gly Lys Tyr  Asp Ser Lys
    1460             1465               1470

cgt gct  gag ctc tcc gat gcc  gca tgg cag cag tat  cag gac acc        4464
Arg Ala  Glu Leu Ser Asp Ala  Ala Trp Gln Gln Tyr  Gln Asp Thr
    1475             1480               1485

ttg gtc  aag gct cgc gtg ctc  atc aac aag aat ggc  gca acc gct        4509
Leu Val  Lys Ala Arg Val Leu  Ile Asn Lys Asn Gly  Ala Thr Ala
    1490             1495               1500

gag gac  ttc acc aag gca tac  gac att ctc gtg gcc  ctc gac gag        4554
Glu Asp  Phe Thr Lys Ala Tyr  Asp Ile Leu Val Ala  Leu Asp Glu
    1505             1510               1515

tac atg  aag acc gct ccc ggc  aac gag agc agc gac  aag tac gac        4599
Tyr Met  Lys Thr Ala Pro Gly  Asn Glu Ser Ser Asp  Lys Tyr Asp
    1520             1525               1530

gtg gca  gct gac ggc tcc gat  gag ctg ggt ggc tac  acc gtg gcc        4644
Val Ala  Ala Asp Gly Ser Asp  Glu Leu Gly Gly Tyr  Thr Val Ala
    1535             1540               1545

acg ggc  agc gaa gag cct acc  gca ggc ctg ccg agc  gaa ggc ccg        4689
Thr Gly  Ser Glu Glu Pro Thr  Ala Gly Leu Pro Ser  Glu Gly Pro
    1550             1555               1560

gcc gat  ctg gca cag gat ggc  aac gac agc acc cac  tgg cac acc        4734
Ala Asp  Leu Ala Gln Asp Gly  Asn Asp Ser Thr His  Trp His Thr
    1565             1570               1575

agc tgg  agc gag aac gca gtc  ggc aac ggc acc gca  tgg tat cag        4779
Ser Trp  Ser Glu Asn Ala Val  Gly Asn Gly Thr Ala  Trp Tyr Gln
    1580             1585               1590

ttc aac  ctc aac gaa ccg acc  acc atc aac ggc ctg  cgc tac ctg        4824
Phe Asn  Leu Asn Glu Pro Thr  Thr Ile Asn Gly Leu  Arg Tyr Leu
    1595             1600               1605

ccg cgc  tcc gga ggt atg aac  gcc aac ggc aag atc  aag ggc tac        4869
Pro Arg  Ser Gly Gly Met Asn  Ala Asn Gly Lys Ile  Lys Gly Tyr
    1610             1615               1620

aag atc  acg ctc act ctg gcg  gat ggc acc acc aag  gat gtc gtc        4914
Lys Ile  Thr Leu Thr Leu Ala  Asp Gly Thr Thr Lys  Asp Val Val
    1625             1630               1635

acc gat  gct gag ttc tcc acc  acc acc atg tgg cag  aag gcc agc        4959
Thr Asp  Ala Glu Phe Ser Thr  Thr Thr Met Trp Gln  Lys Ala Ser
    1640             1645               1650

ttc gac  gcc gtc gag aat gtg  acc gcc gta cgc ttg  acc gtc ctg        5004
Phe Asp  Ala Val Glu Asn Val  Thr Ala Val Arg Leu  Thr Val Leu
    1655             1660               1665

tct tcc  gca ggc cag agc gac  tcc cag gcc aac aag  ttc gca tcc        5049
Ser Ser  Ala Gly Gln Ser Asp  Ser Gln Ala Asn Lys  Phe Ala Ser
    1670             1675               1680

gct gcc  gaa ctg cgt ttg acc  acg gac cgc gag gtt  gag gaa gag        5094
Ala Ala  Glu Leu Arg Leu Thr  Thr Asp Arg Glu Val  Glu Glu Glu
    1685             1690               1695

act gtc  gct ccg gac aag acc  gac ctc aac gac acc  atc gcc aag        5139
Thr Val  Ala Pro Asp Lys Thr  Asp Leu Asn Asp Thr  Ile Ala Lys
    1700             1705               1710

gct aac  ggt ctt aag gaa tcc  gac tac acg gct gaa  agc tgg act        5184
Ala Asn  Gly Leu Lys Glu Ser  Asp Tyr Thr Ala Glu  Ser Trp Thr
    1715             1720               1725
```

131

```
gct ctg gtc aag gcc cgc gaa  gct gca cag gcc gtg  gcg gat aac      5229
Ala Leu Val Lys Ala Arg Glu  Ala Ala Gln Ala Val  Ala Asp Asn
    1730            1735                1740

gat aag gcc acc gct tac gat  gtg gct ctg gcg ctg  acg aac ctc      5274
Asp Lys Ala Thr Ala Tyr Asp  Val Ala Leu Ala Leu  Thr Asn Leu
    1745            1750                1755

gaa tcc gct atc gct ggt ctc  gag aag acc ggt gag  gag cct ggc      5319
Glu Ser Ala Ile Ala Gly Leu  Glu Lys Thr Gly Glu  Glu Pro Gly
    1760            1765                1770

cca ggc ccg gtt gag gtg aac  aag acc gac ctg cag  act gca gtg      5364
Pro Gly Pro Val Glu Val Asn  Lys Thr Asp Leu Gln  Thr Ala Val
    1775            1780                1785

aac aag gca agc aag ctc gag  aag gcc gat tac acg  acc aac tcg      5409
Asn Lys Ala Ser Lys Leu Glu  Lys Ala Asp Tyr Thr  Thr Asn Ser
    1790            1795                1800

tgg gaa gct ttc gcc gag gca  ctg aag gct gca cag  cag gtg ctc      5454
Trp Glu Ala Phe Ala Glu Ala  Leu Lys Ala Ala Gln  Gln Val Leu
    1805            1810                1815

gac aac aag aac gcc acc cag  cag gat gtg gat acc  gca ctg agc      5499
Asp Asn Lys Asn Ala Thr Gln  Gln Asp Val Asp Thr  Ala Leu Ser
    1820            1825                1830

gct ctt cag gac gcc atc tcc  aag ctg gaa gct acc  gcc gag ccg      5544
Ala Leu Gln Asp Ala Ile Ser  Lys Leu Glu Ala Thr  Ala Glu Pro
    1835            1840                1845

aag ccg aat ccg gaa ccg ggc  gtg gtg gac aag gct  gct ctg aac      5589
Lys Pro Asn Pro Glu Pro Gly  Val Val Asp Lys Ala  Ala Leu Asn
    1850            1855                1860

gcg acc atc aac aag gcc gcc  gcc atc aac ctg ggt  ctc tac acc      5634
Ala Thr Ile Asn Lys Ala Ala  Ala Ile Asn Leu Gly  Leu Tyr Thr
    1865            1870                1875

gac gac tcc gcc aac gct ctg  cgc gcc gcg ctg aag  aag gcc cgt      5679
Asp Asp Ser Ala Asn Ala Leu  Arg Ala Ala Leu Lys  Lys Ala Arg
    1880            1885                1890

gag gtc tcc gac aac agc aac  gcc acg cag aag cag  gtc gac gca      5724
Glu Val Ser Asp Asn Ser Asn  Ala Thr Gln Lys Gln  Val Asp Ala
    1895            1900                1905

gct cgc gag gct ctc gag aag  gca att gcc ggc ttg  gtg aag cgt      5769
Ala Arg Glu Ala Leu Glu Lys  Ala Ile Ala Gly Leu  Val Lys Arg
    1910            1915                1920

acc gct gcc aag ggt gat ggc  aac gtt gtt tcc aac  acg ggc tcc      5814
Thr Ala Ala Lys Gly Asp Gly  Asn Val Val Ser Asn  Thr Gly Ser
    1925            1930                1935

gat gtc gcc acg atc gct ctg  gct gga ctg ctg ctg  gca ggt gct      5859
Asp Val Ala Thr Ile Ala Leu  Ala Gly Leu Leu Leu  Ala Gly Ala
    1940            1945                1950

ggc gcc gcc atc gcc tac cgt  cgc aat cgc gag caa  ctg tga          5901
Gly Ala Ala Ile Ala Tyr Arg  Arg Asn Arg Glu Gln  Leu
    1955            1960                1965
```

<210> 6

<211> 1966

<212> PRT

<213> Bifidobacterium longum NCC2705

<400> 6

Met Lys Lys Lys Lys Thr Ile Ser Ala Ala Leu Ala Thr Ala Leu Ala
1                   5                   10                  15

```
Leu Thr Cys Met Gly Ser Gly Gly Gly Thr Ala Phe Ala Val Pro Leu
            20              25              30


Ser Asp Ala Asp Leu Gln Thr Leu Ala Ser Gln Ile Gln Gln Ile Asn
        35              40              45


Asp Thr Ser Asp Ser Ala Thr Ala Ser Glu Thr Pro Ser Ala Gln Ala
    50              55              60


Asp Ala Val Glu Gly Trp Thr Ile Asp Ser Asn Ile Ala Gln Gly Asp
65              70              75              80


Glu Ile Leu Glu Met Ala Asn Gly Trp Leu His Leu Lys Ser Thr Ala
                85              90                  95


Ser Asn Gly Asn Ala Ala Ala Asn Pro Ser Ser Ser Asn Asn Trp Pro
            100             105             110


Ala Val Ala Val Trp Gly Thr Asp Tyr Asp Phe Ser Lys Ala Gly Ser
            115             120             125


Phe His Ala Thr Ile Lys Ser Pro Gln Glu Gly Ser Ala Asn Arg Phe
    130             135             140


Gly Phe Tyr Leu Gly Tyr Asn Asp Pro Gly Ser Gly Leu Phe Ile Gly
145             150             155             160


Tyr Asp Ser Asp Gly Trp Phe Trp Gln Thr Tyr Thr Gly Gly Gly Ser
            165             170             175


Gly Ser Trp Tyr Ser Gly Ala Arg Ile Ala Ala Pro Ser Ala Asn Glu
            180             185             190


Glu His Asp Ile Gln Val Ser Trp Thr Asp Ala Lys Val Ala Thr Leu
        195             200             205


Thr Val Asp Gly Gln Lys Ala Phe Asp Val Asp Tyr Ser Ala Met Thr
    210             215             220


Asn Leu Ser Asn Lys Leu Ala Ile Lys Ala Gly Ser Trp Lys Gly Leu
225             230             235             240


Asn Gln Val Thr Asp Val Tyr Ile Lys Asp Phe Pro Glu Val Val Glu
            245             250             255


Ala Ala Lys His Ala Val Ser Gly Lys Val Val Asp Ala Gly Gly Ala
            260             265             270


Ala Ile Glu Gly Ala Thr Val Arg Leu Asp Lys Thr Lys Val Lys Thr
        275             280             285


Gly Ala Asp Gly Thr Phe Ser Phe Ala Asp Ile Glu Glu Gly Glu His
    290             295             300


Thr Leu Ser Ile Ala Lys Glu Gly Tyr Glu Asp Val Ser Gln Gln Val
305             310             315             320
```

```
Ala Val Ser Gly Ala Asp Leu Ala Ile Asp Pro Ile Thr Leu Asn Lys
             325             330             335

Thr Val Gln Val Ala Ser Glu Thr Leu Lys Thr Lys Lys Met Glu Val
             340             345             350

Gln Ile Lys Lys Asn Phe Pro Ser Val Leu Gln Tyr Thr Met Thr Asp
         355             360             365

Gly Lys Val Met Tyr Gly Gln Thr Lys Asp Val Arg Thr Val Glu Ile
     370             375             380

Asn Gly Thr Asn Ile Glu Leu Thr Asp Asp Asp Val Thr Phe Lys Lys
385             390             395             400

Val Ser Asp Thr Glu Ala Thr Tyr Thr Leu Lys Val Lys Asp Glu Ala
             405             410             415

Lys Lys Ile Asp Ala Val Ile Thr Val Gln Ile Thr Val Lys Ala Asn
         420             425             430

Gln Leu His Leu Asn Val Thr Lys Ile Lys Asn Asn Leu Ser Glu Gly
         435             440             445

Ile Pro Glu Gly Asn Gly Val Glu Glu Asn Ala Ile Gln Thr Leu Ser
     450             455             460

Phe Pro Asn Gln Ser Leu Val Ser Val Arg Ser Ser Gln Glu Asn Ala
465             470             475             480

Gln Phe Thr Gly Ala Arg Met Ser Ser Asn Thr Gln Lys Pro Gly Asp
             485             490             495

Thr Asn Phe Ala Val Thr Glu Asp Thr Asn Val Thr Asp Ser Asp Tyr
             500             505             510

Thr Tyr Gly Phe Ile Ser Gly Ala Gly Leu Ser Ala Gly Leu Trp Ser
     515             520             525

Asn Ser Glu His Asp Gly Thr Tyr Val Ala Ala Pro Val Arg Gly Gly
     530             535             540

Ser Gln Asn Thr Arg Val Tyr Ala Thr Thr Gln Gln Thr Gly Asp Ala
545             550             555             560

Thr Ser Leu Gly Leu Ala Ser Ala Pro Trp Tyr Tyr His Arg Thr Val
             565             570             575

Thr Asp Ser Lys Gly Lys Lys Tyr Thr Val Ala Glu Thr Ala Leu Pro
         580             585             590

Gln Met Ala Val Ala Ile Ala Gly Asp Glu Asn Gly Asp Gly Ala Val
     595             600             605

Asn Trp Gln Asp Gly Ala Ile Ala Tyr Arg Asp Ile Met Asn Asn Pro
     610             615             620
```

Tyr Lys Ser Glu Glu Val Pro Glu Leu Val Ala Trp Arg Ile Ala Met
625　　　　　　630　　　　　　635　　　　　　640

Asn Phe Gly Ser Gln Ala Gln Asn Pro Phe Leu Thr Thr Leu Asp Asn
　　　　　645　　　　　　650　　　　　　655

Val Lys Lys Val Ala Leu Asn Thr Asp Gly Leu Gly Gln Ser Val Leu
　　　　660　　　　　　665　　　　　　670

Leu Lys Gly Tyr Gly Asn Glu Gly His Asp Ser Gly His Pro Asp Tyr
　　　675　　　　　　680　　　　　　685

Gly Asp Ile Gly Gln Arg Leu Gly Gly Ala Asp Asp Met Asn Thr Met
　　690　　　　　　695　　　　　　700

Met Glu Glu Gly Ser Lys Tyr Gly Ala Arg Phe Gly Val His Val Asn
705　　　　　　710　　　　　　715　　　　　　720

Ala Ser Glu Met Tyr Pro Glu Ala Lys Ala Phe Ser Glu Asp Met Val
　　　　　725　　　　　　730　　　　　　735

Arg Arg Asn Ser Ala Gly Gly Leu Ser Tyr Gly Trp Asn Trp Leu Asp
　　　　740　　　　　　745　　　　　　750

Gln Gly Val Gly Ile Asp Gly Ile Tyr Asp Leu Ala Ser Gly Ser Arg
　　　755　　　　　　760　　　　　　765

Val Ser Arg Phe Ala Asp Leu Ser Lys Glu Val Gly Asp Asn Met Asp
　　770　　　　　　775　　　　　　780

Phe Ile Tyr Leu Asp Val Trp Gly Asn Leu Thr Ser Ser Gly Ser Glu
785　　　　　　790　　　　　　795　　　　　　800

Asp Ser Trp Glu Thr Arg Lys Met Ser Lys Met Ile Asn Asp Asn Gly
　　　　805　　　　　　810　　　　　　815

Trp Arg Met Thr Thr Glu Trp Gly Ser Gly Asn Glu Tyr Asp Ser Thr
　　　　820　　　　　　825　　　　　　830

Phe Gln His Trp Ala Ala Asp Leu Thr Tyr Gly Gly Tyr Thr Ser Lys
　　　835　　　　　　840　　　　　　845

Gly Glu Asn Ser Glu Val Met Arg Phe Leu Arg Asn His Gln Lys Asp
　　850　　　　　　855　　　　　　860

Ser Trp Val Gly Asp Tyr Pro Gln Tyr Gly Gly Ala Ala Asn Ala Pro
865　　　　　　870　　　　　　875　　　　　　880

Leu Leu Gly Gly Tyr Asn Met Lys Asp Phe Glu Gly Trp Gln Gly Arg
　　　　　885　　　　　　890　　　　　　895

Asn Asp Tyr Ala Ala Tyr Ile Lys Asn Leu Tyr Thr His Asp Val Ser
　　　　900　　　　　　905　　　　　　910

Thr Lys Phe Ile Gln His Phe Lys Val Thr Arg Trp Val Asn Asn Pro
　　　915　　　　　　920　　　　　　925

Leu Leu Thr Ala Asp Asn Gly Asn Ala Ala Ala Val Ser Asp Pro Asn
930                935                940

Thr Asn Asn Gly Asn Glu Gln Ile Thr Leu Lys Asp Ser Asn Gly Asn
945                950                955                960

Val Val Val Val Ser Arg Gly Ser Asn Asp Thr Ser Ser Ala Ala Tyr
965                970                975

Arg Gln Arg Thr Ile Thr Phe Asn Gly Val Lys Val Ala Ser Gly Val
980                985                990

Val Ser Ala Gly Asp Gly Ser Ala Thr Gly Asp Glu Ser Tyr Leu Leu
995                1000                1005

Pro Trp Met Trp Asp Ser Phe Thr Gly Lys Leu Val Lys Asp Ser
1010                1015                1020

Glu Gln Lys Leu Tyr His Trp Asn Thr Lys Gly Gly Thr Thr Thr
1025                1030                1035

Trp Thr Leu Pro Asp Ser Trp Lys Asn Leu Ser Ser Val Lys Val
1040                1045                1050

Tyr Gln Leu Thr Asp Gln Gly Lys Thr Asn Glu Gln Thr Val Ala
1055                1060                1065

Val Ser Gly Gly Lys Val Thr Leu Thr Ala Asp Ala Glu Thr Pro
1070                1075                1080

Tyr Val Val Tyr Lys Gly Glu Ala Lys Gln Ile Gln Val Asn Trp
1085                1090                1095

Ser Glu Gly Met His Val Val Asp Ala Gly Phe Asn Gly Gly Ser
1100                1105                1110

Asn Thr Leu Thr Asp Asn Trp Thr Val Ala Gly Thr Gly Lys Ala
1115                1120                1125

Glu Val Glu Gly Asp Asn Asn Ala Met Leu Arg Leu Thr Gly Lys
1130                1135                1140

Val Asp Val Ser Gln Arg Leu Thr Asp Leu Lys Ala Gly Gln Lys
1145                1150                1155

Tyr Ala Leu Tyr Val Gly Val Asp Asn Arg Ser Thr Gly Asp Ala
1160                1165                1170

Ser Val Thr Val Thr Ser Gly Gly Lys Val Leu Ala Ala Asn Ser
1175                1180                1185

Thr Gly Lys Ser Ile Ala Lys Asn Tyr Ile Lys Ala Tyr Gly His
1190                1195                1200

Asn Thr Asn Ser Asn Thr Glu Asn Gly Ser Ser Tyr Phe Gln Asn
1205                1210                1215

```
Met Tyr  Val Phe Phe Thr Ala  Pro Glu Asn Gly Asp  Ala Thr Val
    1220                 1225             1230

Thr Leu  Ser His Lys Ser Thr  Asp Gly Ala His Thr  Tyr Phe Asp
    1235                 1240             1245

Asp Val  Arg Ile Val Glu Asn  Gln Tyr Ser Gly Ile  Thr Tyr Glu
    1250                 1255             1260

Lys Asp  Gly Thr Leu Lys Ser  Leu Thr Asn Gly Phe  Glu Asn Asn
    1265                 1270             1275

Ala Gln  Gly Ile Trp Pro Phe  Val Val Ser Gly Ser  Glu Gly Val
    1280                 1285             1290

Glu Asp  Asn Arg Ile His Leu  Ser Glu Leu His Ala  Pro Phe Thr
    1295                 1300             1305

Gln Ala  Gly Trp Asp Val Lys  Lys Met Asp Asp Val  Leu Asp Gly
    1310                 1315             1320

Thr Trp  Ser Val Lys Val Asn  Gly Leu Thr Gln Lys  Gly Thr Leu
    1325                 1330             1335

Val Tyr  Gln Thr Ile Pro Gln  Asn Val Lys Phe Glu  Ala Gly Ala
    1340                 1345             1350

Lys Tyr  Lys Val Ser Phe Asp  Tyr Gln Ser Gly Ser  Asp Asp Ile
    1355                 1360             1365

Tyr Ala  Ile Ala Val Gly Gln  Gly Glu Tyr Ser Ala  Gly Ser Val
    1370                 1375             1380

Lys Leu  Thr Asn Leu Lys Lys  Ala Leu Gly Glu Thr  Gly Lys Ala
    1385                 1390             1395

Glu Phe  Glu Leu Thr Gly Gly  Val Asn Gly Asp Ser  Trp Phe Gly
    1400                 1405             1410

Ile Tyr  Ser Thr Ala Thr Ala  Pro Asp Leu Gln Gly  Ser Thr Gly
    1415                 1420             1425

Asn Ala  Gln Asp Phe Gly Gly  Tyr Lys Asp Phe Val  Leu Asp Asn
    1430                 1435             1440

Leu Lys  Ile Glu Arg Ile Glu  Ser Gln Thr Arg Thr  Lys Ala Glu
    1445                 1450             1455

Ala Gln  Asp Lys Val Lys Glu  Ile Arg Gly Lys Tyr  Asp Ser Lys
    1460                 1465             1470

Arg Ala  Glu Leu Ser Asp Ala  Ala Trp Gln Gln Tyr  Gln Asp Thr
    1475                 1480             1485

Leu Val  Lys Ala Arg Val Leu  Ile Asn Lys Asn Gly  Ala Thr Ala
    1490                 1495             1500
```

```
Glu Asp  Phe Thr Lys Ala Tyr  Asp Ile Leu Val Ala  Leu Asp Glu
    1505             1510              1515

Tyr Met  Lys Thr Ala Pro Gly  Asn Glu Ser Ser Asp  Lys Tyr Asp
    1520             1525              1530

Val Ala  Ala Asp Gly Ser Asp  Glu Leu Gly Gly Tyr  Thr Val Ala
    1535             1540              1545

Thr Gly  Ser Glu Glu Pro Thr  Ala Gly Leu Pro Ser  Glu Gly Pro
    1550             1555              1560

Ala Asp  Leu Ala Gln Asp Gly  Asn Asp Ser Thr His  Trp His Thr
    1565             1570              1575

Ser Trp  Ser Glu Asn Ala Val  Gly Asn Gly Thr Ala  Trp Tyr Gln
    1580             1585              1590

Phe Asn  Leu Asn Glu Pro Thr  Thr Ile Asn Gly Leu  Arg Tyr Leu
    1595             1600              1605

Pro Arg  Ser Gly Gly Met Asn  Ala Asn Gly Lys Ile  Lys Gly Tyr
    1610             1615              1620

Lys Ile  Thr Leu Thr Leu Ala  Asp Gly Thr Thr Lys  Asp Val Val
    1625             1630              1635

Thr Asp  Ala Glu Phe Ser Thr  Thr Thr Met Trp Gln  Lys Ala Ser
    1640             1645              1650

Phe Asp  Ala Val Glu Asn Val  Thr Ala Val Arg Leu  Thr Val Leu
    1655             1660              1665

Ser Ser  Ala Gly Gln Ser Asp  Ser Gln Ala Asn Lys  Phe Ala Ser
    1670             1675              1680

Ala Ala  Glu Leu Arg Leu Thr  Thr Asp Arg Glu Val  Glu Glu Glu
    1685             1690              1695

Thr Val  Ala Pro Asp Lys Thr  Asp Leu Asn Asp Thr  Ile Ala Lys
    1700             1705              1710

Ala Asn  Gly Leu Lys Glu Ser  Asp Tyr Thr Ala Glu  Ser Trp Thr
    1715             1720              1725

Ala Leu  Val Lys Ala Arg Glu  Ala Ala Gln Ala Val  Ala Asp Asn
    1730             1735              1740

Asp Lys  Ala Thr Ala Tyr Asp  Val Ala Leu Ala Leu  Thr Asn Leu
    1745             1750              1755

Glu Ser  Ala Ile Ala Gly Leu  Glu Lys Thr Gly Glu  Glu Pro Gly
    1760             1765              1770

Pro Gly  Pro Val Glu Val Asn  Lys Thr Asp Leu Gln  Thr Ala Val
    1775             1780              1785
```

139

Asn Lys Ala Ser Lys Leu Glu Lys Ala Asp Tyr Thr Thr Asn Ser
    1790            1795            1800

Trp Glu Ala Phe Ala Glu Ala Leu Lys Ala Ala Gln Gln Val Leu
    1805            1810            1815

Asp Asn Lys Asn Ala Thr Gln Gln Asp Val Asp Thr Ala Leu Ser
    1820            1825            1830

Ala Leu Gln Asp Ala Ile Ser Lys Leu Glu Ala Thr Ala Glu Pro
    1835            1840            1845

Lys Pro Asn Pro Glu Pro Gly Val Val Asp Lys Ala Ala Leu Asn
    1850            1855            1860

Ala Thr Ile Asn Lys Ala Ala Ala Ile Asn Leu Gly Leu Tyr Thr
    1865            1870            1875

Asp Asp Ser Ala Asn Ala Leu Arg Ala Ala Leu Lys Lys Ala Arg
    1880            1885            1890

Glu Val Ser Asp Asn Ser Asn Ala Thr Gln Lys Gln Val Asp Ala
    1895            1900            1905

Ala Arg Glu Ala Leu Glu Lys Ala Ile Ala Gly Leu Val Lys Arg
    1910            1915            1920

Thr Ala Ala Lys Gly Asp Gly Asn Val Val Ser Asn Thr Gly Ser
    1925            1930            1935

Asp Val Ala Thr Ile Ala Leu Ala Gly Leu Leu Leu Ala Gly Ala
    1940            1945            1950

Gly Ala Ala Ile Ala Tyr Arg Arg Asn Arg Glu Gln Leu
    1955            1960            1965

<210> 7
<211> 5061
<212> DNA
<213> Clostridium perfringens str. 13

<220>
<221> CDS
<222> (1).. (5061)

<400> 7

EP 1 767 645 B1

```
atg ggt aga aaa tgc atg aat aag aag att gcc gca ata ata gca gct        48
Met Gly Arg Lys Cys Met Asn Lys Lys Ile Ala Ala Ile Ile Ala Ala
1               5               10                  15

gca gtt att gta gga caa tta cca att tca gta ctt gct aca cct gtt        96
Ala Val Ile Val Gly Gln Leu Pro Ile Ser Val Leu Ala Thr Pro Val
                20              25                  30

aat gaa gct gga gat gag att aat agt gaa tca gct gaa att tta act       144
Asn Glu Ala Gly Asp Glu Ile Asn Ser Glu Ser Ala Glu Ile Leu Thr
            35                  40                  45

aat agt gat gaa gaa gct gag gct tat att caa aac tat gat aga cca       192
Asn Ser Asp Glu Glu Ala Glu Ala Tyr Ile Gln Asn Tyr Asp Arg Pro
        50                  55                  60

gag ggg att act tgg aca aaa tta gca ggc tca gga agt gtt gag gta       240
Glu Gly Ile Thr Trp Thr Lys Leu Ala Gly Ser Gly Ser Val Glu Val
```

141

```
              65                    70                    75                    80
        act gat gga ttt tta tca gtt act aat aat gga gat tat aga att atg        288
        Thr Asp Gly Phe Leu Ser Val Thr Asn Asn Gly Asp Tyr Arg Ile Met
                        85                    90                    95

        gaa gat caa tcg cct aat ata aaa aat ggt gag tta gaa agt aaa ttt        336
        Glu Asp Gln Ser Pro Asn Ile Lys Asn Gly Glu Leu Glu Ser Lys Phe
                        100                   105                   110

        aca gtt gga ggt tct caa act gga ata ata ttt aga gca act gag tca        384
        Thr Val Gly Gly Ser Gln Thr Gly Ile Ile Phe Arg Ala Thr Glu Ser
                        115                   120                   125

        aac tat gga atg atc aac tat aac tca ggt act ggc tgg gtt ata gaa        432
        Asn Tyr Gly Met Ile Asn Tyr Asn Ser Gly Thr Gly Trp Val Ile Glu
                130                   135                   140

        aat aaa aat agt tgg gag gat att aca gga cca aaa cta aat aat gga        480
        Asn Lys Asn Ser Trp Glu Asp Ile Thr Gly Pro Lys Leu Asn Asn Gly
        145                   150                   155                   160

        gat gtt gta aca gtt aag gct act ttt gtt gaa aag cat tta act gtt        528
        Asp Val Val Thr Val Lys Ala Thr Phe Val Glu Lys His Leu Thr Val
                        165                   170                   175

        aat gtt tct gta aat gat gga gaa ttt gaa act ata tat gat aaa gaa        576
        Asn Val Ser Val Asn Asp Gly Glu Phe Glu Thr Ile Tyr Asp Lys Glu
                        180                   185                   190

        tca gat tta att cca tta caa gct ggt aaa gtt gga tat aga gga tgg        624
        Ser Asp Leu Ile Pro Leu Gln Ala Gly Lys Val Gly Tyr Arg Gly Trp
                        195                   200                   205

        ggt aac gct aaa act acc aaa ttt gat tat att aag tat gct cct atg        672
        Gly Asn Ala Lys Thr Thr Lys Phe Asp Tyr Ile Lys Tyr Ala Pro Met
                210                   215                   220

        act ata gat aag gga cct ata gta tca ata aat gaa gta aat gta gaa        720
        Thr Ile Asp Lys Gly Pro Ile Val Ser Ile Asn Glu Val Asn Val Glu
        225                   230                   235                   240

        act tat cca aga gtt aag cct att tta cca tca agt gtt aca gta aat        768
        Thr Tyr Pro Arg Val Lys Pro Ile Leu Pro Ser Ser Val Thr Val Asn
                        245                   250                   255

        cat gaa aat ggt atg tct agt att aag gat gtt tct tgg aat tat ata        816
        His Glu Asn Gly Met Ser Ser Ile Lys Asp Val Ser Trp Asn Tyr Ile
                        260                   265                   270

        cct aag gaa agt tat tca aag cca ggt aca ttc aaa gtt gaa ggt aca        864
        Pro Lys Glu Ser Tyr Ser Lys Pro Gly Thr Phe Lys Val Glu Gly Thr
                        275                   280                   285

        gtt gaa ggt aca gat gta aaa gca ata gct aat gta act gta agt tca        912
        Val Glu Gly Thr Asp Val Lys Ala Ile Ala Asn Val Thr Val Ser Ser
                290                   295                   300

        gat tta gca tat tat gaa act aat ttt gaa aca gaa gaa aca aga gga        960
        Asp Leu Ala Tyr Tyr Glu Thr Asn Phe Glu Thr Glu Glu Thr Arg Gly
        305                   310                   315                   320

        gat tgg caa gtt gta caa gga gga gga tct cca agt tat gaa gag ggt       1008
        Asp Trp Gln Val Val Gln Gly Gly Gly Ser Pro Ser Tyr Glu Glu Gly
                        325                   330                   335

        aaa gta aaa ata cct atg aat gga gta tca atc gct gtt gat atg aat       1056
        Lys Val Lys Ile Pro Met Asn Gly Val Ser Ile Ala Val Asp Met Asn
                        340                   345                   350

        tct cca gag gtt aag aac ttt act tat gaa act gat ttt tct gtt gat       1104
        Ser Pro Glu Val Lys Asn Phe Thr Tyr Glu Thr Asp Phe Ser Val Asp
                        355                   360                   365

        aat aat gga gga aga ata ggt cta tta ttt aga tat gta tca gaa act       1152
        Asn Asn Gly Gly Arg Ile Gly Leu Leu Phe Arg Tyr Val Ser Glu Thr
```

370        375        380

```
gag tgg gga gct gtt tgc tat gat aat ggt tct tgg gta tgg aaa act   1200
Glu Trp Gly Ala Val Cys Tyr Asp Asn Gly Ser Trp Val Trp Lys Thr
385                 390                 395                 400

gga gat ggc aaa tat ggt aat ttc cca gga aca ttt aca cca gag cca   1248
Gly Asp Gly Lys Tyr Gly Asn Phe Pro Gly Thr Phe Thr Pro Glu Pro
                405                 410                 415

gga aaa act tac aga ata aag ctt aaa gta gaa gat aca aat att act   1296
Gly Lys Thr Tyr Arg Ile Lys Leu Lys Val Glu Asp Thr Asn Ile Thr
                420                 425                 430

atg tgg gtt gat gga gag aaa ata ggg caa gtt gca gta tct aat tta   1344
Met Trp Val Asp Gly Glu Lys Ile Gly Gln Val Ala Val Ser Asn Leu
                435                 440                 445

cca gat gta aga gga aaa gtt ggc tta act gga tgg ttt gga aat aaa   1392
Pro Asp Val Arg Gly Lys Val Gly Leu Thr Gly Trp Phe Gly Asn Lys
        450                 455                 460

aat gtt act tta gat aat ctt gtt gtt gag gaa tta ggt gga ata atg   1440
Asn Val Thr Leu Asp Asn Leu Val Val Glu Glu Leu Gly Gly Ile Met
465                 470                 475                 480

gca cca gaa gta ggt cca tta gaa gaa caa agt ata gaa tca gat tct   1488
Ala Pro Glu Val Gly Pro Leu Glu Glu Gln Ser Ile Glu Ser Asp Ser
                485                 490                 495

atg aaa gtt gtt tta gat aat aga ttc cca act gtt att aga tat gag   1536
Met Lys Val Val Leu Asp Asn Arg Phe Pro Thr Val Ile Arg Tyr Glu
                500                 505                 510

tgg aaa gga act gaa gat gtt tta tca gga gca tct gta gat gat tta   1584
Trp Lys Gly Thr Glu Asp Val Leu Ser Gly Ala Ser Val Asp Asp Leu
                515                 520                 525

gaa gct caa tat atg gtt gaa att aat ggt gaa aag aga att cca aaa   1632
Glu Ala Gln Tyr Met Val Glu Ile Asn Gly Glu Lys Arg Ile Pro Lys
                530                 535                 540

gta act agt gaa ttt gca aat aat gaa ggt ata tat aca tta aac ttt   1680
Val Thr Ser Glu Phe Ala Asn Asn Glu Gly Ile Tyr Thr Leu Asn Phe
545                 550                 555                 560

gaa gat ata gga atg act att act tta aag atg act gtt aat gaa aat   1728
Glu Asp Ile Gly Met Thr Ile Thr Leu Lys Met Thr Val Asn Glu Asn
                565                 570                 575

aaa tta aga atg gaa gtt act gat att caa gaa ggg gat gtt aaa ctt   1776
Lys Leu Arg Met Glu Val Thr Asp Ile Gln Glu Gly Asp Val Lys Leu
                580                 585                 590

caa aca tta aat ttc cca aat cat agt tta gct tca gta agc agt tta   1824
Gln Thr Leu Asn Phe Pro Asn His Ser Leu Ala Ser Val Ser Ser Leu
                595                 600                 605

aat aat ggt aaa aca gcc tct gtt cta aca act ggt gac tgg aat aac   1872
Asn Asn Gly Lys Thr Ala Ser Val Leu Thr Thr Gly Asp Trp Asn Asn
        610                 615                 620

ata aat gaa gag ttt aca gat gtt gct aag gca aaa cca ggg gtt aag   1920
Ile Asn Glu Glu Phe Thr Asp Val Ala Lys Ala Lys Pro Gly Val Lys
625                 630                 635                 640

ggt aaa act tat gca ttt ata aat gat gat aag ttt gct gtt act ata   1968
Gly Lys Thr Tyr Ala Phe Ile Asn Asp Asp Lys Phe Ala Val Thr Ile
                645                 650                 655

aat aat aat act att gaa ggt gga aat agg gtt gta tta aca aca gaa   2016
Asn Asn Asn Thr Ile Glu Gly Gly Asn Arg Val Val Leu Thr Thr Glu
                660                 665                 670

aat gat act ctt cct gat aat aca aac tat aag aaa gtt gga ata tca   2064
Asn Asp Thr Leu Pro Asp Asn Thr Asn Tyr Lys Lys Val Gly Ile Ser
```

```
                675                 680                 685
        aac ggt act tgg act tat aaa gaa ata ctt caa gat aca aca gat caa        2112
        Asn Gly Thr Trp Thr Tyr Lys Glu Ile Leu Gln Asp Thr Thr Asp Gln
            690                 695                 700

        gga agt aag cta tat caa ggt gaa aag cca tgg tca gaa gta att ata        2160
        Gly Ser Lys Leu Tyr Gln Gly Glu Lys Pro Trp Ser Glu Val Ile Ile
        705                 710                 715                 720

        gca aga gat gag aat gaa gat ggt caa gta gat tgg caa gat ggt gct        2208
        Ala Arg Asp Glu Asn Glu Asp Gly Gln Val Asp Trp Gln Asp Gly Ala
                        725                 730                 735

        att caa tat aga aaa aat atg aaa att cca gta ggt gga gaa gaa ata        2256
        Ile Gln Tyr Arg Lys Asn Met Lys Ile Pro Val Gly Gly Glu Glu Ile
                    740                 745                 750

        aaa aat caa atg tca tat att gat ttt aat att gga tac act caa aat        2304
        Lys Asn Gln Met Ser Tyr Ile Asp Phe Asn Ile Gly Tyr Thr Gln Asn
                    755                 760                 765

        cca ttc tta aga tca tta gat aca att aaa aag ctt tca aat tat aca        2352
        Pro Phe Leu Arg Ser Leu Asp Thr Ile Lys Lys Leu Ser Asn Tyr Thr
            770                 775                 780

        gat gga ttt gga caa tta gtt ctt cat aag gga tat caa gga gaa gga        2400
        Asp Gly Phe Gly Gln Leu Val Leu His Lys Gly Tyr Gln Gly Glu Gly
        785                 790                 795                 800

        cat gac gac tct cat cca gac tat ggc gga cat att ggt atg aga caa        2448
        His Asp Asp Ser His Pro Asp Tyr Gly Gly His Ile Gly Met Arg Gln
                        805                 810                 815

        ggt ggt aag gaa gac ttc aat acc tta ata gaa caa ggt aag gaa tat        2496
        Gly Gly Lys Glu Asp Phe Asn Thr Leu Ile Glu Gln Gly Lys Glu Tyr
                        820                 825                 830

        aat gct aaa ata ggt gtt cat ata aat gca acc gaa tat act atg gat        2544
        Asn Ala Lys Ile Gly Val His Ile Asn Ala Thr Glu Tyr Thr Met Asp
                    835                 840                 845

        gcg ttt gaa tat cca act gaa tta gtt aac gaa aat gct cca gga tgg        2592
        Ala Phe Glu Tyr Pro Thr Glu Leu Val Asn Glu Asn Ala Pro Gly Trp
            850                 855                 860

        gga tgg tta gac caa gct tat tat gta aat caa aga gga gat ata act        2640
        Gly Trp Leu Asp Gln Ala Tyr Tyr Val Asn Gln Arg Gly Asp Ile Thr
        865                 870                 875                 880

        agt ggt gaa tta ttc aga aga ctt gat atg tta atg gaa gat gca cca        2688
        Ser Gly Glu Leu Phe Arg Arg Leu Asp Met Leu Met Glu Asp Ala Pro
                        885                 890                 895

        gaa cta gga tgg att tac gtt gac gtt tat aca ggt aat gga tgg aat        2736
        Glu Leu Gly Trp Ile Tyr Val Asp Val Tyr Thr Gly Asn Gly Trp Asn
                    900                 905                 910

        gct cat caa tta gga gaa aag ata aat gat tat gga att atg att gca        2784
        Ala His Gln Leu Gly Glu Lys Ile Asn Asp Tyr Gly Ile Met Ile Ala
            915                 920                 925

        aca gaa atg aat gga cca tta gag caa cat gtt cca tgg act cac tgg        2832
        Thr Glu Met Asn Gly Pro Leu Glu Gln His Val Pro Trp Thr His Trp
            930                 935                 940

        ggt gga gat cct gca tat cca aac aag gga aat gca agt aaa ata atg        2880
        Gly Gly Asp Pro Ala Tyr Pro Asn Lys Gly Asn Ala Ser Lys Ile Met
        945                 950                 955                 960

        aga ttt atg aaa aat gat act caa gat tca ttc tta gca gat cca tta        2928
        Arg Phe Met Lys Asn Asp Thr Gln Asp Ser Phe Leu Ala Asp Pro Leu
                    965                 970                 975

        gta aaa ggt aat aag cat tta tta tca ggt gga tgg gga act aga cat        2976
        Val Lys Gly Asn Lys His Leu Leu Ser Gly Gly Trp Gly Thr Arg His
```

```
                    980                 985                 990
    gat ata gaa ggt gct tat ggt aca  gaa gta ttc tat aat  caa gta tta   3024
    Asp Ile Glu Gly Ala Tyr Gly Thr  Glu Val Phe Tyr Asn  Gln Val Leu
        995                 1000                 1005

    cct act aag tat tta caa cac  ttc caa ata act aag  atg agc gaa        3069
    Pro Thr Lys Tyr Leu Gln His  Phe Gln Ile Thr Lys  Met Ser Glu
        1010                 1015                 1020

    aat gaa gta tta ttt gaa aat  gga gtt aag gct gtt  aga gaa aac        3114
    Asn Glu Val Leu Phe Glu Asn  Gly Val Lys Ala Val  Arg Glu Asn
        1025                 1030                 1035

    tct aat ata aat tac tat aga  aat gat aga tta gtt  gct act act        3159
    Ser Asn Ile Asn Tyr Tyr Arg  Asn Asp Arg Leu Val  Ala Thr Thr
        1040                 1045                 1050

    cca gaa aat tca att gga aat  act ggt ata ggg gat  act caa tta        3204
    Pro Glu Asn Ser Ile Gly Asn  Thr Gly Ile Gly Asp  Thr Gln Leu
        1055                 1060                 1065

    ttc tta cca tgg aat cct gtt  gat gag gca aat agt  gaa aag ata        3249
    Phe Leu Pro Trp Asn Pro Val  Asp Glu Ala Asn Ser  Glu Lys Ile
        1070                 1075                 1080

    tat cac tgg aat cca tta gga  act act tca gaa tgg  act tta cca        3294
    Tyr His Trp Asn Pro Leu Gly  Thr Thr Ser Glu Trp  Thr Leu Pro
        1085                 1090                 1095

    gaa gga tgg act tct aat gac  aaa gtt tat tta tat  gaa tta tca        3339
    Glu Gly Trp Thr Ser Asn Asp  Lys Val Tyr Leu Tyr  Glu Leu Ser
        1100                 1105                 1110

    gat tta gga aga act tta gtt  aag gaa gta cca gtt  gta gat gga        3384
    Asp Leu Gly Arg Thr Leu Val  Lys Glu Val Pro Val  Val Asp Gly
        1115                 1120                 1125

    aaa gtt aat tta gaa gta aaa  caa gat act cct tat  ata gtt act        3429
    Lys Val Asn Leu Glu Val Lys  Gln Asp Thr Pro Tyr  Ile Val Thr
        1130                 1135                 1140

    aag gaa aaa gtt gaa gag aag  aga ata gag gat tgg  gga tat ggc        3474
    Lys Glu Lys Val Glu Glu Lys  Arg Ile Glu Asp Trp  Gly Tyr Gly
        1145                 1150                 1155

    tca gaa ata gct gat cca gga  ttt gat tct caa act  ttt gat aag        3519
    Ser Glu Ile Ala Asp Pro Gly  Phe Asp Ser Gln Thr  Phe Asp Lys
        1160                 1165                 1170

    tgg aat aaa gaa tct act gct  gaa aat aca gat cat  ata act att        3564
    Trp Asn Lys Glu Ser Thr Ala  Glu Asn Thr Asp His  Ile Thr Ile
        1175                 1180                 1185

    gaa aat gaa agt gtt caa aag  aga tta ggt aat gat  gtt ctt aaa        3609
    Glu Asn Glu Ser Val Gln Lys  Arg Leu Gly Asn Asp  Val Leu Lys
        1190                 1195                 1200

    ata agt gga aat gaa ggc gct  gat gct aaa att tct  caa agc ata        3654
    Ile Ser Gly Asn Glu Gly Ala  Asp Ala Lys Ile Ser  Gln Ser Ile
        1205                 1210                 1215

    agt gga tta gaa gaa gga gta  act tat tca gta tct  gca tgg gtt        3699
    Ser Gly Leu Glu Glu Gly Val  Thr Tyr Ser Val Ser  Ala Trp Val
        1220                 1225                 1230

    aaa aat gat aat aat aga gaa  gtt aca cta gga gtt  aat gtt ggt        3744
    Lys Asn Asp Asn Asn Arg Glu  Val Thr Leu Gly Val  Asn Val Gly
        1235                 1240                 1245

    gga aaa gat ttc act aat gta  ata aca agt ggt ggt  aaa gta aga        3789
    Gly Lys Asp Phe Thr Asn Val  Ile Thr Ser Gly Gly  Lys Val Arg
        1250                 1255                 1260

    caa gga gaa ggt gtt aag tat  att gac gat act ttc  gtg aga atg        3834
    Gln Gly Glu Gly Val Lys Tyr  Ile Asp Asp Thr Phe  Val Arg Met
```

```
              1265                 1270                 1275
   gaa gta  gaa ttt act gta cct  aaa gga gta aat tca  gct gat gtt    3879
   Glu Val  Glu Phe Thr Val Pro  Lys Gly Val Asn Ser  Ala Asp Val
       1280                 1285                 1290

   tac tta  aag gct tca gaa gga  gat gct gac tca gtt  gta cta gtt    3924
   Tyr Leu  Lys Ala Ser Glu Gly  Asp Ala Asp Ser Val  Val Leu Val
       1295                 1300                 1305

   gat gac  ttt aga ata tgg gat  cat cca gga cac act  aat aga gat    3969
   Asp Asp  Phe Arg Ile Trp Asp  His Pro Gly His Thr  Asn Arg Asp
       1310                 1315                 1320

   gga tat  gta ttc tac gaa gat  ttt gaa aat gta gat  gaa ggt ata    4014
   Gly Tyr  Val Phe Tyr Glu Asp  Phe Glu Asn Val Asp  Glu Gly Ile
       1325                 1330                 1335

   tca cca  ttt tat tta tct cca  gga aga gga cat tca  aat aga tct    4059
   Ser Pro  Phe Tyr Leu Ser Pro  Gly Arg Gly His Ser  Asn Arg Ser
       1340                 1345                 1350

   cac tta  gct gag aag gat ata  tct ata gat gct aat  caa aga atg    4104
   His Leu  Ala Glu Lys Asp Ile  Ser Ile Asp Ala Asn  Gln Arg Met
       1355                 1360                 1365

   aac tgg  gta ctt gat gga aga  ttc tca tta aaa tct  aac caa caa    4149
   Asn Trp  Val Leu Asp Gly Arg  Phe Ser Leu Lys Ser  Asn Gln Gln
       1370                 1375                 1380

   cca aag  gaa ata ggt gaa atg  ttg act acg gat gtt  tca tca ttc    4194
   Pro Lys  Glu Ile Gly Glu Met  Leu Thr Thr Asp Val  Ser Ser Phe
       1385                 1390                 1395

   aaa tta  gaa cca aat aaa act  tat gaa ttt gga ttc  tta tat tca    4239
   Lys Leu  Glu Pro Asn Lys Thr  Tyr Glu Phe Gly Phe  Leu Tyr Ser
       1400                 1405                 1410

   tta gaa  aat gct gct cca gga  tat tct gta aat att  aag aat aga    4284
   Leu Glu  Asn Ala Ala Pro Gly  Tyr Ser Val Asn Ile  Lys Asn Arg
       1415                 1420                 1425

   gat ggt  gaa aag att gta agt  att cct tta gag gct  act ggt tca    4329
   Asp Gly  Glu Lys Ile Val Ser  Ile Pro Leu Glu Ala  Thr Gly Ser
       1430                 1435                 1440

   aat tat  gca caa gat att ttc  act aaa act aaa tct  gta act cac    4374
   Asn Tyr  Ala Gln Asp Ile Phe  Thr Lys Thr Lys Ser  Val Thr His
       1445                 1450                 1455

   gag ttt  aca act gga gat ttt  gct gga gat tac tat  att act tta    4419
   Glu Phe  Thr Thr Gly Asp Phe  Ala Gly Asp Tyr Tyr  Ile Thr Leu
       1460                 1465                 1470

   gaa aaa  ggt gat gga ttt aaa  gaa gta atc tta gat  aat att tat    4464
   Glu Lys  Gly Asp Gly Phe Lys  Glu Val Ile Leu Asp  Asn Ile Tyr
       1475                 1480                 1485

   gtt aag  gaa ata gat aag tca  att gaa tca cct gaa  tta gct cat    4509
   Val Lys  Glu Ile Asp Lys Ser  Ile Glu Ser Pro Glu  Leu Ala His
       1490                 1495                 1500

   gta aac  tta aat aca gta gaa  cat gac tta gaa gtt  gga caa agc    4554
   Val Asn  Leu Asn Thr Val Glu  His Asp Leu Glu Val  Gly Gln Ser
       1505                 1510                 1515

   gtt cca  ttt gct ata aat gca  tta atg aat aat ggt  gca aat gtt    4599
   Val Pro  Phe Ala Ile Asn Ala  Leu Met Asn Asn Gly  Ala Asn Val
       1520                 1525                 1530

   aac tta  gaa gaa gct gaa gtt  gaa tat aaa gtt tca  aaa cca gaa    4644
   Asn Leu  Glu Glu Ala Glu Val  Glu Tyr Lys Val Ser  Lys Pro Glu
       1535                 1540                 1545

   gtt tta  act att gaa aat gga  atg atg act gga gct  tct gaa ggc    4689
   Val Leu  Thr Ile Glu Asn Gly  Met Met Thr Gly Ala  Ser Glu Gly
```

```
                 1550                 1555                 1560
ttt act gat gtc caa gta aat  att act gtt aat gga  aat aaa gtt    4734
Phe Thr Asp Val Gln Val Asn  Ile Thr Val Asn Gly  Asn Lys Val
    1565                 1570                 1575

tct tca aat aca gta aga gtt  aag gtt gga aat cca  gaa gtt gag    4779
Ser Ser Asn Thr Val Arg Val  Lys Val Gly Asn Pro  Glu Val Glu
    1580                 1585                 1590

gaa gaa gaa gtt ata gta aac  cca gta aga aac ttt  aag gtt act    4824
Glu Glu Glu Val Ile Val Asn  Pro Val Arg Asn Phe  Lys Val Thr
    1595                 1600                 1605

gat aag act aag aag aat gta  act gta agc tgg gaa  gag cca gaa    4869
Asp Lys Thr Lys Lys Asn Val  Thr Val Ser Trp Glu  Glu Pro Glu
    1610                 1615                 1620

aaa act tat gga tta gaa ggc  tat gtt ctt tat aaa  gat ggt aag    4914
Lys Thr Tyr Gly Leu Glu Gly  Tyr Val Leu Tyr Lys  Asp Gly Lys
    1625                 1630                 1635

aaa gtt aaa gaa ata ggt gct  gat aaa act gaa ttt  aca ttt aag    4959
Lys Val Lys Glu Ile Gly Ala  Asp Lys Thr Glu Phe  Thr Phe Lys
    1640                 1645                 1650

gga tta aac aga cac act att  tat aac ttt aag att  gct gct aaa    5004
Gly Leu Asn Arg His Thr Ile  Tyr Asn Phe Lys Ile  Ala Ala Lys
    1655                 1660                 1665

tat tct aat ggt gaa ctt tca  act aag gaa tca ata  act gtt aga    5049
Tyr Ser Asn Gly Glu Leu Ser  Thr Lys Glu Ser Ile  Thr Val Arg
    1670                 1675                 1680

act gca aga taa                                                  5061
Thr Ala Arg
    1685
```

<210> 8
<211> 1686
<212> PRT
<213> Clostridium perfringens str. 13

<400> 8

```
Met Gly Arg Lys Cys Met Asn Lys Lys Ile Ala Ala Ile Ile Ala Ala
1             5                 10                15

Ala Val Ile Val Gly Gln Leu Pro Ile Ser Val Leu Ala Thr Pro Val
            20              25              30

Asn Glu Ala Gly Asp Glu Ile Asn Ser Glu Ser Ala Glu Ile Leu Thr
        35              40              45

Asn Ser Asp Glu Glu Ala Glu Ala Tyr Ile Gln Asn Tyr Asp Arg Pro
    50              55              60

Glu Gly Ile Thr Trp Thr Lys Leu Ala Gly Ser Gly Ser Val Glu Val
65              70              75              80

Thr Asp Gly Phe Leu Ser Val Thr Asn Asn Gly Asp Tyr Arg Ile Met
            85              90              95

Glu Asp Gln Ser Pro Asn Ile Lys Asn Gly Glu Leu Glu Ser Lys Phe
        100             105             110

Thr Val Gly Gly Ser Gln Thr Gly Ile Ile Phe Arg Ala Thr Glu Ser
        115             120             125
```

Asn Tyr Gly Met Ile Asn Tyr Asn Ser Gly Thr Gly Trp Val Ile Glu
130             135         140

Asn Lys Asn Ser Trp Glu Asp Ile Thr Gly Pro Lys Leu Asn Asn Gly
145             150         155             160

Asp Val Val Thr Val Lys Ala Thr Phe Val Glu Lys His Leu Thr Val
            165         170             175

Asn Val Ser Val Asn Asp Gly Glu Phe Glu Thr Ile Tyr Asp Lys Glu
        180         185             190

Ser Asp Leu Ile Pro Leu Gln Ala Gly Lys Val Gly Tyr Arg Gly Trp
        195             200             205

Gly Asn Ala Lys Thr Thr Lys Phe Asp Tyr Ile Lys Tyr Ala Pro Met
    210             215             220

Thr Ile Asp Lys Gly Pro Ile Val Ser Ile Asn Glu Val Asn Val Glu
225             230             235             240

Thr Tyr Pro Arg Val Lys Pro Ile Leu Pro Ser Ser Val Thr Val Asn
            245             250             255

His Glu Asn Gly Met Ser Ser Ile Lys Asp Val Ser Trp Asn Tyr Ile
            260             265             270

Pro Lys Glu Ser Tyr Ser Lys Pro Gly Thr Phe Lys Val Glu Gly Thr
        275             280             285

Val Glu Gly Thr Asp Val Lys Ala Ile Ala Asn Val Thr Val Ser Ser
    290             295             300

Asp Leu Ala Tyr Tyr Glu Thr Asn Phe Glu Thr Glu Glu Thr Arg Gly
305             310             315             320

Asp Trp Gln Val Val Gln Gly Gly Gly Ser Pro Ser Tyr Glu Glu Gly
            325             330             335

Lys Val Lys Ile Pro Met Asn Gly Val Ser Ile Ala Val Asp Met Asn
        340             345             350

Ser Pro Glu Val Lys Asn Phe Thr Tyr Glu Thr Asp Phe Ser Val Asp
        355             360             365

Asn Asn Gly Gly Arg Ile Gly Leu Leu Phe Arg Tyr Val Ser Glu Thr
    370             375             380

Glu Trp Gly Ala Val Cys Tyr Asp Asn Gly Ser Trp Val Trp Lys Thr
385             390             395             400

Gly Asp Gly Lys Tyr Gly Asn Phe Pro Gly Thr Phe Thr Pro Glu Pro
            405             410             415

Gly Lys Thr Tyr Arg Ile Lys Leu Lys Val Glu Asp Thr Asn Ile Thr
            420             425             430

```
Met Trp Val Asp Gly Glu Lys Ile Gly Gln Val Ala Val Ser Asn Leu
        435             440             445

Pro Asp Val Arg Gly Lys Val Gly Leu Thr Gly Trp Phe Gly Asn Lys
        450             455             460

Asn Val Thr Leu Asp Asn Leu Val Val Glu Glu Leu Gly Gly Ile Met
465             470             475             480

Ala Pro Glu Val Gly Pro Leu Glu Glu Gln Ser Ile Glu Ser Asp Ser
            485             490             495

Met Lys Val Val Leu Asp Asn Arg Phe Pro Thr Val Ile Arg Tyr Glu
        500             505             510

Trp Lys Gly Thr Glu Asp Val Leu Ser Gly Ala Ser Val Asp Asp Leu
        515             520             525

Glu Ala Gln Tyr Met Val Glu Ile Asn Gly Glu Lys Arg Ile Pro Lys
        530             535             540

Val Thr Ser Glu Phe Ala Asn Asn Glu Gly Ile Tyr Thr Leu Asn Phe
545             550             555             560

Glu Asp Ile Gly Met Thr Ile Thr Leu Lys Met Thr Val Asn Glu Asn
            565             570             575

Lys Leu Arg Met Glu Val Thr Asp Ile Gln Glu Gly Asp Val Lys Leu
            580             585             590

Gln Thr Leu Asn Phe Pro Asn His Ser Leu Ala Ser Val Ser Ser Leu
        595             600             605

Asn Asn Gly Lys Thr Ala Ser Val Leu Thr Thr Gly Asp Trp Asn Asn
        610             615             620

Ile Asn Glu Glu Phe Thr Asp Val Ala Lys Ala Lys Pro Gly Val Lys
625             630             635             640

Gly Lys Thr Tyr Ala Phe Ile Asn Asp Asp Lys Phe Ala Val Thr Ile
            645             650             655

Asn Asn Asn Thr Ile Glu Gly Gly Asn Arg Val Val Leu Thr Thr Glu
            660             665             670

Asn Asp Thr Leu Pro Asp Asn Thr Asn Tyr Lys Lys Val Gly Ile Ser
        675             680             685

Asn Gly Thr Trp Thr Tyr Lys Glu Ile Leu Gln Asp Thr Thr Asp Gln
        690             695             700

Gly Ser Lys Leu Tyr Gln Gly Glu Lys Pro Trp Ser Glu Val Ile Ile
705             710             715             720

Ala Arg Asp Glu Asn Glu Asp Gly Gln Val Asp Trp Gln Asp Gly Ala
            725             730             735
```

```
Ile Gln Tyr Arg Lys Asn Met Lys Ile Pro Val Gly Gly Glu Glu Ile
            740               745               750

Lys Asn Gln Met Ser Tyr Ile Asp Phe Asn Ile Gly Tyr Thr Gln Asn
            755               760               765

Pro Phe Leu Arg Ser Leu Asp Thr Ile Lys Lys Leu Ser Asn Tyr Thr
    770               775               780

Asp Gly Phe Gly Gln Leu Val Leu His Lys Gly Tyr Gln Gly Glu Gly
785               790               795               800

His Asp Asp Ser His Pro Asp Tyr Gly Gly His Ile Gly Met Arg Gln
                805               810               815

Gly Gly Lys Glu Asp Phe Asn Thr Leu Ile Glu Gln Gly Lys Glu Tyr
            820               825               830

Asn Ala Lys Ile Gly Val His Ile Asn Ala Thr Glu Tyr Thr Met Asp
            835               840               845

Ala Phe Glu Tyr Pro Thr Glu Leu Val Asn Glu Asn Ala Pro Gly Trp
    850               855               860

Gly Trp Leu Asp Gln Ala Tyr Tyr Val Asn Gln Arg Gly Asp Ile Thr
865               870               875               880

Ser Gly Glu Leu Phe Arg Arg Leu Asp Met Leu Met Glu Asp Ala Pro
                885               890               895

Glu Leu Gly Trp Ile Tyr Val Asp Val Tyr Thr Gly Asn Gly Trp Asn
            900               905               910

Ala His Gln Leu Gly Glu Lys Ile Asn Asp Tyr Gly Ile Met Ile Ala
            915               920               925

Thr Glu Met Asn Gly Pro Leu Glu Gln His Val Pro Trp Thr His Trp
    930               935               940

Gly Gly Asp Pro Ala Tyr Pro Asn Lys Gly Asn Ala Ser Lys Ile Met
945               950               955               960

Arg Phe Met Lys Asn Asp Thr Gln Asp Ser Phe Leu Ala Asp Pro Leu
                965               970               975

Val Lys Gly Asn Lys His Leu Leu Ser Gly Gly Trp Gly Thr Arg His
            980               985               990

Asp Ile Glu Gly Ala Tyr Gly Thr Glu Val Phe Tyr Asn Gln Val Leu
        995               1000              1005

Pro Thr Lys Tyr Leu Gln His Phe Gln Ile Thr Lys Met Ser Glu
    1010              1015              1020

Asn Glu Val Leu Phe Glu Asn Gly Val Lys Ala Val Arg Glu Asn
    1025              1030              1035
```

Ser Asn Ile Asn Tyr Tyr Arg Asn Asp Arg Leu Val Ala Thr Thr
1040 1045 1050

Pro Glu Asn Ser Ile Gly Asn Thr Gly Ile Gly Asp Thr Gln Leu
1055 1060 1065

Phe Leu Pro Trp Asn Pro Val Asp Glu Ala Asn Ser Glu Lys Ile
1070 1075 1080

Tyr His Trp Asn Pro Leu Gly Thr Thr Ser Glu Trp Thr Leu Pro
1085 1090 1095

Glu Gly Trp Thr Ser Asn Asp Lys Val Tyr Leu Tyr Glu Leu Ser
1100 1105 1110

Asp Leu Gly Arg Thr Leu Val Lys Glu Val Pro Val Val Asp Gly
1115 1120 1125

Lys Val Asn Leu Glu Val Lys Gln Asp Thr Pro Tyr Ile Val Thr
1130 1135 1140

Lys Glu Lys Val Glu Glu Lys Arg Ile Glu Asp Trp Gly Tyr Gly
1145 1150 1155

Ser Glu Ile Ala Asp Pro Gly Phe Asp Ser Gln Thr Phe Asp Lys
1160 1165 1170

Trp Asn Lys Glu Ser Thr Ala Glu Asn Thr Asp His Ile Thr Ile
1175 1180 1185

Glu Asn Glu Ser Val Gln Lys Arg Leu Gly Asn Asp Val Leu Lys
1190 1195 1200

Ile Ser Gly Asn Glu Gly Ala Asp Ala Lys Ile Ser Gln Ser Ile
1205 1210 1215

Ser Gly Leu Glu Glu Gly Val Thr Tyr Ser Val Ser Ala Trp Val
1220 1225 1230

Lys Asn Asp Asn Asn Arg Glu Val Thr Leu Gly Val Asn Val Gly
1235 1240 1245

Gly Lys Asp Phe Thr Asn Val Ile Thr Ser Gly Gly Lys Val Arg
1250 1255 1260

Gln Gly Glu Gly Val Lys Tyr Ile Asp Asp Thr Phe Val Arg Met
1265 1270 1275

Glu Val Glu Phe Thr Val Pro Lys Gly Val Asn Ser Ala Asp Val
1280 1285 1290

Tyr Leu Lys Ala Ser Glu Gly Asp Ala Asp Ser Val Val Leu Val
1295 1300 1305

Asp Asp Phe Arg Ile Trp Asp His Pro Gly His Thr Asn Arg Asp
1310 1315 1320

Gly Tyr Val Phe Tyr Glu Asp Phe Glu Asn Val Asp Glu Gly Ile
    1325              1330              1335

Ser Pro Phe Tyr Leu Ser Pro Gly Arg Gly His Ser Asn Arg Ser
    1340              1345              1350

His Leu Ala Glu Lys Asp Ile Ser Ile Asp Ala Asn Gln Arg Met
    1355              1360              1365

Asn Trp Val Leu Asp Gly Arg Phe Ser Leu Lys Ser Asn Gln Gln
    1370              1375              1380

Pro Lys Glu Ile Gly Glu Met Leu Thr Thr Asp Val Ser Ser Phe
    1385              1390              1395

Lys Leu Glu Pro Asn Lys Thr Tyr Glu Phe Gly Phe Leu Tyr Ser
    1400              1405              1410

Leu Glu Asn Ala Ala Pro Gly Tyr Ser Val Asn Ile Lys Asn Arg
    1415              1420              1425

Asp Gly Glu Lys Ile Val Ser Ile Pro Leu Glu Ala Thr Gly Ser
    1430              1435              1440

Asn Tyr Ala Gln Asp Ile Phe Thr Lys Thr Lys Ser Val Thr His
    1445              1450              1455

Glu Phe Thr Thr Gly Asp Phe Ala Gly Asp Tyr Tyr Ile Thr Leu
    1460              1465              1470

Glu Lys Gly Asp Gly Phe Lys Glu Val Ile Leu Asp Asn Ile Tyr
    1475              1480              1485

Val Lys Glu Ile Asp Lys Ser Ile Glu Ser Pro Glu Leu Ala His
    1490              1495              1500

Val Asn Leu Asn Thr Val Glu His Asp Leu Glu Val Gly Gln Ser
    1505              1510              1515

Val Pro Phe Ala Ile Asn Ala Leu Met Asn Asn Gly Ala Asn Val
    1520              1525              1530

Asn Leu Glu Glu Ala Glu Val Glu Tyr Lys Val Ser Lys Pro Glu
    1535              1540              1545

Val Leu Thr Ile Glu Asn Gly Met Met Thr Gly Ala Ser Glu Gly
    1550              1555              1560

Phe Thr Asp Val Gln Val Asn Ile Thr Val Asn Gly Asn Lys Val
    1565              1570              1575

Ser Ser Asn Thr Val Arg Val Lys Val Gly Asn Pro Glu Val Glu
    1580              1585              1590

Glu Glu Glu Val Ile Val Asn Pro Val Arg Asn Phe Lys Val Thr
    1595              1600              1605

EP 1 767 645 B1

```
Asp Lys  Thr Lys Lys Asn Val  Thr Val Ser Trp Glu  Glu Pro Glu
    1610             1615             1620

Lys Thr  Tyr Gly Leu Glu Gly  Tyr Val Leu Tyr Lys  Asp Gly Lys
    1625             1630             1635

Lys Val  Lys Glu Ile Gly Ala  Asp Lys Thr Glu Phe  Thr Phe Lys
    1640             1645             1650

Gly Leu  Asn Arg His Thr Ile  Tyr Asn Phe Lys Ile  Ala Ala Lys
    1655             1660             1665

Tyr Ser  Asn Gly Glu Leu Ser  Thr Lys Glu Ser Ile  Thr Val Arg
    1670             1675             1680

Thr Ala  Arg
    1685
```

<210> 9
<211> 4086
<212> DNA
<213> Streptomyces coelicolor A3(2)

<220>
<221> CDS
<222> (1)..(4086)

<400> 9

```
atg gca gcg atc aca ccg aag cga tcg gtc aga gtg gac acc tcg aca     48
Met Ala Ala Ile Thr Pro Lys Arg Ser Val Arg Val Asp Thr Ser Thr
1               5                   10                  15

ggc tca tca tct ccc cca acc gga cgc gca cgg gtc cgg cgg cac ggc     96
Gly Ser Ser Ser Pro Pro Thr Gly Arg Ala Arg Val Arg Arg His Gly
                20                  25                  30

gcc gtc gtc gcg gcg ctg ggc ctc acc gcg ggc ctg ctg tcg gcc gcc    144
Ala Val Val Ala Ala Leu Gly Leu Thr Ala Gly Leu Leu Ser Ala Ala
            35                  40                  45

gcc ctg ccc gcg ggc gcg gcc ccg ccc cgc ccc gct gcc gct gcg gcg    192
Ala Leu Pro Ala Gly Ala Ala Pro Pro Arg Pro Ala Ala Ala Ala Ala
        50                  55                  60

ccc gcg ggg gca ccg acg ccg gtg gaa ctg agc cgg ggc ggg ctg acc    240
Pro Ala Gly Ala Pro Thr Pro Val Glu Leu Ser Arg Gly Gly Leu Thr
65                  70                  75                  80

gtc acc gtg gcg aag gag ttc ccc cag gtg atc tcc tac cgg ctg ggc    288
Val Thr Val Ala Lys Glu Phe Pro Gln Val Ile Ser Tyr Arg Leu Gly
                85                  90                  95

cgg cgc gga ctc gac ggg cgg gca acg gcg ctg gac ggc ttc acc gtc    336
Arg Arg Gly Leu Asp Gly Arg Ala Thr Ala Leu Asp Gly Phe Thr Val
                100                 105                 110

aac ggc gag agc cac cgc gcc acc acc acc gtg aag gcg aag ggc agc    384
Asn Gly Glu Ser His Arg Ala Thr Thr Thr Val Lys Ala Lys Gly Ser
        115                 120                 125

agg gcg gtc tac acc tcc acg ttc gag gac ctg ccc ggt ctc acg atc    432
Arg Ala Val Tyr Thr Ser Thr Phe Glu Asp Leu Pro Gly Leu Thr Ile
        130                 135                 140

acc tcc agc atc acg gtc acc aag gag acg acg gtc gtc ttc gcc gtc    480
Thr Ser Ser Ile Thr Val Thr Lys Glu Thr Thr Val Val Phe Ala Val
145                 150                 155                 160
```

```
gag aag atc tcg ggc gag gcc gcg ccg ggc gtg cgc acc ctc gcg atc      528
Glu Lys Ile Ser Gly Glu Ala Ala Pro Gly Val Arg Thr Leu Ala Ile
            165                 170                 175

ccc ggc cag tcc ctc gtc tcc gtc gac tcc gcc gag ccg ggc gcc aac      576
Pro Gly Gln Ser Leu Val Ser Val Asp Ser Ala Glu Pro Gly Ala Asn
            180                 185                 190

ctc gcc cgg acg aag atc tcc acc gac tcg acg acg acc gcc gac cgc      624
Leu Ala Arg Thr Lys Ile Ser Thr Asp Ser Thr Thr Thr Ala Asp Arg
            195                 200                 205

ttc gtc ccc gtc acc ggc gac acc gcc ccg gac aag ggg ccc gtc ggc      672
Phe Val Pro Val Thr Gly Asp Thr Ala Pro Asp Lys Gly Pro Val Gly
            210                 215                 220

acc ccg tac gcg ttc gtc ggc aac gcg cag ttg tcg gcg ggc atc atc      720
Thr Pro Tyr Ala Phe Val Gly Asn Ala Gln Leu Ser Ala Gly Ile Ile
225                 230                 235                 240

acc aac gcg acc gag gac tcg ccg cag gac gac aac acc gac tgg aac      768
Thr Asn Ala Thr Glu Asp Ser Pro Gln Asp Asp Asn Thr Asp Trp Asn
                245                 250                 255

acc cgc ctg cag tcc cgc atc gtc gac gag ggc gag gga cgg cgc cgg      816
Thr Arg Leu Gln Ser Arg Ile Val Asp Glu Gly Glu Gly Arg Arg Arg
                260                 265                 270

gcg gag ctg tcg gcc ggc acg tac acc tac cac ccc gag ggc gcc acc      864
Ala Glu Leu Ser Ala Gly Thr Tyr Thr Tyr His Pro Glu Gly Ala Thr
                275                 280                 285

gat cca cgg gtc gac acc tac gag ctg ccc cgg gcg acg gtg gtc ctc      912
Asp Pro Arg Val Asp Thr Tyr Glu Leu Pro Arg Ala Thr Val Val Leu
            290                 295                 300

gcc gcc gac gcc aac cgg gac ggc acg gtc gac tgg cag gac ggc gcc      960
Ala Ala Asp Ala Asn Arg Asp Gly Thr Val Asp Trp Gln Asp Gly Ala
305                 310                 315                 320

atc gcc cac cgg gag cac atg cgc cgc ccg ctc ggc gcc gac cgg gtg     1008
Ile Ala His Arg Glu His Met Arg Arg Pro Leu Gly Ala Asp Arg Val
            325                 330                 335

ccc gaa cgc gtg gtc cag cgc atc ccg ttc aac ttc gcg agc cag gcc     1056
Pro Glu Arg Val Val Gln Arg Ile Pro Phe Asn Phe Ala Ser Gln Ala
            340                 345                 350

acc aac ccg ttc ctg aag acg ctg gac aac acc aag cgc atc tcc atg     1104
Thr Asn Pro Phe Leu Lys Thr Leu Asp Asn Thr Lys Arg Ile Ser Met
                355                 360                 365

gcc acc gac gac ctc ggg cag tgg gtg ctg gag aag ggg tac gcg agc     1152
Ala Thr Asp Asp Leu Gly Gln Trp Val Leu Glu Lys Gly Tyr Ala Ser
            370                 375                 380

gag ggc cac gac tcc gcc cac ccc gac tac ggc ggc aac gag aac gtc     1200
Glu Gly His Asp Ser Ala His Pro Asp Tyr Gly Gly Asn Glu Asn Val
385                 390                 395                 400

cgc gcg ggc ggc tgg aag gac ctg aac cgc ctc acc cgg acg gga gcc     1248
Arg Ala Gly Gly Trp Lys Asp Leu Asn Arg Leu Thr Arg Thr Gly Ala
                405                 410                 415

ggc tac aac gcg gac ttc gcc gtc cac gtc aac gcc acg gag gcc tac     1296
Gly Tyr Asn Ala Asp Phe Ala Val His Val Asn Ala Thr Glu Ala Tyr
            420                 425                 430

gcc cag gcc agg acc ttc acc gag gac atg gtc gcg ggc cag gcc gac     1344
Ala Gln Ala Arg Thr Phe Thr Glu Asp Met Val Ala Gly Gln Ala Asp
            435                 440                 445

ggc tgg gac tgg ctc aac cag gcc tac cac atc gac cag cgc aag gac     1392
Gly Trp Asp Trp Leu Asn Gln Ala Tyr His Ile Asp Gln Arg Lys Asp
            450                 455                 460
```

ctg ggc acc ggc gcc gtc ctc gac cgc ttc aag cag ctc cgc aag gaa   1440
Leu Gly Thr Gly Ala Val Leu Asp Arg Phe Lys Gln Leu Arg Lys Glu
465           470             475            480

gca ccg ggc atc aga acc gtc tac atc gac gcc tac tac tcc agc ggc   1488
Ala Pro Gly Ile Arg Thr Val Tyr Ile Asp Ala Tyr Tyr Ser Ser Gly
            485            490            495

tgg ctg gcc gac ggt ctg gcc gcc ggg ctg cgt gag atg ggc ttc gag   1536
Trp Leu Ala Asp Gly Leu Ala Ala Gly Leu Arg Glu Met Gly Phe Glu
          500            505           510

gtc gcc acc gag tgg gcg tac aag ttc gag ggc acc tcg gtg tgg tcg   1584
Val Ala Thr Glu Trp Ala Tyr Lys Phe Glu Gly Thr Ser Val Trp Ser
        515          520            525

cac tgg gcg gcc gac aag aac tac ggc ggc gcc acg aac aag ggc atc   1632
His Trp Ala Ala Asp Lys Asn Tyr Gly Gly Ala Thr Asn Lys Gly Ile
        530          535            540

aac tcg gac atc gtc cgg ttc atc gcc aac gcc gac cgc gac gtg tgg   1680
Asn Ser Asp Ile Val Arg Phe Ile Ala Asn Ala Asp Arg Asp Val Trp
545           550           555          560

aac gtg gac ccg ctg ctc ggc ggc gcg agc gtc gtc gag ttc gag ggc   1728
Asn Val Asp Pro Leu Leu Gly Gly Ala Ser Val Val Glu Phe Glu Gly
          565          570          575

tgg acc ggc cag gac gac tgg aac gcc ttc tac cgc aac atc tgg acc   1776
Trp Thr Gly Gln Asp Asp Trp Asn Ala Phe Tyr Arg Asn Ile Trp Thr
        580          585            590

gac aac ctg ccg acc aag ttc ctc cag cac ttc cag gtg ctg gac tgg   1824
Asp Asn Leu Pro Thr Lys Phe Leu Gln His Phe Gln Val Leu Asp Trp
          595          600          605

gac cgc ggc agg tcc gcg agg ctc acc ggc ggg gtg gac gtg aag tcc   1872
Asp Arg Gly Arg Ser Ala Arg Leu Thr Gly Gly Val Asp Val Lys Ser
        610          615            620

gtc gac ggc gag cgg cgg atc tcc atg gac ggc acc gag gtc ctc aag   1920
Val Asp Gly Glu Arg Arg Ile Ser Met Asp Gly Thr Glu Val Leu Lys
625           630           635          640

ggc gac acc tac ctg ctg ccc tgg cag aac gcc ggg aag gac gac ggc   1968
Gly Asp Thr Tyr Leu Leu Pro Trp Gln Asn Ala Gly Lys Asp Asp Gly
          645          650          655

acc tcg tcg ccc cgc gac gcc gac aag atg tac ttc tac agc gcc tcc   2016
Thr Ser Ser Pro Arg Asp Ala Asp Lys Met Tyr Phe Tyr Ser Ala Ser
          660          665          670

ggc ggc gag cac acc ttc gaa ctg acc gga cag ttc gcg ggc acc gag   2064
Gly Gly Glu His Thr Phe Glu Leu Thr Gly Gln Phe Ala Gly Thr Glu
        675          680          685

gac ttc acc ctc tac gaa ctc acc gac cag ggc cgg gcg gag aag gcc   2112
Asp Phe Thr Leu Tyr Glu Leu Thr Asp Gln Gly Arg Ala Glu Lys Ala
        690          695          700

cgg gtg acg gcc cac gag ggg cgg gtg acc ctc acc gcc gag aag ggg   2160
Arg Val Thr Ala His Glu Gly Arg Val Thr Leu Thr Ala Glu Lys Gly
705            710            715          720

cag ccc tac gtc ctc gtg ccg aac ggc ggc agg gca ccg cac cgc gac   2208
Gln Pro Tyr Val Leu Val Pro Asn Gly Gly Arg Ala Pro His Arg Asp
          725          730          735

gcc cac tac ggc gag ttc acc ggg ctg tcc gac ccc ggc ttc aac ggc   2256
Ala His Tyr Gly Glu Phe Thr Gly Leu Ser Asp Pro Gly Phe Asn Gly
        740          745          750

ggg gac ctc gac gcc tgg aac gcg agc ggc ggc gcg gag atc gtc cgg   2304
Gly Asp Leu Asp Ala Trp Asn Ala Ser Gly Gly Ala Glu Ile Val Arg
        755          760          765

```
gcc ggc aac ggg gac aac gtg gtc cgc ctg ggc gag gac gcc tcg ggc    2352
Ala Gly Asn Gly Asp Asn Val Val Arg Leu Gly Glu Asp Ala Ser Gly
770             775             780

atc gcg cag cgg gtc cgc ggc ctg acc ccg ggc gag cgg tac acg ctc    2400
Ile Ala Gln Arg Val Arg Gly Leu Thr Pro Gly Glu Arg Tyr Thr Leu
785             790             795             800

ggc gcg gac gtc ggg atc ggc ccc ggc gag cgc cgg gag acg acg ctg    2448
Gly Ala Asp Val Gly Ile Gly Pro Gly Glu Arg Arg Glu Thr Thr Leu
805             810             815

cgg gtg cgc ggc ggc aag gac agc gag gcc agg acc ttc gac atc acg    2496
Arg Val Arg Gly Gly Lys Asp Ser Glu Ala Arg Thr Phe Asp Ile Thr
820             825             830

ccg gcg cgg aac agg atg gcg tcc gac gag aag cga gac acg tac tcc    2544
Pro Ala Arg Asn Arg Met Ala Ser Asp Glu Lys Arg Asp Thr Tyr Ser
835             840             845

cag cgg gcc tcg gtc tcc ttc acc gcg ccg cgc gac ggc tcg gtc acc    2592
Gln Arg Ala Ser Val Ser Phe Thr Ala Pro Arg Asp Gly Ser Val Thr
850             855             860

gtg gag ctc ggg gcg gtc gcc ggt ggc gcc ccg gtg gtc ctg gac gac    2640
Val Glu Leu Gly Ala Val Ala Gly Gly Ala Pro Val Val Leu Asp Asp
865             870             875             880

gta cgg gtc atg gtg gac acc acc gct ccg ctc ccc cgc tcc cag gac    2688
Val Arg Val Met Val Asp Thr Thr Ala Pro Leu Pro Arg Ser Gln Asp
885             890             895

ggc acg gtc gtc gcc cac gac gac ttc gag ggc aac cgg ccc ggc tgg    2736
Gly Thr Val Val Ala His Asp Asp Phe Glu Gly Asn Arg Pro Gly Trp
900             905             910

gga ccg ttc gtc aag ggc gac gcc ggc ggc gtc acc gac ccc cgc acc    2784
Gly Pro Phe Val Lys Gly Asp Ala Gly Gly Val Thr Asp Pro Arg Thr
915             920             925

agc atc agc gac ctg cac gcg ccc tac agc cag aag gag tgg aag aac    2832
Ser Ile Ser Asp Leu His Ala Pro Tyr Ser Gln Lys Glu Trp Lys Asn
930             935             940

acc tac tcg ccc tac gac acc ggc gcg ctg aag ggc agg gcc gtc gac    2880
Thr Tyr Ser Pro Tyr Asp Thr Gly Ala Leu Lys Gly Arg Ala Val Asp
945             950             955             960

gac gtg ctc gcg ggc cgg cac tcg ctg aag tcc cac gcc gag aac acc    2928
Asp Val Leu Ala Gly Arg His Ser Leu Lys Ser His Ala Glu Asn Thr
965             970             975

ggg ctg gtc cac cgc acg acc cct gcc acc gtg ccg ttc gag gag ggc    2976
Gly Leu Val His Arg Thr Thr Pro Ala Thr Val Pro Phe Glu Glu Gly
980             985             990

cac cgg tac cgg gtc tcc ttc tcg  tac cag acc aac gtc  gag ggc cag  3024
His Arg Tyr Arg Val Ser Phe Ser  Tyr Gln Thr Asn Val  Glu Gly Gln
995             1000            1005

tgg gcc  tgg gtc acc ggc gcg  gac cgg gtc gcc gac  ggg acg acg     3069
Trp Ala  Trp Val Thr Gly Ala  Asp Arg Val Ala Asp  Gly Thr Thr
1010            1015            1020

acc tcg  cgg gac atc acc cgt  gac gtc ctg gca ccc  gcc ctg gac     3114
Thr Ser  Arg Asp Ile Thr Arg  Asp Val Leu Ala Pro  Ala Leu Asp
1025            1030            1035

acg gcg  gcc tac tcc cgc gag  ttc gtc gcc ggc tgc  ggg gac acc     3159
Thr Ala  Ala Tyr Ser Arg Glu  Phe Val Ala Gly Cys  Gly Asp Thr
1040            1045            1050

tgg gtc  ggg ctg cgc aga ctc  ggc agc gcc cgg ggc  acc gat ctc     3204
Trp Val  Gly Leu Arg Arg Leu  Gly Ser Ala Arg Gly  Thr Asp Leu
1055            1060            1065
```

gtc ctc gac gac ttc acg gtg acc gac ctc ggc gag gcg gac acc   3249
Val Leu Asp Asp Phe Thr Val Thr Asp Leu Gly Glu Ala Asp Thr
    1070           1075             1080

ggg gcc gcc tgc gcc gct gtc acg gcc ccg tcc ggc gcc gaa ctg   3294
Gly Ala Ala Cys Ala Ala Val Thr Ala Pro Ser Gly Ala Glu Leu
    1085           1090             1095

agc ccc ggg gtc ccc ggt gag tac gtc acg gcg ttc acc aac cac   3339
Ser Pro Gly Val Pro Gly Glu Tyr Val Thr Ala Phe Thr Asn His
    1100           1105             1110

gag tcc gcc ggc gcc gag aac gtg ggc atc gcc ctc cag ggt ctg   3384
Glu Ser Ala Gly Ala Glu Asn Val Gly Ile Ala Leu Gln Gly Leu
    1115           1120             1125

ccc gag ggc tgg aag gcc gag gtg aag gag aag gac ggc aat ctc   3429
Pro Glu Gly Trp Lys Ala Glu Val Lys Glu Lys Asp Gly Asn Leu
    1130           1135             1140

ttc gag cgc gtg cag ccg ggc gcg acc gtg cgc acc acc tgg ctc   3474
Phe Glu Arg Val Gln Pro Gly Ala Thr Val Arg Thr Thr Trp Leu
    1145           1150             1155

ctc acc ccg ccc gcc ggc acg gcc ggc acc tcc gcc acg tgg cag   3519
Leu Thr Pro Pro Ala Gly Thr Ala Gly Thr Ser Ala Thr Trp Gln
    1160           1165             1170

gtg acg gcc gcc tac gca cac gag gga gcc acc agg acg gtc tcc   3564
Val Thr Ala Ala Tyr Ala His Glu Gly Ala Thr Arg Thr Val Ser
    1175           1180             1185

acc ggg gcc cgc gcc gcg gtg acc gac gaa ccg gta ctg gcc ccg   3609
Thr Gly Ala Arg Ala Ala Val Thr Asp Glu Pro Val Leu Ala Pro
    1190           1195             1200

gct tcg acg acc gcg acc gcc gac tcg gag aac acc tcg tcg ggc   3654
Ala Ser Thr Thr Ala Thr Ala Asp Ser Glu Asn Thr Ser Ser Gly
    1205           1210             1215

gcc tcc gaa ggg ccg gtg tcc aac gtc ctc gac ggt gac gcc ggc   3699
Ala Ser Glu Gly Pro Val Ser Asn Val Leu Asp Gly Asp Ala Gly
    1220           1225             1230

acc atc tgg cac acc gac tac acc acc tcc cag gcg ccg tat ccg   3744
Thr Ile Trp His Thr Asp Tyr Thr Thr Ser Gln Ala Pro Tyr Pro
    1235           1240             1245

cac tgg gtg acg ctg aag ctc ggc ggc gcc gcc gac gtc gac ggg   3789
His Trp Val Thr Leu Lys Leu Gly Gly Ala Ala Asp Val Asp Gly
    1250           1255             1260

ttc ggg tac ctg ggc cga cag agc ggg ggc ccg aac gga cgc gtc   3834
Phe Gly Tyr Leu Gly Arg Gln Ser Gly Gly Pro Asn Gly Arg Val
    1265           1270             1275

gcc gac tac gag gtc gcc gtg tcg gac gac ggc gag gcg tgg acg   3879
Ala Asp Tyr Glu Val Ala Val Ser Asp Asp Gly Glu Ala Trp Thr
    1280           1285             1290

acg gtg gcc acc ggc acc ctg aag gac gtc ccg cgg acc cag cgc   3924
Thr Val Ala Thr Gly Thr Leu Lys Asp Val Pro Arg Thr Gln Arg
    1295           1300             1305

gtc tcc ttc gac cgg gtc cgc gcc tcc tac gtg cgc ttc acc gcc   3969
Val Ser Phe Asp Arg Val Arg Ala Ser Tyr Val Arg Phe Thr Ala
    1310           1315             1320

ctc gac gcg ctc aac ggg cag ccc tac gcg gcc gcc gcg gag atg   4014
Leu Asp Ala Leu Asn Gly Gln Pro Tyr Ala Ala Ala Ala Glu Met
    1325           1330             1335

cgc gtg tac ggc gtg ccc gtg gac ctg ccc acg ggc tac ccg ccg   4059
Arg Val Tyr Gly Val Pro Val Asp Leu Pro Thr Gly Tyr Pro Pro
    1340           1345             1350

```
ggc gaa  cgc ccc gcc gac gcc  cgc tga                                    4086
Gly Glu  Arg Pro Ala Asp Ala  Arg
    1355              1360
```

<210> 10
<211> 1361
<212> PRT
<213> Streptomyces coelicolor A3(2)

<400> 10

Met Ala Ala Ile Thr Pro Lys Arg Ser Val Arg Val Asp Thr Ser Thr
1           5               10              15

Gly Ser Ser Ser Pro Pro Thr Gly Arg Ala Arg Val Arg Arg His Gly
              20              25              30

Ala Val Val Ala Ala Leu Gly Leu Thr Ala Gly Leu Leu Ser Ala Ala
        35              40              45

Ala Leu Pro Ala Gly Ala Ala Pro Pro Arg Pro Ala Ala Ala Ala
    50              55              60

Pro Ala Gly Ala Pro Thr Pro Val Glu Leu Ser Arg Gly Gly Leu Thr
65              70              75              80

Val Thr Val Ala Lys Glu Phe Pro Gln Val Ile Ser Tyr Arg Leu Gly
            85              90              95

Arg Arg Gly Leu Asp Gly Arg Ala Thr Ala Leu Asp Gly Phe Thr Val
            100             105             110

Asn Gly Glu Ser His Arg Ala Thr Thr Thr Val Lys Ala Lys Gly Ser
        115             120             125

Arg Ala Val Tyr Thr Ser Thr Phe Glu Asp Leu Pro Gly Leu Thr Ile
    130             135             140

Thr Ser Ser Ile Thr Val Thr Lys Glu Thr Thr Val Val Phe Ala Val
145             150             155             160

Glu Lys Ile Ser Gly Glu Ala Ala Pro Gly Val Arg Thr Leu Ala Ile
            165             170             175

Pro Gly Gln Ser Leu Val Ser Val Asp Ser Ala Glu Pro Gly Ala Asn
            180             185             190

Leu Ala Arg Thr Lys Ile Ser Thr Asp Ser Thr Thr Thr Ala Asp Arg
    195             200             205

Phe Val Pro Val Thr Gly Asp Thr Ala Pro Asp Lys Gly Pro Val Gly
    210             215             220

Thr Pro Tyr Ala Phe Val Gly Asn Ala Gln Leu Ser Ala Gly Ile Ile
225             230             235             240

Thr Asn Ala Thr Glu Asp Ser Pro Gln Asp Asp Asn Thr Asp Trp Asn
            245             250             255

Thr Arg Leu Gln Ser Arg Ile Val Asp Glu Gly Glu Gly Arg Arg Arg
        260               265             270

Ala Glu Leu Ser Ala Gly Thr Tyr Thr Tyr His Pro Glu Gly Ala Thr
        275             280            285

Asp Pro Arg Val Asp Thr Tyr Glu Leu Pro Arg Ala Thr Val Val Leu
        290             295            300

Ala Ala Asp Ala Asn Arg Asp Gly Thr Val Asp Trp Gln Asp Gly Ala
305           310           315          320

Ile Ala His Arg Glu His Met Arg Arg Pro Leu Gly Ala Asp Arg Val
          325           330          335

Pro Glu Arg Val Val Gln Arg Ile Pro Phe Asn Phe Ala Ser Gln Ala
        340             345           350

Thr Asn Pro Phe Leu Lys Thr Leu Asp Asn Thr Lys Arg Ile Ser Met
        355             360          365

Ala Thr Asp Asp Leu Gly Gln Trp Val Leu Glu Lys Gly Tyr Ala Ser
        370             375          380

Glu Gly His Asp Ser Ala His Pro Asp Tyr Gly Gly Asn Glu Asn Val
385           390           395          400

Arg Ala Gly Gly Trp Lys Asp Leu Asn Arg Leu Thr Arg Thr Gly Ala
          405           410          415

Gly Tyr Asn Ala Asp Phe Ala Val His Val Asn Ala Thr Glu Ala Tyr
        420             425          430

Ala Gln Ala Arg Thr Phe Thr Glu Asp Met Val Ala Gly Gln Ala Asp
        435             440          445

Gly Trp Asp Trp Leu Asn Gln Ala Tyr His Ile Asp Gln Arg Lys Asp
        450             455          460

Leu Gly Thr Gly Ala Val Leu Asp Arg Phe Lys Gln Leu Arg Lys Glu
465           470           475          480

Ala Pro Gly Ile Arg Thr Val Tyr Ile Asp Ala Tyr Tyr Ser Ser Gly
          485           490          495

Trp Leu Ala Asp Gly Leu Ala Ala Gly Leu Arg Glu Met Gly Phe Glu
        500             505          510

Val Ala Thr Glu Trp Ala Tyr Lys Phe Glu Gly Thr Ser Val Trp Ser
        515             520          525

His Trp Ala Ala Asp Lys Asn Tyr Gly Gly Ala Thr Asn Lys Gly Ile
        530             535          540

Asn Ser Asp Ile Val Arg Phe Ile Ala Asn Ala Asp Arg Asp Val Trp
545           550           555          560

Asn Val Asp Pro Leu Leu Gly Gly Ala Ser Val Val Glu Phe Glu Gly
565 570 575

Trp Thr Gly Gln Asp Asp Trp Asn Ala Phe Tyr Arg Asn Ile Trp Thr
580 585 590

Asp Asn Leu Pro Thr Lys Phe Leu Gln His Phe Gln Val Leu Asp Trp
595 600 605

Asp Arg Gly Arg Ser Ala Arg Leu Thr Gly Gly Val Asp Val Lys Ser
610 615 620

Val Asp Gly Glu Arg Arg Ile Ser Met Asp Gly Thr Glu Val Leu Lys
625 630 635 640

Gly Asp Thr Tyr Leu Leu Pro Trp Gln Asn Ala Gly Lys Asp Asp Gly
645 650 655

Thr Ser Ser Pro Arg Asp Ala Asp Lys Met Tyr Phe Tyr Ser Ala Ser
660 665 670

Gly Gly Glu His Thr Phe Glu Leu Thr Gly Gln Phe Ala Gly Thr Glu
675 680 685

Asp Phe Thr Leu Tyr Glu Leu Thr Asp Gln Gly Arg Ala Glu Lys Ala
690 695 700

Arg Val Thr Ala His Glu Gly Arg Val Thr Leu Thr Ala Glu Lys Gly
705 710 715 720

Gln Pro Tyr Val Leu Val Pro Asn Gly Gly Arg Ala Pro His Arg Asp
725 730 735

Ala His Tyr Gly Glu Phe Thr Gly Leu Ser Asp Pro Gly Phe Asn Gly
740 745 750

Gly Asp Leu Asp Ala Trp Asn Ala Ser Gly Gly Ala Glu Ile Val Arg
755 760 765

Ala Gly Asn Gly Asp Asn Val Val Arg Leu Gly Glu Asp Ala Ser Gly
770 775 780

Ile Ala Gln Arg Val Arg Gly Leu Thr Pro Gly Glu Arg Tyr Thr Leu
785 790 795 800

Gly Ala Asp Val Gly Ile Gly Pro Gly Glu Arg Arg Glu Thr Thr Leu
805 810 815

Arg Val Arg Gly Gly Lys Asp Ser Glu Ala Arg Thr Phe Asp Ile Thr
820 825 830

Pro Ala Arg Asn Arg Met Ala Ser Asp Glu Lys Arg Asp Thr Tyr Ser
835 840 845

Gln Arg Ala Ser Val Ser Phe Thr Ala Pro Arg Asp Gly Ser Val Thr
850 855 860

Val Glu Leu Gly Ala Val Ala Gly Gly Ala Pro Val Val Leu Asp Asp
865           870           875           880

Val Arg Val Met Val Asp Thr Thr Ala Pro Leu Pro Arg Ser Gln Asp
            885           890           895

Gly Thr Val Val Ala His Asp Asp Phe Glu Gly Asn Arg Pro Gly Trp
            900           905           910

Gly Pro Phe Val Lys Gly Asp Ala Gly Gly Val Thr Asp Pro Arg Thr
            915           920           925

Ser Ile Ser Asp Leu His Ala Pro Tyr Ser Gln Lys Glu Trp Lys Asn
            930           935           940

Thr Tyr Ser Pro Tyr Asp Thr Gly Ala Leu Lys Gly Arg Ala Val Asp
945           950           955           960

Asp Val Leu Ala Gly Arg His Ser Leu Lys Ser His Ala Glu Asn Thr
            965           970           975

Gly Leu Val His Arg Thr Thr Pro Ala Thr Val Pro Phe Glu Glu Gly
            980           985           990

His Arg Tyr Arg Val Ser Phe Ser Tyr Gln Thr Asn Val Glu Gly Gln
            995           1000           1005

Trp Ala Trp Val Thr Gly Ala Asp Arg Val Ala Asp Gly Thr Thr
            1010           1015           1020

Thr Ser Arg Asp Ile Thr Arg Asp Val Leu Ala Pro Ala Leu Asp
            1025           1030           1035

Thr Ala Ala Tyr Ser Arg Glu Phe Val Ala Gly Cys Gly Asp Thr
            1040           1045           1050

Trp Val Gly Leu Arg Arg Leu Gly Ser Ala Arg Gly Thr Asp Leu
            1055           1060           1065

Val Leu Asp Asp Phe Thr Val Thr Asp Leu Gly Glu Ala Asp Thr
            1070           1075           1080

Gly Ala Ala Cys Ala Ala Val Thr Ala Pro Ser Gly Ala Glu Leu
            1085           1090           1095

Ser Pro Gly Val Pro Gly Glu Tyr Val Thr Ala Phe Thr Asn His
            1100           1105           1110

Glu Ser Ala Gly Ala Glu Asn Val Gly Ile Ala Leu Gln Gly Leu
            1115           1120           1125

Pro Glu Gly Trp Lys Ala Glu Val Lys Glu Lys Asp Gly Asn Leu
            1130           1135           1140

Phe Glu Arg Val Gln Pro Gly Ala Thr Val Arg Thr Thr Trp Leu
            1145           1150           1155

```
Leu Thr Pro Pro Ala Gly Thr Ala Gly Thr Ser Ala  Thr Trp Gln
    1160            1165            1170

Val Thr Ala Ala Tyr Ala His Glu Gly Ala Thr Arg  Thr Val Ser
    1175            1180            1185

Thr Gly Ala Arg Ala Ala Val Thr Asp Glu Pro Val  Leu Ala Pro
    1190            1195            1200

Ala Ser Thr Thr Ala Thr Ala Asp Ser Glu Asn Thr  Ser Ser Gly
    1205            1210            1215

Ala Ser Glu Gly Pro Val Ser Asn Val Leu Asp Gly  Asp Ala Gly
    1220            1225            1230

Thr Ile Trp His Thr Asp Tyr Thr Thr Ser Gln Ala  Pro Tyr Pro
    1235            1240            1245

His Trp Val Thr Leu Lys Leu Gly Gly Ala Ala Asp  Val Asp Gly
    1250            1255            1260

Phe Gly Tyr Leu Gly Arg Gln Ser Gly Gly Pro Asn  Gly Arg Val
    1265            1270            1275

Ala Asp Tyr Glu Val Ala Val Ser Asp Asp Gly Glu  Ala Trp Thr
    1280            1285            1290

Thr Val Ala Thr Gly Thr Leu Lys Asp Val Pro Arg  Thr Gln Arg
    1295            1300            1305

Val Ser Phe Asp Arg Val Arg Ala Ser Tyr Val Arg  Phe Thr Ala
    1310            1315            1320

Leu Asp Ala Leu Asn Gly Gln Pro Tyr Ala Ala Ala  Ala Glu Met
    1325            1330            1335

Arg Val Tyr Gly Val Pro Val Asp Leu Pro Thr Gly  Tyr Pro Pro
    1340            1345            1350

Gly Glu Arg Pro Ala Asp Ala Arg
    1355            1360
```

<210> 11
<211> 3975
<212> DNA
<213> Enterococcus faecalis V583

<220>
<221> CDS
<222> (1).. (3975)

<400> 11

```
atg aaa cat gga aaa ata aaa cga ttt agt aca ttg aca cta ttg gca        48
Met Lys His Gly Lys Ile Lys Arg Phe Ser Thr Leu Thr Leu Leu Ala
1               5               10              15

agc gca acg att tta gta cca tta agt acg tct gca gaa gaa aca aca        96
Ser Ala Thr Ile Leu Val Pro Leu Ser Thr Ser Ala Glu Glu Thr Thr
                20              25              30

aac agc agc act gaa aca agc tca tca atg gtg gaa cct acc gca aca        144
Asn Ser Ser Thr Glu Thr Ser Ser Ser Met Val Glu Pro Thr Ala Thr
```

```
                    35                  40                  45

gaa gaa aaa ttg tgg caa tct gat ttt cca gga ggg aaa act ggt gag    192
Glu Glu Lys Leu Trp Gln Ser Asp Phe Pro Gly Gly Lys Thr Gly Glu
        50                  55                  60

tgg caa gat gtg att ggt aaa aca aat cga gaa tta gca gga gag tca    240
Trp Gln Asp Val Ile Gly Lys Thr Asn Arg Glu Leu Ala Gly Glu Ser
65                  70                  75                  80

ttg gcg att tca cga gat gca gca gca ggt aat aat gcc gta tct tta    288
Leu Ala Ile Ser Arg Asp Ala Ala Ala Gly Asn Asn Ala Val Ser Leu
                    85                  90                  95

aat tta gat tcc cct aaa tta gct gat ggt gaa gta gaa aca aag ttt    336
Asn Leu Asp Ser Pro Lys Leu Ala Asp Gly Glu Val Glu Thr Lys Phe
                100                 105                 110

aaa tac act gct gga agc ggt cga acg ggt gtg att ata cgt ggt aat    384
Lys Tyr Thr Ala Gly Ser Gly Arg Thr Gly Val Ile Ile Arg Gly Asn
            115                 120                 125

acc aaa gat agc tgg gtt ttt gta ggc tat aac gcg aat ggc aaa tgg    432
Thr Lys Asp Ser Trp Val Phe Val Gly Tyr Asn Ala Asn Gly Lys Trp
    130                 135                 140

tta gtt gaa agc cct aat tcg tgg aat gat tca att tct ggg cca acg    480
Leu Val Glu Ser Pro Asn Ser Trp Asn Asp Ser Ile Ser Gly Pro Thr
145                 150                 155                 160

tta aat gaa gat acg aat tat ttg ttg aaa gta cgt tat gtt ggt gaa    528
Leu Asn Glu Asp Thr Asn Tyr Leu Leu Lys Val Arg Tyr Val Gly Glu
                165                 170                 175

aaa att act atc tgg ctt aac acc acg ttg att tac gaa gga gaa cct    576
Lys Ile Thr Ile Trp Leu Asn Thr Thr Leu Ile Tyr Glu Gly Glu Pro
                180                 185                 190

gtt ttg gcc aat gga gac aaa att cca aca gaa gcc gga cat gtg ggt    624
Val Leu Ala Asn Gly Asp Lys Ile Pro Thr Glu Ala Gly His Val Gly
            195                 200                 205

gtc cgt ttg tgg tac gac aaa aaa att gtc aat tat gac tat ttt aaa    672
Val Arg Leu Trp Tyr Asp Lys Lys Ile Val Asn Tyr Asp Tyr Phe Lys
    210                 215                 220

aat ggc ccc gta gat agc att cca gaa att gtg cca gaa gtg aca caa    720
Asn Gly Pro Val Asp Ser Ile Pro Glu Ile Val Pro Glu Val Thr Gln
225                 230                 235                 240

att gcg cca gtc aaa gtt ttt aca aaa att ggt gtc gca cca aaa tta    768
Ile Ala Pro Val Lys Val Phe Thr Lys Ile Gly Val Ala Pro Lys Leu
            245                 250                 255

ccg aaa caa gta aaa gtg acc tat aat act ggt aaa gaa gcc aat gaa    816
Pro Lys Gln Val Lys Val Thr Tyr Asn Thr Gly Lys Glu Ala Asn Glu
            260                 265                 270

gca gtc cgt tgg aat gaa atc gat cct gat gca tat aaa gaa cca gga    864
Ala Val Arg Trp Asn Glu Ile Asp Pro Asp Ala Tyr Lys Glu Pro Gly
            275                 280                 285

act ttt gaa gtc gac ggt act ttg gaa aat aca aac atc aaa gca aaa    912
Thr Phe Glu Val Asp Gly Thr Leu Glu Asn Thr Asn Ile Lys Ala Lys
    290                 295                 300

gcc agc att gtt gtt gct aaa gac aat gaa gct gaa aaa ggc gac aaa    960
Ala Ser Ile Val Val Ala Lys Asp Asn Glu Ala Glu Lys Gly Asp Lys
305                 310                 315                 320

atc tct tcc gca gat tta aca gcc gtg gtt gat cca caa ttt cca cga   1008
Ile Ser Ser Ala Asp Leu Thr Ala Val Val Asp Pro Gln Phe Pro Arg
                325                 330                 335

att att cgc tac gaa gac cct cag agt aat caa gtg att ttt aat ggc   1056
Ile Ile Arg Tyr Glu Asp Pro Gln Ser Asn Gln Val Ile Phe Asn Gly
```

```
                    340                 345                 350

caa cac gag aaa att gac caa gta atg att gat ggc aaa gca tat aaa     1104
Gln His Glu Lys Ile Asp Gln Val Met Ile Asp Gly Lys Ala Tyr Lys
        355                 360                 365

gca act gct gaa aaa cag aag agt gaa gca aat caa gcc gtt tat aac     1152
Ala Thr Ala Glu Lys Gln Lys Ser Glu Ala Asn Gln Ala Val Tyr Asn
    370                 375                 380

gta gct gtc cca gaa att ggt ttg cgt ttc aca acg aca ttg act gtt     1200
Val Ala Val Pro Glu Ile Gly Leu Arg Phe Thr Thr Thr Leu Thr Val
385                 390                 395                 400

tcc gaa ggc caa gaa tta gct atg aaa ctc tca gat att cgt gaa gaa     1248
Ser Glu Gly Gln Glu Leu Ala Met Lys Leu Ser Asp Ile Arg Glu Glu
                405                 410                 415

gga acc aaa ata cac aca att tca att cca aat caa ggc ttg att tct     1296
Gly Thr Lys Ile His Thr Ile Ser Ile Pro Asn Gln Gly Leu Ile Ser
                420                 425                 430

gtc aat agt aca gat gaa ggg gcg act ttt gct ggc gtt gtg atg aat     1344
Val Asn Ser Thr Asp Glu Gly Ala Thr Phe Ala Gly Val Val Met Asn
            435                 440                 445

act ggg aca aat gca aat aac gga aat aaa aat ggt gat act atc caa     1392
Thr Gly Thr Asn Ala Asn Asn Gly Asn Lys Asn Gly Asp Thr Ile Gln
    450                 455                 460

gat tta act aca aca agc caa gaa gaa acg aaa aaa tat atg tat ggt     1440
Asp Leu Thr Thr Thr Ser Gln Glu Glu Thr Lys Lys Tyr Met Tyr Gly
465                 470                 475                 480

ttc tta aat acg gcg aat tat gct gca agt ttt tgg acg aac gcc tat     1488
Phe Leu Asn Thr Ala Asn Tyr Ala Ala Ser Phe Trp Thr Asn Ala Tyr
                485                 490                 495

gga gac ggc tct gtc gat ggt agt gac aac aat cga atc cat aaa caa     1536
Gly Asp Gly Ser Val Asp Gly Ser Asp Asn Asn Arg Ile His Lys Gln
                500                 505                 510

aca aaa gaa gcg gcg act ggt ttt gta aca acc ttg tca agt ggg gca     1584
Thr Lys Glu Ala Ala Thr Gly Phe Val Thr Thr Leu Ser Ser Gly Ala
        515                 520                 525

tgg acc tat cga cca ttt gat gca ccg gaa gat tac aca act gga gaa     1632
Trp Thr Tyr Arg Pro Phe Asp Ala Pro Glu Asp Tyr Thr Thr Gly Glu
        530                 535                 540

acg cca gaa gtg aaa gtt aaa ttc tca aaa gat agc aac gac gac aat     1680
Thr Pro Glu Val Lys Val Lys Phe Ser Lys Asp Ser Asn Asp Asp Asn
545                 550                 555                 560

cgg gtg gat tgg caa gat gcg gca att ggg ttc cgt tca att atg aat     1728
Arg Val Asp Trp Gln Asp Ala Ala Ile Gly Phe Arg Ser Ile Met Asn
                565                 570                 575

aac cca atg ggt gcg gaa aaa gtc cct gaa tta gtc aac caa cgg att     1776
Asn Pro Met Gly Ala Glu Lys Val Pro Glu Leu Val Asn Gln Arg Ile
                580                 585                 590

cct ttt aac ttt gct agt cag gcg aca aac cca ttc tta gtg acg tta     1824
Pro Phe Asn Phe Ala Ser Gln Ala Thr Asn Pro Phe Leu Val Thr Leu
                595                 600                 605

gac gaa tca aaa cgt att tac aat tta aca gat gga tta gga caa atg     1872
Asp Glu Ser Lys Arg Ile Tyr Asn Leu Thr Asp Gly Leu Gly Gln Met
        610                 615                 620

aat tta cta aaa ggg tat caa aat gaa gga cat gat tct gcg cat cca     1920
Asn Leu Leu Lys Gly Tyr Gln Asn Glu Gly His Asp Ser Ala His Pro
625                 630                 635                 640

gat tac ggt gct att ggt cag cga cct ggt ggg gaa caa gcg ttg aat     1968
Asp Tyr Gly Ala Ile Gly Gln Arg Pro Gly Gly Glu Gln Ala Leu Asn
```

645 650 655

```
caa tta att gat gaa gga cat aaa tta aat gcc gtt ttc ggt gtg cat    2016
Gln Leu Ile Asp Glu Gly His Lys Leu Asn Ala Val Phe Gly Val His
        660             665             670

att aat gac acc gag tct tac cca gaa gca aaa gga ttt aat gag gaa    2064
Ile Asn Asp Thr Glu Ser Tyr Pro Glu Ala Lys Gly Phe Asn Glu Glu
        675             680             685

tta gtt gat cca acg aag cgt ggc tgg gat tgg tta gat ccg tct tat    2112
Leu Val Asp Pro Thr Lys Arg Gly Trp Asp Trp Leu Asp Pro Ser Tyr
        690             695             700

ttt att aaa caa aga ccc gat aca ttg agt ggt cgt cgc tat gag cgc    2160
Phe Ile Lys Gln Arg Pro Asp Thr Leu Ser Gly Arg Arg Tyr Glu Arg
705             710             715             720

ttt aaa gaa tta aaa caa aaa gca ccg aat cta gat tat att tat gtt    2208
Phe Lys Glu Leu Lys Gln Lys Ala Pro Asn Leu Asp Tyr Ile Tyr Val
                725             730             735

gat gtt tgg ggc aac caa ggc gaa tca ggt tgg gca agt cgt caa cta    2256
Asp Val Trp Gly Asn Gln Gly Glu Ser Gly Trp Ala Ser Arg Gln Leu
            740             745             750

agt aaa gaa att aat tca ctc ggt tgg ttt aca acc aat gaa ttt ccg    2304
Ser Lys Glu Ile Asn Ser Leu Gly Trp Phe Thr Thr Asn Glu Phe Pro
        755             760             765

aat gct tta gag tat gac tcg gtt tgg aac cat tgg tct gca gaa aaa    2352
Asn Ala Leu Glu Tyr Asp Ser Val Trp Asn His Trp Ser Ala Glu Lys
        770             775             780

gat tac ggc ggt aca aca acg aaa ggc ttt aac agt aca atc gtt cgt    2400
Asp Tyr Gly Gly Thr Thr Thr Lys Gly Phe Asn Ser Thr Ile Val Arg
785             790             795             800

ttt att cgg aat cat caa aaa gac act tgg att att tcc gac aac cct    2448
Phe Ile Arg Asn His Gln Lys Asp Thr Trp Ile Ile Ser Asp Asn Pro
                805             810             815

ttg tta ggt gga gct gaa ttt gaa gcc tat gaa ggt tgg gtg ggt aaa    2496
Leu Leu Gly Gly Ala Glu Phe Glu Ala Tyr Glu Gly Trp Val Gly Lys
            820             825             830

acc aat ttt aat acc tat cgc caa aaa act ttt gcc att aat gtc cca    2544
Thr Asn Phe Asn Thr Tyr Arg Gln Lys Thr Phe Ala Ile Asn Val Pro
        835             840             845

act aag ttc tta caa cat tac caa att aca aac tgg gaa act aca aca    2592
Thr Lys Phe Leu Gln His Tyr Gln Ile Thr Asn Trp Glu Thr Thr Thr
850             855             860

gca gca gat ggt caa atc tat ggc aca att aaa tta gcg aac ggt gct    2640
Ala Ala Asp Gly Gln Ile Tyr Gly Thr Ile Lys Leu Ala Asn Gly Ala
865             870             875             880

gaa aaa gtg acc gtt act caa gca gat gct aat tcg cca aga agc att    2688
Glu Lys Val Thr Val Thr Gln Ala Asp Ala Asn Ser Pro Arg Ser Ile
                885             890             895

acg tta aat gag aca gaa gtt cta aaa ggt gat gcg tat cta ctg cct    2736
Thr Leu Asn Glu Thr Glu Val Leu Lys Gly Asp Ala Tyr Leu Leu Pro
            900             905             910

tgg aat gtc aat ggt caa gac aaa cta tat cac tgg aat cca aaa ggc    2784
Trp Asn Val Asn Gly Gln Asp Lys Leu Tyr His Trp Asn Pro Lys Gly
        915             920             925

ggt acc agc act tgg tca ttg gat aag aaa atg caa gga aaa acg aat    2832
Gly Thr Ser Thr Trp Ser Leu Asp Lys Lys Met Gln Gly Lys Thr Asn
930             935             940

tta cat tta tat gaa tta aca gat caa ggg cgt att gac aaa ggc gca    2880
Leu His Leu Tyr Glu Leu Thr Asp Gln Gly Arg Ile Asp Lys Gly Ala
```

```
          945              950              955              960
att gcc act aca aat aac caa gtg acc atc caa gcc gag gct aat aca    2928
Ile Ala Thr Thr Asn Asn Gln Val Thr Ile Gln Ala Glu Ala Asn Thr
                965                  970                  975

ccg tat gtc att gct gaa cct gac agt att gaa ccg atg aca ttt gga    2976
Pro Tyr Val Ile Ala Glu Pro Asp Ser Ile Glu Pro Met Thr Phe Gly
            980                  985                  990

aca gga aca cca ttt aaa gat cct gga ttt aat gaa gcc aat acc tta    3024
Thr Gly Thr Pro Phe Lys Asp Pro Gly Phe Asn Glu Ala Asn Thr Leu
        995                  1000                 1005

aaa aat aac tgg aaa gtt ttc cga ggt gat gga gag gtt aaa aaa    3069
Lys Asn Asn Trp Lys Val Phe Arg Gly Asp Gly Glu Val Lys Lys
    1010                 1015                 1020

gat gcc aat ggt gat tat gtc ttt agt tca gaa aaa gaa aga acc    3114
Asp Ala Asn Gly Asp Tyr Val Phe Ser Ser Glu Lys Glu Arg Thr
    1025                 1030                 1035

gaa atc aaa caa gat atc aat ctt cct aaa cca gga aaa tat agt    3159
Glu Ile Lys Gln Asp Ile Asn Leu Pro Lys Pro Gly Lys Tyr Ser
    1040                 1045                 1050

ttg tat cta aac aca gaa aca cat gat cgt aaa gcc aca gta act    3204
Leu Tyr Leu Asn Thr Glu Thr His Asp Arg Lys Ala Thr Val Thr
    1055                 1060                 1065

gtt aaa att ggt ggt aag aaa tat acg cgg aca gtg aat aat tcg    3249
Val Lys Ile Gly Gly Lys Lys Tyr Thr Arg Thr Val Asn Asn Ser
    1070                 1075                 1080

gtt gcc caa aac tac att cag gca gat att aac cat aca agc agg    3294
Val Ala Gln Asn Tyr Ile Gln Ala Asp Ile Asn His Thr Ser Arg
    1085                 1090                 1095

aaa aat ccg cag tat atg caa aat atg cga att gat ttt gaa atc    3339
Lys Asn Pro Gln Tyr Met Gln Asn Met Arg Ile Asp Phe Glu Ile
    1100                 1105                 1110

cca gat aat gcc aaa aaa ggc tcg gtg aca tta gcg gtt gat aaa    3384
Pro Asp Asn Ala Lys Lys Gly Ser Val Thr Leu Ala Val Asp Lys
    1115                 1120                 1125

ggc aat tcc gtt aca aaa ttt gat gat tta cga att gtt gag cgt    3429
Gly Asn Ser Val Thr Lys Phe Asp Asp Leu Arg Ile Val Glu Arg
    1130                 1135                 1140

caa acg gat atc atg aac cca gac aaa caa aca gtt att aag caa    3474
Gln Thr Asp Ile Met Asn Pro Asp Lys Gln Thr Val Ile Lys Gln
    1145                 1150                 1155

gat ttt gaa gac aca caa gca gtt ggg tta tat ccg ttt gtt aaa    3519
Asp Phe Glu Asp Thr Gln Ala Val Gly Leu Tyr Pro Phe Val Lys
    1160                 1165                 1170

ggc tca gct ggt ggt gta gaa gat cca cgg att cat tta tca gaa    3564
Gly Ser Ala Gly Gly Val Glu Asp Pro Arg Ile His Leu Ser Glu
    1175                 1180                 1185

aga aat gaa cct tac aca caa tat ggt tgg aat gga aac ctt gtt    3609
Arg Asn Glu Pro Tyr Thr Gln Tyr Gly Trp Asn Gly Asn Leu Val
    1190                 1195                 1200

tca gat gta tta gaa ggc aac tgg tcc ttg aaa gcc cat aaa caa    3654
Ser Asp Val Leu Glu Gly Asn Trp Ser Leu Lys Ala His Lys Gln
    1205                 1210                 1215

gga gca gga ttg atg ctt caa aca att ccg caa aat att aaa ttt    3699
Gly Ala Gly Leu Met Leu Gln Thr Ile Pro Gln Asn Ile Lys Phe
    1220                 1225                 1230

gaa ccg aac aag aaa tat acg gtc caa ttt gat tat caa act gat    3744
Glu Pro Asn Lys Lys Tyr Thr Val Gln Phe Asp Tyr Gln Thr Asp
```

170

```
                1235                    1240                    1245

     ggt gaa  aat gtc ttt act gct  ggg acc att aat ggg  gag ttg aaa        3789
     Gly Glu  Asn Val Phe Thr Ala  Gly Thr Ile Asn Gly  Glu Leu Lys
          1250                    1255                    1260

     aat aac  aat gac ttt aag cca  gtc ggt gag tta act  tcg aca gca        3834
     Asn Asn  Asn Asp Phe Lys Pro  Val Gly Glu Leu Thr  Ser Thr Ala
          1265                    1270                    1275

     gca gat  ggt caa acc aag cat  tat gaa gca gaa ata  att ggg gat        3879
     Ala Asp  Gly Gln Thr Lys His  Tyr Glu Ala Glu Ile  Ile Gly Asp
          1280                    1285                    1290

     gct tca  gga aac act acg ttt  ggt att ttt aca aca  ggt gcc gat        3924
     Ala Ser  Gly Asn Thr Thr Phe  Gly Ile Phe Thr Thr  Gly Ala Asp
          1295                    1300                    1305

     aaa gat  ttc att atg gat aac  ttt acg gtc aca gtg  gaa tca aaa        3969
     Lys Asp  Phe Ile Met Asp Asn  Phe Thr Val Thr Val  Glu Ser Lys
          1310                    1315                    1320

     aaa taa                                                               3975
     Lys
```

<210> 12
<211> 1324
<212> PRT
<213> Enterococcus faecalis V583

<400> 12

```
Met Lys His Gly Lys Ile Lys Arg Phe Ser Thr Leu Thr Leu Leu Ala
1               5                   10                  15

Ser Ala Thr Ile Leu Val Pro Leu Ser Thr Ser Ala Glu Glu Thr Thr
            20              25              30

Asn Ser Ser Thr Glu Thr Ser Ser Ser Met Val Glu Pro Thr Ala Thr
        35              40              45

Glu Glu Lys Leu Trp Gln Ser Asp Phe Pro Gly Gly Lys Thr Gly Glu
    50              55              60

Trp Gln Asp Val Ile Gly Lys Thr Asn Arg Glu Leu Ala Gly Glu Ser
65              70              75              80

Leu Ala Ile Ser Arg Asp Ala Ala Ala Gly Asn Asn Ala Val Ser Leu
            85              90              95

Asn Leu Asp Ser Pro Lys Leu Ala Asp Gly Glu Val Glu Thr Lys Phe
            100             105             110

Lys Tyr Thr Ala Gly Ser Gly Arg Thr Gly Val Ile Ile Arg Gly Asn
        115             120             125

Thr Lys Asp Ser Trp Val Phe Val Gly Tyr Asn Ala Asn Gly Lys Trp
    130             135             140

Leu Val Glu Ser Pro Asn Ser Trp Asn Asp Ser Ile Ser Gly Pro Thr
145             150             155             160

Leu Asn Glu Asp Thr Asn Tyr Leu Leu Lys Val Arg Tyr Val Gly Glu
            165             170             175
```

```
Lys Ile Thr Ile Trp Leu Asn Thr Thr Leu Ile Tyr Glu Gly Glu Pro
        180                   185               190

Val Leu Ala Asn Gly Asp Lys Ile Pro Thr Glu Ala Gly His Val Gly
        195                   200               205

Val Arg Leu Trp Tyr Asp Lys Lys Ile Val Asn Tyr Asp Tyr Phe Lys
        210                   215               220

Asn Gly Pro Val Asp Ser Ile Pro Glu Ile Val Pro Glu Val Thr Gln
225                   230               235               240

Ile Ala Pro Val Lys Val Phe Thr Lys Ile Gly Val Ala Pro Lys Leu
                245               250               255

Pro Lys Gln Val Lys Val Thr Tyr Asn Thr Gly Lys Glu Ala Asn Glu
                260               265               270

Ala Val Arg Trp Asn Glu Ile Asp Pro Asp Ala Tyr Lys Glu Pro Gly
                275               280               285

Thr Phe Glu Val Asp Gly Thr Leu Glu Asn Thr Asn Ile Lys Ala Lys
        290               295               300

Ala Ser Ile Val Val Ala Lys Asp Asn Glu Ala Glu Lys Gly Asp Lys
305               310               315               320

Ile Ser Ser Ala Asp Leu Thr Ala Val Val Asp Pro Gln Phe Pro Arg
                325               330               335

Ile Ile Arg Tyr Glu Asp Pro Gln Ser Asn Gln Val Ile Phe Asn Gly
                340               345               350

Gln His Glu Lys Ile Asp Gln Val Met Ile Asp Gly Lys Ala Tyr Lys
        355               360               365

Ala Thr Ala Glu Lys Gln Lys Ser Glu Ala Asn Gln Ala Val Tyr Asn
        370               375               380

Val Ala Val Pro Glu Ile Gly Leu Arg Phe Thr Thr Thr Leu Thr Val
385               390               395               400

Ser Glu Gly Gln Glu Leu Ala Met Lys Leu Ser Asp Ile Arg Glu Glu
                405               410               415

Gly Thr Lys Ile His Thr Ile Ser Ile Pro Asn Gln Gly Leu Ile Ser
                420               425               430

Val Asn Ser Thr Asp Glu Gly Ala Thr Phe Ala Gly Val Val Met Asn
        435               440               445

Thr Gly Thr Asn Ala Asn Asn Gly Asn Lys Asn Gly Asp Thr Ile Gln
        450               455               460

Asp Leu Thr Thr Thr Ser Gln Glu Glu Thr Lys Lys Tyr Met Tyr Gly
465               470               475               480
```

Phe Leu Asn Thr Ala Asn Tyr Ala Ala Ser Phe Trp Thr Asn Ala Tyr
           485              490          495

Gly Asp Gly Ser Val Asp Gly Ser Asp Asn Asn Arg Ile His Lys Gln
          500          505          510

Thr Lys Glu Ala Ala Thr Gly Phe Val Thr Thr Leu Ser Ser Gly Ala
        515          520          525

Trp Thr Tyr Arg Pro Phe Asp Ala Pro Glu Asp Tyr Thr Thr Gly Glu
   530          535          540

Thr Pro Glu Val Lys Val Lys Phe Ser Lys Asp Ser Asn Asp Asp Asn
545          550          555          560

Arg Val Asp Trp Gln Asp Ala Ala Ile Gly Phe Arg Ser Ile Met Asn
        565          570          575

Asn Pro Met Gly Ala Glu Lys Val Pro Glu Leu Val Asn Gln Arg Ile
        580          585          590

Pro Phe Asn Phe Ala Ser Gln Ala Thr Asn Pro Phe Leu Val Thr Leu
        595          600          605

Asp Glu Ser Lys Arg Ile Tyr Asn Leu Thr Asp Gly Leu Gly Gln Met
      610          615          620

Asn Leu Leu Lys Gly Tyr Gln Asn Glu Gly His Asp Ser Ala His Pro
625          630          635          640

Asp Tyr Gly Ala Ile Gly Gln Arg Pro Gly Gly Glu Gln Ala Leu Asn
        645          650          655

Gln Leu Ile Asp Glu Gly His Lys Leu Asn Ala Val Phe Gly Val His
        660          665          670

Ile Asn Asp Thr Glu Ser Tyr Pro Glu Ala Lys Gly Phe Asn Glu Glu
        675          680          685

Leu Val Asp Pro Thr Lys Arg Gly Trp Asp Trp Leu Asp Pro Ser Tyr
      690          695          700

Phe Ile Lys Gln Arg Pro Asp Thr Leu Ser Gly Arg Arg Tyr Glu Arg
705          710          715          720

Phe Lys Glu Leu Lys Gln Lys Ala Pro Asn Leu Asp Tyr Ile Tyr Val
        725          730          735

Asp Val Trp Gly Asn Gln Gly Glu Ser Gly Trp Ala Ser Arg Gln Leu
        740          745          750

Ser Lys Glu Ile Asn Ser Leu Gly Trp Phe Thr Thr Asn Glu Phe Pro
        755          760          765

Asn Ala Leu Glu Tyr Asp Ser Val Trp Asn His Trp Ser Ala Glu Lys
        770          775          780

Asp Tyr Gly Gly Thr Thr Thr Lys Gly Phe Asn Ser Thr Ile Val Arg
785                790                795                800

Phe Ile Arg Asn His Gln Lys Asp Thr Trp Ile Ile Ser Asp Asn Pro
                805                810                815

Leu Leu Gly Gly Ala Glu Phe Glu Ala Tyr Glu Gly Trp Val Gly Lys
            820                825                830

Thr Asn Phe Asn Thr Tyr Arg Gln Lys Thr Phe Ala Ile Asn Val Pro
            835                840                845

Thr Lys Phe Leu Gln His Tyr Gln Ile Thr Asn Trp Glu Thr Thr Thr
    850                855                860

Ala Ala Asp Gly Gln Ile Tyr Gly Thr Ile Lys Leu Ala Asn Gly Ala
865                870                875                880

Glu Lys Val Thr Val Thr Gln Ala Asp Ala Asn Ser Pro Arg Ser Ile
            885                890                895

Thr Leu Asn Glu Thr Glu Val Leu Lys Gly Asp Ala Tyr Leu Leu Pro
            900                905                910

Trp Asn Val Asn Gly Gln Asp Lys Leu Tyr His Trp Asn Pro Lys Gly
            915                920                925

Gly Thr Ser Thr Trp Ser Leu Asp Lys Lys Met Gln Gly Lys Thr Asn
    930                935                940

Leu His Leu Tyr Glu Leu Thr Asp Gln Gly Arg Ile Asp Lys Gly Ala
945                950                955                960

Ile Ala Thr Thr Asn Asn Gln Val Thr Ile Gln Ala Glu Ala Asn Thr
            965                970                975

Pro Tyr Val Ile Ala Glu Pro Asp Ser Ile Glu Pro Met Thr Phe Gly
            980                985                990

Thr Gly Thr Pro Phe Lys Asp Pro Gly Phe Asn Glu Ala Asn Thr Leu
    995                1000                1005

Lys Asn Asn Trp Lys Val Phe Arg Gly Asp Gly Glu Val Lys Lys
    1010                1015                1020

Asp Ala Asn Gly Asp Tyr Val Phe Ser Ser Glu Lys Glu Arg Thr
    1025                1030                1035

Glu Ile Lys Gln Asp Ile Asn Leu Pro Lys Pro Gly Lys Tyr Ser
    1040                1045                1050

Leu Tyr Leu Asn Thr Glu Thr His Asp Arg Lys Ala Thr Val Thr
    1055                1060                1065

Val Lys Ile Gly Gly Lys Lys Tyr Thr Arg Thr Val Asn Asn Ser
    1070                1075                1080

**175**

Val Ala Gln Asn Tyr Ile Gln Ala Asp Ile Asn His Thr Ser Arg
1085 1090 1095

Lys Asn Pro Gln Tyr Met Gln Asn Met Arg Ile Asp Phe Glu Ile
1100 1105 1110

Pro Asp Asn Ala Lys Lys Gly Ser Val Thr Leu Ala Val Asp Lys
1115 1120 1125

Gly Asn Ser Val Thr Lys Phe Asp Asp Leu Arg Ile Val Glu Arg
1130 1135 1140

Gln Thr Asp Ile Met Asn Pro Asp Lys Gln Thr Val Ile Lys Gln
1145 1150 1155

Asp Phe Glu Asp Thr Gln Ala Val Gly Leu Tyr Pro Phe Val Lys
1160 1165 1170

Gly Ser Ala Gly Gly Val Glu Asp Pro Arg Ile His Leu Ser Glu
1175 1180 1185

Arg Asn Glu Pro Tyr Thr Gln Tyr Gly Trp Asn Gly Asn Leu Val
1190 1195 1200

Ser Asp Val Leu Glu Gly Asn Trp Ser Leu Lys Ala His Lys Gln
1205 1210 1215

Gly Ala Gly Leu Met Leu Gln Thr Ile Pro Gln Asn Ile Lys Phe
1220 1225 1230

Glu Pro Asn Lys Lys Tyr Thr Val Gln Phe Asp Tyr Gln Thr Asp
1235 1240 1245

Gly Glu Asn Val Phe Thr Ala Gly Thr Ile Asn Gly Glu Leu Lys
1250 1255 1260

Asn Asn Asn Asp Phe Lys Pro Val Gly Glu Leu Thr Ser Thr Ala
1265 1270 1275

Ala Asp Gly Gln Thr Lys His Tyr Glu Ala Glu Ile Ile Gly Asp
1280 1285 1290

Ala Ser Gly Asn Thr Thr Phe Gly Ile Phe Thr Thr Gly Ala Asp
1295 1300 1305

Lys Asp Phe Ile Met Asp Asn Phe Thr Val Thr Val Glu Ser Lys
1310 1315 1320

Lys

<210> 13
<211> 26
<212> DNA
<213> Artificial

<220>
<223> Primer

<400> 13
ttaacctcca tgggcagcgg gggagg 26

<210> 14
<211> 30
<212> DNA
<213> Artificial

<220>
<223> Primer

<400> 14
aacctgcggc cgccagttgc tcgcgattgc 30

<210> 15
<211> 20
<212> DNA
<213> Artificial

<220>
<223> Primer

<400> 15
tgcgattcat cgcctagcag 20

<210> 16
<211> 8
<212> PRT
<213> Homo sapiens

<220>
<223> Primer

<400> 16

```
          Ala Ser Thr Thr Thr Asn Tyr Thr
          1                   5
```

<210> 17
<211> 12
<212> PRT
<213> Homo sapiens

<220>
<223> Primer

<400> 17

```
          Phe Arg Lys Lys Trp Asn Lys Trp Ala Leu Ser Arg
          1                   5                   10
```

<210> 18
<211> 9
<212> PRT

<213> Digitalis purpurea

<400> 18

```
Arg Pro Pro Gly Phe Ser Pro Phe Arg
1               5
```